# EUROPEAN PATENT SPECIFICATION

(11) **EP 4 347 618 B1**
(45) Date of publication and mention of the grant of the patent: **29.01.2025**
(21) Application number: 22735060.0
(22) Date of filing: 01.06.2022
(51) Int. Cl.: C07K 7/08, A61P 31/04, A61K 38/00

(54) **CYCLIC PEPTIDE ANTIBIOTICS**
ZYKLISCHE PEPTIDANTIBIOTIKA
ANTIBIOTIQUES PEPTIDIQUES CYCLIQUES

(30) Priority: 03.06.2021 US 202163196374 P
(43) Date of publication of application: 10.04.2024
(73) Proprietor: Genentech, Inc., South San Francisco, CA 94080-4990 (US)
(72) Inventor: CUNNINGHAM, Christian Nathaniel, South San Francisco, California 94080-4990 (US); PAYANDEH, Jian Mehr-Dean, South San Francisco, California 94080-4990 (US); RUTHERFORD, Steven Thomas, South San Francisco, California 94080-4990 (US); STOREK, Kelly Marie Colvin, South San Francisco, California 94080-4990 (US); REID, Patrick C., Kawasaki-shi Kanagawa, 210--0821 (JP); YANAGIDA, Hayato, Kawasaki-shi Kanagawa, 210--0821 (JP); NISHIKAWA, Junichi, Kawasaki-shi Kanagawa, 210--0821 (JP)
(74) Representative: Neuhaus, Christian Michel
(86) International application number: PCT/US2022/031732
(87) International publication number: WO 2022/256380

(56) References cited:
- WO-A1-2016/023895
- WO-A1-97/46248
- WO-A2-2017/218949
- THOMSEN THOMAS ET AL: "Analogues of a Cyclic Antimicrobial Peptide with a Flexible Linker Show Promising Activity against Pseudomonas aeruginosa and Staphylococcus aureus", ANTIBIOTICS, vol. 9, no. 7, 30 June 2020 (2020-06-30), pages 366, XP055973393, DOI: 10.3390/antibiotics9070366
- HE RUNZE ET AL: "Design, crystal structure and atomic force microscopy study of thioether ligated D,L-cyclic antimicrobial peptides against multidrug resistant Pseudomonas aeruginosa", CHEMICAL SCIENCE, vol. 8, no. 11, 1 January 2017 (2017-01-01), United Kingdom, pages 7464 - 7475, XP055809670, ISSN: 2041-6520, DOI: 10.1039/C7SC01599B
- ABDEL MONAIM SHIMAA A. H. ET AL: "Bacteria Hunt Bacteria through an Intriguing Cyclic Peptide", CHEMMEDCHEM COMMUNICATIONS, vol. 14, no. 1, 10 December 2018 (2018-12-10), DE, pages 24 - 51, XP055973391, ISSN: 1860-7179, Retrieved from the Internet <URL:https://onlinelibrary.wiley.com/doi/full-xml/10.1002/cmdc.201800597> DOI: 10.1002/cmdc.201800597

## Description

### BACKGROUND

Most Gram-negative bacteria are inherently more resistant to antibacterials than Gram-positive bacteria and currently pose a problem in anti-infective therapy. Initially, this 'intrinsic resistance' was attributed to the outer membrane (OM) permeation barrier. However, OM permeability alone is not a sufficient explanation because most drug molecules can even equilibrate, across the rather impermeable OM of *P. aeruginosa,* in less than a minute. (H. Nikado, Antimicrob. Agent Chemother. 1989 33(11):1831) The intrinsic drug resistance of Gram-negative bacteria has been shown to result from the cooperation between the OM barrier and the expression of broad-specificity multidrug efflux pumps (H. I. Zgurskaya and H. Nikado, Proc. Nat. Acad. Sci. 1999 96(13):7190). Gram-negative bacteria also possess drugspecific efflux pumps that mediate resistance to certain classes of antibacterials. (X.-Z.Li and H. Nikaido, Drugs 2004 64:159-204). WO 2017/218949 discloses a macrocyclic peptide, wherein cyclisation may occur via a thioether linkage and that the compounds are useful for the treatment of an antibacterial infection that may be caused by a Gram-negative bacteria.

### SUMMARY OF THE DISCLOSURE

Described herein are novel cyclic peptides useful for treating microbial infections. In various embodiments, the present disclosure provides macrocyclic peptides for the treatment of bacterial infections. In various embodiments, the present disclosure provides classes and subclasses of chemical compounds for the treatment of Gram-negative bacterial infections. The macrocyclic compounds act by inhibiting folding and insertion of the nascent proteins into the OM that is mediated by the OPM BAM complex. The scope of the present invention is defined by the appended set of claims.

In some embodiments of the invention there is provided a cyclic dodecapeptide of Formula (I), or a pharmaceutically acceptable salt, solvate, or stereoisomer thereof: 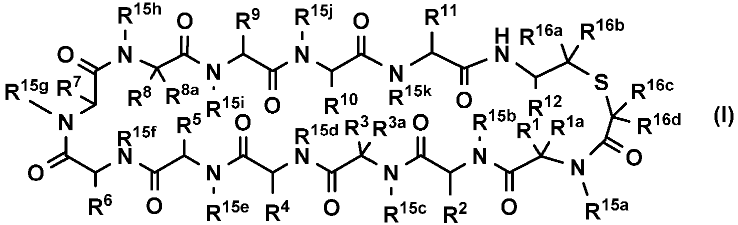 wherein:
R¹ is C₁₋₆alkyl, hydroxyl-C₁₋₆alkyl or optionally substituted benzyl;
R^{1a} is hydrogen or C₁₋₆alkyl;
R² is hydrogen, C₁₋₆alkyl, or optionally substituted (imidazol-4-yl)methyl;
R³ is bicyclic aryl, bicyclic heteroaryl, C₁₋₆alkyl, or hydroxyl-C₁₋₆alkyl wherein the bicyclic aryl and bicyclic heteroaryl is optionally substituted once or twice with C₁₋₆alkyl, hydroxyl or halo;
R^{3a} is hydrogen or C₁₋₆alkyl;
R⁴ is C₁₋₆ alkyl or optionally substituted (imidazol-4-yl)methyl;
R⁵ is hydrogen, C₁₋₆alkyl, hydroxyl-C₁₋₆alkyl, optionally substituted (imidazol-4-yl)methyl or optionally substituted guanidino-C₂₋₄alkyl;
R⁶ is hydroxyl-C₁₋₆alkyl or amino-C₁₋₆alkyl;
R⁷ is C₁₋₆alkyl, hydroxyl-C₁₋₆alkyl or optionally substituted guanidino-C₂₋₄alkyl;
R³ is optionally substituted benzyl;
R^{8a} is hydrogen or C₁₋₆alkyl;
R⁹ is optionally substituted benzyl or optionally substituted 1H-imidazol-5-ylmethyl;
R¹⁰ is C₁₋₆alkyl, amino-C₁₋₆alkyl, optionally substituted 1H-imidazol-5-ylmethyl, optionally substituted guanidino-C₂₋₄alkyl or optionally substituted C₁₋₂alkyl-carboxamide;
R¹¹ is optionally substituted 1- C₁₋₆alkyl-1H-imidazol-5-yl)methyl benzyl or wherein R^{b} is hydrogen, or C₁₋₆alkyl;
R¹² is optionally substituted N-(3-amino-3-oxo-(CH₂)₂₋₃)carboxamide;
R^{15a}, R^{15b}, R^{15c}, R^{15d}, R^{15f}, R^{15g}, R^{15h}, R¹⁵ⁱ, R^{15j}, R^{15k}, R^{16a}, R^{16b}, R^{16c} and R^{16d} each independently in each occurrence hydrogen, or C₁₋₆alkyl.

In other embodiments of the invention there is provided a cyclic tetradecapeptide of Formula (II), or a pharmaceutically acceptable salt, solvate, or stereoisomer thereof, wherein: 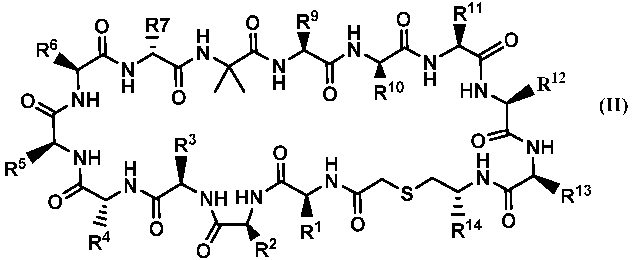
R¹ is C₁₋₆alkyl, hydroxyl-C₁₋₆alkyl or optionally substituted benzyl;
R^{1a} is hydrogen or C₁₋₆alkyl;
R² is hydrogen, C₁₋₆alkyl or optionally substituted (imidazol-4-yl)methyl;
R³ is bicyclic aryl, bicyclic heteroaryl, C₁₋₆alkyl or hydroxyl-C₁₋₆alkyl wherein the bicyclic aryl and bicyclic heteroaryl are optionally substituted once or twice with C₁₋₆alkyl, hydroxyl or halo;
R^{3a} is hydrogen or C₁₋₆alkyl;
R⁴ is C₁₋₆alkyl or optionally substituted (imidazol-4-yl)methyl;
R⁵ is hydrogen, C₁₋₆alkyl, hydroxyl-C₁₋₆alkyl, optionally substituted (imidazol-4-yl)methyl or optionally substituted guanidino-C₂₋₄alkyl;
R⁶ is hydroxyl-C₁₋₆alkyl or amino-C₁₋₆alkyl;
R³ is C₁₋₆alkyl, hydroxyl-C₁₋₆alkyl or optionally substituted guanidino-C₂₋₄alkyl;
R⁸ is optionally substituted benzyl;
R^{8a} is hydrogen or C₁₋₆alkyl;
R⁹ is optionally substituted benzyl or optionally substituted (imidazol-4-yl)methyl;
R¹⁰ is C₁₋₆alkyl, amino-C₁₋₆alkyl, optionally substituted (imidazol-4-yl)methyl, optionally substituted guanidino-C₂₋₄alkyl, or optionally substituted C₁₋₂alkyl-carboxamide;
R¹¹ is optionally substituted benzyl;
R¹² is 1H-indolyl-3-methyl or optionally substituted benzyl;
R¹³ is optionally substituted benzyl;
R¹⁴ is optionally substituted N-(3-amino-3-oxo-(CH₂)₂₋₃)carboxamide;
R^{b,}, R^{c}, R^{d}, R^{e}, R^{f}, R^{15a}, R^{15b}, R^{15c}, R^{15d}, R^{15e}, R^{15f}, R^{15g}, R^{15h}, R¹⁵ⁱ, R^{15j}, R^{15k}, R^{15l}, R^{15m}, R¹⁵ⁿ, R^{16a}, R^{16b}, R^{16c} and R^{16d} are in each occurrence independently hydrogen or C₁₋₆alkyl;
R^{a} is hydrogen, C₁₋₆alkyl, hydroxyl or halo;
n is 0 to 2;
q is 0 or 1; and,
r is 1 or 2.

### BRIEF DESCRIPTION OF THE FIGURES

Figure 1a and 1b - Programs used for preparation of Example 1 and Example 2 in an automated peptide synthesizer
Figure 2a and 2b - Programs used for preparation of Example 3 and Example 4 in an automated peptide synthesizer
Figure 3a and 3b - Programs used for preparation of Example 5 and Example 6 in an automated peptide synthesizer

### DETAILED DESCRIPTION OF THE DISCLOSURE

Antibiotic resistance is a serious and growing phenomenon in contemporary medicine and has emerged as a major public health concern in the 21st century posing a significant obstacle to therapy. Many Gram-negative (GN) bacteria are more inherently resistant to antibacterials than Gram-positive bacteria. This enhanced resistance is in part due to the cell wall. Gram-positive bacteria have cell walls made of a thick layer of peptidoglycan surrounding a cytoplasmic lipid membrane. In contrast, the cell walls of gram-negative bacteria contain only a thin layer of peptidoglycan, but they also have an outer membrane (OM) that is absent in gram-positive bacteria. The outer membrane of Gram-negative bacteria provides rigidity to the cell and serves as a permeability barrier to cytotoxic molecules including antibiotics. In addition the OM contains integral outer membrane proteins (OMPs), porins, that have a characteristic β-barrel structure and perform essential functions, including-the efflux of toxic molecules from the cell. Thus the presence of BamA in the outer membrane presents a potential target for small molecule inhibitors which inhibit formation of antibiotic efflux pumps. (K. M. Lehman and M. Grabowicz, Antibiotics 2019 8:163)

A library cyclic peptides was prepared using mRNA display. (Roberts, R. W. and Szostak, Proc. Nat. Acad. Sci. USA 1997 94:12297, Sohrabi, C. et al., Nature Rev. Chem. 2020 4:90; Josephson, K et al., Drug Discov. Today, 2014 19(4):388). A thioether-macrocyclic peptide library was constructed by using N-chloroacetyl D-phenylalanine (ClAc-F) as an initiator in a genetically reprogrammed in vitro translation system (Kashiwagi, K and Reid, P., WO2011/049157). The resulting library of macrocyclic peptides was screened resulting in the identification of peptide targeting Bam A 1 and peptide targeting Bam A 2.

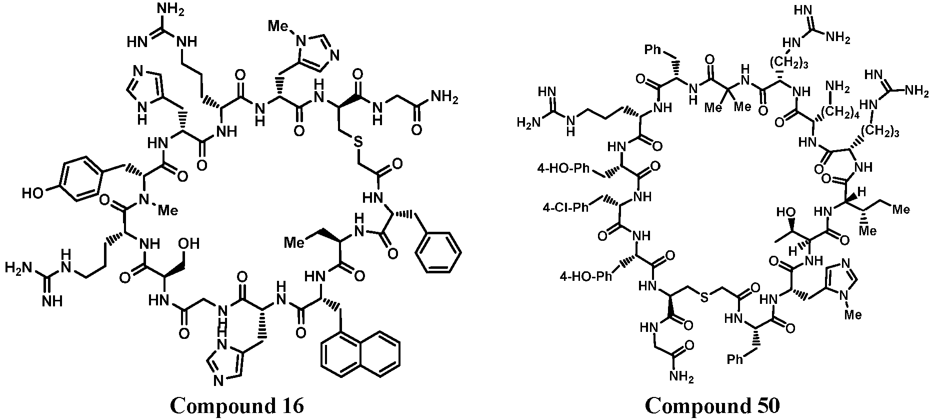

### Definitions

As used herein and in the appended claims, the singular forms "a," "and," and "the" include plural referents unless the context clearly dictates otherwise. Thus, for example, reference to "an agent" includes a plurality of such agents, and reference to "the cell" includes reference to one or more cells (or to a plurality of cells) and equivalents thereof known to those skilled in the art, and so forth. When ranges are used herein for physical properties, such as molecular weight, or chemical properties, such as chemical formulae, all combinations and subcombinations of ranges and specific embodiments therein are intended to be included. The term "about" when referring to a number or a numerical range means that the number or numerical range referred to is an approximation within experimental variability (or within statistical experimental error), and thus the number or numerical range, in some instances, will vary between 1% and 15% of the stated number or numerical range. The term "comprising" (and related terms such as "comprise" or "comprises" or "having" or "including") is not intended to exclude that in other certain embodiments, for example, an embodiment of any composition of matter, composition, method, or process, or the like, described herein, "consist of" or "consist essentially of' the described features.

As used in the specification and appended claims, unless specified to the contrary, the following terms have the meaning indicated below.

"Alkyl" refers to an optionally substituted straight-chain, or optionally substituted branched-chain saturated hydrocarbon monoradical having from one to about ten carbon atoms, or from one to six carbon atoms. Examples include, but are not limited to, methyl, ethyl, n-propyl, isopropyl, 2-methyl-1-propyl, 2-methyl-2-propyl, 2-methyl-1-butyl, 3-methyl-1-butyl, 2-methyl-3-butyl, 2,2-dimethyl-1-propyl, 2-methyl-1-pentyl, 3-methyl-1-pentyl, 4-methyl-1-pentyl, 2-methyl-2-pentyl, 3-methyl-2-pentyl, 4-methyl-2-pentyl, 2,2-dimethyl-1-butyl, 3,3-dimethyl-1-butyl, 2-ethyl-1-butyl, n-butyl, isobutyl, sec-butyl, t-butyl, n-pentyl, isopentyl, neopentyl, tert-amyl and hexyl, and longer alkyl groups, such as heptyl, octyl, and the like. Whenever it appears herein, a numerical range such as "C₁-C₆ alkyl" means that the alkyl group consists of 1 carbon atom, 2 carbon atoms, 3 carbon atoms, 4 carbon atoms, 5 carbon atoms or 6 carbon atoms, although the present definition also covers the occurrence of the term "alkyl" where no numerical range is designated. In some embodiments, the alkyl is a C₁-C₁₀ alkyl, a C₁-C₉ alkyl, a C₁-C₈ alkyl, a C₁-C₇ alkyl, a C₁-C₆ alkyl, a C₁-C₅ alkyl, a C₁-C₄ alkyl, a C₁-C₃ alkyl, a C₁-C₂ alkyl, or a C₁ alkyl. Unless stated otherwise specifically in the specification, an alkyl group is optionally substituted as described below, for example, with oxo, halogen, amino, nitrile, nitro, hydroxyl, haloalkyl, alkoxy, aryl, cycloalkyl, heterocycloalkyl, heteroaryl, and the like. In some embodiments, the alkyl is optionally substituted with oxo, halogen, -CN, -CF₃, -OH, -OMe, -NH₂, or -NO₂. In some embodiments, the alkyl is optionally substituted with oxo, halogen, -CN, -CF₃, -OH, or -OMe. In some embodiments, the alkyl is optionally substituted with halogen.

"Alkylene" refers to a straight or branched divalent hydrocarbon chain. Unless stated otherwise specifically in the specification, an alkylene group may be optionally substituted as described below, for example, with oxo, halogen, amino, nitrile, nitro, hydroxyl, haloalkyl, alkoxy, aryl, cycloalkyl, heterocycloalkyl, heteroaryl, and the like. In some embodiments, an alkylene is optionally substituted with oxo, halogen, -CN, -CF₃, -OH, -OMe, -NH₂, or -NO₂. In some embodiments, an alkylene is optionally substituted with oxo, halogen, -CN, -CF₃, -OH, or -OMe. In some embodiments, the alkylene is optionally substituted with halogen.

"Alkoxy" refers to a radical of the formula -ORₐ where Rₐ is an alkyl radical as defined herein. Unless stated otherwise specifically in the specification, an alkoxy group may be optionally substituted as described below, for example, with oxo, halogen, amino, nitrile, nitro, hydroxyl, haloalkyl, alkoxy, aryl, cycloalkyl, heterocycloalkyl, heteroaryl, and the like. In some embodiments, an alkoxy is optionally substituted with oxo, halogen, -CN, -CF₃, -OH, -OMe, -NH₂, or -NO₂. In some embodiments, an alkoxy is optionally substituted with oxo, halogen, -CN, -CF₃, -OH, or -OMe. In some embodiments, the alkoxy is optionally substituted with halogen.

"Aryl" refers to a radical derived from a hydrocarbon ring system comprising hydrogen, 6 to 30 carbon atoms and at least one aromatic ring. The aryl radical may be a monocyclic, bicyclic, tricyclic or tetracyclic ring system, which may include fused (when fused with a cycloalkyl or heterocycloalkyl ring, the aryl is bonded through an aromatic ring atom) or bridged ring systems. In some embodiments, the aryl is a 6- to 10-membered aryl. In some embodiments, the aryl is a 6-membered aryl. Aryl radicals include, but are not limited to, aryl radicals derived from the hydrocarbon ring systems of anthrylene, naphthylene, phenanthrylene, anthracene, azulene, benzene, chrysene, fluoranthene, fluorene, as-indacene, s-indacene, indane, indene, naphthalene, phenalene, phenanthrene, pleiadene, pyrene, and triphenylene. In some embodiments, the aryl is phenyl. Unless stated otherwise specifically in the specification, an aryl may be optionally substituted as described below, for example, with halogen, amino, nitrile, nitro, hydroxyl, alkyl, alkenyl, alkynyl, haloalkyl, alkoxy, aryl, cycloalkyl, heterocycloalkyl, heteroaryl, and the like. In some embodiments, an aryl is optionally substituted with halogen, methyl, ethyl, -CN, -CF₃, -OH, -OMe, -NH₂, or -NO₂. In some embodiments, an aryl is optionally substituted with halogen, methyl, ethyl, -CN, -CF₃, -OH, or -OMe. In some embodiments, the aryl is optionally substituted with halogen or methyl. In some embodiments, the aryl is optionally substituted with halogen.

"Halo" or "halogen" refers to bromo, chloro, fluoro, or iodo. In some embodiments, halogen is fluoro or chloro. In some embodiments, halogen is fluoro.

"Haloalkyl" refers to an alkyl radical, as defined above, that is substituted by one or more halo radicals, as defined above, e.g., trifluoromethyl, difluoromethyl, fluoromethyl, trichloromethyl, 2,2,2-trifluoroethyl, 1,2-difluoroethyl, 3-bromo-2-fluoropropyl, 1,2-dibromoethyl, and the like.

"Aminoalkyl" refers to an alkyl radical, as defined above, that is substituted by one or more -NH₂, *e.g.,-*CH₂NH₂, -CH₂CH₂NH₂, -CH₂CH₂CH₂NH₂, -CH(NH₂)CH₃, -CH₂CH(NH₂)CH₃, -CH(NH₂)CH₂CH₃, and the like.

"Hydroxyalkyl" refers to an alkyl radical, as defined above, that is substituted by one or more -OH, *e.g., -* CH₂OH, -CH₂CH₂OH, -CH₂CH₂CH₂OH, -CH(OH)CH₃, -CH₂CH(OH)CH₃, -CH(OH)CH₂CH₃, and the like.

"Heteroaryl" refers to a 5- to 14-membered ring system radical comprising hydrogen atoms, one to thirteen carbon atoms, one to six heteroatoms selected from the group consisting of nitrogen, oxygen, phosphorous and sulfur, and at least one aromatic ring. The heteroaryl radical may be a monocyclic, bicyclic, tricyclic or tetracyclic ring system, which may include fused (when fused with a cycloalkyl or heterocycloalkyl ring, the heteroaryl is bonded through an aromatic ring atom) or bridged ring systems; and the nitrogen, carbon or sulfur atoms in the heteroaryl radical may be optionally oxidized; the nitrogen atom may be optionally quaternized. In some embodiments, the heteroaryl is a 5- to 10-membered heteroaryl. In some embodiments, the heteroaryl is a 5- to 6-membered heteroaryl. Examples include, but are not limited to, azepinyl, acridinyl, benzimidazolyl, benzothiazolyl, benzindolyl, benzodioxolyl, benzofuranyl, benzooxazolyl, benzothiazolyl, benzothiadiazolyl, benzo[b][1,4]dioxepinyl, 1,4-benzodioxanyl, benzonaphthofuranyl, benzoxazolyl, benzodioxolyl, benzodioxinyl, benzopyranyl, benzopyranonyl, benzofuranyl, benzofuranonyl, benzothienyl (benzothiophenyl), benzotriazolyl, benzo[4,6]imidazo[1,2-a]pyridinyl, carbazolyl, cinnolinyl, dibenzofuranyl, dibenzothiophenyl, furanyl, furanonyl, isothiazolyl, imidazolyl, indazolyl, indolyl, indazolyl, isoindolyl, indolinyl, isoindolinyl, isoquinolyl, indolizinyl, isoxazolyl, naphthyridinyl, oxadiazolyl, 2-oxoazepinyl, oxazolyl, oxiranyl, 1-oxidopyridinyl, 1-oxidopyrimidinyl, 1-oxidopyrazinyl, 1-oxidopyridazinyl, 1-phenyl-1H-pyrrolyl, phenazinyl, phenothiazinyl, phenoxazinyl, phthalazinyl, pteridinyl, purinyl, pyrrolyl, pyrazolyl, pyridinyl, pyrazinyl, pyrimidinyl, pyridazinyl, quinazolinyl, quinoxalinyl, quinolinyl, quinuclidinyl, isoquinolinyl, tetrahydroquinolinyl, thiazolyl, thiadiazolyl, triazolyl, tetrazolyl, triazinyl, and thiophenyl (i.e., thienyl). Unless stated otherwise specifically in the specification, a heteroaryl is optionally substituted as described below, for example, with halogen, amino, nitrile, nitro, hydroxyl, alkyl, alkenyl, alkynyl, haloalkyl, alkoxy, aryl, cycloalkyl, heterocycloalkyl, heteroaryl, and the like. In some embodiments, a heteroaryl is optionally substituted with halogen, methyl, ethyl, -CN, -CF₃, -OH, -OMe, -NH₂, or -NO₂. In some embodiments, a heteroaryl is optionally substituted with halogen, methyl, ethyl, -CN, -CF₃, -OH, or - OMe. In some embodiments, the heteroaryl is optionally substituted with halogen or methyl. In some embodiments, the heteroaryl is optionally substituted with halogen.

The following nomenclature is used herein.

Optionally substituted benzyl refers to benzyl optionally substituted with C₁₋₆ alkyl, hydroxyl, C₁₋₃ alkoxy, halo. C₁₋₆ haloalkyl or C₁₋₆ haloalkyloxy.

1H-imidazol-5-ylmethyl refers to (*ii*) wherein R^{b} is hydrogen and optionally substituted (imidazol-4-yl)methyl refers to (*ii*) wherein R^{b} is hydrogen or C₁₋₆ alkyl. Both (3-methylimidazol-4-yl)methyl and 1-methyl-1H-imidazol-5-yl)methyl refer to (*iib*) wherein R^{b} is methyl.

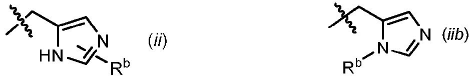

Guanidino-C₂₋₄alkyl refers to-a moiety -(CH₂)₂₋₄-NH(C=NH)NH₂; optionally substituted guanidino-C₂₋₄alkyl refers to-a moiety -(CH₂)ₚ-NR^{c}(C=NR^{c})N(R^{c})₂ (*iii*) wherein R^{c} is independently in each occurrence hydrogen or C₁₋₆alkyl and p is 0 to 2, preferably R^{c} is hydrogen or methyl and p is 1.

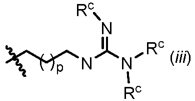

Optionally substituted C₁₋₂alkyl-carboxamide (iv) refers to 3-amino-3-oxo-propyl (-(CH₂)₂C(=O)N(R^{d})₂) and 2-amino-2-oxo-ethyl refer to (-CH₂C(=O)N(R^{d})₂) respectively wherein R^{d} is independently in each occurrence hydrogen or C₁₋₆alkyl and preferably R^{d} is hydrogen or methyl and q is 0 or 1.

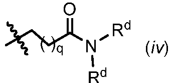

N-(3-amino-3-oxo-(CH₂)₂₋₃)carboxamide-refers to (v) wherein r is 1 or 2 and R^{e} hydrogen. N-(3-amino-3-oxo-propyl)carboxamide refers -C(=O)NR^{e}(CH₂)₂C(=O)N(R^{e})₂ and N-(2-amino-2-oxoethyl)carboxamide refers to -C(=O)NR^{e}CH₂C(=O)N(R^{e})₂ wherein R^{e} is hydrogen. When these moieties are optionally substituted R^{e} is independently C₁₋₆alkyl or hydrogen

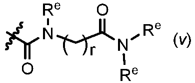

1H-indolyl-3-methyl refers to (vi) wherein R^{f} is hydrogen and optionally substituted 1H-indolyl-3-methyl refers to (vi) wherein R^{f} is hydrogen or C₁₋₆alkyl;

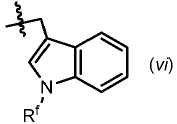

The term aromatic amino acid refers to a compound of formula H₂NCH(CH₂R^{a}) CO₂H wherein R^{a} is benzyl, phenyl, 2-phenethyl, pyridinyl, pyrimidinyl, pyrazinyl or pyridazinyl wherein the aromatic ring is optionally substituted with 1 to 3 groups independently selected from C₁₋₆ alkyl, halogens, C₁₋₆-haloalkyl, hydroxyl, C₁₋₆-alkoxy, C₁₋₆-haloalkoxy, C₁₋₆-thioalkyl, C₁₋₆-alkylsulfonyl and cyano. An N-methyl-aromatic amino acid is a compound of formula MeHNCH(CH₂R^{a}) CO₂H.

The term aliphatic amino acid refers to a compound of formula H₂NCH(CH₂R^{a}) CO₂H wherein R^{a} is hydrogen or C₁ straight or branched alkyl, C₁₋₄alkoxy-C₁₋₂alkyl or methionine.

The term basic amino acid refers to a a compound of formula H₂NCH(CH₂R^{a}) CO₂H wherein R^{a} is C₁₋₆-aminoalkyl, guanidino-C₂₋₄alkyl or optionally substituted C₂₋₄alkyl-(guanidine) wherein R^{c} is independent hydrogen or C₁₋₆alkyl, pyrrolidin-3-yl.

The term heteroaryl amino acid refers to histidine, tryptophan, N-alkyl-histidine or N-alkyl tryptophan wherein the alkyl group is 1-3 carbons.

The term polar amino acid refers to serine, threonine, cysteine, asparagine, or glutamine.

Abbreviations used herein include 4-ClPh (4-chlorophenyl); F4C (4-Chloro-L-phenylalanine); 4-HO-Ph (4-hydroxyphenyl); Boc (t-butoxycarbonyl; Pbf (2,2,4,6,7-pentamethyldihydrobenzofuran-5-sulfonyl); Trt (Trityl); Abu ((S)-2-Aminobutyric acid); H3Me (3-Methyl-L-histidine); MeY (N-Methyl-L-tyrosine); MeF (N-Methyl-L-phenylalanine); TFA (trifluoroacetic acid); MeCN (acetonitrile) ; TEA (triethylamine); DCM (dichloromethane); DMF (N,N-dimethylformamide)

The terms "treat," "prevent," "ameliorate," and "inhibit," as well as words stemming therefrom, as used herein, do not necessarily imply 100% or complete treatment, prevention, amelioration, or inhibition. Rather, there are varying degrees of treatment, prevention, amelioration, and inhibition of which one of ordinary skill in the art recognizes as having a potential benefit or therapeutic effect. In this respect, the disclosed methods can provide any amount of any level of treatment, prevention, amelioration, or inhibition of the disorder in a mammal. For example, a disorder, including symptoms or conditions thereof, may be reduced by, for example, about 100%, about 90%, about 80%, about 70%, about 60%, about 50%, about 40%, about 30%, about 20%, or about 10%. Furthermore, the treatment, prevention, amelioration, or inhibition provided by the methods disclosed herein can include treatment, prevention, amelioration, or inhibition of one or more conditions or symptoms of the disorder, e.g., cancer or an inflammatory disease. Also, for purposes herein, "treatment," "prevention," "amelioration," or "inhibition" encompass delaying the onset of the disorder, or a symptom or condition thereof.

The terms "effective amount" or "therapeutically effective amount," as used herein, refer to a sufficient amount of a compound disclosed herein being administered which will relieve to some extent one or more of the symptoms of the disease or condition being treated, e.g., cancer or an inflammatory disease. In some embodiments, the result is a reduction and/or alleviation of the signs, symptoms, or causes of a disease, or any other desired alteration of a biological system. For example, an "effective amount" for therapeutic uses is the amount of the composition comprising a compound disclosed herein required to provide a clinically significant decrease in disease symptoms. In some embodiments, an appropriate "effective" amount in any individual case is determined using techniques, such as a dose escalation study.

The term as defined hereinabove refers the broadest definition described in the summary of the invention or to the larger embodiment to which it refers

"Prodrug" as the term is used herein means a compound with one or more moieties that can be metabolized *in vivo* to produce the active drug. For example, some prodrugs are metabolized *in vivo* by esterases or by other mechanisms to active drugs. Examples of prodrugs and their uses are well known in the art (see, e.g., Berge et al. (1977) "Pharmaceutical Salts", J. Pharm. Sci. 66:1-19). Prodrugs can be prepared *in situ* during the final isolation and purification of the compounds, or by separately reacting the purified compound in its free acid form or hydroxyl with a suitable esterifying agent. Hydroxyl groups can be converted into esters via treatment with a carboxylic acid. Examples of prodrug moieties include substituted and unsubstituted, branch or unbranched lower alkyl ester moieties, (e.g., propionoic acid esters), lower alkenyl esters, di-lower alkyl-amino lower-alkyl esters (e.g., dimethylaminoethyl ester), acylamino lower alkyl esters (e.g., acetyloxymethyl ester), acyloxy lower alkyl esters (e.g., pivaloyloxymethyl ester), aryl esters (phenyl ester), aryl-lower alkyl esters (e.g., benzyl ester), substituted (e.g., with methyl, halo, or methoxy substituents) aryl and aryl-lower alkyl esters, amides, lower-alkyl amides, di-lower alkyl amides, and hydroxy amides.

"Substantially" as the term is used herein means completely or almost completely; for example, a composition that is "substantially free" of a component either has none of the component or contains such a trace amount that any relevant functional property of the composition is unaffected by the presence of the trace amount, or a compound is "substantially pure" is there are only negligible traces of impurities present.

In some embodiments, the compounds described herein exist as their pharmaceutically acceptable salts. In some embodiments, the methods disclosed herein include methods of treating diseases by administering such pharmaceutically acceptable salts. In some embodiments, the methods disclosed herein include methods of treating diseases by administering such pharmaceutically acceptable salts as pharmaceutical compositions.

In some embodiments, the compounds described herein possess acidic or basic groups and therefor react with any of a number of inorganic or organic bases, and inorganic and organic acids, to form a pharmaceutically acceptable salt. In some embodiments, these salts are prepared *in situ* during the final isolation and purification of the compounds disclosed herein, or by separately reacting a purified compound in its free form with a suitable acid or base, and isolating the salt thus formed.

Examples of pharmaceutically acceptable salts include those salts prepared by reaction of the compounds described herein with a mineral, organic acid, or inorganic base, such salts including acetate, acrylate, adipate, alginate, aspartate, benzoate, benzenesulfonate, bisulfate, bisulfite, bromide, butyrate, butyn-1,4-dioate, camphorate, camphorsulfonate, caproate, caprylate, chlorobenzoate, chloride, citrate, cyclopentanepropionate, decanoate, digluconate, dihydrogenphosphate, dinitrobenzoate, dodecylsulfate, ethanesulfonate, formate, fumarate, glucoheptanoate, glycerophosphate, glycolate, hemisulfate, heptanoate, hexanoate, hexyne-1,6-dioate, hydroxybenzoate, γ-hydroxybutyrate, hydrochloride, hydrobromide, hydroiodide, 2-hydroxyethanesulfonate, iodide, isobutyrate, lactate, maleate, malonate, methanesulfonate, mandelate metaphosphate, methanesulfonate, methoxybenzoate, methylbenzoate, monohydrogenphosphate, 1-napthalenesulfonate, 2-napthalenesulfonate, nicotinate, nitrate, palmoate, pectinate, persulfate, 3-phenylpropionate, phosphate, picrate, pivalate, propionate, pyrosulfate, pyrophosphate, propiolate, phthalate, phenylacetate, phenylbutyrate, propanesulfonate, salicylate, succinate, sulfate, sulfite, succinate, suberate, sebacate, sulfonate, tartrate, thiocyanate, tosylateundeconate, and xylenesulfonate.

In some embodiments, the compounds described herein exist as solvates. The disclosure provides for methods of treating diseases by administering such solvates. The disclosure further provides for methods of treating diseases by administering such solvates as pharmaceutical compositions.

Solvates contain either stoichiometric or non-stoichiometric amounts of a solvent, and, in some embodiments, are formed during the process of crystallization with pharmaceutically acceptable solvents such as water, ethanol, and the like. Hydrates are formed when the solvent is water, or alcoholates are formed when the solvent is alcohol. Solvates of the compounds described herein can be conveniently prepared or formed during the processes described herein. By way of example only, hydrates of the compounds described herein can be conveniently prepared by recrystallization from an aqueous/organic solvent mixture, using organic solvents including, but not limited to, dioxane, tetrahydrofuran, or methanol. In addition, the compounds provided herein can exist in unsolvated as well as solvated forms. In general, the solvated forms are considered equivalent to the unsolvated forms for the purposes of the compounds and methods provided herein.

In some situations, compounds exist as tautomers. The compounds described herein include all possible tautomers within the formulas described herein. Tautomers are compounds that are interconvertible by migration of a hydrogen atom, accompanied by a switch of a single bond and adjacent double bond. In bonding arrangements where tautomerization is possible, a chemical equilibrium of the tautomers will exist. All tautomeric forms of the compounds disclosed herein are contemplated. The exact ratio of the tautomers depends on several factors, including temperature, solvent, and pH.

In another aspect compounds are hydrates or metabolites of any of the aforementioned compounds. In another aspect are pharmaceutical compositions comprising any of the aforementioned compounds together with a pharmaceutically acceptable excipient.

### COMPOUNDS

In one embodiment of the invention there is provided a cyclic dodecapeptide of Formula (I), or a pharmaceutically acceptable salt, solvate, or stereoisomer thereof as disclosed above in the Summary of the Disclosure.

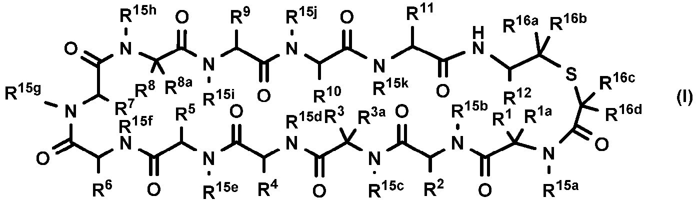

In one embodiment of a compound of Formula (I), R¹² is N-(2-amino-2-oxo-ethyl)carboxamide, R^{1a}, R^{3a} and R^{8a} are hydrogen, R⁴ and R⁹ are 1H-imidazol-5-ylmethyl and R² is C₁₋₆ alkyl. In one embodiment of a compound of Formula (I), R² is ethyl, isopropyl or *sec*-butyl.

In some embodiments of a compound of Formula (I), R¹ is optionally substituted benzyl. In some embodiments of a compound of Formula (I), R¹ is unsubstituted benzyl, hydroxybenzyl or chlorobenzyl. In some embodiments of a compound of Formula (I), R¹ is 4-hydroxybenzyl or 4-chlorobenzyl. In some embodiments of a compound of Formula (I), R¹ is C₁₋₆ alkyl. In some embodiments of a compound of Formula (I), R¹ is butyl. In some embodiments of a compound of Formula (I), R¹ is C₁₋₆ hydroxyalkyl or hydroxymethyl. In some embodiments of a compound of Formula (I), R¹ is hydroxymethyl.

In some embodiments of a compound of formula (I) R³ is bicyclic aryl, bicyclic heteroaryl, C₁₋₆alkyl, or hydroxyl-C₁₋₆alkyl wherein the bicyclic aryl and bicyclic heteroaryl is optionally substituted once or twice with C₁₋₆alkyl, hydroxyl or halo. In some embodiments R³ is 1-naphthylmethyl either substituted by one to three halogens; or unsubstituted 1-naphthylmethyl. In some embodiments R³ is 2-naphthmethyl either substituted by one to three halogens; or unsubstituted 2-naphthmethyl. In some embodiments R³ is optionally substituted-3-indolylmethyl wherein R^{f} is hydrogen or C₁₋₆alkyl. In some embodiments R³ is 3-indolylmethyl. In some embodiments R³ is N-substituted-3-indolylmethyl wherein R^{f} is methyl. In some embodiment R³ is optionally substituted benzyl. In some embodiment R³ is 4-phenylphenyl)methyl.

In some embodiments of a compound of formula (I), R⁴ is C₁₋₆alkyl or optionally substituted (imidazol-4-yl)methyl. In some embodiments of a compound of formula (I), R⁴ is 4-(imidazolyl)methyl optionally substituted by C₁₋₆alkyl. In some embodiments of a compound of formula (I), R⁴ is 4-(imidazolyl)methyl. In some embodiments of a compound of formula (I) R⁴ is 4-(imidazolyl)methyl with a N-substituted methyl. In some embodiments of a compound of formula (I) R⁴ is C₁₋₆alkyl. In some embodiments of a compound of formula (I) R⁴ is methyl.

In some embodiments of a compound of formula (I), R⁵ is hydrogen. In some embodiments of a compound of formula (I), R⁵ is hydroxy-C₁₋₆alkyl. In some embodiments of a compound of formula (I), R⁵ is hydroxymethyl. In some embodiments of a compound of formula (I), R⁵ is C₁₋₆alkyl. In some embodiments of a compound of formula (I), R⁵ is methyl. In some embodiments of a compound of formula (I) R⁵ is optionally substituted 3-guanido-C₂₋₄alkyl wherein R^{c} is independently is hydrogen, or C₁₋₆alkyl and p is 2-4. In some embodiments of a compound of formula (I), R⁵ is 3-guanidopropyl.

In some embodiments of a compound of formula (I), R⁶ is hydroxyl-C₁₋₆alkyl. In some embodiments of a compound of formula (I), R⁶ is hydroxymethyl or hydroxyethyl. In some embodiments of a compound of formula (I), R⁶ is 1-hydroxy-ethyl. In some embodiments of a compound of formula (I), R⁶ is amino-C₁₋₆alkyl. In some embodiments of a compound of formula (I), R⁶ is 4-aminobutyl. In some embodiments of a compound of formula (I), R⁶ is optionally substituted guanidino-C₂₋₄alkyl wherein R^{c} is independently is hydrogen or C₁₋₆alkyl or optionally substituted 3-guanido-propyl. In some embodiments of a compound of formula (I), R³ is 3-guanidopropyl.

In some embodiments of a compound of formula (I), R⁷ is optionally substituted 3-guanido-C₂₋₄alkyl wherein R^{c} is independently is hydrogen or C₁₋₆alkyl. In some embodiments of a compound of formula (I), R⁵ is 3-guanidopropyl. In some embodiments of a compound of formula (I), R⁷ is C₁₋₆alkyl. In some embodiments of a compound of formula (I), R⁷ is methyl or ethyl. In some embodiments of a compound of formula (I), R⁷ is methyl. In some embodiments of a compound of formula (I), R⁷ is ethyl. In some embodiments of a compound of formula (I), R⁷ is hydroxylC₁₋₆alkyl. In some embodiments of a compound of formula (I), R⁷ is 1-hydroxyethyl.

In some embodiments of a compound of formula (I), R³ is optionally substituted benzyl. In some embodiments of a compound of formula (I), R⁸ is hydroxybenzyl. In some embodiments of a compound of formula (I), R⁸ is 4-hydroxybenzyl. In some embodiments of a compound of formula (I), R⁸ is halo substituted benzyl. In some embodiments of a compound of formula (I), R⁸ is benzyl substituted by 1 or 2 halogens. In some embodiments of a compound of formula (I), R⁸ is benzyl substituted by 1 or 2 chlorines. In some embodiments of a compound of formula (I), R⁸ is 4-chlorobenzyl.

In some embodiments of a compound of formula (I), R⁹ is optionally substituted (imidazol-4-yl)methyl. In some embodiments of a compound of formula (I), R⁹ is 4-(imidazolyl)methyl optionally substituted by C₁₋₆alkyl. In some embodiments of a compound of formula (I), R⁴ is 4-(imidazolyl)methyl. In some embodiments of a compound of formula (I), R⁴ is 4-(imidazolyl)methyl with a N-substituted methyl. In some embodiments of a compound of formula (I), R⁹ is C₁₋₆alkyl. In some embodiments of a compound of formula (I), R⁹ is methyl.

In some embodiments of a compound of formula (I), R¹⁰ is optionally substituted 3-guanido-C₁₋₆alkyl wherein R^{c} is independently is hydrogen or C₁₋₆alkyl. In some embodiments of a compound of formula (I), R⁵ is 3-guanidopropyl. In some embodiments of a compound of formula (I), R¹⁰ is - CH₂(CH₂)_{q}C(=O)NH₂ wherein q is 0 or 1. In some embodiments of a compound of formula (I), 3-oxo-3-aminopropyl. In some embodiments of a compound of formula (I), R¹⁰ is amino-C₁₋₆alkyl. In some embodiments of a compound of formula (I), R¹⁰ is aminomethyl, 2-aminoethyl or 4-aminobutyl. In some embodiments R¹⁰ is 3-(methylimidazol-4-yl)methyl *(iib* wherein R*^{b}* is methyl). In some embodiments of a compound of formula (I), R¹⁰ is C₁₋₆alkyl. In some embodiments of a compound of formula (I), R¹⁰ is ethyl.

In some embodiments of a compound of formula (I), R¹¹ is 4-(imidazolyl)methyl optionally substituted with C₁₋₆alkyl. In some embodiments of a compound of formula (I), R¹¹ is C₁₋₆alkyl. In some embodiments of a compound of formula (I), R¹¹ is 4-(imidazolyl)methyl with a N-substituted methyl. In some embodiments of a compound of formula (I), R¹¹⁰ is 3-(methylimidazol-4-yl)methyl *(iib* wherein R*^{b}* is methyl). In some embodiments of a compound of formula (I), R¹¹ is 4-(imidazolyl)methyl.

In some embodiments of a compound of formula (I), R^{b}, R^{c}, R^{d}, R^{e}, R^{f}, R^{1a}, R^{3a}, R^{8a}, R^{15a}, R^{15b}, R^{15c}, R^{15d}, R^{15e}, R^{15f}, R^{15g}, R¹⁵ⁱ, R^{15j}, R^{15k}, R^{16a}, R^{16b}, R^{16c} and R^{16d} are hydrogen or C₁₋₆ alkyl; in some embodiments R^{b}, R^{c}, R^{d}, R^{e}, R^{f}, R^{1a}, R^{3a}, R^{8a}, R^{15a,} R^{15b}, R^{15c}, R^{15d}, R^{15e}, R^{15f}, R^{15g}, R¹⁵ⁱ, R^{15j}, R^{15k}, R^{16a}, R^{16b}, R^{16c} and R^{16d} are hydrogen or methyl. In some embodiments of a compound of formula (I), R^{a} is hydrogen, C₁₋₆alkyl, hydroxyl or halo; in some embodiment R^{a} is chloro. In some embodiments of a compound of formula (I) R^{f} is hydrogen, C₁₋₆alkyl. In some embodiments of a compound of formula (I), R^{f} is hydrogen. In some embodiments of a compound of formula (I), R^{f} is methyl. In some embodiments of a compound of formula (I), n and p are independently from 0 to 2. In some embodiments of a compound of formula (I), n and p are 1 or 0. In some embodiments of a compound of formula (I), n is 1. In some embodiments of a compound of formula (I), n is 0. In some embodiments of a compound of formula (I), q is independently from 0 or 1. In some embodiments of a compound of formula (I), in some embodiments q is 1. In some embodiments of a compound of formula (I), r is independently from 1 or 2. In some embodiments of a compound of formula (I), r is 1.

In some embodiments there is a compound of formula (I) wherein the compound is selected from Table 1.

| TABLE 1 | | | |
|---|---|---|---|
| # | Structure | Compound Name | m/e |
| 1 | 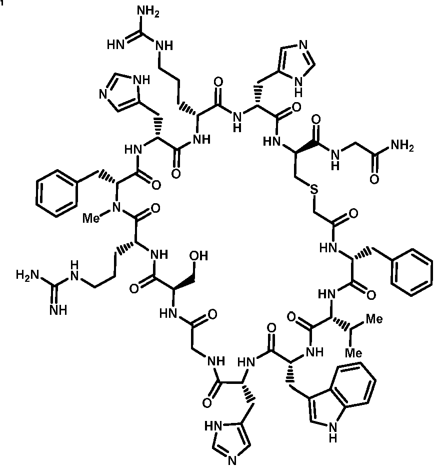 | rac-(3R,6S,9S,12S,15S,18S,21S,27S,30S,3 3S,36S)-N-(2-amino-2-oxo-ethyl)-15,36-dibenzyl-9,18-bis(3-guanidinopropyl)-21-(hydroxymethyl)-6,12,27-tris(1H-imidazol-5-ylmethyl)-30-(1H-indol-3-ylmethyl)-33-isopropyl-16-methyl-5,8,11,14,17,20,23,26,29,32,35,38-dodecaoxo-1-thia-4,7,10,13,16,19,22,25,28,31,34,37-dodecazacyclononatriacontane-3-carboxamide | 1678.78 |
| 2 | 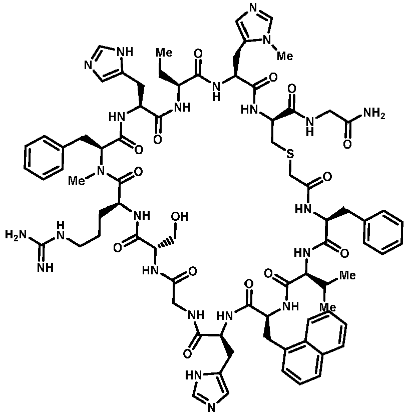 | (3R,6S,9S,12S,15S,18S,21S,27S,30S,3 3S,36S)-N-(2-amino-2-oxo-ethyl)-15,36-dibenzyl-9-ethyl-18-(3-guanidinopropyl)-21-(hydroxymethyl)-12,27-bis(1H-imidazol-4-ylmethyl)-33-isopropyl-16-methyl-6-[(3-methylimidazol-4-yl)methyl]-30-(1-naphthylmethyl)-5,8,11,14,17,20,23,26,29,32,35,38-dodecaoxo-1-thia-4,7,10,13,16,19,22,25,28,31,34,37-dodecazacyclononatriacontane-3-carboxamide | 1622.88 |
| 3 | 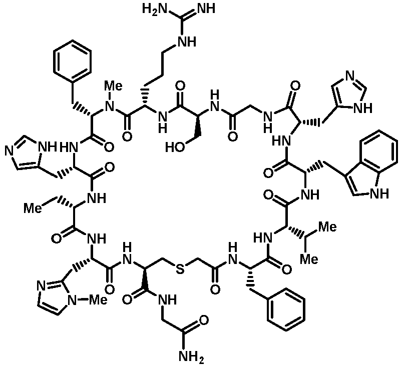 | (3R,6S,9S,12S,15S,18S,21S,27S,30S,3 3S,36S)-N-(2-amino-2-oxo-ethyl)-15,36-dibenzyl-9-ethyl-18-(3-guanidinopropyl)-21-(hydroxymethyl)-12,27-bis(1H-imidazol-4-ylmethyl)-30-(1H-indol-3-ylmethyl)-33-isopropyl-16-methyl-6-[(3-methylimidazol-4-yl)methyl]-5,8,11,14,17,20,23,26,29,32,35,38-dodecaoxo-1-thia-4,7,10,13,16,19,22,25,28,31,34,37-dodecazacyclononatriacontane-3-carboxamide | 1621.76 |
| 4 | 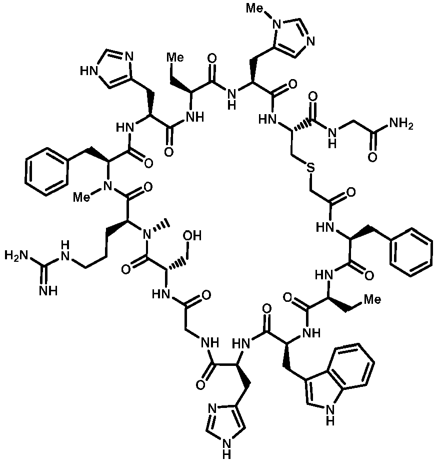 | (3R,6S,9S,12S,15S,18S,21S,27S,30S,3 3S,36S)-N-(2-amino-2-oxo-ethyl)-15,36-dibenzyl-9,33-diethyl-18-(3-guanidinopropyl)-21-(hydroxymethyl)-12,27-bis(1H-imidazol-4-ylmethyl)-30-(1H-indol-3-ylmethyl)-16-methyl-6-[(3-methylimidazol-4-yl)methyl]-5,8,11,14,17,20,23,26,29,32,35,38-dodecaoxo-1-thia-4,7,10,13,16,19,22,25,28,31,34,37-dodecazacyclononatriacontane-3-carboxamide | 1607.83 |
| 5 | 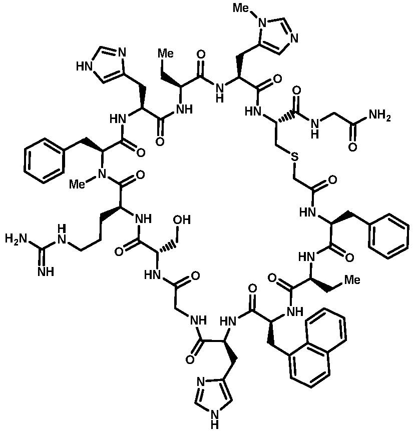 | (3R,6S,9S,12S,15S,18S,21S,27S,30S,3 3S,36S)-N-(2-amino-2-oxo-ethyl)-15,36-dibenzyl-9,33-diethyl-18-(3-guanidinopropyl)-21-(hydroxymethyl)-12,27-bis(1H-imidazol-4-ylmethyl)-16-methyl-6-[(3-methylimidazol-4-yl)methyl]-30-(1-naphthylmethyl)-5,8,11,14,17,20,23,26,29,32,35,38-dodecaoxo-1-thia-4,7,10,13,16,19,22,25,28,31,34,37-dodecazacyclononatriacontane-3-carboxamide | 1618.86 |
| 6 | 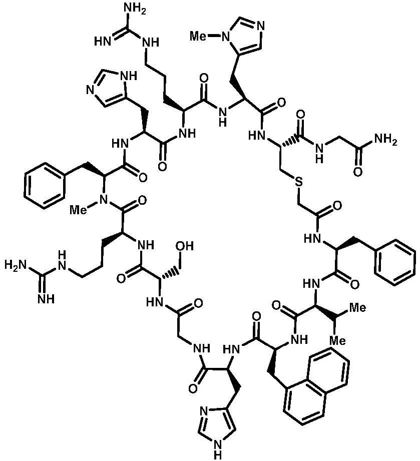 | (3R,6S,9S,12S,15S,18S,21S,27S,30S,3 3S,36S)-N-(2-amino-2-oxo-ethyl)-15,36-dibenzyl-9,18-bis(3-guanidinopropyl)-21-(hydroxymethyl)-12,27-bis(1H-imidazol-4-ylmethyl)-33-isopropyl-16-methyl-6-[(3-methylimidazol-4-yl)methyl]-30-(1-naphthylmethyl)-5,8,11,14,17,20,23,26,29,32,35,38-dodecaoxo-1-thia-4,7,10,13,16,19,22,25,28,31,34,37-dodecazacyclononatriacontane-3-carboxamide | 1703.8 |
| 7 | 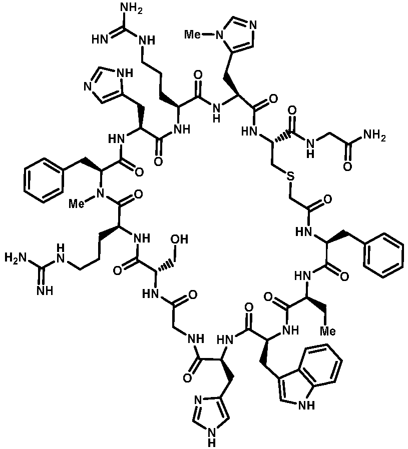 | (3R,6S,9S,12S,15S,18S,21S,27S,30S,3 3S,36S)-N-(2-amino-2-oxo-ethyl)-15,36-dibenzyl-33-ethyl-9,18-bis(3-guanidinopropyl)-21-(hydroxymethyl)-12,27-bis(1H-imidazol-4-ylmethyl)-30-(1H-indol-3-ylmethyl)-16-methyl-6-[(3-methylimidazol-4-yl)methyl]-5,8,11,14,17,20,23,26,29,32,35,38-dodecaoxo-1-thia-4,7,10,13,16,19,22,25,28,31,34,37-dodecazacyclononatriacontane-3-carboxamide | 1678.78 |
| 8 | 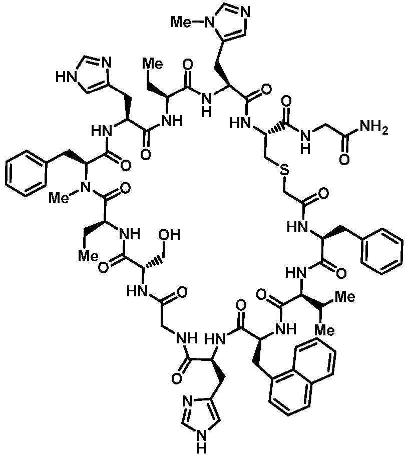 | (3R,6S,9S,12S,15S,18S,21S,27S,30S,3 3S,36S)-N-(2-amino-2-oxo-ethyl)-15,36-dibenzyl-9,18-diethyl-21-(hydroxymethyl)-12,27-bis(1H-imidazol-4-ylmethyl)-33-isopropyl-16-methyl-6-[(3-methylimidazol-4-yl)methyl]-30-(1-naphthylmethyl)-5,8,11,14,17,20,23,26,29,32,35,38-dodecaoxo-1-thia-4,7,10,13,16,19,22,25,28,31,34,37-dodecazacyclononatriacontane-3-carboxamide | 1561.71 |
| 9 | 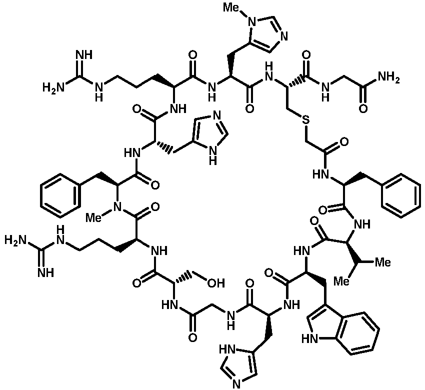 | rac-(3R,6S,9S,12S,15S,18S,21S,27S,30S,3 3S,36S)-N-(2-amino-2-oxo-ethyl)-15,36-dibenzyl-9,18-bis(3-guanidinopropyl)-21-(hydroxymethyl)-12,27-bis(1H-imidazol-5-ylmethyl)-30-(1H-indol-3-ylmethyl)-33-isopropyl-16-methyl-6-[(3-methylimidazol-4-yl)methyl]-5,8,11,14,17,20,23,26,29,32,35,38-dodecaoxo-1-thia-4,7,10,13,16,19,22,25,28,31,34,37-dodecazacyclononatriacontane-3-carboxamide | 1692.80 |
| 10 | 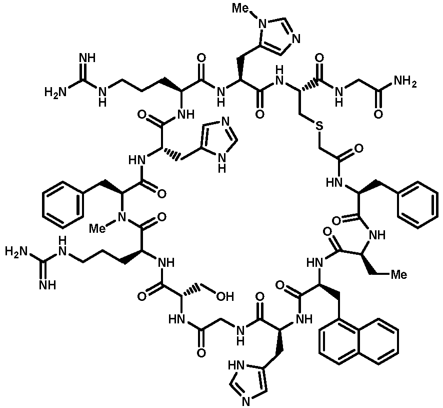 | rac-(3R,6S,9S,12S,15S,18S,21S,27S,30S,3 3S,36S)-N-(2-amino-2-oxo-ethyl)-15,36-dibenzyl-33-ethyl-9,18-bis(3-guanidinopropyl)-21-(hydroxymethyl)-12,27-bis(1H-imidazol-5-ylmethyl)-16-methyl-6-[(3-methylimidazol-4-yl)methyl]-30-(1-naphthylmethyl)-5,8,11,14,17,20,23,26,29,32,35,38-dodecaoxo-1-thia-4,7,10,13,16,19,22,25,28,31,34,37-dodecazacyclononatriacontane-3-carboxamide | 1689.79 |
| 11 | 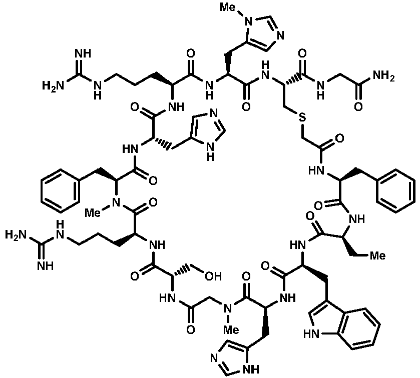 | rac-(3R,6S,9S,12S,15S,18S,21S,27S,30S,3 3S,36S)-N-(2-amino-2-oxo-ethyl)-15,36-dibenzyl-33-ethyl-9,18-bis(3-guanidinopropyl)-21-(hydroxymethyl)-12,27-bis(1H-imidazol-5-ylmethyl)-30-(1H-indol-3-ylmethyl)-16,25-dimethyl-6-[(3-methylimidazol-4-yl)methyl]-5,8,11,14,17,20,23,26,29,32,35,38-dodecaoxo-1-thia-4,7,10,13,16,19,22,25,28,31,34,37-dodecazacyclononatriacontane-3-carboxamide | 1692.80 |
| 12 | 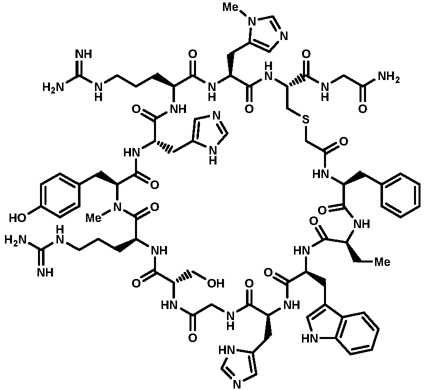 | rac-(3R,6S,9S,12S,15S,18S,21S,27S,30S,3 3S,36S)-N-(2-amino-2-oxo-ethyl)-36-benzyl-33-ethyl-9,18-bis(3-guanidinopropyl)-21-(hydroxymethyl)-15-[(4-hydroxyphenyl)methyl]-12,27-bis(1H-imidazol-5-ylmethyl)-30-(1H-indol-3-ylmethyl)-16-methyl-6-[(3-methylimidazol-4-yl)methyl]-5,8,11,14,17,20,23,26,29,32,35,38-dodecaoxo-1-thia-4,7,10,13,16,19,22,25,28,31,34,37-dodecazacyclononatriacontane-3-carboxamide | 1694.78 |
| 13 | 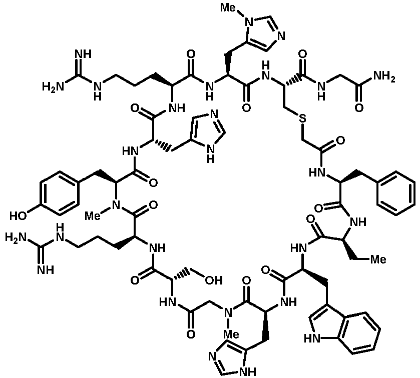 | rac-(3R,6S,9S,12S,15S,18S,21S,27S,30S,3 3S,36S)-N-(2-amino-2-oxo-ethyl)-36-benzyl-33-ethyl-9,18-bis(3-guanidinopropyl)-21-(hydroxymethyl)-15-[(4-hydroxyphenyl)methyl]-12,27-bis(1H-imidazol-5-ylmethyl)-30-(1H-indol-3-ylmethyl)-16,25-dimethyl-6-[(3-methylimidazol-4-yl)methyl]-5,8,11,14,17,20,23,26,29,32,35,38-dodecaoxo-1-thia-4,7,10,13,16,19,22,25,28,31,34,37-dodecazacyclononatriacontane-3-carboxamide | 1708.80 |
| 14 | 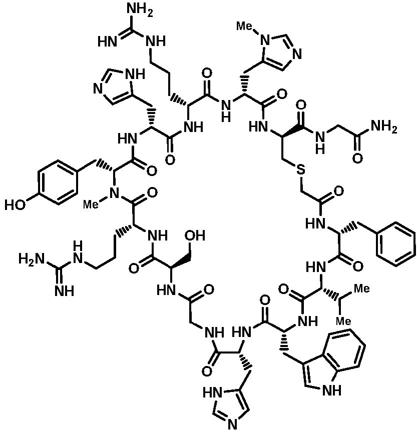 | rac-(3R,6S,9S,12S,15S,18S,21S,27S,30S,3 3S,36S)-N-(2-amino-2-oxo-ethyl)-36-benzyl-9,18-bis(3-guanidinopropyl)-21-(hydroxymethyl)-15-[(4-hydroxyphenyl)methyl]-12,27-bis(1H-imidazol-5-ylmethyl)-30-(1H-indol-3-ylmethyl)-33-isopropyl-16-methyl-6-[(3-methylimidazol-4-yl)methyl]-5,8,11,14,17,20,23,26,29,32,35,38-dodecaoxo-1-thia-4,7,10,13,16,19,22,25,28,31,34,37-dodecazacyclononatriacontane-3-carboxamide | 1708.79 |
| 15 | 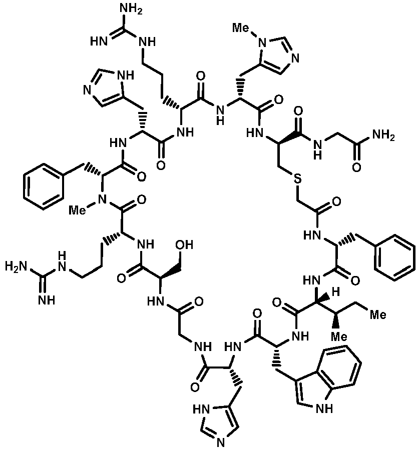 | rac-(3R,6S,9S,12S,15S,18S,21S,27S,30S,3 3S,36S)-N-(2-amino-2-oxo-ethyl)-15,36-dibenzyl-9,18-bis(3-guanidinopropyl)-21-(hydroxymethyl)-12,27-bis(1H-imidazol-5-ylmethyl)-30-(1H-indol-3-ylmethyl)-16-methyl-6-[(3-methylimidazol-4-yl)methyl]-5,8,11,14,17,20,23,26,29,32,35,38-dodecaoxo-33-[rac-(1S)-1-methylpropyl]-1-thia-4,7,10,13,16,19,22,25,28,31,34,37-dodecazacyclononatriacontane-3-carboxamide | 1706.82 |
| 16 | 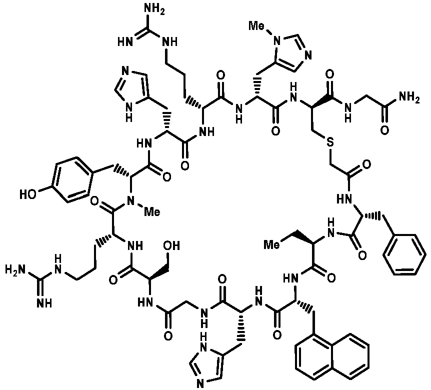 | rac-(3R,6S,9S,12S,15S,18S,21S,27S,30S,3 3S,36S)-N-(2-amino-2-oxo-ethyl)-36-benzyl-33-ethyl-9,18-bis(3-guanidinopropyl)-21-(hydroxymethyl)-15-[(4-hydroxyphenyl)methyl]-12,27-bis(1H-imidazol-5-ylmethyl)-16-methyl-6-[(3-methylimidazol-4-yl)methyl]-30-(1-naphthylmethyl)-5,8,11,14,17,20,23,26,29,32,35,38-dodecaoxo-1-thia-4,7,10,13,16,19,22,25,28,31,34,37-dodecazacyclononatriacontane-3-carboxamide | 1705.78 |
| 17 | 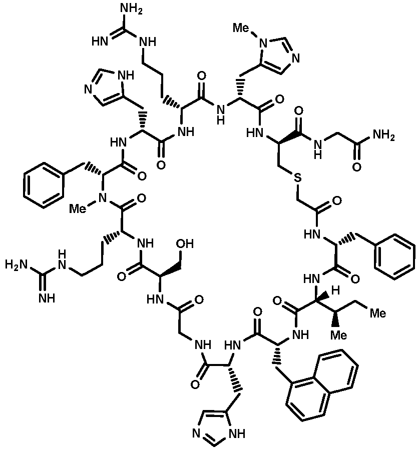 | rac-(3R,6S,9S,12S,15S,18S,21S,27S,30S,3 3S,36S)-N-(2-amino-2-oxo-ethyl)-15,36-dibenzyl-9,18-bis(3-guanidinopropyl)-21-(hydroxymethyl)-12,27-bis(1H-imidazol-5-ylmethyl)-16-methyl-6-[(3-methylimidazol-4-yl)methyl]-30-(1-naphthylmethyl)-5,8,11,14,17,20,23,26,29,32,35,38-dodecaoxo-33-[rac-(1S)-1-methylpropyl]-1-thia-4,7,10,13,16,19,22,25,28,31,34,37-dodecazacyclononatriacontane-3-carboxamide | 1717.82 |
| 18 | 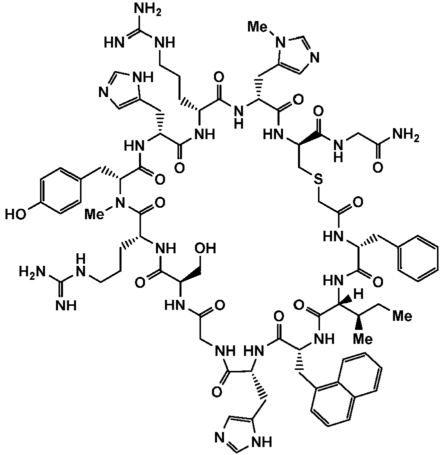 | rac-(3R,6S,9S,12S,15S,18S,21S,27S,30S,3 3S,36S)-N-(2-amino-2-oxo-ethyl)-36-benzyl-9,18-bis(3-guanidinopropyl)-21-(hydroxymethyl)-15-[(4-hydroxyphenyl)methyl]-12,27-bis(1H-imidazol-5-ylmethyl)-16-methyl-6-[(3-methylimidazol-4-yl)methyl]-30-(1-naphthylmethyl)-5,8,11,14,17,20,23,26,29,32,35,38-dodecaoxo-33-[rac-(1S)-1-methylpropyl]-1-thia-4,7,10,13,16,19,22,25,28,31,34,37-dodecazacyclononatriacontane-3-carboxamide | 1733.82 |
| 19 | 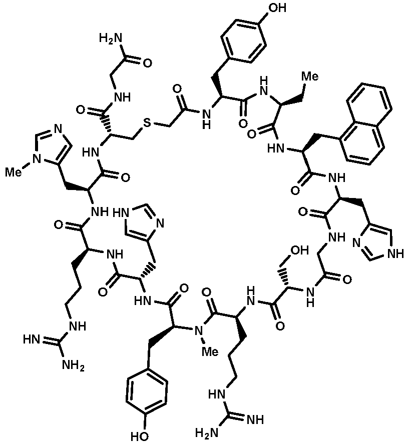 | (3R,6S,9S,12S,15S,18S,21S,27S,30S,3 3S,36S)-N-(2-amino-2-oxo-ethyl)-33-ethyl-9,18-bis(3-guanidinopropyl)-21-(hydroxymethyl)-15,36-bis[(4-hydroxyphenyl)methyl]-12,27-bis(1H-imidazol-4-ylmethyl)-16-methyl-6-[(3-methylimidazol-4-yl)methyl]-30-(1-naphthylmethyl)-5,8,11,14,17,20,23,26,29,32,35,38-dodecaoxo-1-thia-4,7,10,13,16,19,22,25,28,31,34,37-dodecazacyclononatriacontane-3-carboxamide | 1721.78 |
| 20 | 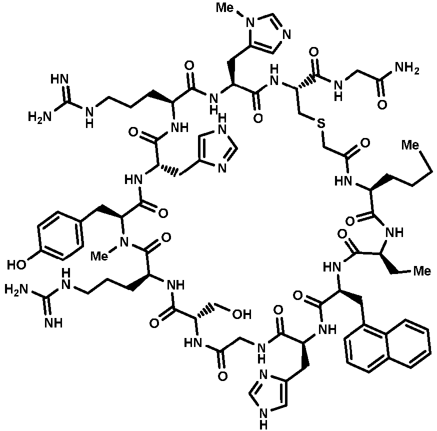 | (3R,6S,9S,12S,15S,18S,21S,27S,30S,3 3S,36S)-N-(2-amino-2-oxo-ethyl)-36-butyl-33-ethyl-9,18-bis(3-guanidinopropyl)-21-(hydroxymethyl)-15-[(4-hydroxyphenyl)methyl]-12,27-bis(1H-imidazol-4-ylmethyl)-16-methyl-6-[(3-methylimidazol-4-yl)methyl]-30-(1-naphthylmethyl)-5,8,11,14,17,20,23,26,29,32,35,38-dodecaoxo-1-thia-4,7,10,13,16,19,22,25,28,31,34,37-dodecazacyclononatriacontane-3-carboxamide | 1671.80 |
| 21 | 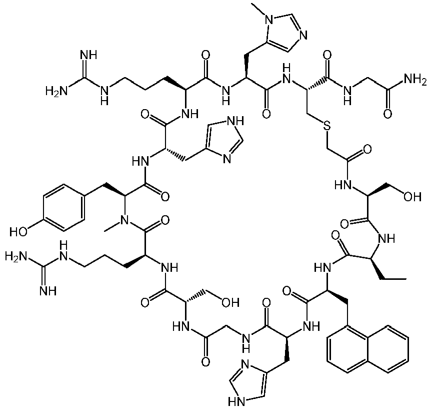 | (3R,6S,9S,12S,15S,18S,21S,27S,30S,3 3S,36S)-N-(2-amino-2-oxo-ethyl)-33-ethyl-9,18-bis(3-guanidinopropyl)-21,36-bis(hydroxymethyl)-15-[(4-hydroxyphenyl)methyl]-12,27-bis(1H-imidazol-4-ylmethyl)-16-methyl-6-[(3-methylimidazol-4-yl)methyl]-30-(1-naphthylmethyl)-5,8,11,14,17,20,23,26,29,32,35,38-dodecaoxo-1-thia-4,7,10,13,16,19,22,25,28,31,34,37-dodecazacyclononatriacontane-3-carboxamide | 1645.75 |
| 22 | 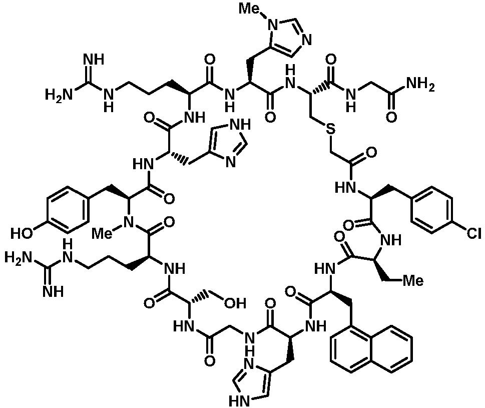 | (3R,6S,9S,12S,15S,18S,21S,27S,30S,3 3S,36S)-N-(2-amino-2-oxo-ethyl)-36-[(4-chlorophenyl)methyl]-33-ethyl-9,18-bis(3-guanidinopropyl)-21-(hydroxymethyl)-15-[(4-hydroxyphenyl)methyl]-12,27-bis(1H-imidazol-4-ylmethyl)-16-methyl-6-[(3-methylimidazol-4-yl)methyl]-30-(1-naphthylmethyl)-5,8,11,14,17,20,23,26,29,32,35,38-dodecaoxo-1-thia-4,7,10,13,16,19,22,25,28,31,34,37-dodecazacyclononatriacontane-3-carboxamide | 1739.75 |
| 23 | 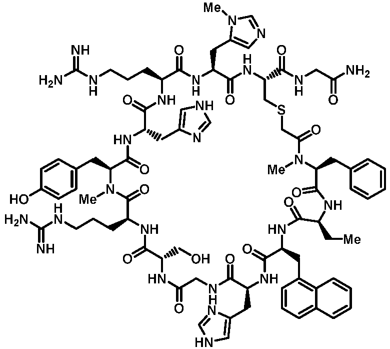 | (3R,6S,9S,12S,15S,18S,21S,27S,30S,3 3S,36S)-N-(2-amino-2-oxo-ethyl)-36-benzyl-33-ethyl-9,18-bis(3-guanidinopropyl)-21-(hydroxymethyl)-15-[(4-hydroxyphenyl)methyl]-12,27-bis(1H-imidazol-4-ylmethyl)-16,37-dimethyl-6-[(3-methylimidazol-4-yl)methyl]-30-(1-naphthylmethyl)-5,8,11,14,17,20,23,26,29,32,35,38-dodecaoxo-1-thia-4,7,10,13,16,19,22,25,28,31,34,37-dodecazacyclononatriacontane-3-carboxamide | 1719.80 |
| 24 | 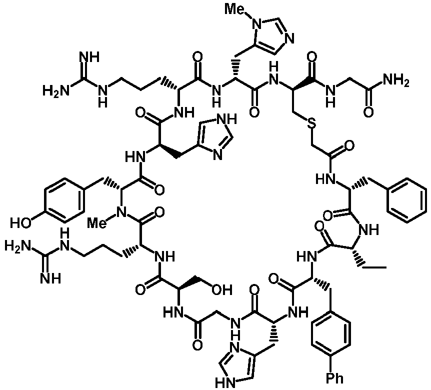 | (3R,6S,9S,12S,15S,18S,21S,27S,30S,3 3S,36S)-N-(2-amino-2-oxo-ethyl)-36-benzyl-33-ethyl-9,18-bis(3-guanidinopropyl)-21-(hydroxymethyl)-15-[(4-hydroxyphenyl)methyl]-12,27-bis(1H-imidazol-4-ylmethyl)-16-methyl-6-[(3-methylimidazol-4-yl)methyl]-5,8,11,14,17,20,23,26,29,32,35,38-dodecaoxo-30-[(4-phenylphenyl)methyl]-1-thia-4,7,10,13,16,19,22,25,28,31,34,37-dodecazacyclononatriacontane-3-carboxamide | 1731.80 |
| 25 | 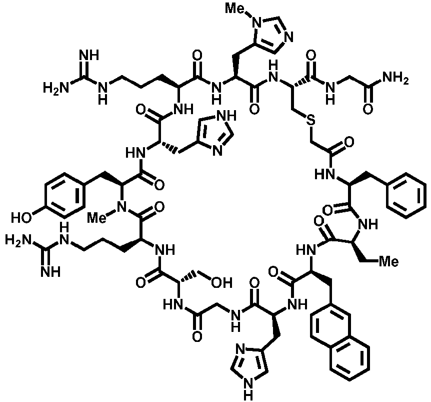 | (3R,6S,9S,12S,15S,18S,21S,27S,30S,3 3S,36S)-N-(2-amino-2-oxo-ethyl)-36-benzyl-33-ethyl-9,18-bis(3-guanidinopropyl)-21-(hydroxymethyl)-15-[(4-hydroxyphenyl)methyl]-12,27-bis(1H-imidazol-4-ylmethyl)-16-methyl-6-[(3-methylimidazol-4-yl)methyl]-30-(2-naphthylmethyl)-5,8,11,14,17,20,23,26,29,32,35,38-dodecaoxo-1-thia-4,7,10,13,16,19,22,25,28,31,34,37-dodecazacyclononatriacontane-3-carboxamide | 1705.78 |
| 26 | 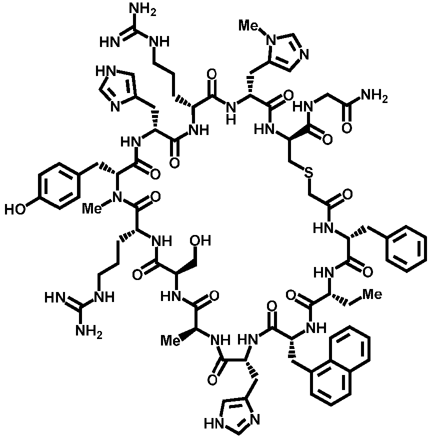 | (3R,6S,9S,12S,15S,18S,21S,24R,27S, 30S,33S,36S)-N-(2-amino-2-oxoethyl)-36-benzyl-33-ethyl-9,18-bis(3-guanidinopropyl)-21-(hydroxymethyl)-15-[(4-hydroxyphenyl)methyl]-12,27-bis(1H-imidazol-4-ylmethyl)-16,24-dimethyl-6-[(3-methylimidazol-4-yl)methyl]-30-(1-naphthylmethyl)-5,8,11,14,17,20,23,26,29,32,35,38-dodecaoxo-1-thia-4,7,10,13,16,19,22,25,28,31,34,37-dodecazacyclononatriacontane-3-carboxamide | 1719.80 |
| 27 | 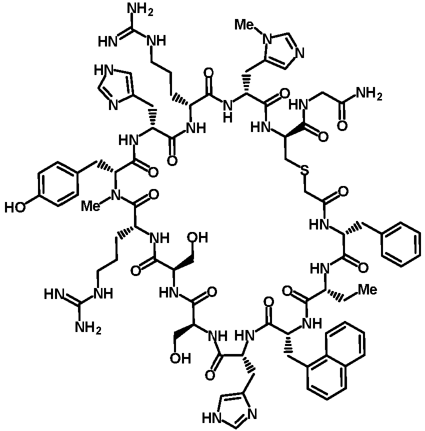 | (3R,6S,9S,12S,15S,18S,21S,24R,27S, 30S,33S,36S)-N-(2-amino-2-oxoethyl)-36-benzyl-33-ethyl-9,18-bis(3-guanidinopropyl)-21,24-bis(hydroxymethyl)-15-[(4-hydroxyphenyl)methyl]-12,27-bis(1H-imidazol-4-ylmethyl)-16-methyl-6-[(3-methylimidazol-4-yl)methyl]-30-(1-naphthylmethyl)-5,8,11,14,17,20,23,26,29,32,35,38-dodecaoxo-1-thia-4,7,10,13,16,19,22,25,28,31,34,37-dodecazacyclononatriacontane-3-carboxamide | 1735.79 |
| 28 | 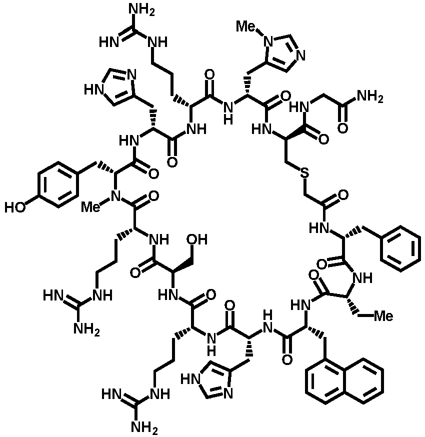 | (3R,6S,9S,12S,15S,18S,21S,24S,27S,3 0S,33S,36S)-N-(2-amino-2-oxoethyl)-36-benzyl-33-ethyl-9,18,24-tris(3-guanidinopropyl)-21-(hydroxymethyl)-15-[(4-hydroxyphenyl)methyl]-12,27-bis(1H-imidazol-4-ylmethyl)-16-methyl-6-[(3-methylimidazol-4-yl)methyl]-30-(1-naphthylmethyl)-5,8,11,14,17,20,23,26,29,32,35,38-dodecaoxo-1-thia-4,7,10,13,16,19,22,25,28,31,34,37-dodecazacyclononatriacontane-3-carboxamide | 1804.86 |
| 29 | 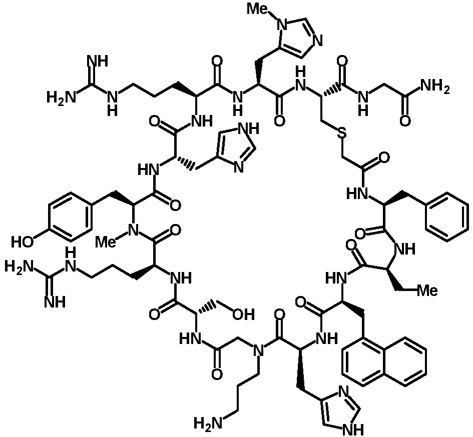 | (3R,6S,9S,12S,15S,18S,21S,27S,30S,3 3S,36S)-N-(2-amino-2-oxo-ethyl)-25-(3-aminopropyl)-36-benzyl-33-ethyl-9,18-bis(3-guanidinopropyl)-21-(hydroxymethyl)-15-[(4-hydroxyphenyl)methyl]-12,27-bis(1H-imidazol-4-ylmethyl)-16-methyl-6-[(3-methylimidazol-4-yl)methyl]-30-(1-naphthylmethyl)-5,8,11,14,17,20,23,26,29,32,35,38-dodecaoxo-1-thia-4,7,10,13,16,19,22,25,28,31,34,37-dodecazacyclononatriacontane-3-carboxamide | 1762.84 |
| 30 | 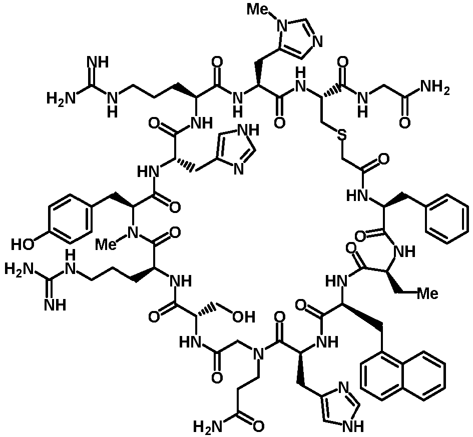 | (3R,6S,9S,12S,15S,18S,21S,27S,30S,3 3S,36S)-N-(2-amino-2-oxo-ethyl)-25-(3-amino-3-oxo-propyl)-36-benzyl-33-ethyl-9,18-bis(3-guanidinopropyl)-21-(hydroxymethyl)-15-[(4-hydroxyphenyl)methyl]-12,27-bis(1H-imidazol-4-ylmethyl)-16-methyl-6-[(3-methylimidazol-4-yl)methyl]-30-(1-naphthylmethyl)-5,8,11,14,17,20,23,26,29,32,35,38-dodecaoxo-1-thia-4,7,10,13,16,19,22,25,28,31,34,37-dodecazacyclononatriacontane-3-carboxamide | 1776.82 |
| **31** | 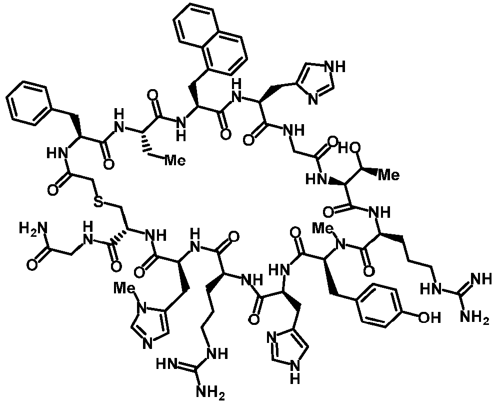 | (3R,6S,9S,12S,15S,18S,21S,27S,30S,3 3S,36S)-N-(2-amino-2-oxo-ethyl)-36-benzyl-33-ethyl-9,18-bis(3-guanidinopropyl)-21-[(1S)-1-hydroxyethyl]-15-[(4-hydroxyphenyl)methyl]-12,27-bis(1H-imidazol-4-ylmethyl)-16-methyl-6-[(3-methylimidazol-4-yl)methyl]-30-(1-naphthylmethyl)-5,8,11,14,17,20,23,26,29,32,35,38-dodecaoxo-1-thia-4,7,10,13,16,19,22,25,28,31,34,37-dodecazacyclononatriacontane-3-carboxamide | 1719.80 |
| **32** | 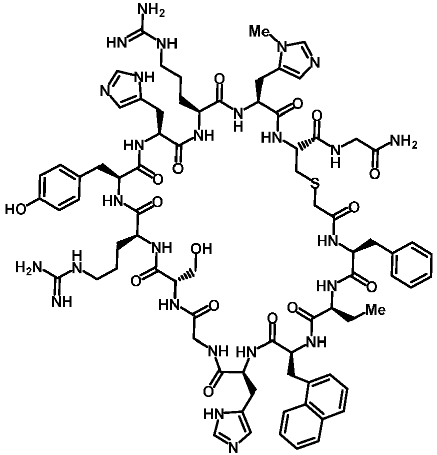 | (3R,6S,9S,12S,15S,18S,21S,27S,30S,3 3S,36S)-N-(2-amino-2-oxo-ethyl)-36-benzyl-33-ethyl-9,18-bis(3-guanidinopropyl)-21-(hydroxymethyl)-15-[(4-hydroxyphenyl)methyl]-12,27-bis(1H-imidazol-4-ylmethyl)-6-[(3-methylimidazol-4-yl)methyl]-30-(1-naphthylmethyl)-5,8,11,14,17,20,23,26,29,32,35,38-dodecaoxo-1-thia-4,7,10,13,16,19,22,25,28,31,34,37-dodecazacyclononatriacontane-3-carboxamide | 1691.77 |
| **33** | 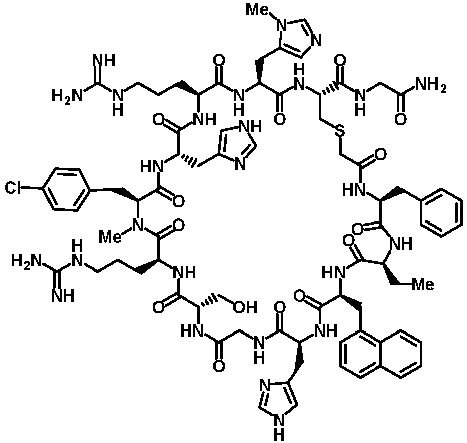 | (3R,6S,9S,12S,15S,18S,21S,27S,30S,3 3S,36S)-N-(2-amino-2-oxo-ethyl)-36-benzyl-15-[(4-chlorophenyl)methyl]-33-ethyl-9,18-bis(3-guanidinopropyl)-21-(hydroxymethyl)-12,27-bis(1H-imidazol-4-ylmethyl)-16-methyl-6-[(3-methylimidazol-4-yl)methyl]-30-(1-naphthylmethyl)-5,8,11,14,17,20,23,26,29,32,35,38-dodecaoxo-1-thia-4,7,10,13,16,19,22,25,28,31,34,37-dodecazacyclononatriacontane-3-carboxamide | 1723.75 |
| **34** | 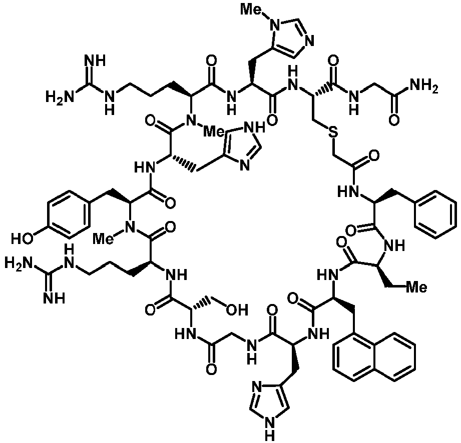 | (3R,6S,9S,12S,15S,18S,21S,27S,30S,3 3S,36S)-N-(2-amino-2-oxo-ethyl)-36-benzyl-33-ethyl-9,18-bis(3-guanidinopropyl)-21-(hydroxymethyl)-15-[(4-hydroxyphenyl)methyl]-12,27-bis(1H-imidazol-4-ylmethyl)-10,16-dimethyl-6-[(3-methylimidazol-4-yl)methyl]-30-(1-naphthylmethyl)-5,8,11,14,17,20,23,26,29,32,35,38-dodecaoxo-1-thia-4,7,10,13,16,19,22,25,28,31,34,37-dodecazacyclononatriacontane-3-carboxamide | 1719.80 |
| **35** | 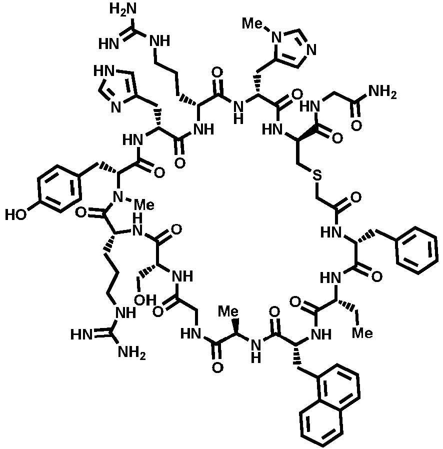 | (3R,6S,9S,12S,15S,18S,21S,27S,30S,3 3S,36S)-N-(2-amino-2-oxo-ethyl)-36-benzyl-33-ethyl-9,18-bis(3-guanidinopropyl)-21-(hydroxymethyl)-15-[(4-hydroxyphenyl)methyl]-12-(1H-imidazol-4-ylmethyl)-16,27-dimethyl-6-[(3-methylimidazol-4-yl)methyl]-30-(1-naphthylmethyl)-5,8,11,14,17,20,23,26,29,32,35,38-dodecaoxo-1-thia-4,7,10,13,16,19,22,25,28,31,34,37-dodecazacyclononatriacontane-3-carboxamide | 1639.76 |
| **36** | 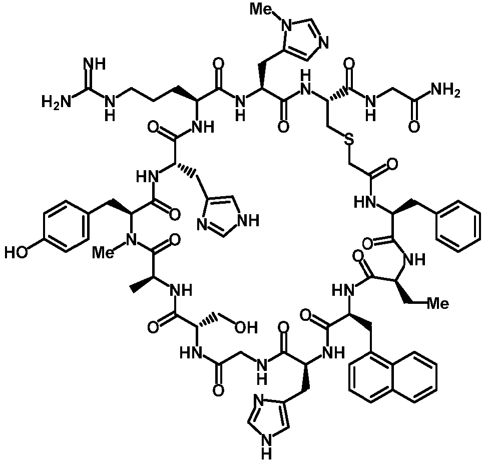 | (3R,6S,9S,12S,15S,18S,21S,27S,30S,3 3S,36S)-N-(2-amino-2-oxo-ethyl)-36-benzyl-33-ethyl-9-(3-guanidinopropyl)-21-(hydroxymethyl)-15-[(4-hydroxyphenyl)methyl]-12,27-bis(1H-imidazol-4-ylmethyl)-16,18-dimethyl-6-[(3-methylimidazol-4-yl)methyl]-30-(1-naphthylmethyl)-5,8,11,14,17,20,23,26,29,32,35,38-dodecaoxo-1-thia-4,7,10,13,16,19,22,25,28,31,34,37-dodecazacyclononatriacontane-3-carboxamide | 1620.72 |
| **37** | 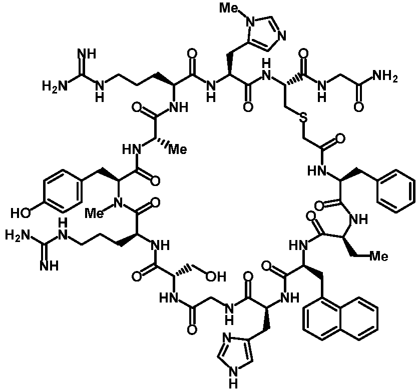 | (3R,6S,9S,12S,15S,18S,21S,27S,30S,3 3S,36S)-N-(2-amino-2-oxo-ethyl)-36-benzyl-33-ethyl-9,18-bis(3-guanidinopropyl)-21-(hydroxymethyl)-15-[(4-hydroxyphenyl)methyl]-27-(1H-imidazol-4-ylmethyl)-12,16-dimethyl-6-[(3-methylimidazol-4-yl)methyl]-30-(1-naphthylmethyl)-5,8,11,14,17,20,23,26,29,32,35,38-dodecaoxo-1-thia-4,7,10,13,16,19,22,25,28,31,34,37-dodecazacyclononatriacontane-3-carboxamide | 1639.76 |
| **38** | 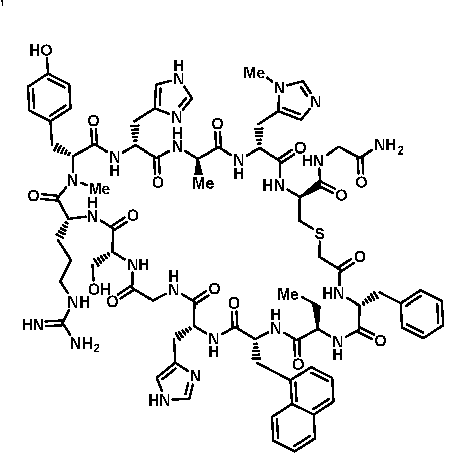 | (3R,6S,9S,12S,15S,18S,21S,27S,30S,3 3S,36S)-N-(2-amino-2-oxo-ethyl)-36-benzyl-33-ethyl-18-(3-guanidinopropyl)-21-(hydroxymethyl)-15-[(4-hydroxyphenyl)methyl]-12,27-bis(1H-imidazol-4-ylmethyl)-9,16-dimethyl-6-[(3-methylimidazol-4-yl)methyl]-30-(1-naphthylmethyl)-5,8,11,14,17,20,23,26,29,32,35,38-dodecaoxo-1-thia-4,7,10,13,16,19,22,25,28,31,34,37-dodecazacyclononatriacontane-3-carboxamide | 1620.72 |
| **39** | 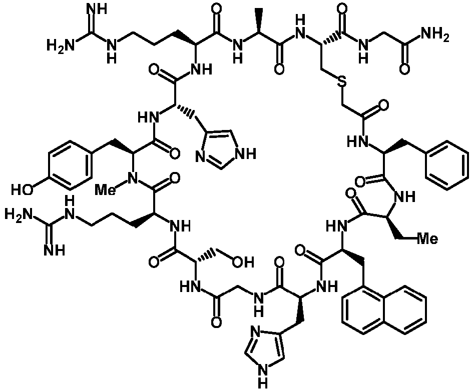 | (3R,6S,9S,12S,15S,18S,21S,27S,30S,3 3S,36S)-N-(2-amino-2-oxo-ethyl)-36-benzyl-33-ethyl-9,18-bis(3-guanidinopropyl)-21-(hydroxymethyl)-15-[(4-hydroxyphenyl)methyl]-12,27-bis(1H-imidazol-4-ylmethyl)-6,16-dimethyl-30-(1-naphthylmethyl)-5,8,11,14,17,20,23,26,29,32,35,38-dodecaoxo-1-thia-4,7,10,13,16,19,22,25,28,31,34,37-dodecazacyclononatriacontane-3-carboxamide | 1625.75 |
| **40** | 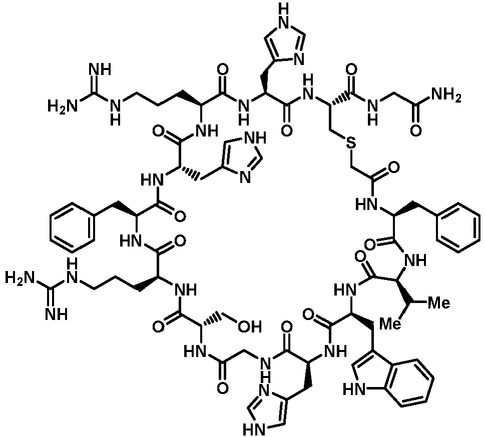 | (3R,6S,9S,12S,15S,18S,21S,27S,30S,3 3S,36S)-N-(2-amino-2-oxo-ethyl)-15,36-dibenzyl-9,18-bis(3-guanidinopropyl)-21-(hydroxymethyl)-6,12,27-tris(1H-imidazol-4-ylmethyl)-30-(1H-indol-3-ylmethyl)-33-isopropyl-5,8,11,14,17,20,23,26,29,32,35,38-dodecaoxo-1-thia-4,7,10,13,16,19,22,25,28,31,34,37-dodecazacyclononatriacontane-3-carboxamide | 1664.77 |
| **41** | 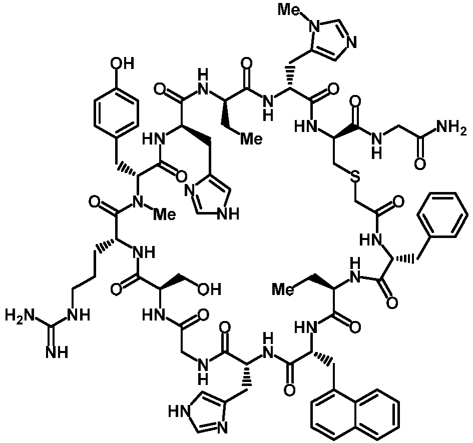 | (3R,6S,9S,12S,15S,18S,21S,27S,30S,3 3S,36S)-N-(2-amino-2-oxo-ethyl)-36-benzyl-9,33-diethyl-18-(3-guanidinopropyl)-21-(hydroxymethyl)-15-[(4-hydroxyphenyl)methyl]-12,27-bis(1H-imidazol-4-ylmethyl)-16-methyl-6-[(3-methylimidazol-4-yl)methyl]-30-(1-naphthylmethyl)-5,8,11,14,17,20,23,26,29,32,35,38-dodecaoxo-1-thia-4,7,10,13,16,19,22,25,28,31,34,37-dodecazacyclononatriacontane-3-carboxamide | 1634.74 |
| **42** | 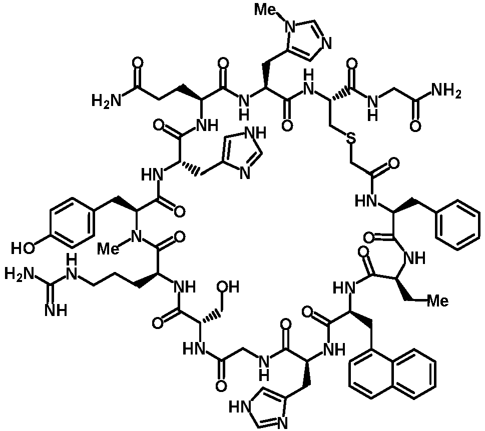 | rac-(3R,6S,9S,12S,15S,18S,21S,27S,30S,3 3S,36S)-N-(2-amino-2-oxo-ethyl)-9-(3-amino-3-oxo-propyl)-36-benzyl-33-ethyl-18-(3-guanidinopropyl)-21-(hydroxymethyl)-15-[(4-hydroxyphenyl)methyl]-12,27-bis(1H-imidazol-4-ylmethyl)-16-methyl-6-[(3-methylimidazol-4-yl)methyl]-30-(1-naphthylmethyl)-5,8,11,14,17,20,23,26,29,32,35,38-dodecaoxo-1-thia-4,7,10,13,16,19,22,25,28,31,34,37-dodecazacyclononatriacontane-3-carboxamide | 1677.74 |
| **43** | 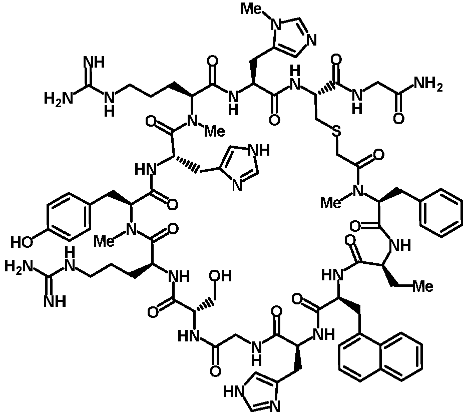 | ac-(3R,6S,9S,12S,15S,18S,21S,27S,30S,3 3S,36S)-N-(2-amino-2-oxo-ethyl)-36-benzyl-33-ethyl-9,18-bis(3-guanidinopropyl)-21-(hydroxymethyl)-15-[(4-hydroxyphenyl)methyl]-12,27-bis(1H-imidazol-4-ylmethyl)-10,16,37-trimethyl-6-[(3-methylimidazol-4-yl)methyl]-30-(1-naphthylmethyl)-5,8,11,14,17,20,23,26,29,32,35,38-dodecaoxo-1-thia-4,7,10,13,16,19,22,25,28,31,34,37-dodecazacyclononatriacontane-3-carboxamide | 1733.82 |
| **44** | 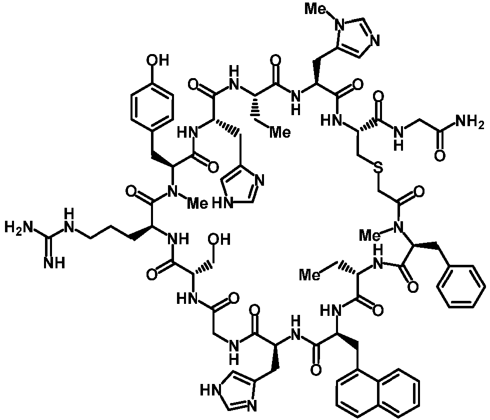 | rac-(3R,6S,9S,12S,15S,18S,21S,27S,30S,3 3S,36S)-N-(2-amino-2-oxo-ethyl)-36-benzyl-9,33-diethyl-18-(3-guanidinopropyl)-21-(hydroxymethyl)-15-[(4-hydroxyphenyl)methyl]-12,27-bis(1H-imidazol-4-ylmethyl)-16,37-dimethyl-6-[(3-methylimidazol-4-yl)methyl]-30-(1-naphthylmethyl)-5,8,11,14,17,20,23,26,29,32,35,38-dodecaoxo-1-thia-4,7,10,13,16,19,22,25,28,31,34,37-dodecazacyclononatriacontane-3-carboxamide | 1648.75 |
| **45** | 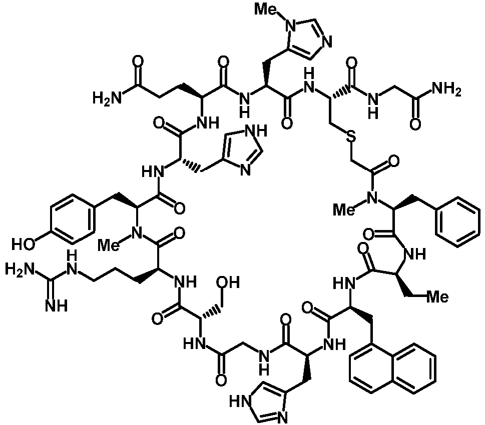 | rac-(3R,6S,9S,12S,15S,18S,21S,27S,30S,3 3S,36S)-N-(2-amino-2-oxo-ethyl)-9-(3-amino-3-oxo-propyl)-36-benzyl-33-ethyl-18-(3-guanidinopropyl)-21-(hydroxymethyl)-15-[(4-hydroxyphenyl)methyl]-12,27-bis(1H-imidazol-4-ylmethyl)-16,37-dimethyl-6-[(3-methylimidazol-4-yl)methyl]-30-(1-naphthylmethyl)-5,8,11,14,17,20,23,26,29,32,35,38-dodecaoxo-1-thia-4,7,10,13,16,19,22,25,28,31,34,37-dodecazacyclononatriacontane-3-carboxamide | 1691.76 |
| **46** | 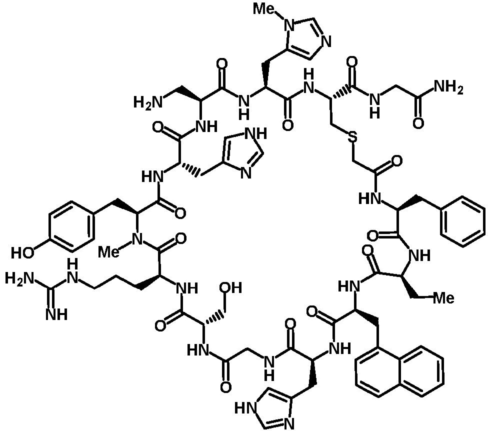 | rac-(3R,6S,9S,12S,15S,18S,21S,27S,30S,3 3S,36S)-9-(aminomethyl)-N-(2-amino-2-oxo-ethyl)-36-benzyl-33-ethyl-18-(3-guanidinopropyl)-21-(hydroxymethyl)-15-[(4-hydroxyphenyl)methyl]-12,27-bis(1H-imidazol-4-ylmethyl)-16-methyl-6-[(3-methylimidazol-4-yl)methyl]-30-(1-naphthylmethyl)-5,8,11,14,17,20,23,26,29,32,35,38-dodecaoxo-1-thia-4,7,10,13,16,19,22,25,28,31,34,37-dodecazacyclononatriacontane-3-carboxamide | 1635.73 |
| **47** | 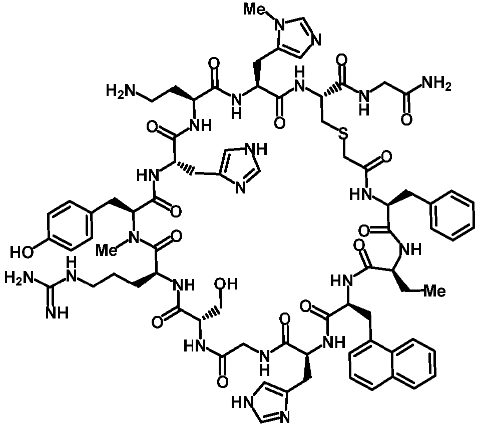 | rac-(3R,6S,9S,12S,15S,18S,21S,27S,30S,3 3S,36S)-9-(2-aminoethyl)-N-(2-amino-2-oxo-ethyl)-36-benzyl-33-ethyl-18-(3-guanidinopropyl)-21-(hydroxymethyl)-15-[(4-hydroxyphenyl)methyl]-12,27-bis(1H-imidazol-4-ylmethyl)-16-methyl-6-[(3-methylimidazol-4-yl)methyl]-30-(1-naphthylmethyl)-5,8,11,14,17,20,23,26,29,32,35,38-dodecaoxo-1-thia-4,7,10,13,16,19,22,25,28,31,34,37-dodecazacyclononatriacontane-3-carboxamide | 1635.73 |
| 48 | 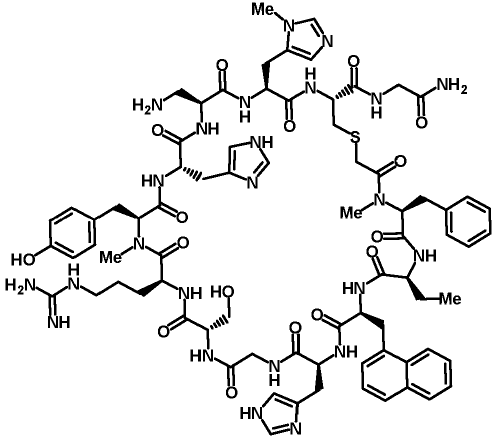 | rac-(3R,6S,9S,12S,15S,18S,21S,27S,30S,3 3S,36S)-9-(aminomethyl)-N-(2-amino-2-oxo-ethyl)-36-benzyl-33-ethyl-18-(3-guanidinopropyl)-21-(hydroxymethyl)-15-[(4-hydroxyphenyl)methyl]-12,27-bis(1H-imidazol-4-ylmethyl)-16,37-dimethyl-6-[(3-methylimidazol-4-yl)methyl]-30-(1-naphthylmethyl)-5,8,11,14,17,20,23,26,29,32,35,38-dodecaoxo-1-thia-4,7,10,13,16,19,22,25,28,31,34,37-dodecazacyclononatriacontane-3-carboxamide | 1649.75 |
| 49 | 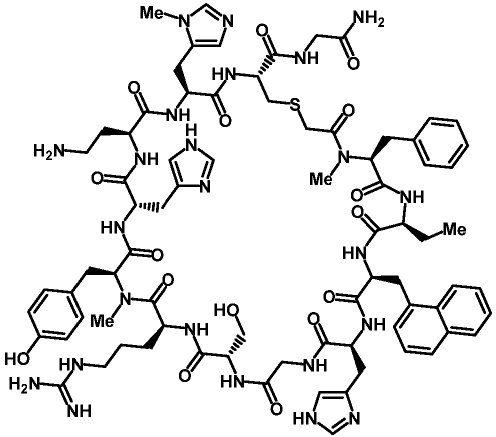 | rac-(3R,6S,9S,12S,15S,18S,21S,27S,30S,3 3S,36S)-9-(2-aminoethyl)-N-(2-amino-2-oxo-ethyl)-36-benzyl-33-ethyl-18-(3-guanidinopropyl)-21-(hydroxymethyl)-15-[(4-hydroxyphenyl)methyl]-12,27-bis(1H-imidazol-4-ylmethyl)-16,37-dimethyl-6-[(3-methylimidazol-4-yl)methyl]-30-(1-naphthylmethyl)-5,8,11,14,17,20,23,26,29,32,35,38-dodecaoxo-1-thia-4,7,10,13,16,19,22,25,28,31,34,37-dodecazacyclononatriacontane-3-carboxamide | 1663.76 |

In another embodiment of the invention, there is a cyclic dodecapeptide of Formula (Ia), or a pharmaceutically acceptable salt, solvate, or stereoisomer thereof:

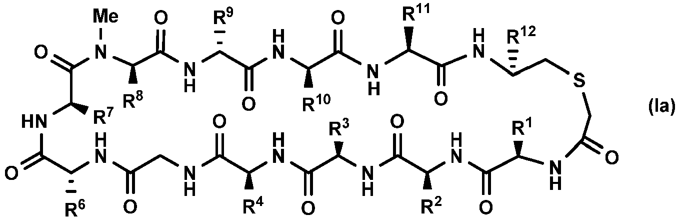

In some embodiments there is a compound of formula (Ia) wherein in R¹, R², R³, R⁴, R⁵, R⁶, R⁷, R⁸, R⁹, R¹⁰, R¹¹ and R¹² are as described above.

In one embodiment of the present invention there is a compound of formula (Ib) wherein: 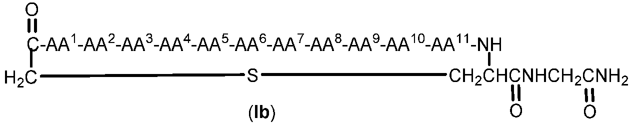
- AA¹: is an aromatic amino acid or N-methyl-aromatic amino acid wherein the aromatic amino acid is phenylalanine wherein the phenyl ring of either is optionally substituted by 1 to 3 C₁₋₆ alkyl, halogens, C₁₋₆-haloalkyl, hydroxyl, C₁₋₆-alkoxy, C₁₋₆-haloalkoxy, C₁₋₆-thioalkyl, C₁₋₆-alkylsulfonyl or cyano;
- AA²: is an aliphatic amino acid of formula H₂NCHR^{a}CO₂H wherein R^{a} is C₁₋₆alkyl or C₁₋₃alkoxy-C₁₋₃alkyl;
- AA³: is an aromatic amino acid of formula H₂NCHR^{a}CO₂H wherein R⁸ is 1-naphthylmethyl, 2-naphthylmethyl or 4-phenylbenzyl and R⁸ is optionally substituted by 1 to 3 C₁₋₆ alkyl, halogens, C₁₋₆-haloalkyl, hydroxyl, C₁₋₆-alkoxy, C₁₋₆-haloalkoxy, C₁₋₆-thioalkyl, C₁₋₆-alkylsulfonyl or cyano, or a heteroarylamino acid wherein R^{a} is 3-indolemethyl optionally C1-3 N-alkyl-3-indolemethyl ;
- AA⁴: is histidine;
- AA⁵: is an aliphatic amino acid wherein said aliphatic amino acid is a compound of formula H₂NCHR^{a}CO₂H wherein R^{a} is hydrogen, methyl or hydroxymethyl, preferably, AA⁵is glycine;
- AA⁶: is arginine an amino acid of formula H₂NCHR^{a}CO₂H wherein R⁸ is C₁₋₆ hydroxyalkyl, preferably AA⁷ is serine;
- AA⁷: is a basic amino acid f formula H₂NCHR^{a}CO₂H wherein R^{a} is guanidino-C₁₋₄-alkyl, preferably AA⁸ is arginine
- AA³: is an aromatic amino acid or N-methyl-aromatic amino acid wherein the aromatic amino acid is phenylalanine, optionally substituted by 1 to three C₁₋₆ alkyl, halogens, C₁₋₆-haloalkyl, C₁₋₆-alkoxy, C₁₋₆-haloalkoxy, C₁₋₆-thioalkyl; preferably AA⁹ is phenylalanine;
- AA⁹: is a heteroaryl amino acid wherein said heteroaryl amino acid is a compound of formula H₂NCHR^{a}CO₂H wherein R^{a} is 1H-imidazole-5-ylmethyl, 3-methylimidazol-4-yl-methyl, preferably AA¹⁰ is histidine;
- AA¹⁰: is a basic amino acid wherein said basic amino acid is a compound of formula H₂NCHR^{a}CO₂H wherein R^{a} is C₁₋₆aminoalkyl, guanidino-C₁₋₄-alkyl, (CH₂)₁₋₃CONH₂, or C₁₋₆ alkyl;.
- AA¹¹: is a heteroaryl amino acid wherein said heteroaryl amino acid is a compound of formula H₂NCHR^{a}CO₂H wherein R^{a} is 1H-imidazole-5-ylmethyl, 3-methylimidazol-4-yl-methyl, preferably R^{a} is 3-methylimidazol-4-yl-methyl.

In another embodiment there is afforded a compound of formula (Ib) wherein:
AA¹ is phenylalanine; AA² is an aliphatic amino acid of formula H₂NCHR^{a}CO₂H wherein R^{a} is is ethyl, isopropyl or sec-butyl; AA³ is 2-amino-3-(naphthalen-1-yl)propanoic acid or tryptophan; AA⁴ is histidine; AA⁵ is glycine; AA⁶ is an aliphatic amino acid of formula H₂NCHR^{a}CO₂H wherein R^{a} is hydroxylmethyl or --CH(OH)Me; AA⁷ is arginine; AA⁸ is phenylalanine optionally substituted with hydroxyl or halo; AA⁹ is histidine; AA¹⁰ is arginine, amino methyl, aminoethyl or amino butyl; AA¹¹ is a basic amino acid of formula H₂NCHR^{a}CO₂H wherein R^{a} is 3-methylimidazol-4-yl)methyl.

In other embodiments of the invention, a cyclic tetradecapeptide of Formula (II), or a pharmaceutically acceptable salt, solvate, or stereoisomer thereof as disclosed above in the Summary of the Disclosure.

In one embodiment of a compound of Formula (II), R^{1a} is methyl or hydrogen. In one embodiment of a compound of Formula (II), R^{1a} is hydrogen. In another embodiment of a compound of Formula (II), R^{1a} is methyl.

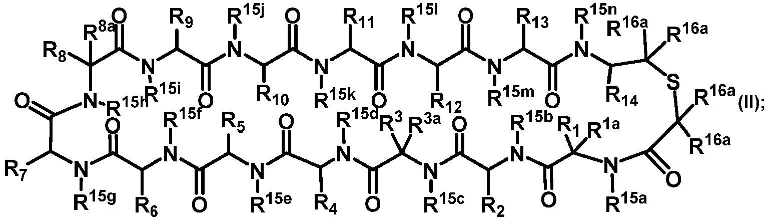

In some embodiments of a compound of Formula (II), R¹ is optionally substituted benzyl. In some embodiments of a compound of Formula (II), R¹ is unsubstituted benzyl.

In some embodiments of a compound of Formula (II), R² is 4-(imidazolyl)methyl optionally substituted with C₁₋₆alkyl. In some embodiments of a compound of Formula (II), R² is 4-(imidazolyl)methyl with a N-substituted methyl. In some embodiments of a compound of Formula (II), 3-(methylimidazol-4-yl)methyl *(iib* wherein *R^{b}* is methyl). In some embodiments of a compound of Formula (II), R² is 4-(imidazolyl)methyl.

In some embodiments of a compound of formula (II) R³ is C₁₋₆alkyl. In some embodiments of a compound of Formula (II), R³ is (1S)-methylpropyl. In some embodiments of a compound of Formula (II), in some embodiments R³ is methyl. In some embodiments of a compound of Formula (II), R³ is C₁₋₆alkyl hydroxyalkyl. In some embodiments of a compound of Formula (II), R³ is (1R)-1-hydroxyethyl.

In some embodiments of a compound of formula (II) R⁴ is C₁₋₆ alkyl; in some embodiments R⁴ is (1S)-methylpropyl; in some embodiments R⁴ is methyl.

In some embodiments of a compound of formula (II), R⁵ is 4-(imidazolyl)methyl optionally substituted with C₁₋₆alkyl. n some embodiments of a compound of formula (II), R⁵ is 4-(imidazolyl)methyl with a N-substituted methyl. n some embodiments of a compound of formula (II), R⁵ is 3-(methylimidazol-4-yl)methyl *(iib* wherein *R^{b}* is methyl). In some embodiments of a compound of formula (II), R⁵ is 4-(imidazolyl)methyl. In some embodiments of a compound of formula (II), R⁵ is optionally substituted 3-guanidino-C₂₋₄alkyl. In some embodiments of a compound of formula (II), R⁵ is 3-guanidinopropyl.

In some embodiments of a compound of formula (II), R⁶ is optionally substituted 3-guanido-C₂₋₄alkyl. In some embodiments of a compound of formula (II), R⁶ is 3-guanidopropyl. In some embodiments of a compound of formula (II), R⁶ is amino-C₁₋₆alkyl. In some embodiments of a compound of formula (II), R⁶ is 4-aminobutyl.

In some embodiments of a compound of formula (II), R⁷ is hydroxy-C₁₋₆alkyl. In some embodiments of a compound of formula (II), R⁷ is (1R)-1-hydroxyethyl. In some embodiments of a compound of formula (II), R⁷ is 3-guanido-C₂₋₄alkyl. In some embodiments of a compound of formula (II), R⁶ is 3-guanidopropyl.

In some embodiments of a compound of formula (II), R⁸ is optionally substituted benzyl. In some embodiments of a compound of formula (II), R⁸ is hydroxybenzyl. In some embodiments of a compound of formula (II), R⁸ is halo substituted benzyl. In some embodiments of a compound of formula (II), R⁸ is benzyl substituted by 1 or 2 halogens. In some embodiments of a compound of formula (II), R⁸ is benzyl substituted by 1 or 2 chlorines. In some embodiments of a compound of formula (II), R⁸ is 4-chlorobenzyl.

In some embodiments of a compound of formula (II), R⁹ is optionally substituted benzyl. In some embodiments of a compound of formula (II), R⁹ is hydroxybenzyl. In some embodiments of a compound of formula (II), R⁹ is 4-hydroxybenzyl. In some embodiments of a compound of formula (II), R⁹ is halo substituted benzyl. In some embodiments of a compound of formula (II), R⁹ is benzyl substituted by 1 or 2 halogens. In some embodiments of a compound of formula (II)in some embodiments R⁹ is benzyl substituted by 1 or 2 chlorines. In some embodiments of a compound of formula (II), R⁹ is 4-chlorobenzyl. In some embodiments of a compound of formula (II), R⁹ is unsubstituted benzyl.

In some embodiments of a compound of formula (II), R¹⁰ is optionally substituted 3-guanido-C₁₋₆alkyl. In some embodiments of a compound of formula (II), R¹⁰ is 3-guanidopropyl. In some embodiments of a compound of formula (II), R¹⁰ is amino-C₁₋₆alkyl. In some embodiments of a compound of formula (II), R¹⁰ is 4-aminobutyl. In some embodiments of a compound of formula (II), R¹⁰ is 4(imidazolyl)methyl optionally substituted with C₁₋₆alkyl. In some embodiments of a compound of formula (II), R¹⁰ is 4-(imidazolyl)methyl with a N-substituted methyl. In some embodiments of a compound of formula (II), R¹⁰ is 3-(methylimidazol-4-yl)methyl (*iib* wherein R^{b} is methyl). In some embodiments of a compound of formula (II), R¹⁰ is 4-(imidazolyl)methyl.

In some embodiments of a compound of formula (II), R¹¹ is optionally substituted benzyl. In some embodiments of a compound of formula (II), R¹¹ is hydroxybenzyl. In some embodiments of a compound of formula (II), R¹¹ is 4-hydroxybenzyl. In some embodiments of a compound of formula (II), R¹¹ is halo substituted benzyl. In some embodiments of a compound of formula (II), R¹¹ is benzyl substituted by 1 or 2 halogens. In some embodiments of a compound of formula (II), in some embodiments R¹¹ is benzyl substituted by 1 or 2 chlorines. In some embodiments of a compound of formula (II),R¹¹ is 4-chlorobenzyl. In some embodiments of a compound of formula (II), R¹¹ is unsubstituted benzyl.

In some embodiments of a compound of formula (II), R¹² is optionally substituted benzyl. In some embodiments of a compound of formula (II), R¹² is hydroxybenzyl. In some embodiments of a compound of formula (II), R¹² is 4-hydroxybenzyl. In some embodiments of a compound of formula (II), R¹² is halo substituted benzyl. In some embodiments of a compound of formula (II), R¹² is benzyl substituted by 1 or 2 halogens. In some embodiments of a compound of formula (II), R¹² is benzyl substituted by 1 or 2 chlorines. In some embodiments of a compound of formula (II), R¹² is 4-chlorobenzyl. In some embodiments of a compound of formula (II), R¹² is N-substituted-3-indolylmethyl wherein R^{f} is hydrogen or C₁₋₆alkyl. In some embodiments R¹² is N-substituted-3-indolylmethyl wherein R^{f} is hydrogen is hydrogen. In some embodiments R^{f} methyl. In some embodiments of a compound of formula (II), R¹² is indolyl-3-methyl (*vi* wherein R^{f} is hydrogen). In some embodiments of a compound of formula (II), R¹² is optionally substituted benzyl. In some embodiments of a compound of formula (II), R¹² is hydroxybenzyl. In some embodiments of a compound of formula (II), R¹² is 4-hydroxybenzyl. In some embodiments of a compound of formula (II), R¹² is halo substituted benzyl. In some embodiments of a compound of formula (II), R¹² is benzyl substituted by 1 or 2 halogens. In some embodiments of a compound of formula (II), R¹² is benzyl substituted by 1 or 2 chlorines. In some embodiments of a compound of formula (II), R¹² is 4-chlorobenzyl. In some embodiments of a compound of formula (II), R¹² is unsubstituted benzyl.

In some embodiments of a compound of formula (II), R¹³ is optionally substituted benzyl. In some embodiments of a compound of formula (II), R¹³ is hydroxybenzyl. In some embodiments of a compound of formula (II), R¹³ is 4-hydroxybenzyl. In some embodiments of a compound of formula (II), R¹³ is halo substituted benzyl. In some embodiments of a compound of formula (II), R¹³ is benzyl substituted by 1 or 2 halogens. In some embodiments of a compound of formula (II), R¹³ is benzyl substituted by 1 or 2 chlorines. In some embodiments of a compound of formula (II), R¹³ is 4-chlorobenzyl. In some embodiments of a compound of formula (II), R¹³ is unsubstituted benzyl.

In some embodiments of a compound of formula (II), R¹⁴ is N-(2-amino-2-oxo-ethyl)carboxamide.

In some embodiments of a compound of formula (II) R^{b}, R^{c}, R^{d}, R^{e}, R^{f}, R^{1a}, R^{3a}, R^{8a}, R^{15a}, R^{15b}, R^{15c}, R^{15d}, R^{15e}, R^{15f}, R^{15g}, R¹⁵ⁱ, R^{15j}, R^{15k} R^{16a}, R^{16b}, R^{16c} and R^{16d} are hydrogen or C₁₋₆ alkyl; in some embodiment R^{b}, R^{c}, R^{d}, R^{e}, R^{f}, R^{1a}, R^{3a}, R^{8a}, R^{15a}, R^{15b}, R^{15c}, R^{15d}, R^{15e}, R^{15f}, R^{15g}, R¹⁵ⁱ, R^{15j}, R^{15k}, R^{16a}, R^{16b}, R^{16c} and R^{16d} are hydrogen or methyl. In some embodiments of a compound of formula (II), R³ is hydrogen, C₁₋₆alkyl, hydroxyl or halo. In some embodiments of a compound of formula (II), R^{a} is chloro. In some embodiments of a compound of formula (II), R^{f} is hydrogen or C₁₋₆alkyl. In some embodiments of a compound of formula (II), R^{f} is hydrogen. In some embodiments of a compound of formula (II), R^{f} is methyl. In some embodiments of a compound of formula (II), n and p are independently from 0 to 2. i In some embodiments of a compound of formula (II), n and p are 1 or 0. In some embodiments of a compound of formula (II), n is 1. In some embodiments of a compound of formula (II), n is 0. In some embodiments of a compound of formula (II) q is independently from 0 or 1. In some embodiments of a compound of formula (II), q is 1. In some embodiments of a compound of formula (II), r is independently from 1 or 2. In some embodiments of a compound of formula (II), r is 1.

In another embodiment of the present invention there is provided a compound of formula (I) wherein R¹ is C₁₋₆alkyl, hydroxyl-C₁₋₆alkyl or benzyl; R^{1a} is hydrogen or C₁₋₆alkyl; R² is C₁₋₆alkyl; R³ is 1-naphthylmethyl, 2-naphthylmethyl, (4-phenylphenyl)methyl or indolyl-3-methyl; R^{3a} is hydrogen; R⁴ is *(iic);* R⁵ is hydrogen or 3-guanidopropyl; R⁶ is hydroxymethyl, (1R)-1-hydroxyethyl or 4-aminobutyl; R⁷ is C₁₋₆ alkyl or optionally substituted guanidino-C₂₋₄alkyl; R⁸ is (*i*); R^{8a} is hydrogen; R⁹ is (*iic*); R¹⁰ is C₁₋₆alkyl, amino-C₁₋₆alkyl, optionally substituted guanidino-C₂₋₄₃alkyl or (*iv*); R¹¹ is (*iib*) or (*iic*); R¹² is (*v*); R^{1a}, R^{3a}, R^{8a}, R^{15a}, R^{15b}, R^{15c}, R^{15d}, R^{15e}, R^{15f}, R^{15g}, R¹⁵ⁱ, R^{15j}, R^{15k}, R^{16a}, R^{16b}, R^{16c} and R^{16d} are hydrogen; R^{15h} is methyl.

In a subembodiment R³ is 1H-indol-3-ylmethyl; R⁴ and R⁹ is (1H-imidazol-4-yl) methyl; R⁵ is hydrogen; R⁶ is hydroxymethyl, R⁷ and R¹⁰ are 3-guanidinyl-propyl; R¹¹ is 3-methylimidazol-4-yl)methyl; R¹² is N-(2-amino-2-oxo-ethyl)-carboxamide and the remaining substituents not further limited are as described herein above.

In a subembodiment R³ is 1-naphthylmethyl; R⁴ and R⁹ is (1H-imidazol-4-yl) methyl; R⁵ is hydrogen; R⁶ is hydroxymethyl, R⁷ and R¹⁰ are 3-guanidinyl-propyl; R¹¹ is 3-methylimidazol-4-yl)methyl; R¹² is N-(2-amino-2-oxo-ethyl)-carboxamide and the remaining substituents not further limited are as described herein above.

In some embodiments there is a compound of formula (II) wherein the compound is selected from Table 2

| TABLE 2 | | | |
|---|---|---|---|
| # | Structure | Compound Name | m/e |
| 50 | 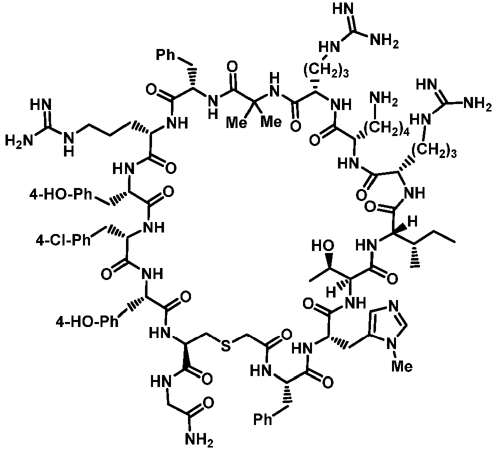 | rac-(3R,6S,9S,12S,15S,18S,21S,24S,27 S,30S,33S,36S,42S)-27-(4-aminobutyl)-N-(2-amino-2-oxoethyl)-6,18,42-tribenzyl-15,21,24-tris(3-guanidinopropyl)-12-[(4-hydroxyphenyl)methyl]-30-(1H-imidazol-5-ylmethyl)-9-(1H-indol-3-ylmethyl)-5,8,11,14,17,20,23,26,29,32,35,38 ,41,44-tetradecaoxo-36-[rac-(1R)-1-hydroxyethyl]-33-[rac-(1S)-1-methylpropyl]-1-thia-4,7,10,13,16,19,22,25,28,31,34,37 ,40,43-tetradecazacyclopentatetraconta ne-3-carboxamide | 2013.01 |
| 51 | 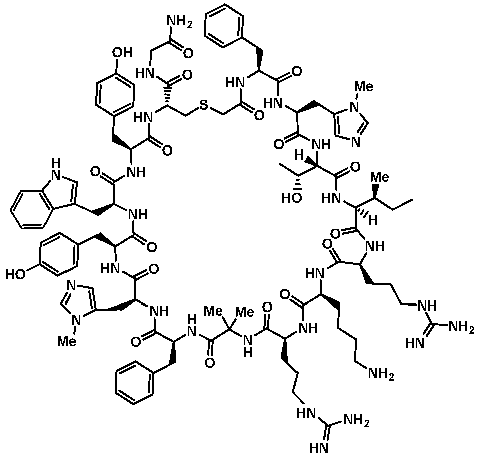 | (3R,6S,9S,12S,15S,18S,24S,27S,30 S,33S,36S,39S,42S)-27-(4-aminobutyl)-N-(2-amino-2-oxoethyl)-18,42-dibenzyl-24,30-bis(3-guanidinopropyl)-36-[(1R)-1-hydroxyethyl]-6,12-bis[(4-hydroxyphenyl)methyl]-9-(1H-indol-3-ylmethyl)-21,21-dimethyl-15,39-bis[(3-methylimidazol-4-yl)methyl]-33-[(1S)-1-methylpropyl]-5,8,11,14,17,20,23,26,29,32,35,38 ,41,44-tetradecaoxo-1-thia-4,7,10,13,16,19,22,25,28,31,34,37 ,40,43-tetradecazacyclopentatetraconta ne-3-carboxamide | 2066.02 |
| 52 | 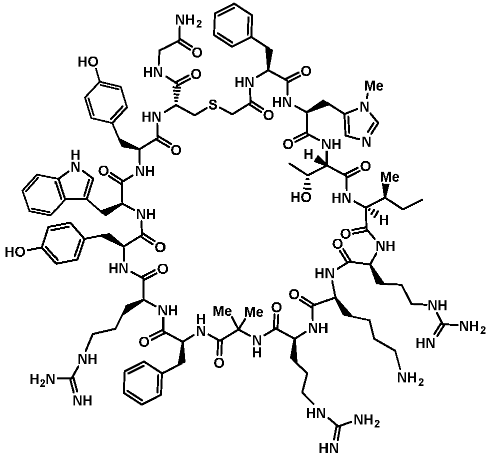 | (3R,6S,9S,12S,15S,18S,24S,27S,30 S,33S,36S,39S,42S)-27-(4-aminobutyl)-N-(2-amino-2-oxoethyl)-18,42-dibenzyl-15,24,30-tris(3-guanidinopropyl)-36-[(1R)-1-hydroxyethyl]-6,12-bis[(4-hydroxyphenyl)methyl]-9-(1H-indol-3-ylmethyl)-21,21-dimethyl-39-[(3-methylimidazol-4-yl)methyl]-33-[(1S)-1-methylpropyl]-5,8,11,14,17,20,23,26,29,32,35,38 ,41,44-tetradecaoxo-1-thia-4,7,10,13,16,19,22,25,28,31,34,37 ,40,43-tetradecazacyclopentatetraconta ne-3-carboxamide | 2071.05 |
| 53 | 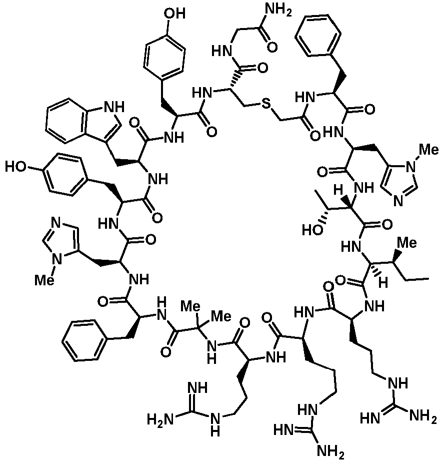 | (3R,6S,9S,12S,15S,18S,24S,27S,30 S,33S,36S,39S,42S)-N-(2-amino-2-oxo-ethyl)-18,42-dibenzyl-24,27,30-tris(3-guanidinopropyl)-36-[(1R)-1-hydroxyethyl]-6,12-bis[(4-hydroxyphenyl)methyl]-9-(1H-indol-3-ylmethyl)-21,21-dimethyl-15,39-bis[(3-methylimidazol-4-yl)methyl]-33-[(1S)-1-methylpropyl]-5,8,11,14,17,20,23,26,29,32,35,38 ,41,44-tetradecaoxo-1-thia-4,7,10,13,16,19,22,25,28,31,34,37 ,40,43-tetradecazacyclopentatetraconta ne-3-carboxamide | 2094.03 |
| 54 | 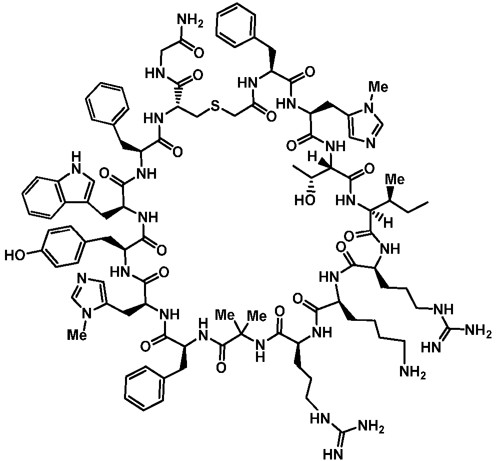 | (3R,6S,9S,12S,15S,18S,24S,27S,30 S,33S,36S,39S,42S)-27-(4-aminobutyl)-N-(2-amino-2-oxoethyl)-6,18,42-tribenzyl-24,30-bis(3-guanidinopropyl)-36-[(1R)-1-hydroxyethyl]-12-[(4-hydroxyphenyl)methyl]-9-(1H-indol-3-ylmethyl)-21,21-dimethyl-15,39-bis[(3-methylimidazol-4-yl)methyl]-33-[(1S)-1-methylpropyl]-5,8,11,14,17,20,23,26,29,32,35,38 ,41,44-tetradecaoxo-1-thia-4,7,10,13,16,19,22,25,28,31,34,37 ,40,43-tetradecazacyclopentatetraconta ne-3-carboxamide;2,2,2-trifluoroacetic acid | 2050.03 |
| 55 | 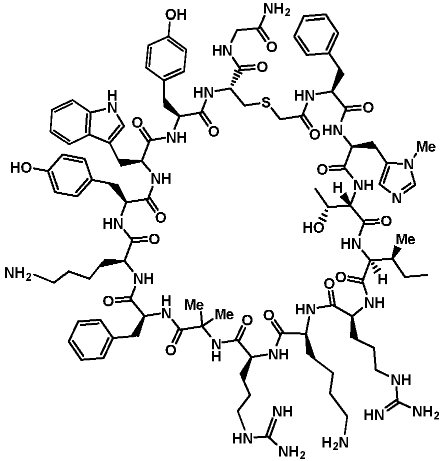 | (3R,6S,9S,12S,15S,18S,24S,27S,30 S,33S,36S,39S,42S)-15,27-bis(4-aminobutyl)-N-(2-amino-2-oxoethyl)-18,42-dibenzyl-24,30-bis(3-guanidinopropyl)-36-[(1R)-1-hydroxyethyl]-6,12-bis[(4-hydroxyphenyl)methyl]-9-(1H-indol-3-ylmethyl)-21,21-dimethyl-39-[(3-methylimidazol-4-yl)methyl]-33-[(1S)-1-methylpropyl]-5,8,11,14,17,20,23,26,29,32,35,38 ,41,44-tetradecaoxo-1-thia-4,7,10,13,16,19,22,25,28,31,34,37 ,40,43-tetradecazacyclopentatetraconta ne-3-carboxamide | 2046.04 |
| 56 | 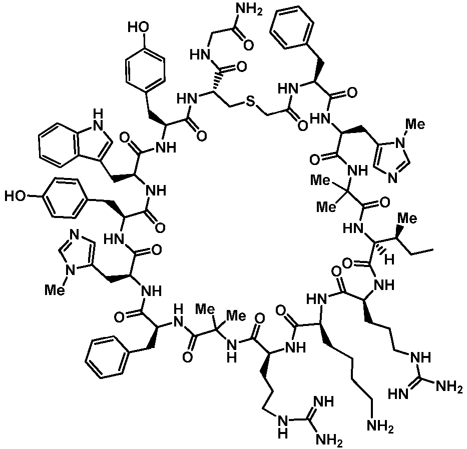 | (3R,6S,9S,12S,15S,18S,24S,27S,30 S,33S,39S,42S)-27-(4-aminobutyl)-N-(2-amino-2-oxo-ethyl)-18,42-dibenzyl-24,30-bis(3-guanidinopropyl)-6,12-bis[(4-hydroxyphenyl)methyl]-9-(1H-indol-3-ylmethyl)-21,21,36,36-tetramethyl-15,39-bis[(3-methylimidazol-4-yl)methyl]-33-[(1S)-1-methylpropyl]-5,8,11,14,17,20,23,26,29,32,35,38 ,41,44-tetradecaoxo-1-thia-4,7,10,13,16,19,22,25,28,31,34,37 ,40,43-tetradecazacyclopentatetraconta ne-3-carboxamide | 2050.03 |
| 57 | 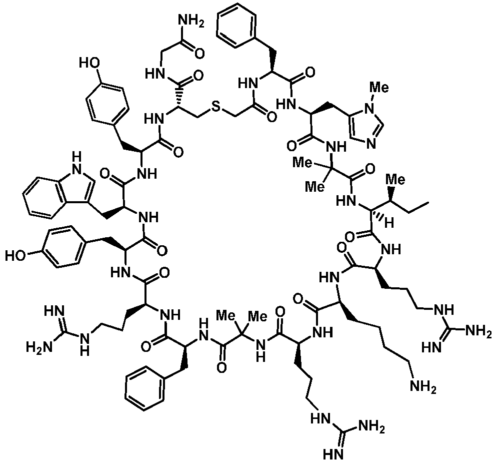 | (3R,6S,9S,12S,15S,18S,24S,27S,30 S,33S,39S,42S)-27-(4-aminobutyl)-N-(2-amino-2-oxo-ethyl)-18,42-dibenzyl-15,24,30-tris(3-guanidinopropyl)-6,12-bis[(4-hydroxyphenyl)methyl]-9-(1H-indol-3-ylmethyl)-21,21,36,36-tetramethyl-39-[(3-methylimidazol-4-yl)methyl]-33-[(1S)-1-methylpropyl]-5,8,11,14,17,20,23,26,29,32,35,38 ,41,44-tetradecaoxo-1-thia-4,7,10,13,16,19,22,25,28,31,34,37 ,40,43-tetradecazacyclopentatetraconta ne-3-carboxamide | 2055.05 |
| 58 | 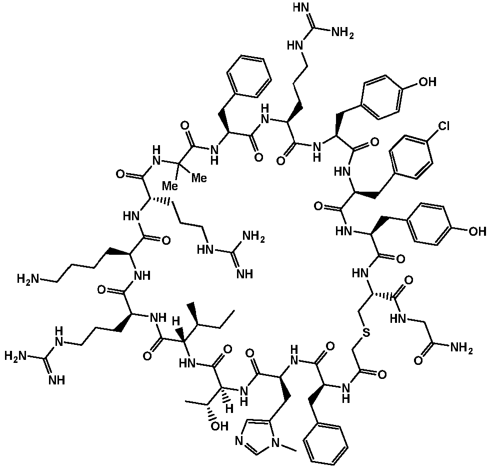 | (3R,6S,9S,12S,15S,18S,24S,27S,30 S,33S,36S,39S,42S)-27-(4-aminobutyl)-N-(2-amino-2-oxoethyl)-18,42-dibenzyl-9-[(4-chlorophenyl)methyl]-15,24,30-tris(3-guanidinopropyl)-36-[(1R)-1-hydroxyethyl]-6,12-bis[(4-hydroxyphenyl)methyl]-21,21-dimethyl-39-[(3-methylimidazol-4-yl)methyl]-33-[(1S)-1-methylpropyl]-5,8,11,14,17,20,23,26,29,32,35,38 ,41,44-tetradecaoxo-1-thia-4,7,10,13,16,19,22,25,28,31,34,37 ,40,43-tetradecazacyclopentatetraconta ne-3-carboxamide | 2066.00 |
| 59 | 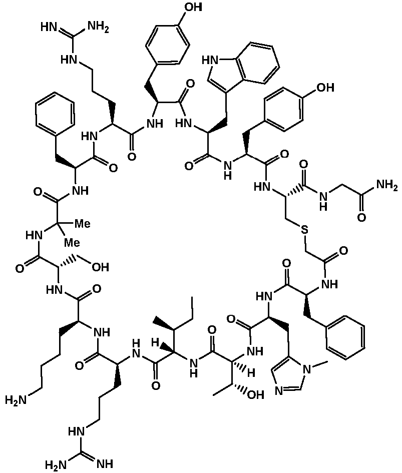 | rac-(3R,6S,9S,12S,15S,18S,24S,27S,30 S,33S,36S,39S,42S)-27-(4-aminobutyl)-N-(2-amino-2-oxoethyl)-18,42-dibenzyl-15,30-bis(3-guanidinopropyl)-24-(hydroxymethyl)-6,12-bis[(4-hydroxyphenyl)methyl]-9-(1H-indol-3-ylmethyl)-21,21-dimethyl-39-[(3-methylimidazol-4-yl)methyl]-5,8,11,14,17,20,23,26,29,32,35,38 ,41,44-tetradecaoxo-36-[rac-(1R)-1-hydroxyethyl]-33-[rac-(1S)-1-methylpropyl]-1-thia-4,7,10,13,16,19,22,25,28,31,34,37 ,40,43-tetradecazacyclopentatetraconta ne-3-carboxamide | 2001.98 |
| 60 | 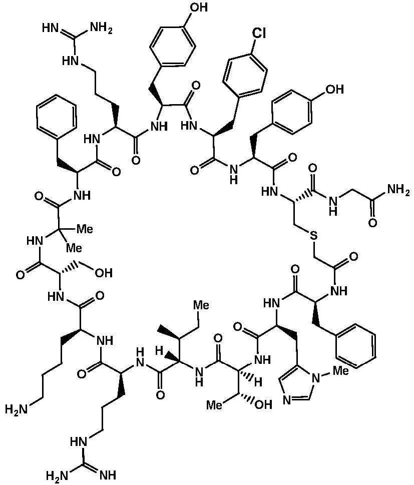 | (3R,6S,9S,12S,15S,18S,24S,27S,30 S,33S,36S,39S,42S)-27-(4-aminobutyl)-N-(2-amino-2-oxoethyl)-18,42-dibenzyl-9-[(4-chlorophenyl)methyl]-15,30-bis(3-guanidinopropyl)-24-(hydroxymethyl)-6,12-bis[(4-hydroxyphenyl)methyl]-21,21-dimethyl-39-[(3-methylimidazol-4-yl)methyl]-5,8,11,14,17,20,23,26,29,32,35,38 ,41,44-tetradecaoxo-36-[rac-(1R)-1-hydroxyethyl]-33-[rac-(1S)-1-methylpropyl]-1-thia-4,7,10,13,16,19,22,25,28,31,34,37 ,40,43-tetradecazacyclopentatetraconta ne-3-carboxamide | 1996.93 |
| 61 | 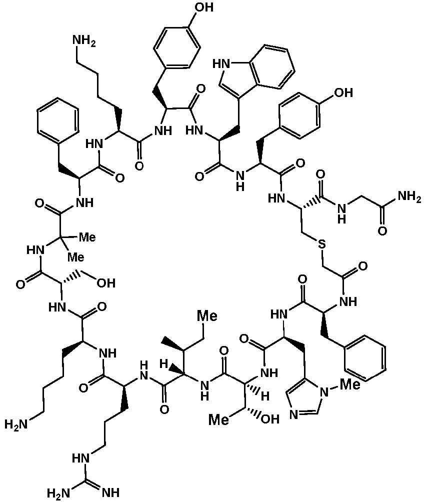 | rac-(3R,6S,9S,12S,15S,18S,24S,27S,30 S,33S,36S,39S,42S)-15,27-bis(4-aminobutyl)-N-(2-amino-2-oxoethyl)-18,42-dibenzyl-30-(3-guanidinopropyl)-24-(hydroxymethyl)-6,12-bis[(4-hydroxyphenyl)methyl]-9-(1H-indol-3-ylmethyl)-21,21-dimethyl-39-[(3-methylimidazol-4-yl)methyl]-5,8,11,14,17,20,23,26,29,32,35,38 ,41,44-tetradecaoxo-36-[rac-(1R)-1-hydroxyethyl]-33-[rac-(1S)-1-methylpropyl]-1-thia-4,7,10,13,16,19,22,25,28,31,34,37 ,40,43-tetradecazacyclopentatetraconta ne-3-carboxamide | 1973.98 |

In some embodiments of the invention, a cyclic tetradecapeptide of Formula (IIa), or a pharmaceutically acceptable salt, solvate, or stereoisomer thereof:

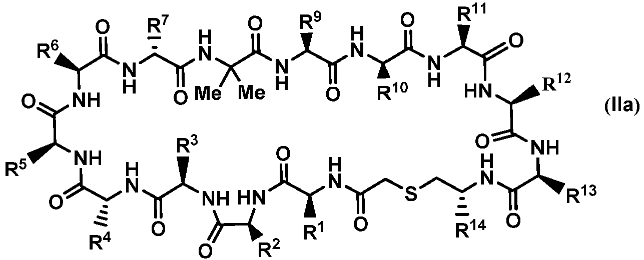

In one embodiment of a compound of Formula (IIa), R¹⁴ is N-(2-amino-2-oxo-ethyl)carboxamide *(v,* r =1, *R^{e}* = H); R⁴ is (1S)-1-methylpropyl, R² is 1-methyl-1H-imidazol-5-yl)methyl and R⁹ is benzyl optionally substituted with halo or hydroxyl. In one embodiment of a compound of Formula (II) R⁹ is unsubstituted benzyl.

In one embodiment of the present invention there is a compound of formula (IIb) wherein: 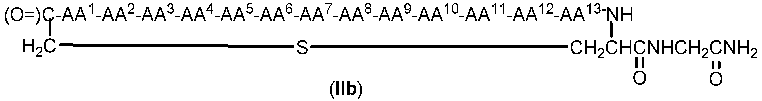
- AA¹: is an aromatic amino acid wherein the aromatic amino acid is phenylalanine optionally substituted by 1 to 3 C₁₋₆ alkyl, halogens, C₁₋₆-haloalkyl, hydroxyl, C₁₋₆-alkoxy, C₁₋₆-haloalkoxy, C₁₋₆-thioalkyl, C₁₋₆-alkylsulfonyl or cyano;
- AA²: is a heteroaryl amino acid wherein said heteroaryl amino acid is a compound of formula H₂NCHR^{a}CO₂H wherein R^{a} is 1H-imidazole-5-ylmethyl, 3-methylimidazol-4-yl-methyl, preferably R^{a} is 3-methylimidazol-4-yl-methyl or AA² is glycine;
- AA³: is an aliphatic amino acid of formula H₂NCHR^{a}CO₂H wherein R^{a} is C₁₋₆ alkyl or C₁₋₅-hydroxyalkyl or an aliphatic amino acid of formula H₂NCR^{a}R^{b}CO₂H wherein R^{a} and R^{b} are independently C₁₋₆ alkyl; preferably α-aminoisobutyric acid (Aib);
- AA⁴: is an aliphatic amino acid of formula H₂NCHR^{a}CO₂H wherein R^{a} is C₁₋₆ alkyl, preferably, AA⁴ is isoleucine;
- AA⁵: is a basic amino acid of formula H₂NCHR^{a} CO₂H wherein R^{a} is H-imidazole-5-ylmethyl or guanidino-C₁₋₄-alkyl, preferably arginine;
- AA⁶: is a basic amino acid of formula H₂NCHR^{a} CO₂H wherein R^{a} is C₁₋₆-aminoalkyl or guanidino-C₁₋₄-alkyl, preferably, aminobutyl;
- AA⁷: is a basic amino acid of formula H₂NCHR^{a} CO₂H wherein R^{a} is C₁₋₆-hydroxyalkyl or guanidino-C₁₋₄-alkyl, preferably, aminobutyl;
- AA⁸: is an aliphatic amino acid of formula H₂NCR^{a}R^{b}CO₂H wherein R^{a} is C₁₋₆ alkyl and R^{b} hydrogen or C₁₋₆ alkyl; preferably α-aminoisobutyric acid (Aib);
- AA⁹: is an aromatic amino acid wherein the aromatic amino acid is phenylalanine wherein the phenyl ring of either is optionally substituted by 1 to 3 C₁₋₆ alkyl, halogens, C₁₋₆-haloalkyl, hydroxyl, C₁₋₆-alkoxy, C₁₋₆-haloalkoxy, C₁₋₆-thioalkyl; preferably phenylalanine;
- AA¹⁰: is a basic amino acid of formula H₂NCHR^{a} CO₂H wherein R^{a} is C₁₋₆-aminoalkyl, guanidino-C₁₋₄-alkyl, 3-methylimidazol-4-yl-methyl;
- AA¹¹: is an aromatic amino acid wherein the aromatic amino acid is phenylalanine wherein the phenyl ring of either is optionally substituted by 1 to 3 C₁₋₆ alkyl, halogens, C₁₋₆-haloalkyl, hydroxyl, C₁₋₆-alkoxy, C₁₋₆-haloalkoxy, C₁₋₆-thioalkyl; preferably phenylalanine;
- AA¹²: is a heteroaryl amino acid of formula H₂NCHR^{a} CO₂H wherein R^{a} is 3-indolemethyl or aromatic amino acid wherein R^{a} is phenyl ring optionally substituted by 1 to 3 C₁₋₆ alkyl, halogens, C₁₋₆-haloalkyl, hydroxyl, C₁₋₆-alkoxy, C₁₋₆-haloalkoxy, C₁₋₆-thioalkyl; preferably tryptophan;
- AA¹³: is phenylalanine wherein the phenyl ring is optionally substituted by one to three C₁₋₆ alkyl, halogens, C₁₋₆-haloalkyl, C₁₋₆-alkoxy, C₁₋₆-haloalkoxy, C₁₋₆-thioalkyl, C₁₋₆-alkylsulfonyl or cyano.

In another embodiment there is afforded a compound of formula (**IIb**) wherein: AA¹ is phenylalanine; AA² is N^{α}-methylhistidine; AA³ is serine or alanine; AA⁴ is isoleucine; AA⁵ is arginine or histidine; AA⁶ is lysine or arginine; AA⁷ is arginine or serine; AA⁸ is α-aminoisobutyric acid; AA⁹ is phenylalanine; AA¹⁰ is arginine, N^{α}-methylhistidine or H₂NCHR^{a}CO₂H wherein Ra is amino methyl, aminoethyl or aminobutyl; AA¹¹ is 4-hydroxy-phenylalanine; AA¹² is tryptophan; AA¹³ is phenylalanine optionally substituted with hydroxyl or halo.

### DOSAGE & ADMINISTRATION

The references to methods of treatment in the subsequent paragraphs of this description are to be interpreted as references to the compounds, pharmaceutical compositions and medicaments of the present invention for use in a method for treatment of the human (or animal) body by therapy.

The present invention provides pharmaceutical compositions or medicaments containing the compounds of the invention and at least one therapeutically inert carrier, diluent or excipient, as well as methods of using the compounds of the invention to prepare such compositions and medicaments. In one example, compounds of Formula **I** with the desired degree of purity may be formulated by mixing with physiologically acceptable carriers, *i.e.,* carriers that are non-toxic to recipients at the dosages and concentrations employed into a dosage form at ambient temperature and at the appropriate pH. The pH of the formulation depends mainly on the particular use and the concentration of compound, but typically ranges anywhere from about 3 to about 8. In one example, a compound of Formula **I** is formulated in an acetate buffer, at pH 5. In another embodiment, the compounds of Formula **I** are sterile. The compound may be stored, for example, as a solid or amorphous composition, as a lyophilized formulation or as an aqueous solution.

Compositions are formulated, dosed, and administered in a fashion consistent with good medical practice. Factors for consideration in this context include the particular disorder being treated, the severity of the disorder, the particular patient being treated, the clinical condition of the individual patient, the cause of the disorder, the site of delivery of the agent, the method of administration, the scheduling of administration, and other factors known to medical practitioners. The "effective amount" of the compound to be administered will be governed by such considerations, and is the minimum amount necessary to provides anti-bacterial activity. Typically such amount may be below the amount that is toxic to normal cells, or the patient as a whole.

The pharmaceutical composition (or formulation) for application may be packaged in a variety of ways depending upon the method used for administering the drug. Generally, an article for distribution includes a container having deposited therein the pharmaceutical formulation in an appropriate form. Suitable containers are well-known to those skilled in the art and include materials such as bottles (plastic and glass), sachets, ampoules, plastic bags, metal cylinders, and the like. The container may also include a tamper-proof assemblage to prevent indiscreet access to the contents of the package. In addition, the container has deposited thereon a label that describes the contents of the container. The label may also include appropriate warnings.

Sustained-release preparations may be prepared. Suitable examples of sustained-release preparations include semipermeable matrices of solid hydrophobic polymers containing a compound of Formula **I,** which matrices are in the form of shaped articles, e.g. films, or microcapsules. Examples of sustained-release matrices include polyesters, hydrogels (for example, poly(2-hydroxyethyl-methacrylate), or poly(vinylalcohol)), polylactides, copolymers of L-glutamic acid and gamma-ethyl-L-glutamate, nondegradable ethylene-vinyl acetate, degradable lactic acid-glycolic acid copolymers such as the LUPRON DEPOT^{™} (injectable microspheres composed of lactic acid-glycolic acid copolymer and leuprolide acetate), and poly-D-(-)-3-hydroxybutyric acid.

A dose to treat human patients may range from about 0.1 mg to about 1000 mg of a compound of formula **I** or **II.** A typical dose may be about 1 mg to about 300 mg of the compound. A dose may be administered once a day (QD), twice per day (BID), or more frequently, depending on the pharmacokinetic and pharmacodynamic properties, including absorption, distribution, metabolism, and excretion of the particular compound. In addition, toxicity factors may influence the dosage and administration regimen.

The compounds of the invention may be administered by any suitable means, including oral, topical (including buccal and sublingual), rectal, vaginal, transdermal, parenteral, subcutaneous, intraperitoneal, intrapulmonary, intradermal, intrathecal, epidural and intranasal, and, if desired for local treatment, intralesional administration. Parenteral infusions include intramuscular, intravenous, intraarterial, intraperitoneal, or subcutaneous administration.

The compounds of the present invention may be administered in any convenient administrative form, *e.g.,* tablets, powders, capsules, solutions, dispersions, suspensions, syrups, sprays, suppositories, gels, emulsions, patches, etc. Such compositions may contain components conventional in pharmaceutical preparations, *e.g*., diluents, carriers, pH modifiers, sweeteners, bulking agents, and further active agents.

A typical formulation is prepared by mixing a compound of the present invention and a carrier or excipient. Suitable carriers and excipients are well known to those skilled in the art and are described in detail in, *e.g.,* Ansel, H. C., et al., Ansel's Pharmaceutical Dosage Forms and Drug Delivery Systems. Philadelphia: Lippincott, Williams & Wilkins, 2004; Gennaro, Alfonso R., et al. Remington: The Science and Practice of Pharmacy. Philadelphia: Lippincott, Williams & Wilkins, 2000; and Rowe, R. C., Handbook of Pharmaceutical Excipients, Chicago, Pharmaceutical Press, 2005**.** The formulations may also include one or more buffers, stabilizing agents, surfactants, wetting agents, lubricating agents, emulsifiers, suspending agents, preservatives, antioxidants, opaquing agents, glidants, processing aids, colorants, sweeteners, perfuming agents, flavoring agents, diluents and other known additives to provide an elegant presentation of the drug (*i.e.,* a compound of the present invention or pharmaceutical composition thereof) or aid in the manufacturing of the pharmaceutical product (*i.e.,* medicament).

In another aspect are methods of treating a mammal in need of such treatment comprising administering to the mammal an antibacterial effective amount of any of the aforementioned compounds at a frequency and for a duration sufficient to provide a beneficial effect to the mammal. In one embodiment, the causative bacteria species of the bacteria infection is an infection involving a Gram-negative bacteria. In such embodiments, the gram-negative bacteria may be, for example, *Escheria coli, Klebsiella pneumoniae, Pseudomonas aeruginosa, Acinetobacter baumanii, Neisseria gonorrhoeae, Neisseria meningitidis, Chlamvdia trachomatis, Moraxella catarrhalis, Haemophilus influenzae, Proteus mirabilis, Enterobacter cloacae, Serratia marcescens, Helicobacter pylori, Salmonella enteritidis, Salmonella typhi , Legionella pneumophila, Haemophilus influenzae, Vibrio cholerae, Pseudomonas stutzeri, Ralstonia solanacearum,* or *Xylella fastidiosa.*

In another aspect the bacterial infection is an infection involving a Gram-negative bacteria wherein the bacteria is *E.coli.*

Injectable preparations, for example, sterile injectable aqueous or oleaginous suspensions are optionally formulated according to the known art using suitable dispersing or wetting agents and suspending agents. The sterile injectable preparation is optionally a sterile injectable solution, suspension or emulsion in a nontoxic parenterally acceptable diluent or solvent, for example, as a solution in 1,3-butanediol. Among the acceptable vehicles and solvents that are optionally employed are water, Ringer's solution, U.S.P. and isotonic sodium chloride solution. In addition, sterile, fixed oils are conventionally employed as a solvent or suspending medium. For this purpose any bland fixed oil can be employed including synthetic mono- or diglycerides. In addition, fatty acids such as oleic acid are used in the preparation of injectables.

The injectable formulations can be sterilized, for example, by filtration through a bacterial-retaining filter, or by incorporating sterilizing agents in the form of sterile solid compositions which can be dissolved or dispersed in sterile water or other sterile injectable medium prior to use.

According to the methods of treatment described herein, bacterial infections are treated or prevented in a patient such as a human or lower mammal by administering to the patient a therapeutically effective amount of a compound described herein, in such amounts and for such time as is necessary to achieve the desired result. By a "therapeutically effective amount" of a compound described herein is meant a sufficient amount of the compound to treat bacterial infections, at a reasonable benefit/risk ratio applicable to any medical treatment. It will be understood, however, that the total daily usage of the compounds and compositions described herein will be decided by the attending physician within the scope of sound medical judgment. The specific therapeutically effective dose level for any particular patient will depend upon a variety of factors including the disorder being treated and the severity of the disorder; the activity of the specific compound employed; the specific composition employed; the age, body weight, general health, sex and diet of the patient; the time of administration, route of administration, and rate of excretion of the specific compound employed; the duration of the treatment; drugs used in combination or coincidental with the specific compound employed; and like factors known in the medical arts.

The total daily dose of the compounds described herein compound described herein (i.e., a compound disclosed herein) administered to a human or other mammal in single or in divided doses can be in amounts, for example, from 0.01 to 50 mg/kg body weight or more usually from 0.1 to 25 mg/kg body weight. Single dose compositions may contain such amounts or submultiples thereof to make up the daily dose. In general, treatment regimens described herein comprise administration to a patient in need of such treatment from about 10 mg to about 2000 mg of the compound(s) described herein per day in single or multiple doses.

### EXPERIMENTAL PROCEDURES

All starting materials, building blocks, reagents, acids, bases, solid phase resins, and solvents used in in the following examples were commercially available products were typically used without any further purification. Standard techniques were used if purification was required. Unless otherwise specified, commercially available protected amino acids were used without additional purification.

The structure cyclic peptides was confirmed by ESI-MS (+) in the mass spectrum analysis. ESI-MS (+) indicates an electrospray ionization mass spectrum analysis method performed in the positive ion mode Cyclic peptides having a molecular weight greater than about 1000 were frequently detected as divalent ions or trivalent ions.

Peptide synthesis carried by standard solid-phase synthesis methodology using Sieber Amide resin or Fmoc-NH-SAL-PEG resin and Fmoc-protected amino acids. Coupling steps typically carried out with 5.2 equivalents of Fmoc-amino acid, 5 equivalents of HATU, and 10 equivalents of DIEA as coupling reagents with automated Liberty Blue automated peptide synthesizer (CEM Inc.) or with 3.2 equivalents of Fmoc-amino acid, 3 equivalents of HATU, and 6 equivalents of DIEA as coupling reagents with Syro I automated Parallel Peptide Synthesizer (Biotage) for peptide elongation. Deprotection was carried either with 20% piperidine in DMF or 5% piperazine in DMF with 0.1 M HOBt.

Cyclic peptides were purified by reverse-phase high-performance liquid chromatography (HPLC) using Waters AutoPurification System or Shimadzu prep-HPLC system. The peptide thus obtained was identified by the mass spectrum obtained in the ESI-positive scan mode and the mass spectrum containing polyvalent ions calculated from the molecular formula of the target object were in agreement within the error range of the mass spectrometer used.

Analysis LCMS conditions: Column: Kinetex EVO C18 100Å 2.6 µm, 2.1 mm ID x150 mm column eluted with 0.025% TFA in water (Mobil Phase A) and 0.025% TFA in MeCN (Mobile phases B) with 5-45% gradient of B over 20 minutes and a 0.25 mL/min flow rate. Eluents were monitored with UV 225 nm detector.

### Representative Examples

### Example 1: Compound 16

Fmoc amino acids used in the synthesis included Fmoc-Gly-OH; Fmoc-Cys(Trt)-OH; Fmoc-H3Me-OH; Fmoc-Arg(Pbf)-OH; Fmoc-His(Boc)-OH; Fmoc-MeTyr(tBu)-OH; Fmoc-Ser(Trt)-OH; Fmoc-Nal1-OH; Fmoc-Abu-OH; Fmoc-Phe-OH.

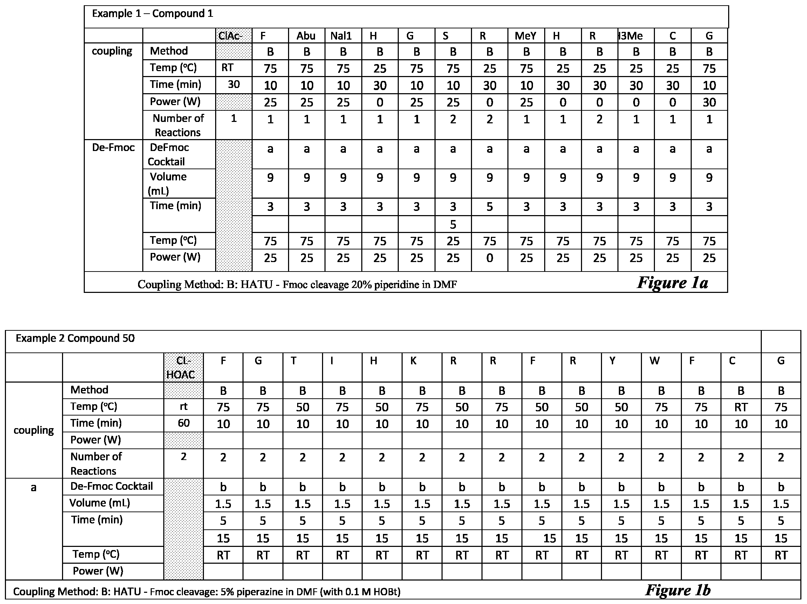

The automated synthesizer program is shown in Figure 1a.

The synthesis was carried out on Sieber Amide resin using 5.2 equivalents of Fmoc-amino acid, 5 equivalents of HATU, and 10 equivalents of DIEA as coupling reagents; 20% piperidine in DMF for Fmoc deprotection on a and Liberty Blue for peptide synthesizer. Double couplings were used at positions 6, 7, and 10 from N-terminal amino acid.

After deprotection of the N-terminal amino acid the resin was washed with DMF, then 5 equiv of 2-chloroacetic acid, 5 equivalents of HATU, and 10 equivalents of DIEA in DMF were added and the resulting mixture was agitated for 30 minutes. The resin was successively washed with DMF and DCM and then dried under high vacuum. Global deprotection was carried out by adding a mixture of TFA/water/TIS/DODT (92.5:2.5:2.5:2.5, v/v/v/v) and agitating the resulting mixture at RT for 1.5 h. The resulting solution was filtered through a disposable filter column and diluted in diisopropyl ether-hexane (1:1, v/v). The precipitated solid was centrifuged and the supernatant solution was decanted. The remaining solid was thrice washed with Et₂O and dried under high vacuum. The crude peptide was dissolved in DMSO to give a final concentration of 5 mM and 10 equivalents of TEA were added. The mixture was stirred at RT for 18 h.

The crude material was purified via the reverse-phase HPLC with a XSelect C18 5 µm 19 mm x150 mm column. The product was eluted with 0.1% TFA in water (Mobile Phase A) and 0.1% TFA in MeCN (Mobile Phase B) with a program gradient of 5-17% B over 3 min, 17-22% B over 8 min, then 22-60% B over 1 min. The flow rate was 17 mL/min with a column temperature of 40 °C. The retention time was 9.06 min. Fractions containing the desired product were combined and dried *via* lyophilization. MS (ESI+) *m*/*z* 853.93 [M+2H]²⁺

### Example 2 Compound 50

Fmoc amino acids used in the synthesis included Fmoc-Gly-OH; Fmoc-Cys(Trt)-OH; Fmoc-Phe-OH; Fmoc-Trp(Boc)-OH; Fmoc-Tyr(tBu)-OH; Fmoc-Arg(Pbf)-OH; Fmoc-Lys(Boc)-OH; Fmoc-His(Trt)-OH; Fmoc-Ile-OH; Fmoc- Thr(tBu)-OH.

The automated synthesizer program is shown in Figure 1b.

The synthesis was carried out on Sieber Amide resin; 3.2 equivalents of Fmoc-amino acid, 3 equivalents of HATU, and 6 equivalents of DIEA as coupling reagents; 5% piperazine in DMF with 0.1 M HOBt was utilized for Fmoc deprotection on a Syro I for automated peptide synthesizer. Double couplings were used at all positions.

After deprotection of the N-terminal Fmoc-Phe-OH, the resin was washed with DMF, then 3.2 equivalents of 2-chloroacetic acid, 3 equivalents of HCTU, and 6 equivalents of DIEA in DMF were added and the resulting mixture was allowed to agitated for 20 min. The resin was successively washed with DMF and DCM and then dried under high vacuum. A TFA-water- TIS-DODT mixture (92.5:2.5:2.5:2.5, v/v/v/v) was added and the resulting mixture was agitated at RT for 2h. The solution was filtered through a disposable filter column and diluted in Et₂O-hexane (1:1, v/v). The precipitated solid was centrifuged and the supernatant solution was decanted and the resulting solid was thrice washed with Et₂O and dried under high vacuum. The crude peptide was dissolved in DMSO-50% aqueous IPA (10:1, v/v) to give a final concentration of 5 mM and 5 equiv of TEA was added. The mixture was stirred at RT for 18 h.

The crude material was purified via the reverse-phase HPLC with a XBridge C18 5 µm 50mm x150 mm column. The product was eluted with 0.1% TFA in water (Mobile Phase A) and 0.1% TFA in MeCN (Mobile Phase B) with a program gradient 5-26% B over 3 minutes, 26-31% B over 8 minutes, then 31-60% B over 1 min. The flow rate was 120 mL/min. The retention time was 13.83 min with a column temperature of 40 °C. Fractions containing the desired product were combined and dried *via* lyophilization. MS (ESI+) *m*/*z* 1007.54 [M+2H]²⁺

### Example 3 - Compound 1

Fmoc amino acids used in the synthesis included Fmoc-Gly-OH; Fmoc-Cys(Trt)-OH; Fmoc-His(Trt)-OH; Fmoc-Arg(Pbf)-OH; Fmoc-MePhe-OH; Fmoc-Ser(tBu)-OH; Fmoc-Trp(Boc)-OH; Fmoc-Val-OH; Fmoc-Phe-OH.

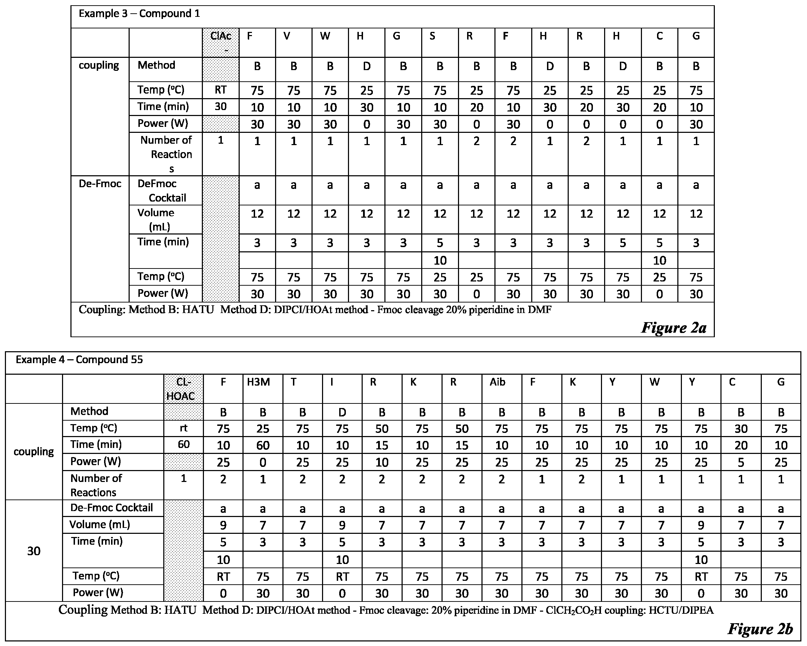

The automated synthesizer program is shown in Figure 2a.

The crude material was purified via the reverse-phase HPLC with a Kinetex EVO C18 21.2mm x150 mm column and eluted with 0.1% TFA in water (Mobile Phase A) and 0.1% TFA in MeCN (Mobile Phase B) with a program gradient of 5-15% B over 3 minutes, 15-20% B over 8 minutes, then 20-60% B over 1 minute. The flow rate was 21 mL/min. The retention time was 13.83 min with a column temperature of 40 °C. Fractions containing the desired product were combined and and lyophilized. MS (ESI+) *m*/*z* 840.31 [M+2H]²⁺⁺

### Example 4 - Compound 55

Fmoc amino acids used in the synthesis included Fmoc-Gly-OH; Fmoc-Cys(Trt)-OH; Fmoc-Tyr(tBu)-OH; Fmoc-Trp(Boc)-OH; Fmoc-Lys(Boc)-OH; Fmoc-Phe-OH; Fmoc-Aib-OH; Fmoc-Arg(Pbf)-OH; Fmoc-Ile-OH; Fmoc-Thr(tBu)-OH; Fmoc-H3Me-OH.

The automated synthesizer program is shown in Figure 2ba.

The crude material was purified via the reverse-phase HPLC with a XBridge C18 5µm 30mmx150mm column and eluted with 0.1% TFA in water (Mobile Phase A) and 0.1% TFA in MeCN (Mobile Phase B) with a program gradient of 5-21% B over 3 minutes, 21-26% B over 8 minutes, then 26-60% B over 1 minute. The flow rate was 45 mL/min. Fractions containing the desired product were combined and dried via lyophilization. MS (ESI+) *m*/*z* 1022.57 [M+2H]²⁺

### Example 5 - Compound 61

Fmoc amino acids used in the synthesis included Fmoc-Gly-OH; Fmoc-Cys(Trt)-OH; Fmoc-Tyr(tBu)-OH; Fmoc-Trp(Boc)-OH; Fmoc-Lys(Boc)-OH; Fmoc-Phe-OH; Fmoc-Aib-OH; Fmoc-Ser(tBu)-OH; Fmoc-Arg(Pbf)-OH; Fmoc-Ile-OH; Fmoc-Thr(tBu)-OH; Fmoc-H3Me-OH.

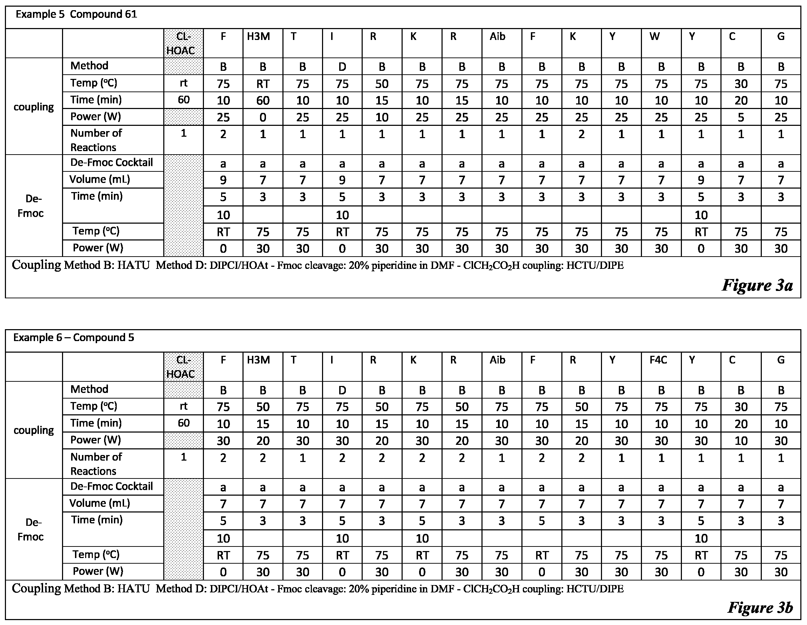

The automated synthesizer program is shown in Figure 3a.

The crude material was purified via the reverse-phase HPLC with a XBridge C18 5µm 19mm x150mm column and eluted with 0.1% TFA in water (Mobile Phase A) and 0.1% TFA in MeCN (Mobile Phase B) with a program gradient of 5-21% B over 3 minutes, 21-26% B over 8 minutes, then 26-60% B over 1 minute. The flow rate was 45 mL/min. Fractions containing the desired product were combined and lyophilized. MS (ESI+) *m*/*z* 988.07 [M+2H]²⁺

### Example 6 - Compound 58

Fmoc amino acids used in the synthesis included Fmoc-Gly-OH; Fmoc-Cys(Trt)-OH; Fmoc-Tyr(tBu)-OH; Fmoc-F4C-OH; Fmoc-Lys(Boc)-OH; Fmoc-Phe-OH; Fmoc-Aib-OH; Fmoc-Arg(Pbf)-OH; Fmoc-Ile-OH; Fmoc-Thr(tBu)-OH; Fmoc-H3Me-OH.

The automated synthesizer program is shown in Figure 3b.

The crude material was purified via the reverse-phase HPLC with a XBridge C18 5µm 30mm x150mm column and eluted with 0.1% TFA in water (Mobil Phase A) and 0.1% TFA in MeCN (Mobile Phase B) with a program gradient of 5-24% B over 3 minutes, 24-29% B over 8 minutes, then 29-60% B over 1 minute. The flow rate was 45 mL/min. Fractions containing the desired product were combined and lyophilized. MS (ESI+) *m*/*z* 1034.23 [M+2H]²⁺

### Biological Assays

### Minimum inhibitory concentration (MIC) determination

MICs were determined by performing two-fold serial dilutions of peptides in LB broth to a final volume of 0.1 ml in round-bottom 96-well assay plates (Corning Life Sciences No 3788). Peptides were initially resuspended in 100% DMSO to 10mM and subsequently diluted in LB medium to the appropriate concentration. Each well was inoculated with 5×10⁵ CFU/ml of the screening stain and incubated at 37° C without agitation for 18 hours. Plates were scored by eye, and the lowest compound concentration preventing visible growth was determined to be the MIC.

| TABLE 3 | | | | | |
|---|---|---|---|---|---|
| compd # | MIC E.coli 25922 WUXI:: MIC | MIC E.cloacae ATCC222 WUXI::MI C | MIC K.pneumoniae ATCC43816 Wuxi::MIC | MIC E.coli BW25113 WUXI::MI C | MIC E.coli 25922 IMP WUXI::MIC |
| 1 | 50 | 6.3 | 50 | 100 | 0.031 |
| 2 | | | | >32 | 0.008 |
| 3 | | | | >32 | 0.064 |
| 4 | | | | >32 | 0.128 |
| 5 | | | | >32 | 0.016 |
| 6 | | | | 2 | <0.000125 |
| 7 | | | | 2 | 0.0005 |
| 8 | | | | >32 | 4 |
| 9 | | | | 8 | 9.5367E-07 |
| 10 | | | | 8 | 2.3842E-07 |
| 11 | | | | 8 | 1.9074E-06 |
| 12 | | | | 1 | 3.8137E-06 |
| 13 | | | | 4 | 0.00 |
| 14 | 25 | 1.19 | 25 | 37.5 | 0.0119 |
| 15 | 50 | 3.1 | 75 | 100 | 0.016 |
| 16 | 27.32 | 0.2633 | 8.5333 | 14.767 | 1.576725 |
| 17 | 13 | 3.1 | 37.5 | 50 | 0.002 |
| 18 | 9.65 | 0.78 | 19 | 19 | 0.00585 |
| 19 | 3.1 | 0.39 | 6.3 | 6.3 | 0.0039 |
| 20 | 13 | 1.6 | 25 | 25 | 0.0078 |
| 21 | 50 | 3.1 | 25 | 50 | 0.063 |
| 22 | 6.3 | 0.39 | 13 | 25 | 0.002 |
| 23 | 25 | | | | 0.016 |
| 24 | 13 | | | | 0.016 |
| 25 | 25 | | | | 0.031 |
| 26 | 3.1 | 0.78 | 25 | 6.3 | 0.0039 |
| 27 | 3.1 | 0.39 | 13 | 13 | 0.002 |
| 28 | 3.1 | 0.2 | 3.1 | 6.3 | 0.002 |
| 29 | 1.6 | 0.1 | 3.1 | 6.3 | 0.001 |
| 30 | 25 | 1.6 | 100 | 50 | 0.016 |
| 31 | 25 | 6.3 | 50 | 50 | 0.016 |
| 32 | 25 | | | | 0.031 |
| 33 | 13 | 1.6 | 25 | 25 | 0.0005 |
| 34 | 41.667 | 6.3 | 25 | 100 | 0.063 |
| 35 | 100 | 100 | 100 | 100 | 3.1 |
| 36 | 100 | | | | 100 |
| 37 | 100 | 100 | 100 | 100 | 25 |
| 38 | 100 | 13 | 100 | 100 | 0.063 |
| 39 | 100 | 100 | 100 | 100 | 6.3 |
| 40 | | | | | |
| 41 | >100 | | | | |
| 42 | 100 | | | | |
| 43 | >100 | | | | |
| 44 | >100 | | | | |
| 45 | >100 | | | | |
| 46 | 100 | | | | |
| 47 | 12.5 | | | | |
| 48 | >100 | | | | |
| 49 | 50 | | | | |
| 50 | 2 | >64 | 32 | 2 | 1 |
| 51 | 8 | >32 | >32 | 8 | 0.5 |
| 52 | | | | 1 | 0.125 |
| 53 | | | | 4 | 0.25 |
| 54 | | | | 8 | 0.5 |
| 55 | | | | 2 | 0.25 |
| 56 | | | | >32 | 8 |
| 57 | | | | >32 | 2 |
| 58 | | | | 2 | 0.25 |
| 59 | 8 | | | 16 | 1 |
| 60 | 16 | | | 16 | 1 |
| 61 | 16 | | | 16 | 1 |

## Claims

1. A compound of formula I 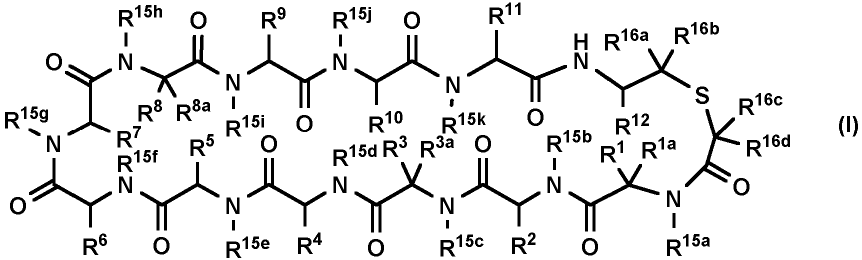
or a stereoisomer or a pharmaceutically acceptable salt thereof, wherein:
R¹ is C₁₋₆ alkyl, hydroxyl-C₁₋₆alkyl or benzyl optionally substituted with halogen or hydroxy;
R^{1a} is hydrogen;
R² is hydrogen, C₁₋₆ alkyl or optionally substituted (imidazol-4-yl)methyl;
R³ is bicyclic aryl, bicyclic heteroaryl, C₁₋₆alkyl, or hydroxyl-C₁₋₆alkyl wherein the bicyclic aryl and bicyclic heteroaryl is optionally substituted once or twice with C₁₋₆alkyl, hydroxyl or halo;
R^{3a} is hydrogen or C₁₋₆alkyl;
R⁴ is C₁₋₆alkyl or optionally substituted (imidazol-4-yl)methyl;
R⁵ is hydrogen; C₁₋₆alkyl, hydroxyl-C₁₋₆alkyl, optionally substituted (imidazol-4-yl)methyl or optionally substituted guanidino-C₂₋₄alkyl;
R⁶ is hydroxyl-C₁₋₆alkyl or amino-C₁₋₆alkyl;
R⁷ is C₁₋₆alkyl, hydroxyl-C₁₋₆alkyl or optionally substituted guanidino-C₂₋₄alkyl R⁸ is optionally substituted benzyl;
R^{8a} is hydrogen or C₁₋₆alkyl;
R⁹ is optionally substituted benzyl or optionally substituted 1H-imidazol-5-ylmethyl;
R¹⁰ is C₁₋₆alkyl, amino-C₁₋₆alkyl, optionally substituted 1H-imidazol-5-ylmethyl, optionally substituted guanidino-C₂₋₄alkyl or optionally substituted C₁₋₂alkyl-carboxamide;
R¹¹ is optionally substituted benzyl or optionally substituted (imidazol-4-yl)methyl;
R¹² is N-(3-amino-3-oxo-propyl)carboxamide or N-(2-amino-2-oxo-ethyl)carboxamide both optionally substituted;
R^{b} R^{c}, R^{d}, R^{e}, R^{f}, R^{15a}, R^{15b}, R^{15c}, R^{15d}, R^{15f}, R^{15g}, R^{15h}, R¹⁵ⁱ, R^{15j}, R^{15k}, R^{16a}, R^{16b}, R^{16c} and R^{16d} are each independently hydrogen, or C₁₋₆alkyl;
R^{15e} is hydrogen, C₁₋₆alkyl, amino-C₁₋₆alkyl, or optionally substituted C₁₋₂alkyl-carboxamide;
R^{a} is hydrogen, C₁₋₆alkyl, hydroxyl or halo;
*n* and *p* are independently from 0 to 2;
q is 0 or 1; and,
r is 1 or 2; or
a compound of Formula (II) 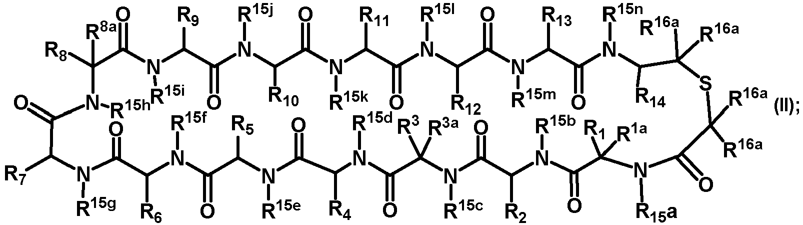
or a stereoisomer or a pharmaceutically acceptable salt thereof, wherein:
R¹ is optionally substituted benzyl;
R^{1a} is hydrogen or C₁₋₆alkyl;
R² is hydrogen or optionally substituted (imidazol-4-yl)methyl;
R³ is C₁₋₆alkyl or hydroxyl-C₁₋₆alkyl;
R^{3a} is hydrogen or C₁₋₆alkyl;
R⁴ is C₁₋₆alkyl or optionally substituted (imidazol-4-yl)methyl;
R⁵ is optionally substituted guanidino-C₂₋₄alkyl or optionally substituted (imidazol-4-yl)methyl;
R⁶ is amino-C₁₋₆alkyl or optionally substituted guanidino-C₂₋₄alkyl;
R⁷ is hydroxyl-C₁₋₆alkyl or optionally substituted guanidino-C₂₋₄alkyl;
R⁸ is methyl or 3-propylguanidine;
R^{8a} is hydrogen or methyl;
R⁹ is optionally substituted benzyl;
R¹⁰ is amino-C₁₋₆alkyl, optionally substituted (imidazol-4-yl)methyl or optionally substituted guanidino-C₂₋₄alkyl;
R¹¹ is optionally substituted benzyl;
R¹² is indolyl-3-methyl or optionally substituted benzyl;
R¹³ is optionally substituted benzyl;
R¹⁴ is is N-(3-amino-3-oxo-propyl)carboxamide or N-(2-amino-2-oxo-ethyl)carboxamide both optionally substituted;
R^{b}, R^{c}, R^{d}, R^{e}, R^{f}, R^{15a}, R^{15b}, R^{15c}, R^{15d}, R^{15e}, R^{15f}, R^{15g}, R^{15h}, R¹⁵ⁱ, R^{15j}, R^{15k}, R^{15l}, R^{15m}, R¹⁵ⁿ, R^{16a}, R^{16b}, R^{16c} and R^{16d} are in each occurrence independently hydrogen or C₁₋₆alkyl;
R^{a} is hydrogen, C₁₋₆alkyl, hydroxyl or halo;
n and p are each independently 0 to 2; and
q is 0 or 1.

2. The compound of claim 1 wherein the compound is of formula I, and R¹² is N-(2-amino-2-oxoethyl)carboxamide, R⁴ and R⁹ are 1H-imidazol-5-ylmethyl and R² is C₁₋₆ alkyl.

3. The compound of either claim 1 or 2 wherein the compound is of formula I, and wherein:
R¹ is C₁₋₆alkyl, hydroxyl-C₁₋₆alkyl or optionally substituted benzyl;
R³ is 1-naphthylmethyl, 2-naphthylmethyl, (4-phenylphenyl)methyl or optionally substituted 1H-indolyl-3-methyl;
R⁵ is hydrogen, or 3-guanidopropyl;
R⁶ is hydroxyl-C₁₋₆alkyl or 4-aminobutyl;
R⁷ is 3-guanidopropyl;
R¹⁰ is 3-guanidino-propyl, methyl, ethyl, aminomethyl, aminoethyl, 4-aminobutyl, 3-methylimidazol-4-yl)methyl or 3-amino-3-oxo-propyl;
R¹¹ is 1H-imidazol-5-ylmethyl or 3-methylimidazol-4-yl)methyl;
R^{b}, R^{c}, R^{e}, R^{1a}, R^{3a}, R^{8a}, R^{15b}, R^{15c}, R^{15d}, R¹⁵f, R^{15g}, R¹⁵ⁱ, R^{15k}, R^{16a}, R^{16b}, R^{16c} and R^{16d} are hydrogen;
R^{15a}, R^{15e}, R^{15h} and R^{15j} are independently hydrogen or methyl;
R^{b} and R^{f} are independently hydrogen or methyl;
R^{15h} is methyl;
or, a stereoisomer or a pharmaceutically acceptable salt thereof.

4. The compound of any of claims 1 to 3 wherein the compound is of formula I, and wherein:
R¹ is benzyl optionally substituted halogen or hydroxy;
R² is hydrogen, ethyl, isopropyl or (1S)-1-methylpropyl;
R³ is 1-naphthylmethyl or 1H-indole-3-methyl;
R⁴ is 1H-imidazol-5-ylmethyl;
R⁵ is hydrogen;
R⁶ is hydroxymethyl or (1R)-1-hydroxyethyl;
R⁷ is 3-guanidinopropyl;
R⁸ is benzyl or 4-hydroxybenzyl;
R⁹ is 1H-imidazol-5-ylmethyl;
R¹⁰ is 3-guanidopropyl, methyl or ethyl;
R¹¹ is 1H-imidazol-5-ylmethyl, 3-methylimidazol-4-yl)methyl;
R¹² is -C(=O)NHCH₂C(=O)NH₂;
or, a stereoisomer or a pharmaceutically acceptable salt thereof.

5. The compound of any of claims 1 to 4 wherein the compound is of formula I, and wherein:
R¹ is benzyl;
R² is ethyl or isopropyl;
R³ is 1-naphthylmethyl;
R⁴ is 1H-imidazol-5-ylmethyl;
R⁵ is hydrogen;
R⁶ is hydroxylmethyl;
R⁷ is 3-guanidopropyl;
R⁸ is 4-hydroxylbenzyl or 4-chlorobenzyl;
R⁹ is 1-methyl-1H-imidazole-5-methylene;
R¹⁰ is 3-guanidopropyl;
*R*¹¹ is (3-methylimidazol-4-yl)methyl;
R¹² is N-(2-amino-2-oxo-ethyl)carboxamide;
R^{b} and R^{f} are independently in each occurrence hydrogen or methyl; and,
or a stereoisomer or a pharmaceutically acceptable salt thereof.

6. The compound of any one of claims 1 to 5 wherein the compound is of formula Ia: 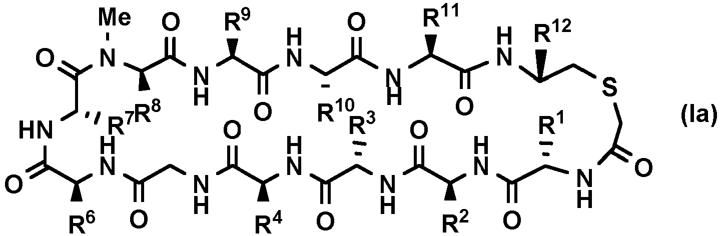 or a stereoisomer or a pharmaceutically acceptable salt thereof.

7. The compound of claim 1, wherein the compound is of Formula II, and wherein:
R¹ is optionally substituted benzyl;
R^{1a} is hydrogen or C₁₋₆alkyl;
R² is (3-methylimidazol-4-yl)methyl;
R³ is C₁₋₆alkyl or hydroxyl-C₁₋₆alkyl;
R^{3a} is hydrogen or C₁₋₆alkyl;
R⁴ is C₁₋₆alkyl;
R⁵ is 3-guanidino-propyl;
R⁶ is3-guanidino-propyl or amino-C₁₋₆alkyl;
R⁷ is 3-guanidino-propyl or hydroxyl-C₁₋₆alkyl;
R⁸ is optionally substituted benzyl;
R^{8a} is methyl;
R⁹ is optionally substituted benzyl;
R¹⁰ is optionally substituted guanidino-C₂₋₄alkyl, (3-methylimidazol-4-yl)methyl or amino-C₁₋₆alkyl;
R¹¹ is 4-hydroxybenzyl or 4-chlorobenzyl;
R¹² is 1H-indole-3-methyl or 4-chlorobenzyl;
R¹³ is optionally substituted benzyl;
R¹⁴ is -C(=O)NHCH₂C(=O)NH₂;
R^{15a}, R^{15b}, R^{15c}, R^{15d}, R^{15e}, R^{15f}, R^{15g}, R^{15h}, R¹⁵ⁱ, R^{15j}, R^{15k}, R^{15l}, R^{15m}, R¹⁵ⁿ, R^{16a}, R^{16b}, R^{16c} and R^{16d} are hydrogen;
or, a stereoisomer or a pharmaceutically acceptable salt thereof.

8. The compound of either claims 1 or 7, wherein the compound is of Formula II**,** and wherein:
R¹ is benzyl;
R^{1a} is hydrogen;
R² is (3-methylimidazol-4-yl)methyl;
R³ is methyl or (1R)-1-hydroxyethyl;
R^{3a} is hydrogen or methyl;
R⁴ is (1S)-1-methylpropyl;
R⁵ is 3-guanidopropyl;
R⁶ is 4-amino-butyl;
R⁷ is 3-guanidopropyl or-(1R)-1-hydroxyethyl;
R⁸ and R^{8a} are methyl;
R⁹ is benzyl;
R¹⁰ is guanidino-propyl, (3-methylimidazol-4-yl)methyl or amino-C₁₋₆alkyl;
R¹¹ is 4-hydroxybenzyl;
R¹² is 1H-indole-3-methyl;
R¹³ is 4-hydroxybenzyl;
R¹⁴ is -C(=O)NHCH₂C(=O)NH₂;
R^{15a}, R^{15b}, R^{15c}, R^{15d},R^{15e}, R^{15f}, R^{15g}, R^{15h}, R¹⁵ⁱ, R^{15j}, R^{15k}, R^{15l}, R^{15m}, R¹⁵ⁿ, R^{16a}, R^{16b}, R^{16c} and
R^{16a} are hydrogen;
or, a stereoisomer or a pharmaceutically acceptable salt thereof.

9. The compound of claim 1, 7 or 8, wherein the compound is of Formula II, and wherein:
R¹ is benzyl;
R^{1a} is hydrogen;
R² is (3-methylimidazol-4-yl)methyl;
R³ is methyl or (1R)-1-hydroxyethyl;
R⁴ is (1S)-1-methylpropyl;
R⁵ is 3-guanidino-propyl;
R⁶ is 4-aminobutyl;
R⁷ is rac-(1R)-1-hydroxyethyl;
R⁸ and R^{8a} are methyl;
R⁹ is benzyl;
R¹⁰ is 3-guanidinopropyl, (3-methylimidazol-4-yl)methyl, aminomethyl, 2-amino-ethyl, 3-aminopropyl or 4 aminobutyl;
R¹¹ is 4-hydroxybenzyl;
R¹² is 1H-indole-3-methyl;
R¹³ is 4-hydroxybenzyl;
R¹⁴ is -C(=O)NHCH₂C(=O)NH.

10. The compound of claim 1 or any of claims 7 to 9, wherein the compound is of Formula II, and wherein:
R¹ is benzyl;
R^{1a} is hydrogen;
R² is (3-methylimidazol-4-yl)methyl;
R³ is methyl or (1R)-1-hydroxyethyl;
R⁴ is (1S)-1-methylpropyl;
R⁵ is 3-guanidino-propyl;
R⁶ is 4-aminobutyl;
R⁷ is rac-(1R)-1-hydroxyethy;
R⁸ and R^{8a} are methyl;
R⁹ is benzyl;
R¹⁰ is 3-guanidinopropyl, (3-methylimidazol-4-yl)methyl, 3-aminopropyl or 4 aminobutyl;
R¹¹ is 4-hydroxybenzyl;
R¹² is 1H-indole-3-methyl;
R¹³ is 4-hydroxybenzyl;
R¹⁴ is -C(=O)NHCH₂C(=O)NH;
or, a stereoisomer or a pharmaceutically acceptable salt thereof.

11. The compound of claim 1 or any of claims 7 to 10 wherein the compound is of formula IIa:

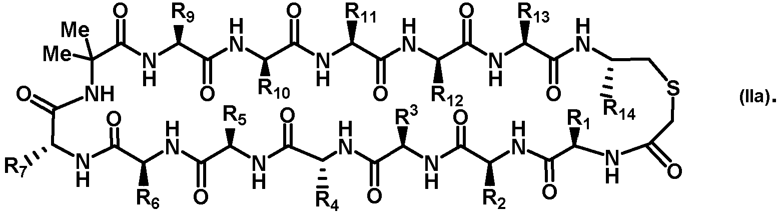

12. The compound according to claim 1 wherein said compound is selected from:
| # | Structure | Compound Name |
|---|---|---|
| 1 | 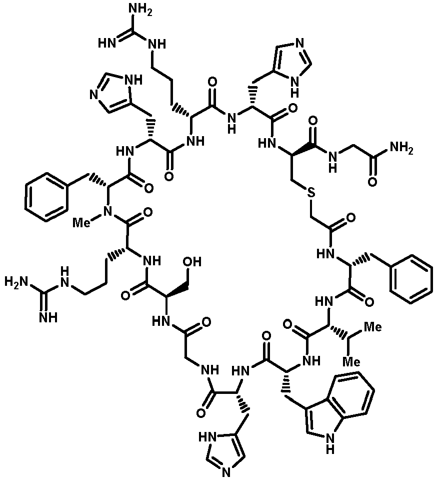 | rac-(3R,6S,9S,12S,15S,18S,21S,27S,30S,33S,36S)-N-(2-amino-2-oxo-ethyl)-15,36-dibenzyl-9,18-bis(3-guanidinopropyl)-21-(hydroxymethyl)-6,12,27-tris(1H-imidazol-5-ylmethyl)-30-(1H-indol-3-ylmethyl)-33-isopropyl-16-methyl-5,8,11,14,17,20,23,26,29,32,35,38-dodecaoxo-1-thia-4,7,10,13,16,19,22,25,28,31,34,37-dodecazacyclononatriacontane-3-carboxamide |
| 2 | 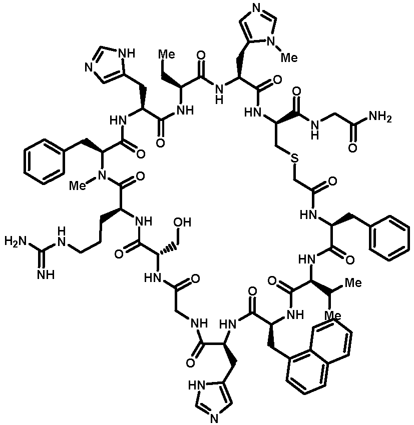 | (3R,6S,9S, 12S, 15S, 18S, 21S,27S,30S,33S,36S)-N-(2-amino-2-oxo-ethyl)-15,36-dibenzyl-9-ethyl-18-(3-guanidinopropyl)-21-(hydroxymethyl)-12,27-bis(1H-imidazol-4-ylmethyl)-33-isopropyl-16-methyl-6-[(3-methylimidazol-4-yl)methyl]-30-(1-naphthylmethyl)-5,8,11,14,17,20,23,26,29,32,35,38-dodecaoxo-1-thia-4,7,10,13,16,19,22,25,28,31,34,37-dodecazacyclononatriacontane-3-carboxamide |
| 3 | 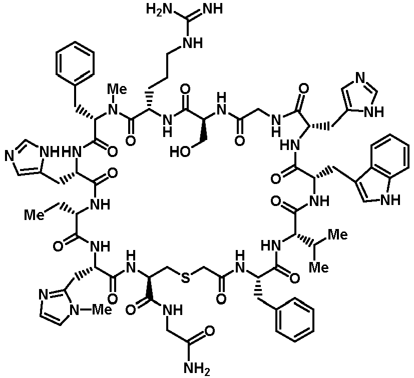 | (3R,6S,9S, 12S, 15S, 18S, 21S,27S,30S,33S,36S)-N-(2-amino-2-oxo-ethyl)-15,36-dibenzyl-9-ethyl-18-(3-guanidinopropyl)-21-(hydroxymethyl)-12,27-bis(1H-imidazol-4-ylmethyl)-30-(1H-indol-3-ylmethyl)-33-isopropyl-16-methyl-6-[(3-methylimidazol-4-yl)methyl]-5,8,11,14,17,20,23,26,29,32,35,38-dodecaoxo-1-thia-4,7,10,13,16,19,22,25,28,31,34,37-dodecazacyclononatriacontane-3-carboxamide |
| 4 | 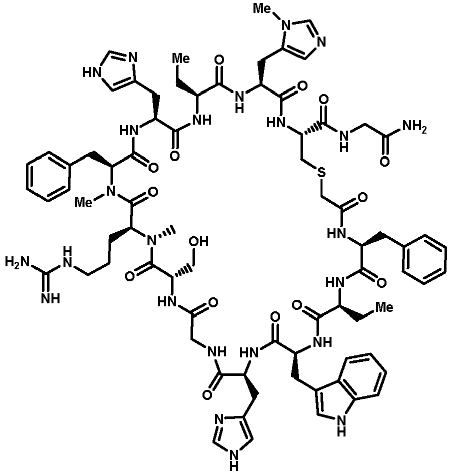 | (3R,6S,9S, 12S, 15S, 18S, 21S,27S,30S,33S,36S)-N-(2-amino-2-oxo-ethyl)-15,36-dibenzyl-9,33-diethyl-18-(3-guanidinopropyl)-21-(hydroxymethyl)-12,27-bis(1H-imidazol-4-ylmethyl)-30-(1H-indol-3-ylmethyl)-16-methyl-6-[(3-methylimidazol-4-yl)methyl]-5,8,11,14,17,20,23,26,29,32,35,38-dodecaoxo-1-thia-4,7,10,13,16,19,22,25,28,31,34,37-dodecazacyclononatriacontane-3-carboxamide |
| 5 | 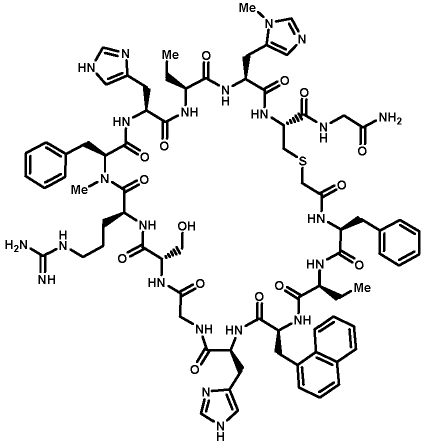 | (3R,6S,9S, 12S, 15S, 18S, 21S,27S,30S,33S,36S)-N-(2-amino-2-oxo-ethyl)-15,36-dibenzyl-9,33-diethyl-18-(3-guanidinopropyl)-21-(hydroxymethyl)-12,27-bis(1H-imidazol-4-ylmethyl)-16-methyl-6-[(3-methylimidazol-4-yl)methyl]-30-(1-naphthylmethyl)-5,8,11,14,17,20,23,26,29,32,35,38-dodecaoxo-1-thia-4,7,10,13,16,19,22,25,28,31,34,37-dodecazacyclononatriacontane-3-carboxamide |
| 6 | 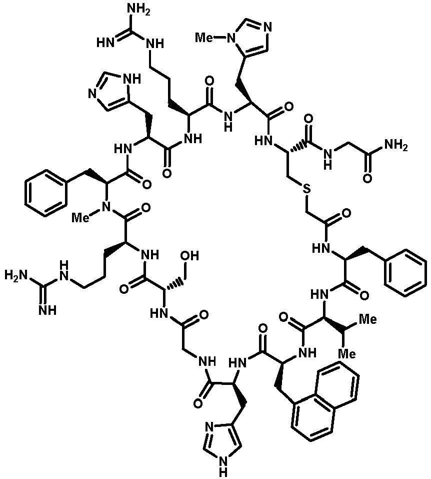 | (3R,6S,9S, 12S, 15S, 18S, 21S,27S,30S,33S,36S)-N-(2-amino-2-oxo-ethyl)-15,36-dibenzyl-9,18-bis(3-guanidinopropyl)-21-(hydroxymethyl)-12,27-bis(1H-imidazol-4-ylmethyl)-33-isopropyl-16-methyl-6-[(3-methylimidazol-4-yl)methyl]-30-(1-naphthylmethyl)-5,8,11,14,17,20,23,26,29,32,35,38-dodecaoxo-1-thia-4,7,10,13,16,19,22,25,28,31,34,37-dodecazacyclononatriacontane-3-carboxamide |
| 7 | 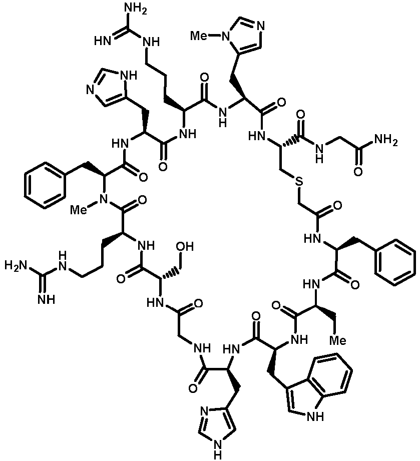 | (3R,6S,9S, 12S, 15S, 18S, 21S,27S,30S,33S,36S)-N-(2-amino-2-oxo-ethyl)-15,36-dibenzyl-33-ethyl-9,18-bis(3-guanidinopropyl)-21-(hydroxymethyl)-12,27-bis(1H-imidazol-4-ylmethyl)-30-(1H-indol-3-ylmethyl)-16-methyl-6-[(3-methylimidazol-4-yl)methyl]-5,8,11,14,17,20,23,26,29,32,35,38-dodecaoxo-1-thia-4,7,10,13,16,19,22,25,28,31,34,37-dodecazacyclononatriacontane-3-carboxamide |
| 8 | 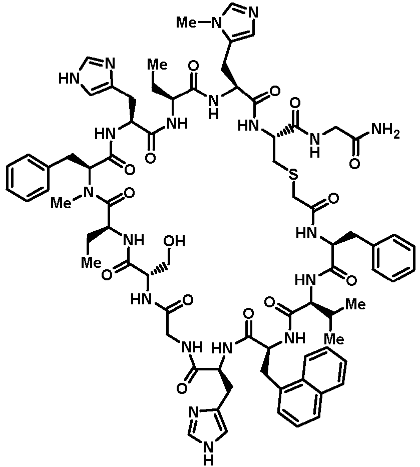 | (3R,6S,9S, 12S, 15S, 18S, 21S,27S,30S,33S,36S)-N-(2-amino-2-oxo-ethyl)-15,36-dibenzyl-9,18-diethyl-21-(hydroxymethyl)-12,27-bis(1H-imidazol-4-ylmethyl)-33-isopropyl-16-methyl-6-[(3-methylimidazol-4-yl)methyl]-30-(1-naphthylmethyl)-5,8,11,14,17,20,23,26,29,32,35,38-dodecaoxo-1-thia-4,7,10,13,16,19,22,25,28,31,34,37-dodecazacyclononatriacontane-3-carboxamide |
| 9 | 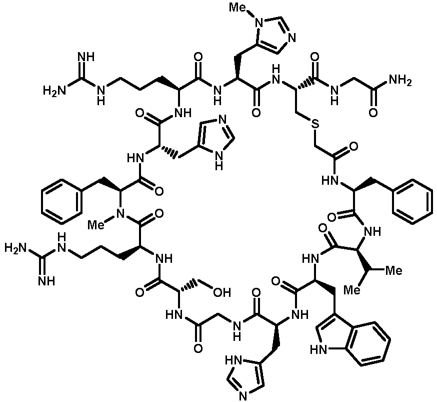 | rac-(3R,6S,9S, 12S, 15S, 18S, 21S,27S,30S,33S,36S)-N-(2-amino-2-oxo-ethyl)-15,36-dibenzyl-9,18-bis(3-guanidinopropyl)-21-(hydroxymethyl)-12,27-bis(1H-imidazol-5-ylmethyl)-30-(1H-indol-3-ylmethyl)-33-isopropyl-16-methyl-6-[(3-methylimidazol-4-yl)methyl]-5,8,11,14,17,20,23,26,29,32,35,38-dodecaoxo-1-thia-4,7,10,13,16,19,22,25,28,31,34,37-dodecazacyclononatriacontane-3-carboxamide |
| 10 | 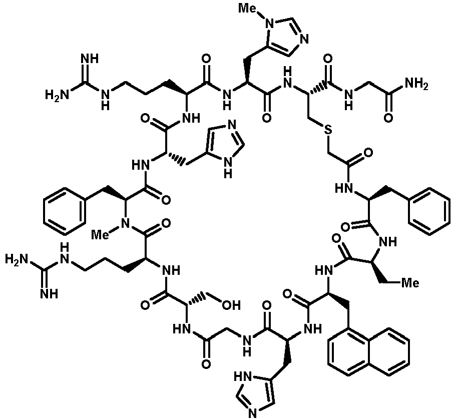 | rac-(3R,6S,9S, 12S, 15S, 18S, 21S,27S,30S,33S,36S)-N-(2-amino-2-oxo-ethyl)-15,36-dibenzyl-33-ethyl-9,18-bis(3-guanidinopropyl)-21-(hydroxymethyl)-12,27-bis(1H-imidazol-5-ylmethyl)-16-methyl-6-[(3-methylimidazol-4-yl)methyl]-30-(1-naphthylmethyl)-5,8,11,14,17,20,23,26,29,32,35,38-dodecaoxo-1-thia-4,7,10,13,16,19,22,25,28,31,34,37-dodecazacyclononatriacontane-3-carboxamide |
| 11 | 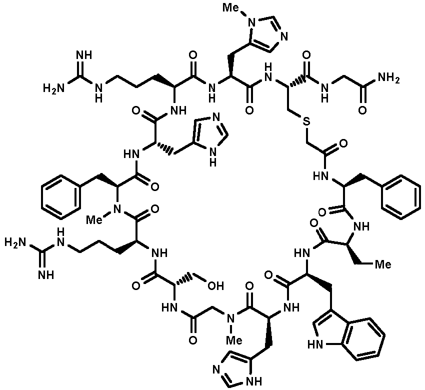 | rac-(3R,6S,9S,12S,15S,18S,21S,27S,30S,33S,36S)-N-(2-amino-2-oxo-ethyl)-15,36-dibenzyl-33-ethyl-9,18-bis(3-guanidinopropyl)-21-(hydroxymethyl)-12,27-bis(1H-imidazol-5-ylmethyl)-30-(1H-indol-3-ylmethyl)-16,25-dimethyl-6-[(3-methylimidazol-4-yl)methyl]-5,8,11,14,17,20,23,26,29,32,35,38-dodecaoxo-1-thia-4,7,10,13,16,19,22,25,28,31,34,37-dodecazacyclononatriacontane-3-carboxamide |
| 12 | 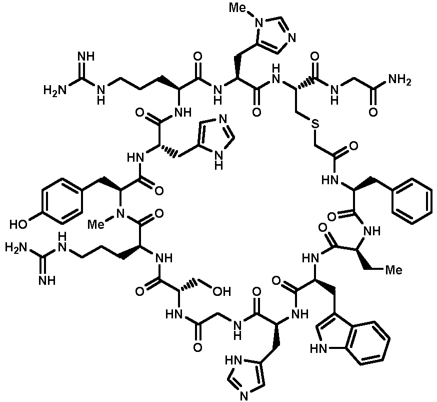 | rac-(3R,6S,9S, 12S, 15S, 18S, 21S,27S,30S,33S,36S)-N-(2-amino-2-oxo-ethyl)-36-benzyl-33-ethyl-9,18-bis(3-guanidinopropyl)-21-(hydroxymethyl)-15-[(4-hydroxyphenyl)methyl]-12,27-bis(1H-imidazol-5-ylmethyl)-30-(1H-indol-3-ylmethyl)-16-methyl-6-[(3-methylimidazol-4-yl)methyl]-5,8,11,14,17,20,23,26,29,32,35,38-dodecaoxo-1-thia-4,7,10,13,16,19,22,25,28,31,34,37-dodecazacyclononatriacontane-3-carboxamide |
| 13 | 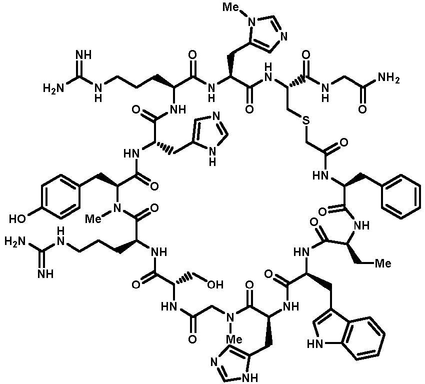 | rac-(3R,6S,9S, 12S, 15S, 18S, 21S,27S,30S,33S,36S)-N-(2-amino-2-oxo-ethyl)-36-benzyl-33-ethyl-9,18-bis(3-guanidinopropyl)-21-(hydroxymethyl)-15-[(4-hydroxyphenyl)methyl]-12,27-bis(1H-imidazol-5-ylmethyl)-30-(1H-indol-3-ylmethyl)-16,25-dimethyl-6-[(3-methylimidazol-4-yl)methyl]-5,8,11,14,17,20,23,26,29,32,35,38-dodecaoxo-1-thia-4,7,10,13,16,19,22,25,28,31,34,37-dodecazacyclononatriacontane-3-carboxamide |
| 14 | 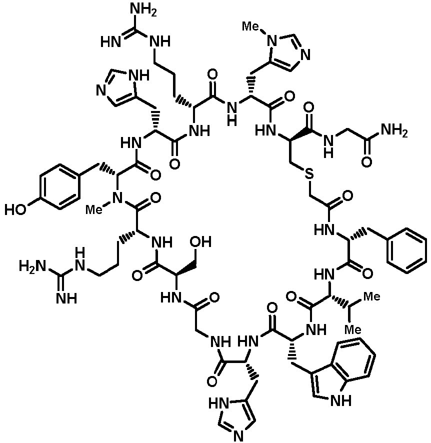 | rac-(3R,6S,9S, 12S, 15S, 18S, 21S,27S,30S,33S,36S)-N-(2-amino-2-oxo-ethyl)-36-benzyl-9,18-bis(3-guanidinopropyl)-21-(hydroxymethyl)-15-[(4-hydroxyphenyl)methyl]-12,27-bis(1H-imidazol-5-ylmethyl)-30-(1H-indol-3-ylmethyl)-33-isopropyl-16-methyl-6-[(3-methylimidazol-4-yl)methyl]-5,8,11,14,17,20,23,26,29,32,35,38-dodecaoxo-1-thia-4,7,10,13,16,19,22,25,28,31,34,37-dodecazacyclononatriacontane-3-carboxamide |
| 15 | 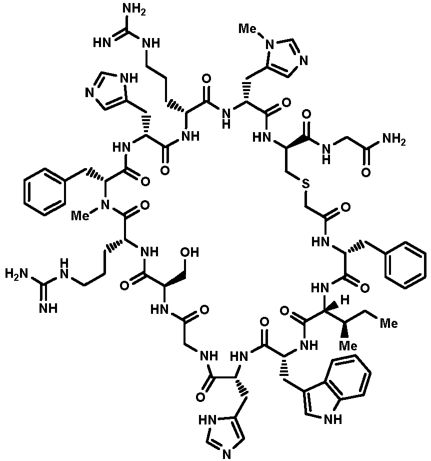 | rac-(3R,6S,9S,12S,15S,18S,21S,27S,30S,33S,36S)-N-(2-amino-2-oxo-ethyl)-15,36-dibenzyl-9,18-bis(3-guanidinopropyl)-21-(hydroxymethyl)-12,27-bis(1H-imidazol-5-ylmethyl)-30-(1H-indol-3-ylmethyl)-16-methyl-6-[(3-methylimidazol-4-yl)methyl]-5,8,11,14,17,20,23,26,29,32,35,38-dodecaoxo-33-[rac-(1S)-1-methylpropyl]-1-thia-4,7,10,13,16,19,22,25,28,31,34,37-dodecazacyclononatriacontane-3-carboxamide |
| 16 | 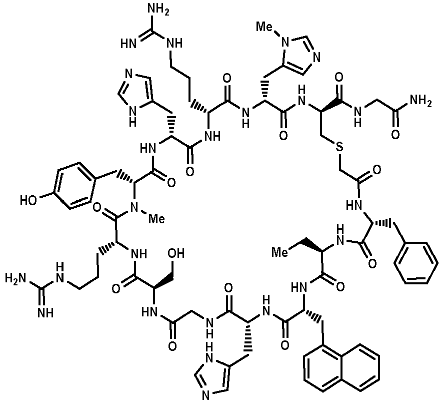 | rac-(3R,6S,9S, 12S, 15S, 18S, 21S,27S,30S,33S,36S)-N-(2-amino-2-oxo-ethyl)-36-benzyl-33-ethyl-9,18-bis(3-guanidinopropyl)-21-(hydroxymethyl)-15-[(4-hydroxyphenyl)methyl]-12,27-bis(1H-imidazol-5-ylmethyl)-16-methyl-6-[(3-methylimidazol-4-yl)methyl]-30-(1-naphthylmethyl)-5,8,11,14,17,20,23,26,29,32,35,38-dodecaoxo-1-thia-4,7,10,13,16,19,22,25,28,31,34,37-dodecazacyclononatriacontane-3-carboxamide |
| 17 | 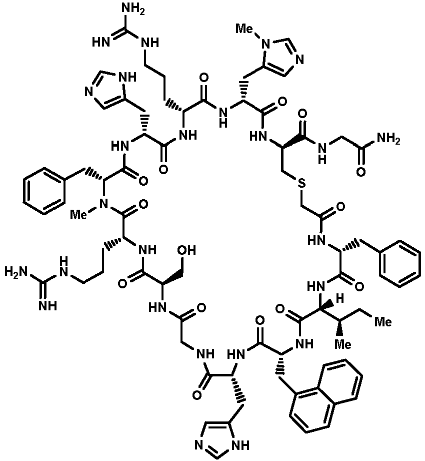 | rac-(3R,6S,9S,12S,15S,18S,21S,27S,30S,33S,36S)-N-(2-amino-2-oxo-ethyl)-15,36-dibenzyl-9,18-bis(3-guanidinopropyl)-21-(hydroxymethyl)-12,27-bis(1H-imidazol-5-ylmethyl)-16-methyl-6-[(3-methylimidazol-4-yl)methyl]-30-(1-naphthylmethyl)-5,8,11,14,17,20,23,26,29,32,35,38-dodecaoxo-33-[rac-(1S)-1-methylpropyl]-1-thia-4,7,10,13,16,19,22,25,28,31,34,37-dodecazacyclononatriacontane-3-carboxamide |
| 18 | 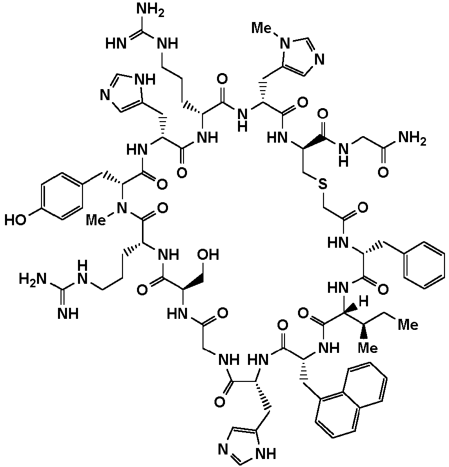 | rac-(3R,6S,9S,12S,15S,18S,21S,27S,30S,33S,36S)-N-(2-amino-2-oxo-ethyl)-36-benzyl-9,18-bis(3-guanidinopropyl)-21-(hydroxymethyl)-15-[(4-hydroxyphenyl)methyl]-12,27-bis(1H-imidazol-5-ylmethyl)-16-methyl-6-[(3-methylimidazol-4-yl)methyl]-30-(1-naphthylmethyl)-5,8,11,14,17,20,23,26,29,32,35,38-dodecaoxo-33-[rac-(1S)-1-methylpropyl]-1-thia-4,7,10,13,16,19,22,25,28,31,34,37-dodecazacyclononatriacontane-3-carboxamide |
| 19 | 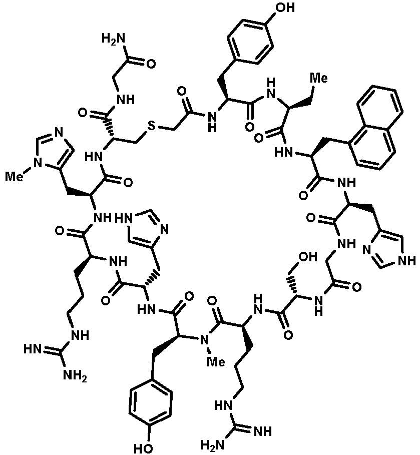 | (3R,6S,9S, 12S, 15S, 18S, 21S,27S,30S,33S,36S)-N-(2-amino-2-oxo-ethyl)-33-ethyl-9,18-bis(3-guanidinopropyl)-21-(hydroxymethyl)-15,36-bis[(4-hydroxyphenyl)methyl]-12,27-bis(1H-imidazol-4-ylmethyl)-16-methyl-6-[(3-methylimidazol-4-yl)methyl]-30-(1-naphthylmethyl)-5,8,11,14,17,20,23,26,29,32,35,38-dodecaoxo-1-thia-4,7,10,13,16,19,22,25,28,31,34,37-dodecazacyclononatriacontane-3-carboxamide |
| 20 | 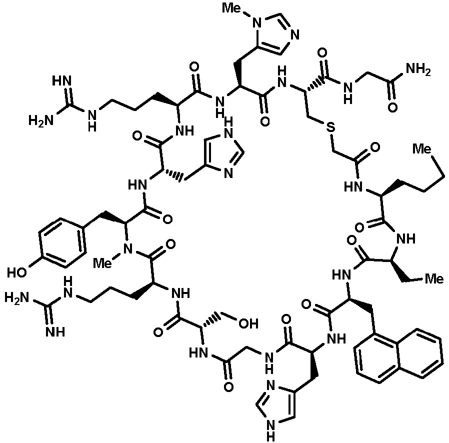 | (3R,6S,9S, 12S, 15S, 18S, 21S,27S,30S,33S,36S)-N-(2-amino-2-oxo-ethyl)-36-butyl-33-ethyl-9,18-bis(3-guanidinopropyl)-21-(hydroxymethyl)-15-[(4-hydroxyphenyl)methyl]-12,27-bis(1H-imidazol-4-ylmethyl)-16-methyl-6-[(3-methylimidazol-4-yl)methyl]-30-(1-naphthylmethyl)-5,8,11,14,17,20,23,26,29,32,35,38-dodecaoxo-1-thia-4,7,10,13,16,19,22,25,28,31,34,37-dodecazacyclononatriacontane-3-carboxamide |
| 21 | 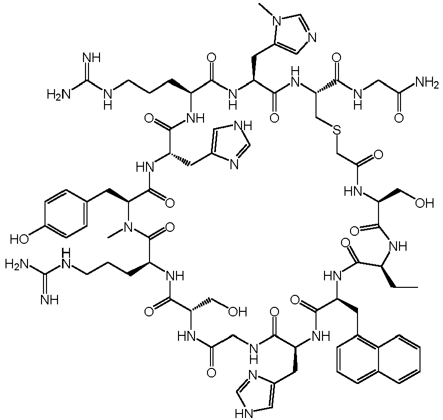 | (3R,6S,9S,12S,15S,18S,21S,27S,30S,33S,36S)-N-(2-amino-2-oxo-ethyl)-33-ethyl-9,18-bis(3-guanidinopropyl)-21,36-bis(hydroxymethyl)-15-[(4-hydroxyphenyl)methyl]-12,27-bis(1H-imidazol-4-ylmethyl)-16-methyl-6-[(3-methylimidazol-4-yl)methyl]-30-(1-naphthylmethyl)-5,8,11,14,17,20,23,26,29,32,35,38-dodecaoxo-1-thia-4,7,10,13,16,19,22,25,28,31,34,37-dodecazacyclononatriacontane-3-carboxamide |
| 22 | 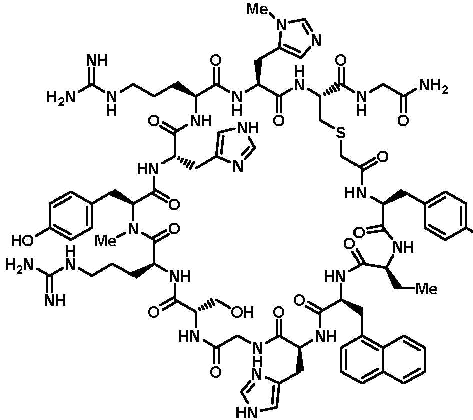 | (3R,6S,9S,12S,15S,18S,21S,27S,30S,33S,36S)-N-(2-amino-2-oxo-ethyl)-36-[(4-chlorophenyl)methyl]-33-ethyl-9,18-bis(3-guanidinopropyl)-21-(hydroxymethyl)-15-[(4-hydroxyphenyl)methyl]-12,27-bis(1H-imidazol-4-ylmethyl)-16-methyl-6-[(3-methylimidazol-4-yl)methyl]-30-(1-naphthylmethyl)-5,8,11,14,17,20,23,26,29,32,35,38-dodecaoxo-1-thia-4,7,10,13,16,19,22,25,28,31,34,37-dodecazacyclononatriacontane-3-carboxamide |
| 23 | 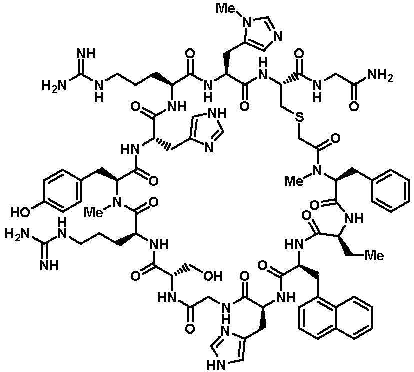 | (3R,6S,9S,12S,15S,18S,21S,27S,30S,33S,36S)-N-(2-amino-2-oxo-ethyl)-36-benzyl-33-ethyl-9,18-bis(3-guanidinopropyl)-21-(hydroxymethyl)-15-[(4-hydroxyphenyl)methyl]-12,27-bis(1H-imidazol-4-ylmethyl)-16,37-dimethyl-6-[(3-methylimidazol-4-yl)methyl]-30-(1-naphthylmethyl)-5,8,11,14,17,20,23,26,29,32,35,38-dodecaoxo-1-thia-4,7,10,13,16,19,22,25,28,31,34,37-dodecazacyclononatriacontane-3-carboxamide |
| 24 | 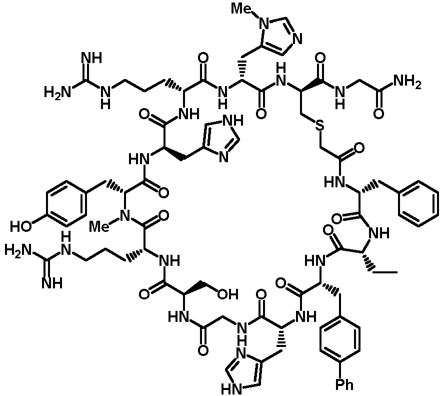 | (3R,6S,9S,12S,15S,18S,21S,27S,30S,33S,36S)-N-(2-amino-2-oxo-ethyl)-36-benzyl-33-ethyl-9,18-bis(3-guanidinopropyl)-21-(hydroxymethyl)-15-[(4-hydroxyphenyl)methyl]-12,27-bis(1H-imidazol-4-ylmethyl)-16-methyl-6-[(3-methylimidazol-4-yl)methyl]-5,8,11,14,17,20,23,26,29,32,35,38-dodecaoxo-30-[(4-phenylphenyl)methyl]-1-thia-4,7,10,13,16,19,22,25,28,31,34,37-dodecazacyclononatriacontane-3-carboxamide |
| 25 | 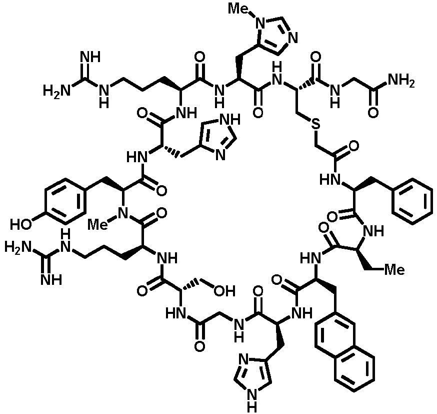 | (3R,6S,9S,12S,15S,18S,21S,27S,30S,33S,36S)-N-(2-amino-2-oxo-ethyl)-36-benzyl-33-ethyl-9,18-bis(3-guanidinopropyl)-21-(hydroxymethyl)-15-[(4-hydroxyphenyl)methyl]-12,27-bis(1H-imidazol-4-ylmethyl)-16-methyl-6-[(3-methylimidazol-4-yl)methyl]-30-(2-naphthylmethyl)-5,8,11,14,17,20,23,26,29,32,35,38-dodecaoxo-1-thia-4,7,10,13,16,19,22,25,28,31,34,37-dodecazacyclononatriacontane-3-carboxamide |
| 26 | 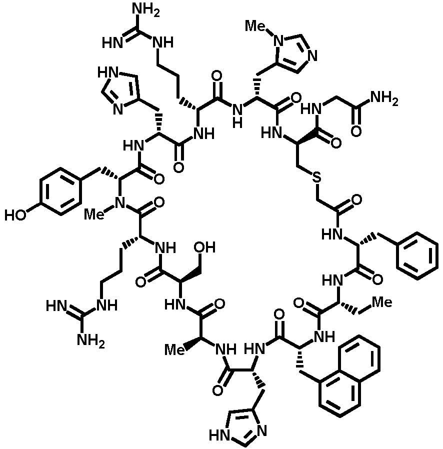 | (3R,6S,9S,12S,15S,18S,21S,24R,27S,30S,33S, 36S)-N-(2-amino-2-oxo-ethyl)-36-benzyl-33-ethyl-9,18-bis(3-guanidinopropyl)-21-(hydroxymethyl)-15-[(4-hydroxyphenyl)methyl]-12,27-bis(1H-imidazol-4-ylmethyl)-16,24-dimethyl-6-[(3-methylimidazol-4-yl)methyl]-30-(1-naphthylmethyl)-5,8,11,14,17,20,23,26,29,32,35,38-dodecaoxo-1-thia-4,7,10,13,16,19,22,25,28,31,34,37-dodecazacyclononatriacontane-3-carboxamide |
| 27 | 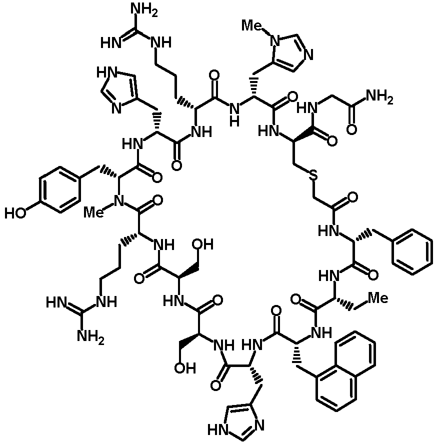 | (3R,6S,9S,12S,15S,18S,21S,24R,27S,30S,33S, 36S)-N-(2-amino-2-oxo-ethyl)-36-benzyl-33-ethyl-9,18-bis(3-guanidinopropyl)-21,24-bis(hydroxymethyl)-15-[(4-hydroxyphenyl)methyl]-12,27-bis(1H-imidazol-4-ylmethyl)-16-methyl-6-[(3-methylimidazol-4-yl)methyl]-30-(1-naphthylmethyl)-5,8,11,14,17,20,23,26,29,32,35,38-dodecaoxo-1-thia-4,7,10,13,16,19,22,25,28,31,34,37-dodecazacyclononatriacontane-3-carboxamide |
| 28 | 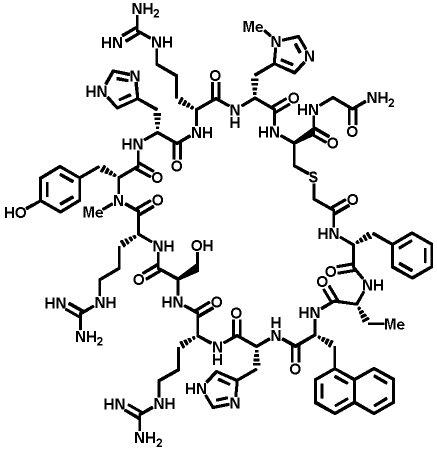 | (3R,6S,9S,12S,15S,18S,21S,24S,27S,30S,33S,3 6S)-N-(2-amino-2-oxo-ethyl)-36-benzyl-33-ethyl-9,18,24-tris(3-guanidinopropyl)-21-(hydroxymethyl)-15-[(4-hydroxyphenyl)methyl]-12,27-bis(1H-imidazol-4-ylmethyl)-16-methyl-6-[(3-methylimidazol-4-yl)methyl]-30-(1-naphthylmethyl)-5,8,11,14,17,20,23,26,29,32,35,38-dodecaoxo-1-thia-4,7,10,13,16,19,22,25,28,31,34,37-dodecazacyclononatriacontane-3-carboxamide |
| 29 | 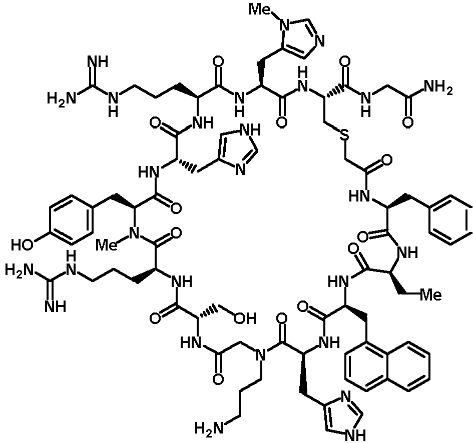 | (3R,6S,9S,12S,15S,18S,21S,27S,30S,33S,36S)-N-(2-amino-2-oxo-ethyl)-25-(3-aminopropyl)-36-benzyl-33-ethyl-9,18-bis(3-guanidinopropyl)-21-(hydroxymethyl)-15-[(4-hydroxyphenyl)methyl]-12,27-bis(1H-imidazol-4-ylmethyl)-16-methyl-6-[(3-methylimidazol-4-yl)methyl]-30-(1-naphthylmethyl)-5,8,11,14,17,20,23,26,29,32,35,38-dodecaoxo-1-thia-4,7,10,13,16,19,22,25,28,31,34,37-dodecazacyclononatriacontane-3-carboxamide |
| 30 | 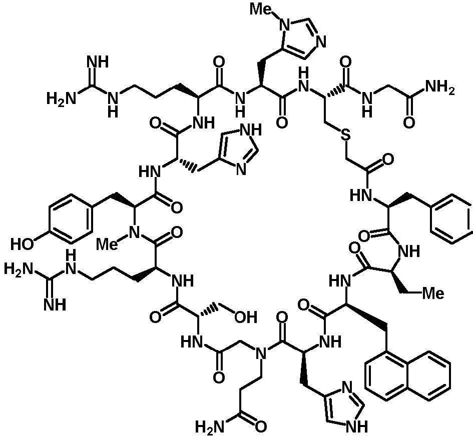 | (3R,6S,9S,12S,15S,18S,21S,27S,30S,33S,36S)-N-(2-amino-2-oxo-ethyl)-25-(3-amino-3-oxopropyl)-36-benzyl-33-ethyl-9,18-bis(3-guanidinopropyl)-21-(hydroxymethyl)-15-[(4-hydroxyphenyl)methyl]-12,27-bis(1H-imidazol-4-ylmethyl)-16-methyl-6-[(3-methylimidazol-4-yl)methyl]-30-(1-naphthylmethyl)-5,8,11,14,17,20,23,26,29,32,35,38-dodecaoxo-1-thia-4,7,10,13,16,19,22,25,28,31,34,37-dodecazacyclononatriacontane-3-carboxamide |
| **31** | 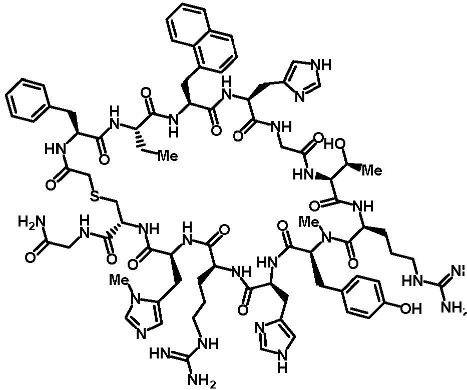 | (3R,6S,9S,12S,15S,18S,21S,27S,30S,33S,36S)-N-(2-amino-2-oxo-ethyl)-36-benzyl-33-ethyl-9,18-bis(3-guanidinopropyl)-21-[(1S)-1-hydroxyethyl]-15-[(4-hydroxyphenyl)methyl]-12,27-bis(1H-imidazol-4-ylmethyl)-16-methyl-6-[(3-methylimidazol-4-yl)methyl]-30-(1-naphthylmethyl)-5,8,11,14,17,20,23,26,29,32,35,38-dodecaoxo-1-thia-4,7,10,13,16,19,22,25,28,31,34,37-dodecazacyclononatriacontane-3-carboxamide |
| **32** | 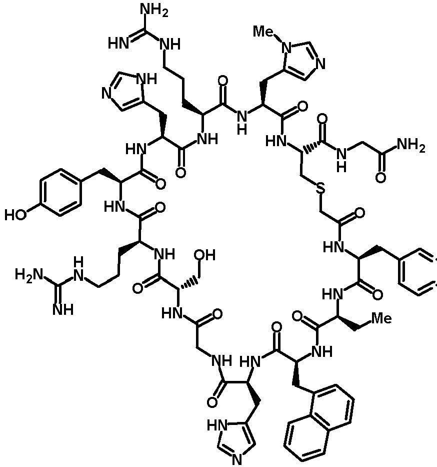 | (3R,6S,9S,12S,15S,18S,21S,27S,30S,33S,36S)-N-(2-amino-2-oxo-ethyl)-36-benzyl-33-ethyl-9,18-bis(3-guanidinopropyl)-21-(hydroxymethyl)-15-[(4-hydroxyphenyl)methyl]-12,27-bis(1H-imidazol-4-ylmethyl)-6-[(3-methylimidazol-4-yl)methyl]-30-(1-naphthylmethyl)-5,8,11,14,17,20,23,26,29,32,35,38-dodecaoxo-1-thia-4,7,10,13,16,19,22,25,28,31,34,37-dodecazacyclononatriacontane-3-carboxamide |
| **33** | 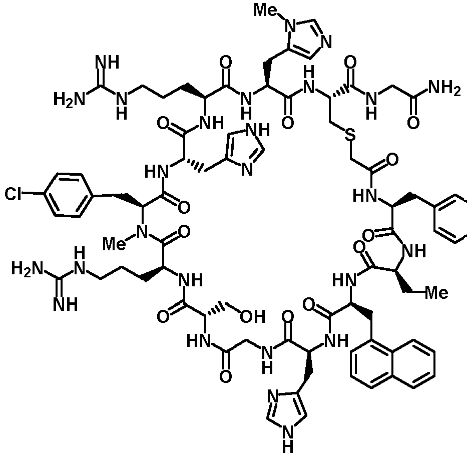 | (3R,6S,9S,12S,15S,18S,21S,27S,30S,33S,36S)-N-(2-amino-2-oxo-ethyl)-36-benzyl-15-[(4-chlorophenyl)methyl]-33-ethyl-9,18-bis(3-guanidinopropyl)-21-(hydroxymethyl)-12,27-bis(1H-imidazol-4-ylmethyl)-16-methyl-6-[(3-methylimidazol-4-yl)methyl]-30-(1-naphthylmethyl)-5,8,11,14,17,20,23,26,29,32,35,38-dodecaoxo-1-thia-4,7,10,13,16,19,22,25,28,31,34,37-dodecazacyclononatriacontane-3-carboxamide |
| **34** | 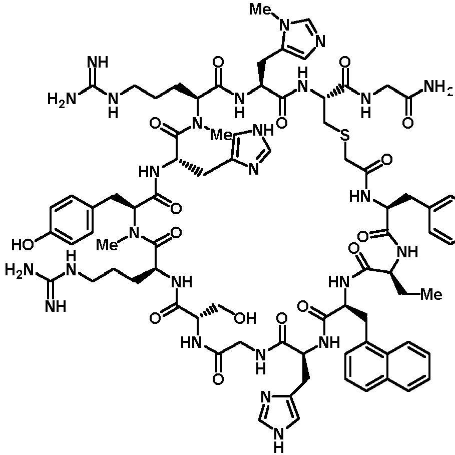 | (3R,6S,9S,12S,15S,18S,21S,27S,30S,33S,36S)-N-(2-amino-2-oxo-ethyl)-36-benzyl-33-ethyl-9,18-bis(3-guanidinopropyl)-21-(hydroxymethyl)-15-[(4-hydroxyphenyl)methyl]-12,27-bis(1H-imidazol-4-ylmethyl)-10,16-dimethyl-6-[(3-methylimidazol-4-yl)methyl]-30-(1-naphthylmethyl)-5,8,11,14,17,20,23,26,29,32,35,38-dodecaoxo-1-thia-4,7,10,13,16,19,22,25,28,31,34,37-dodecazacyclononatriacontane-3-carboxamide |
| **35** | 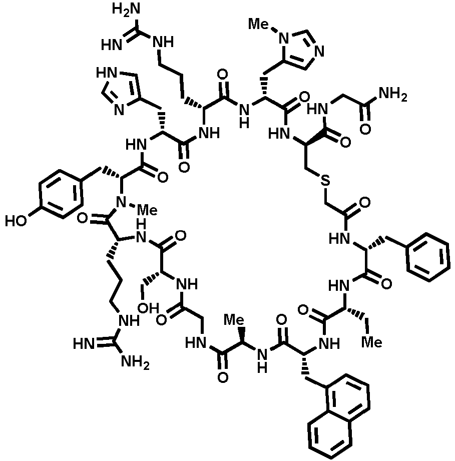 | (3R,6S,9S,12S,15S,18S,21S,27S,30S,33S,36S)-N-(2-amino-2-oxo-ethyl)-36-benzyl-33-ethyl-9,18-bis(3-guanidinopropyl)-21-(hydroxymethyl)-15-[(4-hydroxyphenyl)methyl]-12-(1H-imidazol-4-ylmethyl)-16,27-dimethyl-6-[(3-methylimidazol-4-yl)methyl]-30-(1-naphthylmethyl)-5,8,11,14,17,20,23,26,29,32,35,38-dodecaoxo-1-thia-4,7,10,13,16,19,22,25,28,31,34,37-dodecazacyclononatriacontane-3-carboxamide |
| **36** | 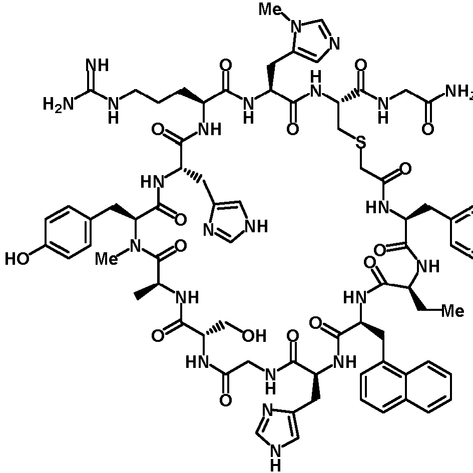 | (3R,6S,9S,12S,15S,18S,21S,27S,30S,33S,36S)-N-(2-amino-2-oxo-ethyl)-36-benzyl-33-ethyl-9-(3-guanidinopropyl)-21-(hydroxymethyl)-15-[(4-hydroxyphenyl)methyl]-12,27-bis(1H-imidazol-4-ylmethyl)-16,18-dimethyl-6-[(3-methylimidazol-4-yl)methyl]-30-(1-naphthylmethyl)-5,8,11,14,17,20,23,26,29,32,35,38-dodecaoxo-1-thia-4,7,10,13,16,19,22,25,28,31,34,37-dodecazacyclononatriacontane-3-carboxamide |
| **37** | 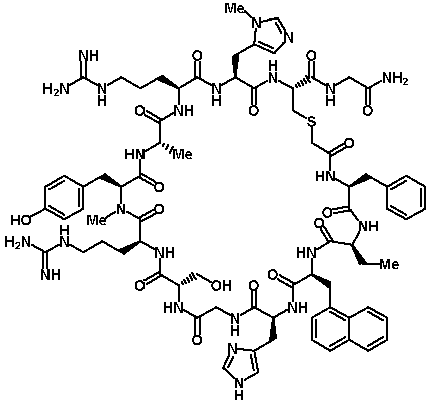 | (3R,6S,9S,12S,15S,18S,21S,27S,30S,33S,36S)-N-(2-amino-2-oxo-ethyl)-36-benzyl-33-ethyl-9,18-bis(3-guanidinopropyl)-21-(hydroxymethyl)-15-[(4-hydroxyphenyl)methyl]-27-(1H-imidazol-4-ylmethyl)-12,16-dimethyl-6-[(3-methylimidazol-4-yl)methyl]-30-(1-naphthylmethyl)-5,8,11,14,17,20,23,26,29,32,35,38-dodecaoxo-1-thia-4,7,10,13,16,19,22,25,28,31,34,37-dodecazacyclononatriacontane-3-carboxamide |
| **38** | 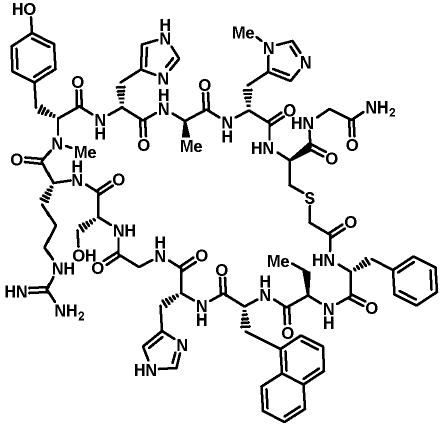 | (3R,6S,9S,12S,15S,18S,21S,27S,30S,33S,36S)-N-(2-amino-2-oxo-ethyl)-36-benzyl-33-ethyl-18-(3-guanidinopropyl)-21-(hydroxymethyl)-15-[(4-hydroxyphenyl)methyl]-12,27-bis(1H-imidazol-4-ylmethyl)-9,16-dimethyl-6-[(3-methylimidazol-4-yl)methyl]-30-(1-naphthylmethyl)-5,8,11,14,17,20,23,26,29,32,35,38-dodecaoxo-1-thia-4,7,10,13,16,19,22,25,28,31,34,37-dodecazacyclononatriacontane-3-carboxamide |
| **39** | 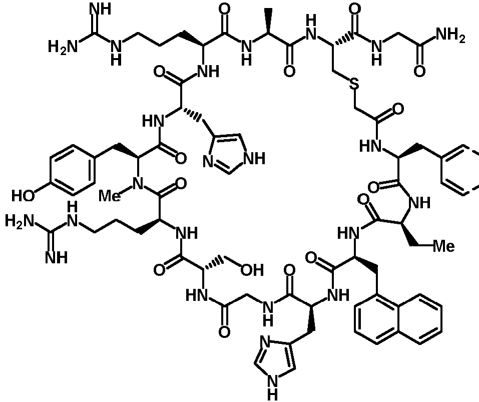 | (3R,6S,9S,12S,15S,18S,21S,27S,30S,33S,36S)-N-(2-amino-2-oxo-ethyl)-36-benzyl-33-ethyl-9,18-bis(3-guanidinopropyl)-21-(hydroxymethyl)-15-[(4-hydroxyphenyl)methyl]-12,27-bis(1H-midazol-4-ylmethyl)-6,16-dimethyl-30-(1-naphthylmethyl)-5,8,11,14,17,20,23,26,29,32,35,38-dodecaoxo-1-thia-4,7,10,13,16,19,22,25,28,31,34,37-dodecazacyclononatriacontane-3-carboxamide |
| **40** | 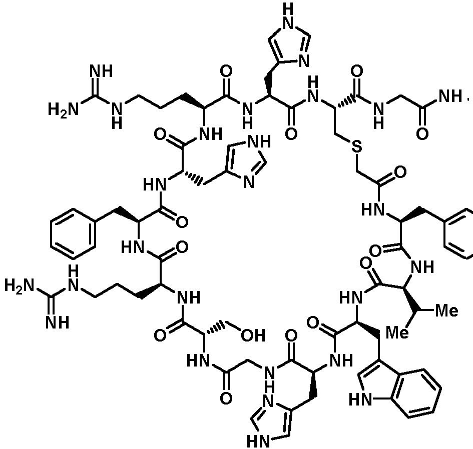 | (3R,6S,9S,12S,15S,18S,21S,27S,30S,33S,36S)-N-(2-amino-2-oxo-ethyl)-15,36-dibenzyl-9,18-bis(3-guanidinopropyl)-21-(hydroxymethyl)-6,12,27-tris(1H-imidazol-4-ylmethyl)-30-(1H-indol-3-ylmethyl)-33-isopropyl-5,8,11,14,17,20,23,26,29,32,35,38-dodecaoxo-1-thia-4,7,10,13,16,19,22,25,28,31,34,37-dodecazacyclononatriacontane-3-carboxamide |
| **41** | 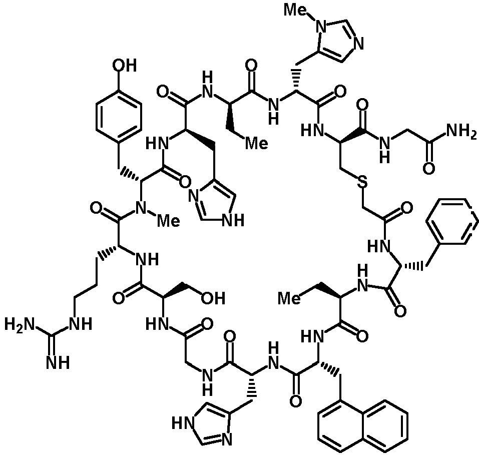 | (3R,6S,9S,12S,15S,18S,21S,27S,30S,33S,36S)-N-(2-amino-2-oxo-ethyl)-36-benzyl-9,33-diethyl-18-(3-guanidinopropyl)-21-(hydroxymethyl)-15-[(4-hydroxyphenyl)methyl]-12,27-bis(1H-imidazol-4-ylmethyl)-16-methyl-6-[(3-methylimidazol-4-yl)methyl]-30-(1-naphthylmethyl)-5,8,11,14,17,20,23,26,29,32,35,38-dodecaoxo-1-thia-4,7,10,13,16,19,22,25,28,31,34,37-dodecazacyclononatriacontane-3-carboxamide |
| **42** | 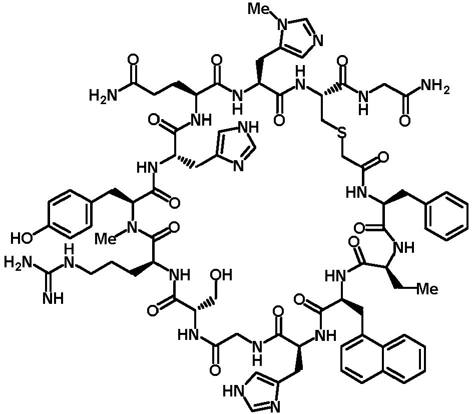 | rac-(3R,6S,9S,12S,15S,18S,21S,27S,30S,33S,36S)-N-(2-amino-2-oxo-ethyl)-9-(3-amino-3-oxopropyl)-36-benzyl-33-ethyl-18-(3-guanidinopropyl)-21-(hydroxymethyl)-15-[(4-hydroxyphenyl)methyl]-12,27-bis(1H-imidazol-4-ylmethyl)-16-methyl-6-[(3-methylimidazol-4-yl)methyl]-30-(1-naphthylmethyl)-5,8,11,14,17,20,23,26,29,32,35,38-dodecaoxo-1-thia-4,7,10,13,16,19,22,25,28,31,34,37-dodecazacyclononatriacontane-3-carboxamide |
| **43** | 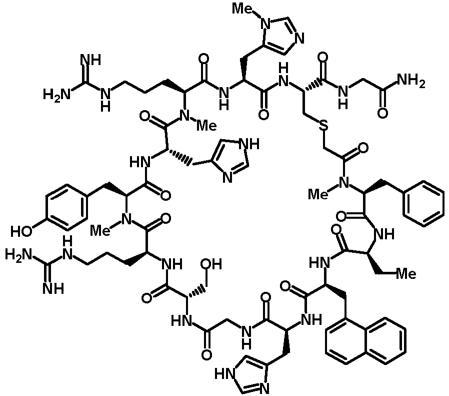 | ac-(3R,6S,9S,12S,15S,18S,21S,27S,30S,33S,36S)-N-(2-amino-2-oxo-ethyl)-36-benzyl-33-ethyl-9,18-bis(3-guanidinopropyl)-21-(hydroxymethyl)-15-[(4-hydroxyphenyl)methyl]-12,27-bis(1H-imidazol-4-ylmethyl)-10,16,37-trimethyl-6-[(3-methylimidazol-4-yl)methyl]-30-(1-naphthylmethyl)-5,8,11,14,17,20,23,26,29,32,35,38-dodecaoxo-1-thia-4,7,10,13,16,19,22,25,28,31,34,37-dodecazacyclononatriacontane-3-carboxamide |
| **44** | 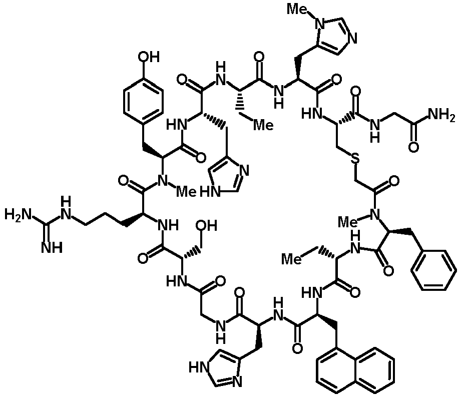 | rac-(3R,6S,9S, 12S, 15S, 18S, 21S,27S,30S,33S,36S)-N-(2-amino-2-oxo-ethyl)-36-benzyl-9,33-diethyl-18-(3-guanidinopropyl)-21-(hydroxymethyl)-15-[(4-hydroxyphenyl)methyl]-12,27-bis(1H-imidazol-4-ylmethyl)-16,37-dimethyl-6-[(3-methylimidazol-4-yl)methyl]-30-(1-naphthylmethyl)-5,8,11,14,17,20,23,26,29,32,35,38-dodecaoxo-1-thia-4,7,10,13,16,19,22,25,28,31,34,37-dodecazacyclononatriacontane-3-carboxamide |
| **45** | 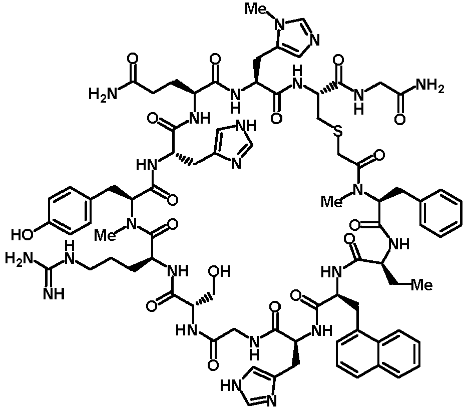 | rac-(3R,6S,9S,12S,15S,18S,21S,27S,30S,33S,36S)-N-(2-amino-2-oxo-ethyl)-9-(3-amino-3-oxopropyl)-36-benzyl-33-ethyl-18-(3-guanidinopropyl)-21-(hydroxymethyl)-15-[(4-hydroxyphenyl)methyl]-12,27-bis(1H-imidazol-4-ylmethyl)-16,37-dimethyl-6-[(3-methylimidazol-4-yl)methyl]-30-(1-naphthylmethyl)-5,8,11,14,17,20,23,26,29,32,35,38-dodecaoxo-1-thia-4,7,10,13,16,19,22,25,28,31,34,37-dodecazacyclononatriacontane-3-carboxamide |
| **46** | 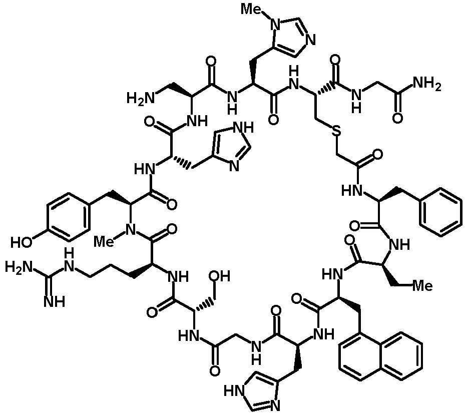 | rac-(3R,6S,9S,12S,15S,18S,21S,27S,30S,33S,36S)-9-(aminomethyl)-N-(2-amino-2-oxo-ethyl)-36-benzyl-33-ethyl-18-(3-guanidinopropyl)-21-(hydroxymethyl)-15-[(4-hydroxyphenyl)methyl]-12,27-bis(1H-imidazol-4-ylmethyl)-16-methyl-6-[(3-methylimidazol-4-yl)methyl]-30-(1-naphthylmethyl)-5,8,11,14,17,20,23,26,29,32,35,38-dodecaoxo-1-thia-4,7,10,13,16,19,22,25,28,31,34,37-dodecazacyclononatriacontane-3-carboxamide |
| **47** | 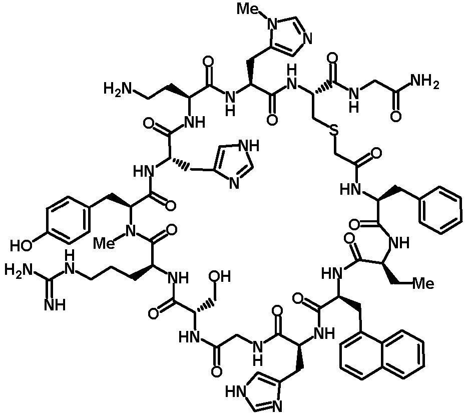 | rac-(3R,6S,9S, 12S, 15S, 18S, 21S,27S,30S,33S,36S)-9-(2-aminoethyl)-N-(2-amino-2-oxo-ethyl)-36-benzyl-33-ethyl-18-(3-guanidinopropyl)-21-(hydroxymethyl)-15-[(4-hydroxyphenyl)methyl]-12,27-bis(1H-imidazol-4-ylmethyl)-16-methyl-6-[(3-methylimidazol-4-yl)methyl]-30-(1-naphthylmethyl)-5,8,11,14,17,20,23,26,29,32,35,38-dodecaoxo-1-thia-4,7,10,13,16,19,22,25,28,31,34,37-dodecazacyclononatriacontane-3-carboxamide |
| 48 | 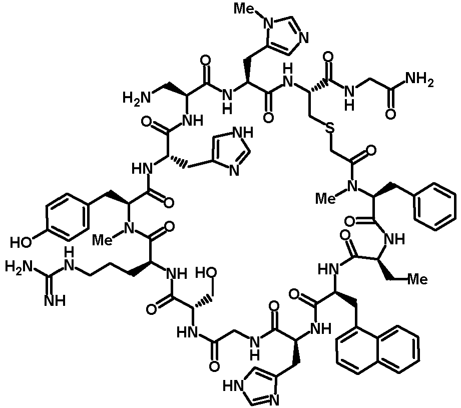 | rac-(3R,6S,9S,12S,15S,18S,21S,27S,30S,33S,36S)-9-(aminomethyl)-N-(2-amino-2-oxo-ethyl)-36-benzyl-33-ethyl-18-(3-guanidinopropyl)-21-(hydroxymethyl)-15-[(4-hydroxyphenyl)methyl]-12,27-bis(1H-imidazol-4-ylmethyl)-16,37-dimethyl-6-[(3-methylimidazol-4-yl)methyl]-30-(1-naphthylmethyl)-5,8,11,14,17,20,23,26,29,32,35,38-dodecaoxo-1-thia-4,7,10,13,16,19,22,25,28,31,34,37-dodecazacyclononatriacontane-3-carboxamide |
| 49 | 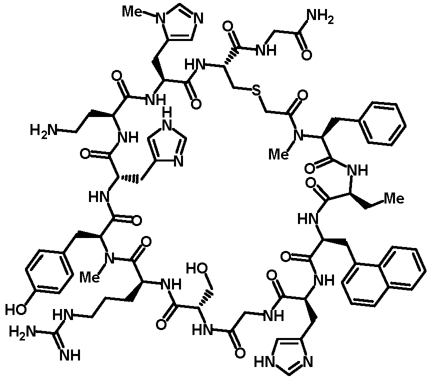 | rac-(3R,6S,9S,12S,15S,18S,21S,27S,30S,33S,36S)-9-(2-aminoethyl)-N-(2-amino-2-oxo-ethyl)-36-benzyl-33-ethyl-18-(3-guanidinopropyl)-21-(hydroxymethyl)-15-[(4-hydroxyphenyl)methyl]-12,27-bis(1H-imidazol-4-ylmethyl)-16,37-dimethyl-6-[(3-methylimidazol-4-yl)methyl]-30-(1-naphthylmethyl)-5,8,11,14,17,20,23,26,29,32,35,38-dodecaoxo-1-thia-4,7,10,13,16,19,22,25,28,31,34,37-dodecazacyclononatriacontane-3-carboxamide |
| 50 | 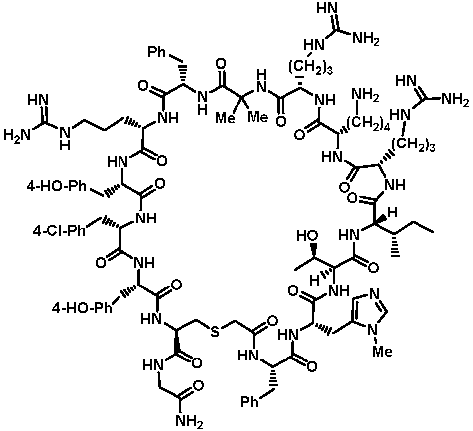 | rac-(3R,6S,9S,12S,15S,18S,21S,24S,27S,30S,33S,3 6S,42S)-27-(4-aminobutyl)-N-(2-amino-2-oxo-ethyl)-6,18,42-tribenzyl-15,21,24-tris(3-guanidinopropyl)-12-[(4-hydroxyphenyl)methyl]-30-(1H-imidazol-5-ylmethyl)-9-(1H-indol-3-ylmethyl)-5,8,11,14,17,20,23,26,29,32,35,38,41,44-tetradecaoxo-36-[rac-(1R)-1-hydroxyethyl]-33-[rac-(1S)-1-methylpropyl]-1-thia-4,7,10,13,16,19,22,25,28,31,34,37,40,43-tetradecazacyclopentatetracontane-3-carboxamide |
| 51 | 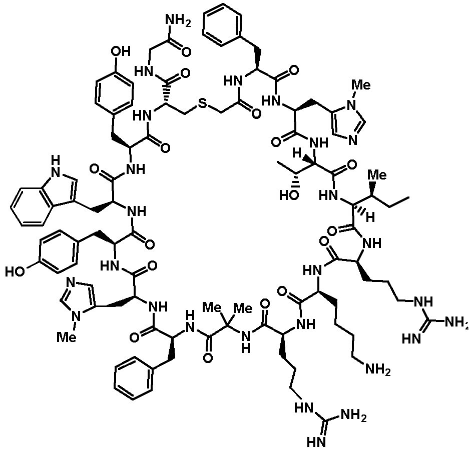 | (3R,6S,9S,12S,15S,18S,24S,27S,30S,33S,36S,3 9S,42S)-27-(4-aminobutyl)-N-(2-amino-2-oxo-ethyl)-18,42-dibenzyl-24,30-bis(3-guanidinopropyl)-36-[(1R)-1-hydroxyethyl]-6,12-bis[(4-hydroxyphenyl)methyl]-9-(1H-indol-3-ylmethyl)-21,21-dimethyl-15,39-bis[(3-methylimidazol-4-yl)methyl]-33-[(1S)-1-methylpropyl]-5,8,11,14,17,20,23,26,29,32,35,38,41,44-tetradecaoxo-1-thia-4,7,10,13,16,19,22,25,28,31,34,37,40,43-tetradecazacyclopentatetracontane-3-carboxamide |
| 52 | 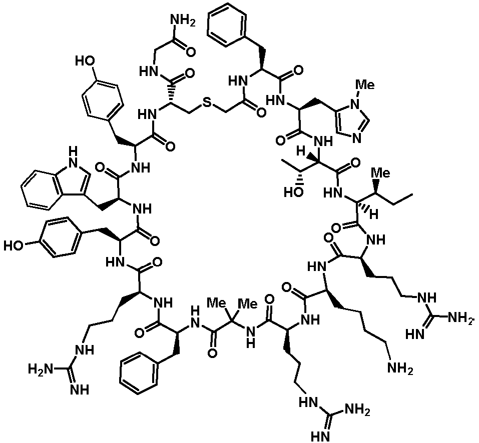 | (3R,6S,9S,12S,15S,18S,24S,27S,30S,33S,36S,3 9S,42S)-27-(4-aminobutyl)-N-(2-amino-2-oxo-ethyl)-18,42-dibenzyl-15,24,30-tris(3-guanidinopropyl)-36-[(1R)-1-hydroxyethyl]-6,12-bis[(4-hydroxyphenyl)methyl]-9-(1H-indol-3-ylmethyl)-21,21-dimethyl-39-[(3-methylimidazol-4-yl)methyl]-33-[(1S)-1-methylpropyl]-5,8,11,14,17,20,23,26,29,32,35,38,41,44-tetradecaoxo-1-thia-4,7,10,13,16,19,22,25,28,31,34,37,40,43-tetradecazacyclopentatetracontane-3-carboxamide |
| 53 | 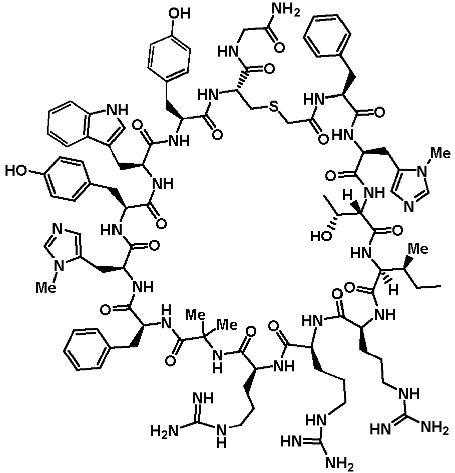 | (3R,6S,9S,12S,15S,18S,24S,27S,30S,33S,36S,3 9S,42S)-N-(2-amino-2-oxo-ethyl)-18,42-dibenzyl-24,27,30-tris(3-guanidinopropyl)-36-[(1R)-1-hydroxyethyl]-6,12-bis[(4-hydroxyphenyl)methyl]-9-(1H-indol-3-ylmethyl)-21,21-dimethyl-15,39-bis[(3-methylimidazol-4-yl)methyl]-33-[(1S)-1-methylpropyl]-5,8,11,14,17,20,23,26,29,32,35,38,41,44-tetradecaoxo-1-thia-4,7,10,13,16,19,22,25,28,31,34,37,40,43-tetradecazacyclopentatetracontane-3-carboxamide |
| 54 | 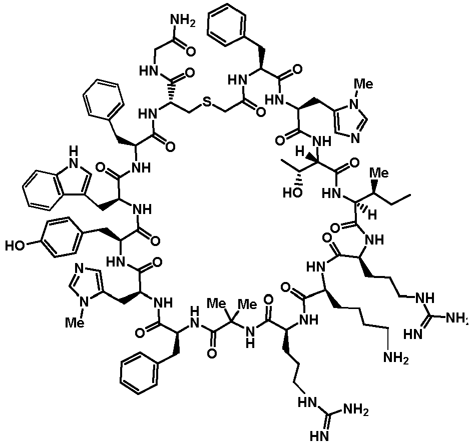 | (3R,6S,9S,12S,15S,18S, 24S,27S,30S,33S,36S,3 95,42S)-27-(4-aminobutyl)-N-(2-amino-2-oxo-ethyl)-6,18,42-tribenzyl-24,30-bis(3-guanidinopropyl)-36-[(1R)-1-hydroxyethyl]-12-[(4-hydroxyphenyl)methyl]-9-(1H-indol-3-ylmethyl)-21,21-dimethyl-15,39-bis[(3-methylimidazol-4-yl)methyl]-33-[(1S)-1-methylpropyl]-5,8,11,14,17,20,23,26,29,32,35,38,41,44-tetradecaoxo-1-thia-4,7,10,13,16,19,22,25,28,31,34,37,40,43-tetradecazacyclopentatetracontane-3-carboxamide;2,2,2-trifluoroacetic acid |
| 55 | 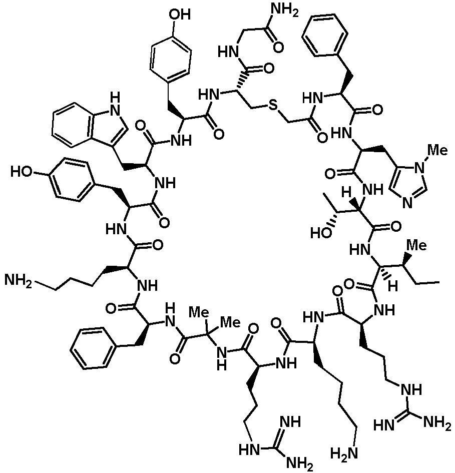 | (3R,6S,9S,12S,15S,18S,24S,27S,30S,33S,36S,3 9S,42S)-15,27-bis(4-aminobutyl)-N-(2-amino-2-oxo-ethyl)-18,42-dibenzyl-24,30-bis(3-guanidinopropyl)-36-[(1R)-1-hydroxyethyl]-6,12-bis[(4-hydroxyphenyl)methyl]-9-(1H-indol-3-ylmethyl)-21,21-dimethyl-39-[(3-methylimidazol-4-yl)methyl]-33-[(1S)-1-methylpropyl]-5,8,11,14,17,20,23,26,29,32,35,38,41,44-tetradecaoxo-1-thia-4,7,10,13,16,19,22,25,28,31,34,37,40,43-tetradecazacyclopentatetracontane-3-carboxamide |
| 56 | 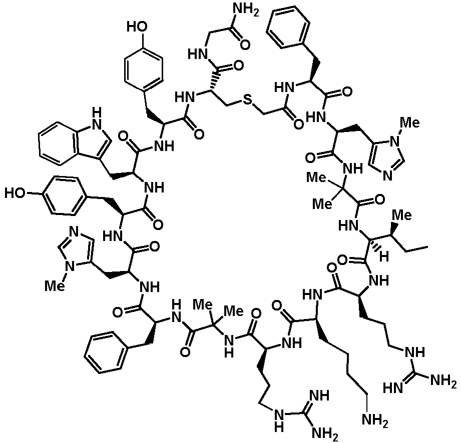 | (3R,6S,9S,12S,15S,18S,24S,27S,30S,33S,39S,4 2S)-27-(4-aminobutyl)-N-(2-amino-2-oxoethyl)-18,42-dibenzyl-24,30-bis(3-guanidinopropyl)-6,12-bis[(4-hydroxyphenyl)methyl]-9-(1H-indol-3-ylmethyl)-21,21,36,36-tetramethyl-15,39-bis[(3-methylimidazol-4-yl)methyl]-33-[(1S)-1-methylpropyl]-5,8,11,14,17,20,23,26,29,32,35,38,41,44-tetradecaoxo-1-thia-4,7,10,13,16,19,22,25,28,31,34,37,40,43-tetradecazacyclopentatetracontane-3-carboxamide |
| 57 | 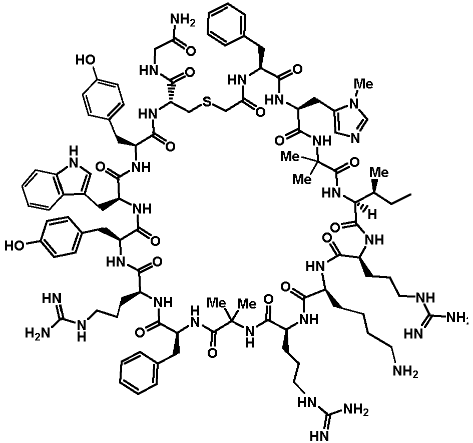 | (3R,6S,9S,12S,15S,18S,24S,27S,30S,33S,39S,4 2S)-27-(4-aminobutyl)-N-(2-amino-2-oxoethyl)-18,42-dibenzyl-15,24,30-tris(3-guanidinopropyl)-6,12-bis[(4-hydroxyphenyl)methyl]-9-(1H-indol-3-ylmethyl)-21,21,36,36-tetramethyl-39-[(3-methylimidazol-4-yl)methyl]-33-[(1S)-1-methylpropyl]-5,8,11,14,17,20,23,26,29,32,35,38,41,44-tetradecaoxo-1-thia-4,7,10,13,16,19,22,25,28,31,34,37,40,43-tetradecazacyclopentatetracontane-3-carboxamide |
| 58 | 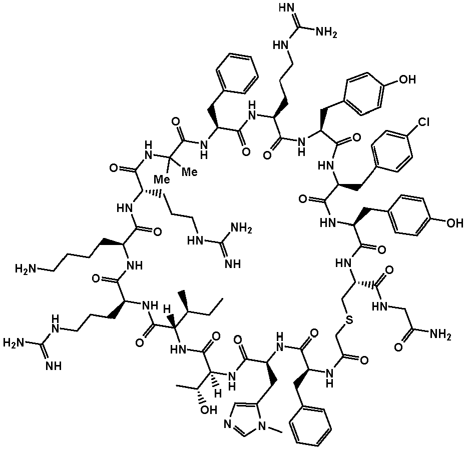 | (3R,6S,9S,12S,15S,18S,24S,27S,30S,33S,36S,3 9S,42S)-27-(4-aminobutyl)-N-(2-amino-2-oxo-ethyl)-18,42-dibenzyl-9-[(4-chlorophenyl)methyl]-15,24,30-tris(3-guanidinopropyl)-36-[(1R)-1-hydroxyethyl]-6,12-bis[(4-hydroxyphenyl)methyl]-21,21-dimethyl-39-[(3-methylimidazol-4-yl)methyl]-33-[(1S)-1-methylpropyl]-5,8,11,14,17,20,23,26,29,32,35,38,41,44-tetradecaoxo-1-thia-4,7,10,13,16,19,22,25,28,31,34,37,40,43-tetradecazacyclopentatetracontane-3-carboxamide |
| 59 | 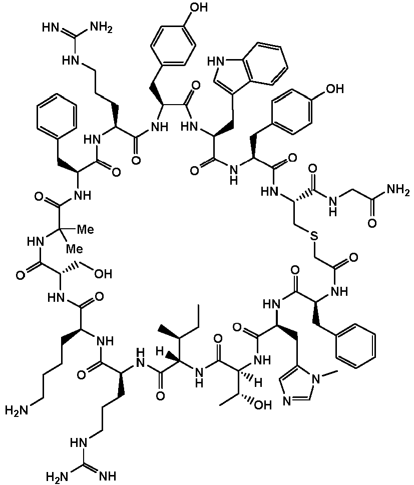 | rac-(3R,6S,9S,12S,15S,18S,24S,27S,30S,33S,36S,3 9S,42S)-27-(4-aminobutyl)-N-(2-amino-2-oxo-ethyl)-18,42-dibenzyl-15,30-bis(3-guanidinopropyl)-24-(hydroxymethyl)-6,12-bis[(4-hydroxyphenyl)methyl]-9-(1H-indol-3-ylmethyl)-21,21-dimethyl-39-[(3-methylimidazol-4-yl)methyl]-5,8,11,14,17,20,23,26,29,32,35,38,41,44-tetradecaoxo-36-[rac-(1R)-1-hydroxyethyl]-33-[rac-(1S)-1-methylpropyl]-1-thia-4,7,10,13,16,19,22,25,28,31,34,37,40,43-tetradecazacyclopentatetracontane-3-carboxamide |
| 60 | 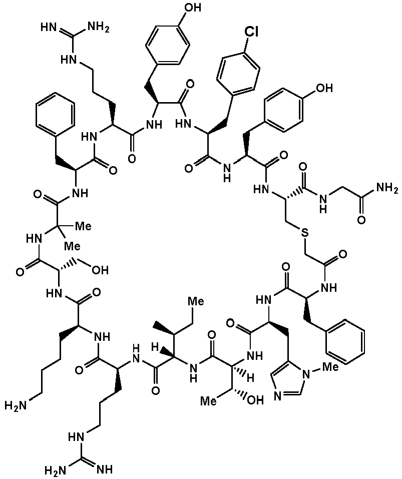 | (3R,6S,9S,12S,15S,18S,24S,27S,30S,33S,36S,3 9S,42S)-27-(4-aminobutyl)-N-(2-amino-2-oxo-ethyl)-18,42-dibenzyl-9-[(4-chlorophenyl)methyl]-15,30-bis(3-guanidinopropyl)-24-(hydroxymethyl)-6,12-bis[(4-hydroxyphenyl)methyl]-21,21-dimethyl-39-[(3-methylimidazol-4-yl)methyl]-5,8,11,14,17,20,23,26,29,32,35,38,41,44-tetradecaoxo-36-[rac-(1R)-1-hydroxyethyl]-33-[rac-(1S)-1-methylpropyl]-1-thia-4,7,10,13,16,19,22,25,28,31,34,37,40,43-tetradecazacyclopentatetracontane-3-carboxamide |
| 61 | 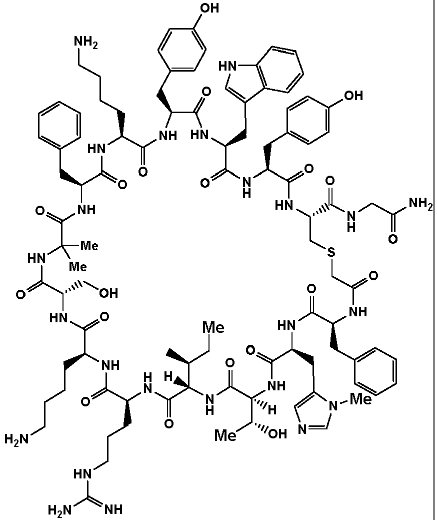 | rac-(3R,6S,9S,12S,15S,18S,24S,27S,30S,33S,36S,3 9S,42S)-15,27-bis(4-aminobutyl)-N-(2-amino-2-oxo-ethyl)-18,42-dibenzyl-30-(3-guanidinopropyl)-24-(hydroxymethyl)-6,12-bis[(4-hydroxyphenyl)methyl]-9-(1H-indol-3-ylmethyl)-21,21-dimethyl-39-[(3-methylimidazol-4-yl)methyl]-5,8,11,14,17,20,23,26,29,32,35,38,41,44-tetradecaoxo-36-[rac-(1R)-1-hydroxyethyl]-33-[rac-(1S)-1-methylpropyl]-1-thia-4,7,10,13,16,19,22,25,28,31,34,37,40,43-tetradecazacyclopentatetracontane-3-carboxamide |

13. A pharmaceutical composition comprising the compound of any one of claims 1 to 12, or a pharmaceutically acceptable salt, solvate, or stereoisomer thereof, and a pharmaceutically acceptable excipient.

14. A compound of any one of claims 1 to 12, or a pharmaceutically acceptable salt, solvate, or stereoisomer thereof, for use in the treatment of a bacterial infection in a mammal, comprising administering to the mammal an effective amount of said compound at a frequency and for a duration sufficient to provide a beneficial effect to the mammal.

15. The compound for use of claim 14 further comprising administering a second therapeutic agent, wherein said second therapeutic agent is an aminoglycoside antibiotic, fluoroquinolone antibiotic, β-lactam antibiotic, macrolide antibiotic, glycopeptide antibiotic, rifampicin, chloramphenicol, fluoramphenicol, colistin, mupirocin, bacitracin, daptomycin, or linezolid.

## Patentansprüche

1. Verbindung der Formel I 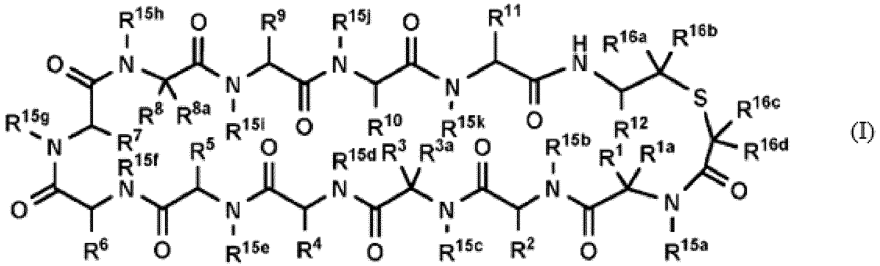
oder ein Stereoisomer oder ein pharmazeutisch unbedenkliches Salz davon, wobei:
R¹ C₁₋₆-Alkyl, Hydroxyl-C₁₋₆-alkyl oder gegebenenfalls mit Halogen oder Hydroxy substituiertes Benzyl ist;
R^{1a} Wasserstoff ist;
R² Wasserstoff, C₁₋₆-Alkyl oder gegebenenfalls substituiertes (Imidazol-4-yl)methyl ist;
R³ bicyclisches Aryl, bicyclisches Heteroaryl, C₁₋₆-Alkyl oder Hydroxyl-C₁₋₆-alkyl ist, wobei das bicyclische Aryl und bicyclische Heteroaryl gegebenenfalls einmal oder zweimal mit C₁₋₆-Alkyl, Hydroxyl oder Halogen substituiertes ist;
R^{3a} Wasserstoff oder C₁₋₆-Alkyl ist;
R⁴ C₁₋₆-Alkyl oder gegebenenfalls substituiertes (Imidazol-4-yl)methyl ist;
R⁵ Wasserstoff; C₁₋₆-Alkyl, Hydroxyl-C₁₋₆-alkyl, gegebenenfalls substituiertes (Imidazol-4-yl)methyl oder gegebenenfalls substituiertes Guanidino-C₂₋₄-alkyl ist;
R⁶ Hydroxyl-C₁₋₆-alkyl oder Amino-C₁₋₆-alkyl ist;
R⁷ C₁₋₆-Alkyl, Hydroxyl-C₁₋₆-alkyl oder gegebenenfalls substituiertes Guanidino-C₂₋₄-alkyl ist;
R⁸ gegebenenfalls substituiertes Benzyl ist;
R^{8a} Wasserstoff oder C₁₋₆-Alkyl ist;
R⁹ gegebenenfalls substituiertes Benzyl oder gegebenenfalls substituiertes 1H-Imidazol-5-ylmethyl ist;
R¹⁰ C₁₋₆-Alkyl, Amino-C₁₋₆-alkyl, gegebenenfalls substituiertes 1H-Imidazol-5-ylmethyl, gegebenenfalls substituiertes Guanidino-C₂₋₄-alkyl oder gegebenenfalls substituiertes C₁₋₂-Alkylcarboxamid ist;
R¹¹ gegebenenfalls substituiertes Benzyl oder gegebenenfalls substituiertes (Imidazol-4-yl)methyl ist;
R¹² N-(3-Amino-3-oxopropyl)carboxamid oder N-(2-Amino-2-oxoethyl)carboxamid ist, die beide gegebenenfalls substituiert sind;
R^{b}, R^{c}, R^{d}, R^{e}, R^{f}, R^{15a}, R^{15b}, R^{15c}, R^{15d}, R^{15f}, R^{15g}, R^{15h}, R¹⁵ⁱ, R^{15j}, R^{15k}, R^{16a}, R^{16b},
R^{16c} und R^{16d} jeweils unabhängig voneinander Wasserstoff oder C₁₋₆-Alkyl sind;
R^{15e} Wasserstoff, C₁₋₆-Alkyl, Amino-C₁₋₆-alkyl oder gegebenenfalls substituiertes C₁₋₂-Alkylcarboxamid ist;
R^{a} Wasserstoff, C₁₋₆-Alkyl, Hydroxyl oder Halogen ist;
*n* und *p* unabhängig voneinander 0 bis 2 sind;
q 0 oder 1 ist und
r 1 oder 2 ist; oder
Verbindung der Formel (II) 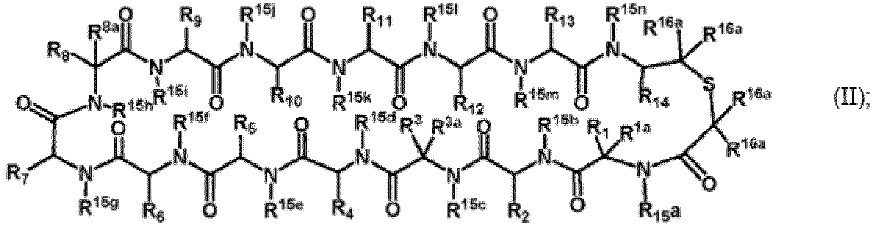
oder ein Stereoisomer oder ein pharmazeutisch unbedenkliches Salz davon, wobei:
R¹ gegebenenfalls substituiertes Benzyl ist;
R^{1a} Wasserstoff oder C₁₋₆-Alkyl ist;
R² Wasserstoff oder gegebenenfalls substituiertes (Imidazol-4-yl)methyl ist;
R³ C₁₋₆-Alkyl oder Hydroxyl-C₁₋₆-alkyl ist;
R^{3a} Wasserstoff oder C₁₋₆-Alkyl ist;
R⁴ C₁₋₆-Alkyl oder gegebenenfalls substituiertes (Imidazol-4-yl)methyl ist;
R⁵ gegebenenfalls substituiertes Guanidino-C₂₋₄-alkyl oder gegebenenfalls substituiertes (Imidazol-4-yl)methyl ist;
R⁶ Amino-C₁₋₆-alkyl oder gegebenenfalls substituiertes Guanidino-C₂₋₄-alkyl ist;
R⁷ Hydroxyl-C₁₋₆-alkyl oder gegebenenfalls substituiertes Guanidino-C₂₋₄-alkyl ist;
R⁸ Methyl oder 3-Propylguanidin ist;
R^{8a} Wasserstoff oder Methyl ist;
R⁹ gegebenenfalls substituiertes Benzyl ist;
R¹⁰ Amino-C₁₋₆-alkyl, gegebenenfalls substituiertes (Imidazol-4-yl)methyl oder gegebenenfalls substituiertes Guanidino-C₂₋₄-alkyl ist;
R¹¹ gegebenenfalls substituiertes Benzyl ist;
R¹² Indolyl-3-methyl oder gegebenenfalls substituiertes Benzyl ist;
R¹³ gegebenenfalls substituiertes Benzyl ist;
R¹⁴ N-(3-Amino-3-oxopropyl)carboxamid oder N-(2-Amino-2-oxoethyl)carboxamid ist, die beide gegebenenfalls substituiert sind;
R^{b}, R^{c}, R^{d}, R^{e}, R^{f}, R^{15a}, R^{15b}, R^{15c}, R^{15d}, R^{15e}, R^{15f}, R^{15g}, R^{15h}, R¹⁵ⁱ, R^{15j}, R^{15k}, R^{15l}, R^{15m}, R¹⁵ⁿ, R^{16a}, R^{16b}, R^{16c} und R^{16d} bei jedem Auftreten unabhängig voneinander Wasserstoff oder C₁₋₆-Alkyl sind;
R^{a} Wasserstoff, C₁₋₆-Alkyl, Hydroxyl oder Halogen ist;
n und p jeweils unabhängig voneinander 0 bis 2 sind und
q 0 oder 1 ist.

2. Verbindung nach Anspruch 1, wobei die Verbindung die Formel I aufweist und R¹² N-(2-Amino-2-oxoethyl)carboxamid ist, R⁴ und R⁹ 1H-Imidazol-5-ylmethyl sind und R² C₁₋₆-Alkyl ist.

3. Verbindung nach Anspruch 1 oder 2, wobei die Verbindung die Formel I aufweist und wobei:
R¹ C₁₋₆-Alkyl, Hydroxyl-C₁₋₆-alkyl oder gegebenenfalls substituiertes Benzyl ist;
R³ 1-Naphthylmethyl, 2-Naphthylmethyl, (4-Phenylphenyl)methyl oder gegebenenfalls substituiertes 1H-Indolyl-3-methyl ist;
R⁵ Wasserstoff oder 3-Guanidopropyl ist;
R⁶ Hydroxyl-C₁₋₆-alkyl oder 4-Aminobutyl ist;
R⁷ 3-Guanidopropyl ist;
R¹⁰ 3-Guanidinopropyl, Methyl, Ethyl, Aminomethyl, Aminoethyl, 4-Aminobutyl, 3-Methylimidazol-4-yl)methyl oder 3-Amino-3-oxopropyl ist;
R¹¹ 1H-Imidazol-5-ylmethyl oder 3-Methylimidazol-4-yl)methyl ist;
R^{b}, R^{c}, R^{e}, R^{1a}, R^{3a}, R^{8a}, R^{15b}, R^{15c}, R^{15d}, R^{15f}, R^{15g}, R¹⁵ⁱ, R^{15k}, R^{16a}, R^{16b}, R^{16c} und
R^{16d} Wasserstoff sind;
R^{15a}, R^{15e}, R^{15h} und R^{15j} unabhängig voneinander Wasserstoff oder Methyl sind;
R^{b} und R^{f} unabhängig voneinander Wasserstoff oder Methyl sind;
R^{15h} Methyl ist;
oder ein Stereoisomer oder ein pharmazeutisch unbedenkliches Salz davon.

4. Verbindung nach einem der Ansprüche 1 bis 3, wobei die Verbindung die Formel I aufweist und wobei:
R¹ gegebenenfalls mit Halogen oder Hydroxy substituiertes Benzyl ist;
R² Wasserstoff, Ethyl, Isopropyl oder (1S)-1-Methylpropyl ist;
R³ 1-Naphthylmethyl oder 1H-Indol-3-methyl ist;
R⁴ 1H-Imidazol-5-ylmethyl ist;
R⁵ Wasserstoff ist;
R⁶ Hydroxymethyl oder (1R)-1-Hydroxyethyl ist;
R⁷ 3-Guanidinopropyl ist;
R⁸ Benzyl oder 4-Hydroxybenzyl ist;
R⁹ 1H-Imidazol-5-ylmethyl ist;
R¹⁰ 3-Guanidopropyl, Methyl oder Ethyl ist;
R¹¹ 1H-Imidazol-5-ylmethyl, 3-Methylimidazol-4-yl)methyl ist;
R¹² -C(=O)NHCH₂C(=O)NH₂ ist;
oder ein Stereoisomer oder ein pharmazeutisch unbedenkliches Salz davon.

5. Verbindung nach einem der Ansprüche 1 bis 4, wobei die Verbindung die Formel I aufweist und wobei:
R¹ Benzyl ist;
R² Ethyl oder Isopropyl ist;
R³ 1-Naphthylmethyl ist;
R⁴ 1H-Imidazol-5-ylmethyl ist;
R⁵ Wasserstoff ist;
R⁶ Hydroxylmethyl ist;
R⁷ 3-Guanidopropyl ist;
R⁸ 4-Hydroxylbenzyl oder 4-Chlorbenzyl ist;
R⁹ 1-Methyl-1H-imidazol-5-methylen ist;
R¹⁰ 3-Guanidopropyl ist;
*R*¹¹ (3-Methylimidazol-4-yl)methyl ist;
R¹² N-(2-Amino-2-oxoethyl)carboxamid ist;
R^{b} und R^{f} unabhängig voneinander bei jedem Auftreten Wasserstoff oder Methyl sind;
oder ein Stereoisomer oder ein pharmazeutisch unbedenkliches Salz davon.

6. Verbindung nach einem der Ansprüche 1 bis 5, wobei die Verbindung die Formel Ia aufweist: 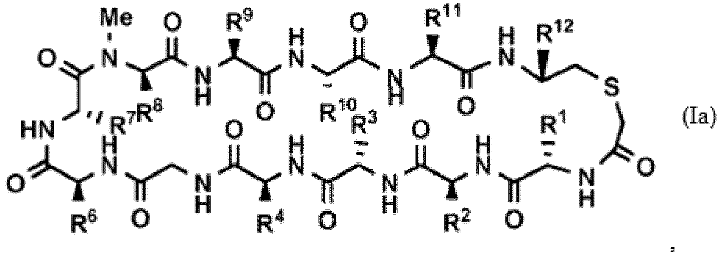 oder ein Stereoisomer oder ein pharmazeutisch unbedenkliches Salz davon.

7. Verbindung nach Anspruch 1, wobei die Verbindung die Formel II aufweist und wobei:
R¹ gegebenenfalls substituiertes Benzyl ist;
R^{1a} Wasserstoff oder C₁₋₆-Alkyl ist;
R² (3-Methylimidazol-4-yl)methyl ist;
R³ C₁₋₆-Alkyl oder Hydroxyl-C₁₋₆-alkyl ist;
R^{3a} Wasserstoff oder C₁₋₆-Alkyl ist;
R⁴ C₁₋₆-Alkyl ist;
R⁵ 3-Guanidinopropyl ist;
R⁶ 3-Guanidinopropyl oder Amino-C₁₋₆-alkyl ist;
R⁷ 3-Guanidinopropyl oder Hydroxyl-C₁₋₆-alkyl ist;
R⁸ gegebenenfalls substituiertes Benzyl ist;
R^{8a} Methyl ist;
R⁹ gegebenenfalls substituiertes Benzyl ist;
R¹⁰ gegebenenfalls substituiertes Guanidino-C₂₋₄-alkyl, (3-Methylimidazol-4-yl)methyl oder Amino-C₁₋₆-alkyl ist;
R¹¹ 4-Hydroxybenzyl oder 4-Chlorbenzyl ist;
R¹² 1H-Indol-3-methyl oder 4-Chlorbenzyl ist;
R¹³ gegebenenfalls substituiertes Benzyl ist;
R¹⁴ -C(=O)NHCH₂C(=O)NH₂ ist;
R^{15b}, R^{15b}, R^{15c}, R^{15d}, R^{15e}, R^{15f}, R^{15g}, R^{15h}, R¹⁵ⁱ, R^{15j}, R^{15k}, R^{15l}, R^{15m}, R¹⁵ⁿ, R^{16a},
R^{16b}, R^{16c} und R^{16d} Wasserstoff sind;
oder ein Stereoisomer oder ein pharmazeutisch unbedenkliches Salz davon.

8. Verbindung nach einem der Ansprüche 1 oder 7, wobei die Verbindung die Formel II aufweist und wobei:
R¹ Benzyl ist;
R^{1a} Wasserstoff ist;
R² (3-Methylimidazol-4-yl)methyl ist;
R³ Methyl oder (1R)-1-Hydroxyethyl ist;
R^{3a} Wasserstoff oder Methyl ist;
R⁴ (1S)-1-Methylpropyl ist;
R⁵ 3-Guanidopropyl ist;
R⁶ 4-Aminobutyl ist;
R⁷ 3-Guanidopropyl oder (1R)-1-Hydroxyethyl ist;
R⁸ und R^{8a} Methyl sind;
R⁹ Benzyl ist;
R¹⁰ Guanidinopropyl, (3-Methylimidazol-4-yl)methyl oder Amino-C₁₋₆-alkyl ist;
R¹¹ 4-Hydroxybenzyl ist;
R¹² 1H-Indol-3-methyl ist;
R¹³ 4-Hydroxybenzyl ist;
R¹⁴ -C(=O)NHCH₂C(=O)NH₂ ist;
R^{15a}, R^{15b}, R^{15c}, R^{15d},R^{15e},R^{15f}, R^{15g}, R^{15h}, R¹⁵ⁱ, R^{15j}, R^{15k}, R^{15l}, R^{15m}, R¹⁵ⁿ, R^{16a}, R^{16b},
R^{16c} und R^{16d} Wasserstoff sind;
oder ein Stereoisomer oder ein pharmazeutisch unbedenkliches Salz davon.

9. Verbindung nach Anspruch 1, 7 oder 8, wobei die Verbindung die Formel II aufweist und wobei:
R¹ Benzyl ist;
R^{1a} Wasserstoff ist;
R² (3-Methylimidazol-4-yl)methyl ist;
R³ Methyl oder (1R)-1-Hydroxyethyl ist;
R⁴ (1S)-1-Methylpropyl ist;
R⁵ 3-Guanidinopropyl ist;
R⁶ 4-Aminobutyl ist;
R⁷ *rac*-(1R)-1-Hydroxyethyl ist;
R⁸ und R^{8a} Methyl sind;
R⁹ Benzyl ist;
R¹⁰ 3-Guanidinopropyl, (3-Methylimidazol-4-yl)methyl, Aminomethyl, 2-Aminoethyl, 3-Aminopropyl oder 4-Aminobutyl ist;
R¹¹ 4-Hydroxybenzyl ist;
R¹² 1H-Indol-3-methyl ist;
R¹³ 4-Hydroxybenzyl ist;
R¹⁴ -C(=O)NHCH₂C(=O)NH ist.

10. Verbindung nach Anspruch 1 oder nach einem der Ansprüche 7 bis 9, wobei die Verbindung die Formel II aufweist und wobei:
R¹ Benzyl ist;
R^{1a} Wasserstoff ist;
R² (3-Methylimidazol-4-yl)methyl ist;
R³ Methyl oder (1R)-1-Hydroxyethyl ist;
R⁴ (1S)-1-Methylpropyl ist;
R⁵ 3-Guanidinopropyl ist;
R⁶ 4-Aminobutyl ist;
R⁷ *rac*-(1R)-1-Hydroxyethyl ist;
R⁸ und R^{8a} Methyl sind;
R⁹ Benzyl ist;
R¹⁰ 3-Guanidinopropyl, (3-Methylimidazol-4-yl)methyl, 3-Aminopropyl oder 4-Aminobutyl ist;
R¹¹ 4-Hydroxybenzyl ist;
R¹² 1H-Indol-3-methyl ist;
R¹³ 4-Hydroxybenzyl ist;
R¹⁴ -C(=O)NHCH₂C(=O)NH ist;
oder ein Stereoisomer oder ein pharmazeutisch unbedenkliches Salz davon.

11. Verbindung nach Anspruch 1 oder nach einem der Ansprüche 7 bis 10, wobei die Verbindung die Formel IIa aufweist:

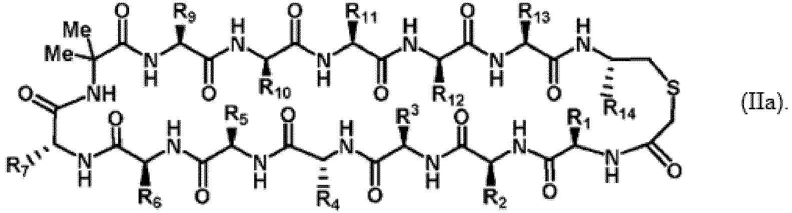

12. Verbindung nach Anspruch 1, wobei die Verbindung aus Folgenden ausgewählt ist:
| # | Struktur | Bezeichnung der Verbindung |
|---|---|---|
| 1 | 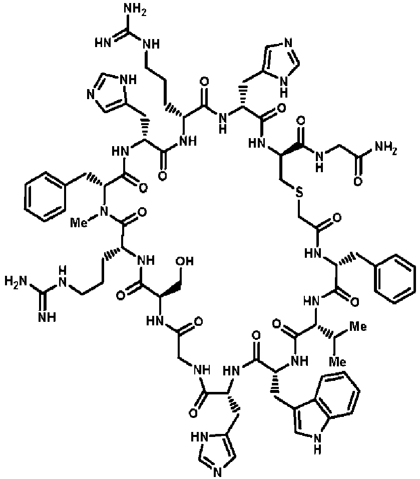 | rac-(3R,6S,9S,12S,15S,18S,21S,27S,30S, 33S,36S)-N-(2-Amino-2-oxoethyl)-15,36-dibenzyl-9,18-bis(3-guanidinopropyl)-21-(hydroxymethyl)-6,12,27-tris(1H-imidazol-5-ylmethyl)-30-(1H-indol-3-ylmethyl)-33-isopropyl-16-methyl-5,8,11,14,17,20,23,26,29,32,35,38-dodecaoxo-1-thia-4,7,10,13,16,19,22,25,28,31,34,37-dodecazacyclononatriacontan-3-carboxamid |
| 2 | 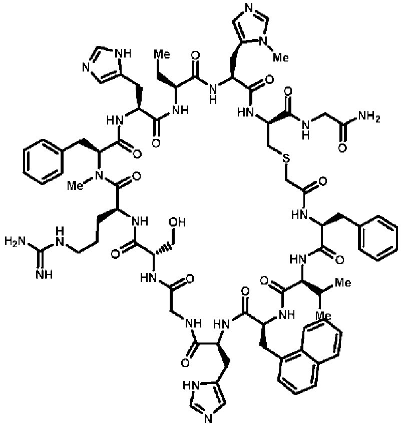 | (3R,6S,9S,12S,15S,18S,21S,27S,30S, 33 S,36S)-N-(2-Amino-2-oxoethyl)-15,36-dibenzyl-9-ethyl-18-(3-guanidinopropyl)-21-(hydroxymethyl)-12,27-bis(1H-imidazol-4-ylmethyl)-33-isopropyl-16-methyl-6-[(3-methylimidazol-4-yl)methyl]-30-(1-naphthylmethyl)-5,8,11,14,17,20,23,26,29,32,35,38-dodecaoxo-1-thia-4,7,10,13,16,19,22,25,28,31,34,37-dodecazacyclononatriacontan-3-carboxamid |
| 3 | 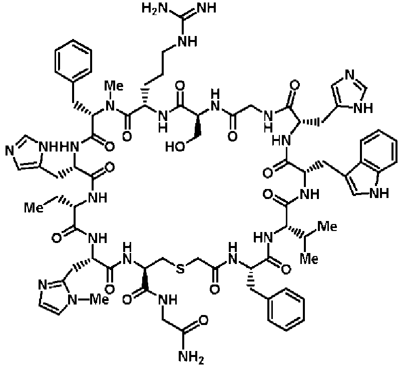 | (3R,6S,9S,12S,15S,18S,21S,27S,30S, 33 S,36S)-N-(2-Amino-2-oxoethyl)-15,36-dibenzyl-9-ethyl-18-(3-guanidinopropyl)-21-(hydroxymethyl)-12,27-bis(1H-imidazol-4-ylmethyl)-30-(1H-indol-3-ylmethyl)-33-isopropyl-16-methyl-6-[(3-methylimidazol-4-yl)methyl]-5,8,11,14,17,20,23,26,29,32,35,38-dodecaoxo-1-thia-4,7,10,13,16,19,22,25,28,31,34,37-dodecazacyclononatriacontan-3-carboxamid |
| 4 | 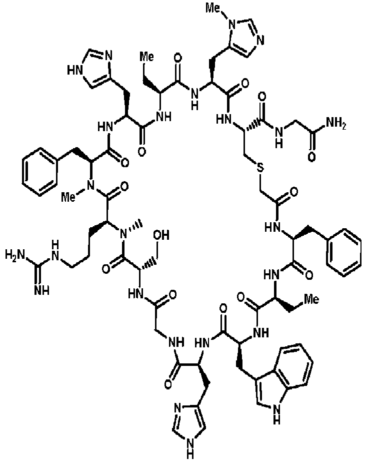 | (3R,6S,9S,12S,15S,18S,21S,27S,30S, 33 S,36S)-N-(2-Amino-2-oxoethyl)-15,36-dibenzyl-9,33-diethyl-18-(3-guanidinopropyl)-21-(hydroxymethyl)-12,27-bis(1H-imidazol-4-ylmethyl)-30-(1H-indol-3-ylmethyl)-16-methyl-6-[(3-methylimidazol-4-yl)methyl]-5,8,11,14,17,20,23,26,29,32,35,38-dodecaoxo-1-thia-4,7,10,13,16,19,22,25,28,31,34,37-dodecazacyclononatriacontan-3-carboxamid |
| 5 | 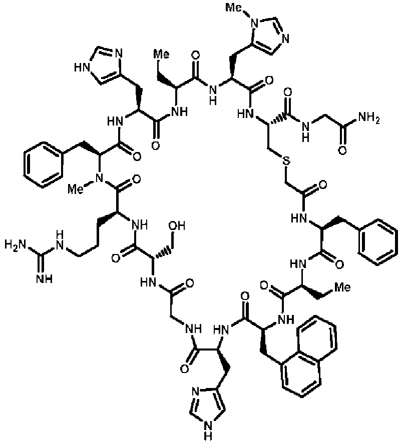 | (3R,6S,9S,12S,15S,18S,21S,27S,30S, 33 S,36S)-N-(2-Amino-2-oxoethyl)-15,36-dibenzyl-9,33-diethyl-18-(3-guanidinopropyl)-21-(hydroxymethyl)-12,27-bis(1H-imidazol-4-ylmethyl)-16-methyl-6-[(3-methylimidazol-4-yl)methyl]-30-(1-naphthylmethyl)-5,8,11,14,17,20,23,26,29,32,35,38-dodecaoxo-1-thia-4,7,10,13,16,19,22,25,28,31,34,37-dodecazacyclononatriacontan-3-carboxamid |
| 6 | 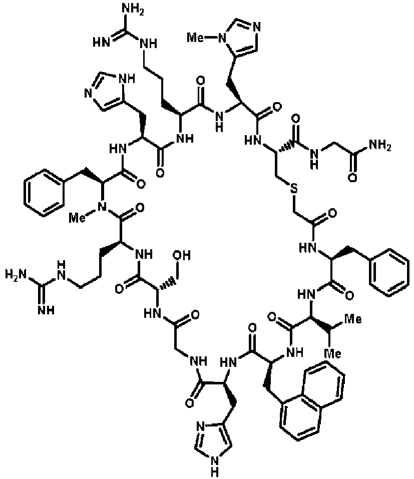 | (3R,6S,9S,12S,15S,18S,21S,27S,30S, 33 S,36S)-N-(2-Amino-2-oxoethyl)-15,36-dibenzyl-9,18-bis(3-guanidinopropyl)-21-(hydroxymethyl)-12,27-bis(1H-imidazol-4-ylmethyl)-33-isopropyl-16-methyl-6-[(3-methylimidazol-4-yl)methyl]-30-(1-naphthylmethyl)-5,8,11,14,17,20,23,26,29,32,35,38-dodecaoxo-1-thia-4,7,10,13,16,19,22,25,28,31,34,37-dodecazacyclononatriacontan-3-carboxamid |
| 7 | 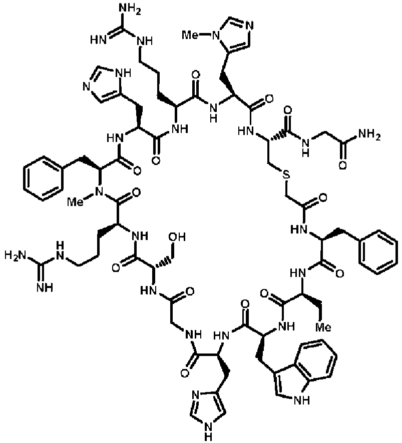 | (3R,6S,9S,12S,15S,18S,21S,27S,30S, 33 S,36S)-N-(2-Amino-2-oxoethyl)-15,36-dibenzyl-33-ethyl-9,18-bis(3-guanidinopropyl)-21-(hydroxymethyl)-12,27-bis(1H-imidazol-4-ylmethyl)-30-(1H-indol-3-ylmethyl)-16-methyl-6-[(3-methylimidazol-4-yl)methyl]-5,8,11,14,17,20,23,26,29,32,35,38-dodecaoxo-1-thia-4,7,10,13,16,19,22,25,28,31,34,37-dodecazacyclononatriacontan-3-carboxamid |
| 8 | 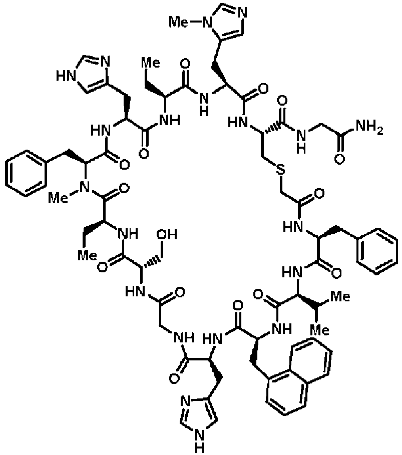 | (3R,6S,9S,12S,15S,18S,21S,27S,30S, 33 S,36S)-N-(2-Amino-2-oxoethyl)-15,36-dibenzyl-9,18-diethyl-21-(hydroxymethyl)-12,27-bis(1H-imidazol-4-ylmethyl)-33-isopropyl-16-methyl-6-[(3-methylimidazol-4-yl)methyl]-30-(1-naphthylmethyl)-5,8,11,14,17,20,23,26,29,32,35,38-dodecaoxo-1-thia-4,7,10,13,16,19,22,25,28,31,34,37-dodecazacyclononatriacontan-3-carboxamid |
| 9 | 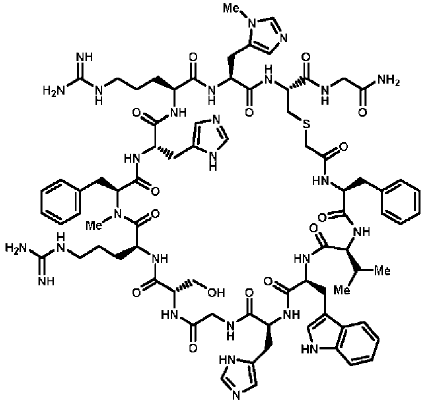 | rac-(3R,6S,9S,12S,15S,18S,21S,27S,30S, 33S,36S)-N-(2-Amino-2-oxoethyl)-15,36-dibenzyl-9,18-bis(3-guanidinopropyl)-21-(hydroxymethyl)-12,27-bis(1H-imidazol-5-ylmethyl)-30-(1H-indol-3-ylmethyl)-33-isopropyl-16-methyl-6-[(3-methylimidazol-4-yl)methyl]-5,8,11,14,17,20,23,26,29,32,35,38-dodecaoxo-1-thia-4,7,10,13,16,19,22,25,28,31,34,37-dodecazacyclononatriacontan-3-carboxamid |
| 10 | 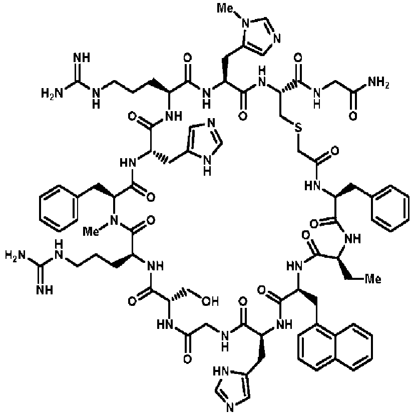 | rac-(3R,6S,9S,12S,15S,18S,21S,27S,30S, 33 S,36S)-N-(2-Amino-2-oxoethyl)-15,36-dibenzyl-33-ethyl-9,18-bis(3-guanidinopropyl)-21-(hydroxymethyl)-12,27-bis(1H-imidazol-5-ylmethyl)-16-methyl-6-[(3-methylimidazol-4-yl)methyl]-30-(1-naphthylmethyl)-5,8,11,14,17,20,23,26,29,32,35,38-dodecaoxo-1-thia-4,7,10,13,16,19,22,25,28,31,34,37-dodecazacyclononatriacontan-3-carboxamid |
| 11 | 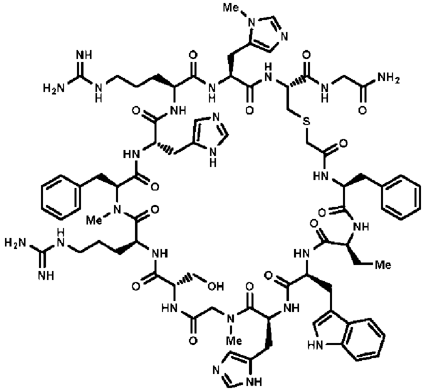 | rac-(3R,6S,9S,12S,15S,18S,21S,27S,30S, 33S,36S)-N-(2-Amino-2-oxoethyl)-15,36-dibenzyl-33-ethyl-9,18-bis(3-guanidinopropyl)-21-(hydroxymethyl)-12,27-bis(1H-imidazol-5-ylmethyl)-30-(1H-indol-3-ylmethyl)-16,25-dimethyl-6-[(3-methylimidazol-4-yl)methyl]-5,8,11,14,17,20,23,26,29,32,35,38-dodecaoxo-1-thia-4,7,10,13,16,19,22,25,28,31,34,37-dodecazacyclononatriacontan-3-carboxamid |
| 12 | 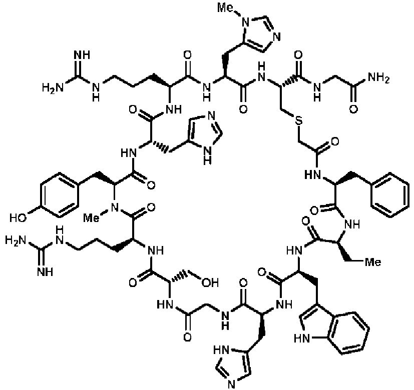 | rac-(3R,6S,9S,12S,15S,18S,21S,27S,30S, 33S,36S)-N-(2-Amino-2-oxoethyl)-36-benzyl-33-ethyl-9,18-bis(3-guanidinopropyl)-21-(hydroxymethyl)-15-[(4-hydroxyphenyl)methyl]-12,27-bis(1H-imidazol-5-ylmethyl)-30-(1H-indol-3-ylmethyl)-16-methyl-6-[(3-methylimidazol-4-yl)methyl]-5,8,11,14,17,20,23,26,29,32,35,38-dodecaoxo-1-thia-4,7,10,13,16,19,22,25,28,31,34,37-dodecazacyclononatriacontan-3-carboxamid |
| 13 | 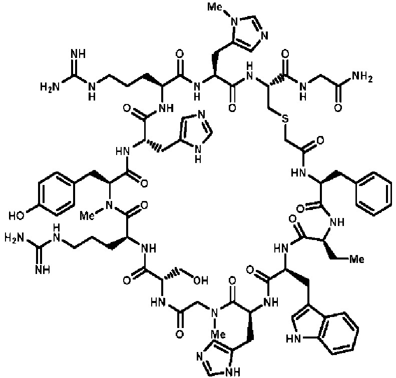 | rac-(3R,6S,9S,12S,15S,18S,21S,27S,30S, 33S,36S)-N-(2-Amino-2-oxoethyl)-36-benzyl-33-ethyl-9,18-bis(3-guanidinopropyl)-21-(hydroxymethyl)-15-[(4-hydroxyphenyl)methyl]-12,27-bis(1H-imidazol-5-ylmethyl)-30-(1H-indol-3-ylmethyl)-16,25-dimethyl-6-[(3-methylimidazol-4-yl)methyl]-5,8,11,14,17,20,23,26,29,32,35,38-dodecaoxo-1-thia-4,7,10,13,16,19,22,25,28,31,34,37-dodecazacyclononatriacontan-3-carboxamid |
| 14 | 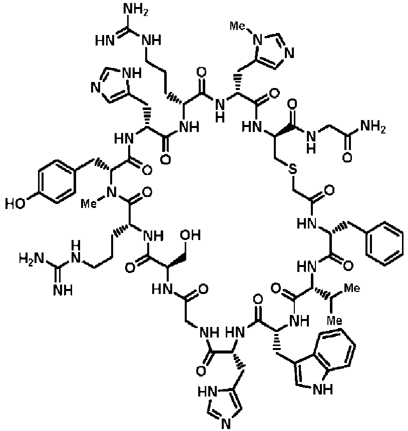 | rac-(3R,6S,9S,12S,15S,18S,21S,27S,30S, 33S,36S)-N-(2-Amino-2-oxoethyl)-36-benzyl-9,18-bis(3-guanidinopropyl)-21-(hydroxymethyl)-15-[(4-hydroxyphenyl)methyl]-12,27-bis(1H-imidazol-5-ylmethyl)-30-(1H-indol-3-ylmethyl)-33-isopropyl-16-methyl-6-[(3-methylimidazol-4-yl)methyl]-5,8,11,14,17,20,23,26,29,32,35,38-dodecaoxo-1-thia-4,7,10,13,16,19,22,25,28,31,34,37-dodecazacyclononatriacontan-3-carboxamid |
| 15 | 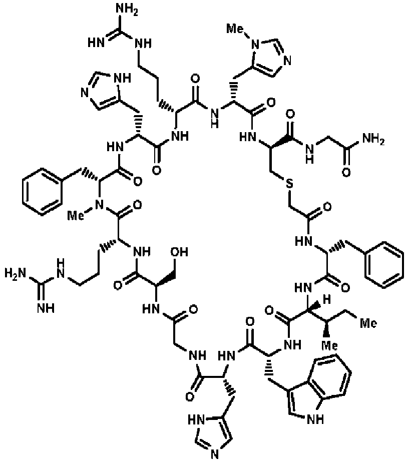 | rac-(3R,6S,9S,12S,15S,18S,21S,27S,30S, 33S,36S)-N-(2-Amino-2-oxoethyl)-15,36-dibenzyl-9,18-bis(3-guanidinopropyl)-21-(hydroxymethyl)-12,27-bis(1H-imidazol-5-ylmethyl)-30-(1H-indol-3-ylmethyl)-16-methyl-6-[(3-methylimidazol-4-yl)methyl]-5,8,11,14,17,20,23,26,29,32,35,38-dodecaoxo-33-[rac-(1S)-1-methylpropyl]-1-thia-4,7,10,13,16,19,22,25,28,31,34,37-dodecazacyclononatriacontan-3-carboxamid |
| 16 | 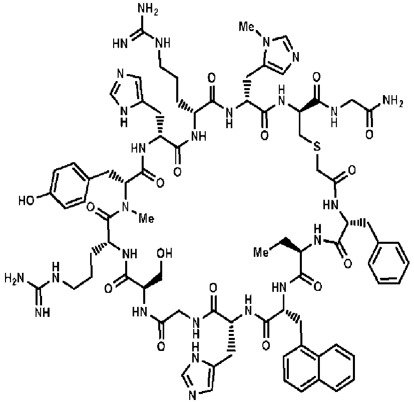 | rac-(3R,6S,9S,12S,15S,18S,21S,27S,30S, 33S,36S)-N-(2-Amino-2-oxoethyl)-36-benzyl-33-ethyl-9,18-bis(3-guanidinopropyl)-21-(hydroxymethyl)-15-[(4-hydroxyphenyl)methyl]-12,27-bis(1H-imidazol-5-ylmethyl)-16-methyl-6-[(3-methylimidazol-4-yl)methyl]-30-(1-naphthylmethyl)-5,8,11,14,17,20,23,26,29,32,35,38-dodecaoxo-1-thia-4,7,10,13,16,19,22,25,28,31,34,37-dodecazacyclononatriacontan-3-carboxamid |
| 17 | 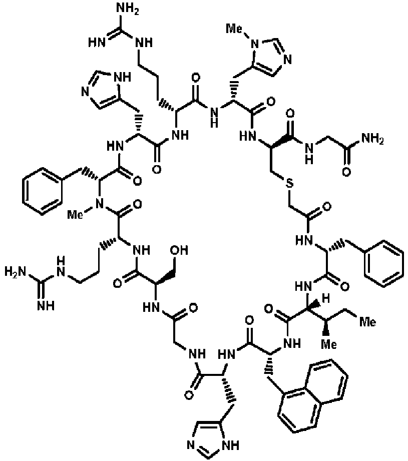 | rac-(3R,6S,9S,12S,15S,18S,21S,27S,30S, 33S,36S)-N-(2-Amino-2-oxoethyl)-15,36-dibenzyl-9,18-bis(3-guanidinopropyl)-21-(hydroxymethyl)-12,27-bis(1H-imidazol-5-ylmethyl)-16-methyl-6-[(3-methylimidazol-4-yl)methyl]-30-(1-naphthylmethyl)-5,8,11,14,17,20,23,26,29,32,35,38-dodecaoxo-33-[rac-(1S)-1-methylpropyl]-1-thia-4,7,10,13,16,19,22,25,28,31,34,37-dodecazacyclononatriacontan-3-carboxamid |
| 18 | 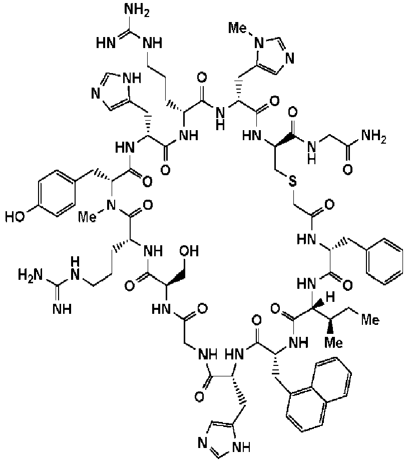 | rac-(3R,6S,9S,12S,15S,18S,21S,27S,30S, 33S,36S)-N-(2-Amino-2-oxoethyl)-36-benzyl-9,18-bis(3-guanidinopropyl)-21-(hydroxymethyl)-15-[(4-hydroxyphenyl)methyl]-12,27-bis(1H-imidazol-5-ylmethyl)-16-methyl-6-[(3-methylimidazol-4-yl)methyl]-30-(1-naphthylmethyl)-5,8,11,14,17,20,23,26,29,32,35,38-dodecaoxo-33-[rac-(1S)-1-methylpropyl]-1-thia-4,7,10,13,16,19,22,25,28,31,34,37-dodecazacyclononatriacontan-3-carboxamid |
| 19 | 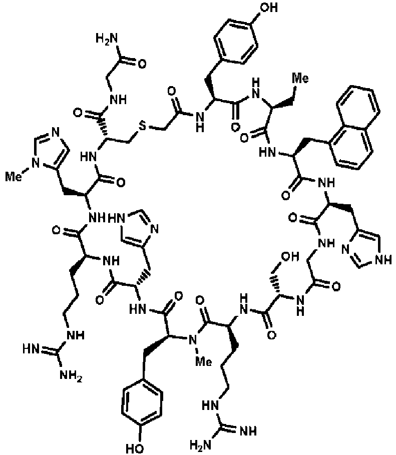 | (3R,6S,9S,12S,15S,18S,21S,27S,30S, 33S,36S)-N-(2-Amino-2-oxoethyl)-33-ethyl-9,18-bis(3-guanidinopropyl)-21-(hydroxymethyl)-15,36-bis[(4-hydroxyphenyl)methyl]-12,27-bis(1H-imidazol-4-ylmethyl)-16-methyl-6-[(3-methylimidazol-4-yl)methyl]-30-(1-naphthylmethyl)-5,8,11,14,17,20,23,26,29,32,35,38-dodecaoxo-1-thia-4,7,10,13,16,19,22,25,28,31,34,37-dodecazacyclononatriacontan-3-carboxamid |
| 20 | 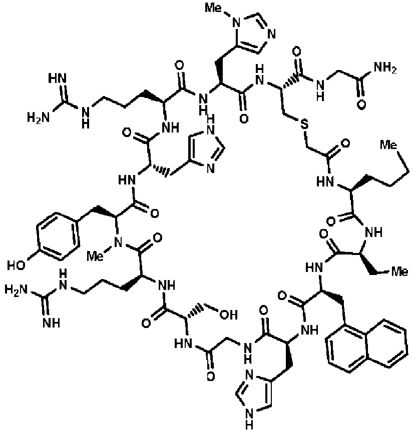 | (3R,6S,9S,12S,15S,18S,21S,27S,30S, 33S,36S)-N-(2-Amino-2-oxoethyl)-36-butyl-33-ethyl-9,18-bis(3-guanidinopropyl)-21-(hydroxymethyl)-15-[(4-hydroxyphenyl)methyl]-12,27-bis(1H-imidazol-4-ylmethyl)-16-methyl-6-[(3-methylimidazol-4-yl)methyl]-30-(1-naphthylmethyl)-5,8,11,14,17,20,23,26,29,32,35,38-dodecaoxo-1-thia-4,7,10,13,16,19,22,25,28,31,34,37-dodecazacyclononatriacontan-3-carboxamid |
| 21 | 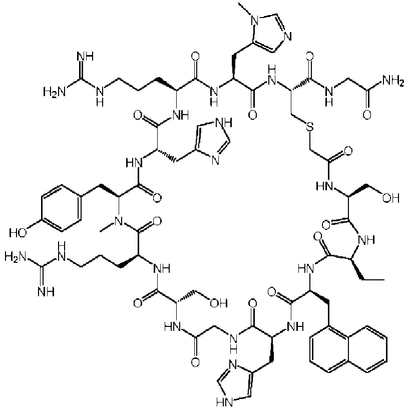 | (3R,6S,9S,12S,15S,18S,21S,27S,30S, 33S,36S)-N-(2-Amino-2-oxoethyl)-33-ethyl-9,18-bis(3-guanidinopropyl)-21,36-bis(hydroxymethyl)-15-[(4-hydroxyphenyl)methyl]-12,27-bis(1H-imidazol-4-ylmethyl)-16-methyl-6-[(3-methylimidazol-4-yl)methyl]-30-(1-naphthylmethyl)-5,8,11,14,17,20,23,26,29,32,35,38-dodecaoxo-1-thia-4,7,10,13,16,19,22,25,28,31,34,37-dodecazacyclononatriacontan-3-carboxamid |
| 22 | 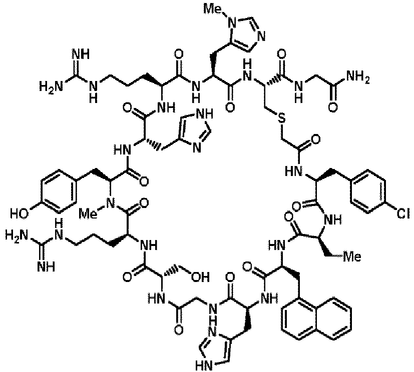 | (3R,6S,9S,12S,15S,18S,21S,27S,30S, 33S,36S)-N-(2-Amino-2-oxoethyl)-36-[(4-chlorphenyl)methyl]-33-ethyl-9,18-bis(3-guanidinopropyl)-21-(hydroxymethyl)-15-[(4-hydroxyphenyl)methyl]-12,27-bis(1H-imidazol-4-ylmethyl)-16-methyl-6-[(3-methylimidazol-4-yl)methyl]-30-(1-naphthylmethyl)-5,8,11,14,17,20,23,26,29,32,35,38-dodecaoxo-1-thia-4,7,10,13,16,19,22,25,28,31,34,37-dodecazacyclononatriacontan-3-carboxamid |
| 23 | 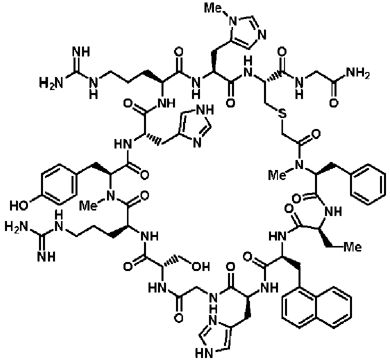 | (3R,6S,9S,12S,15S,18S,21S,27S,30S, 33S,36S)-N-(2-Amino-2-oxoethyl)-36-benzyl-33-ethyl-9,18-bis(3-guanidinopropyl)-21-(hydroxymethyl)-15-[(4-hydroxyphenyl)methyl]-12,27-bis(1H-imidazol-4-ylmethyl)-16,37-dimethyl-6-[(3-methylimidazol-4-yl)methyl]-30-(1-naphthylmethyl)-5,8,11,14,17,20,23,26,29,32,35,38-dodecaoxo-1-thia-4,7,10,13,16,19,22,25,28,31,34,37-dodecazacyclononatriacontan-3-carboxamid |
| 24 | 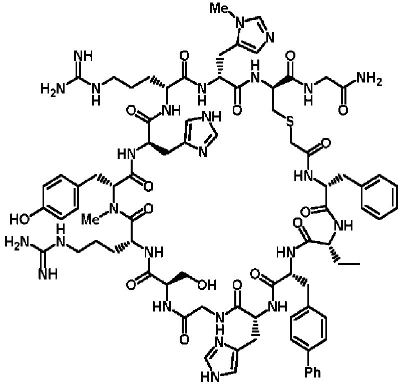 | (3R,6S,9S,12S,15S,18S,21S,27S,30S, 33S,36S)-N-(2-Amino-2-oxoethyl)-36-benzyl-33-ethyl-9,18-bis(3-guanidinopropyl)-21-(hydroxymethyl)-15-[(4-hydroxyphenyl)methyl]-12,27-bis(1H-imidazol-4-ylmethyl)-16-methyl-6-[(3-methylimidazol-4-yl)methyl]-5,8,11,14,17,20,23,26,29,32,35,38-dodecaoxo-30-[(4-phenylphenyl)methyl]-1-thia-4,7,10,13,16,19,22,25,28,31,34,37-dodecazacyclononatriacontan-3-carboxamid |
| 25 | 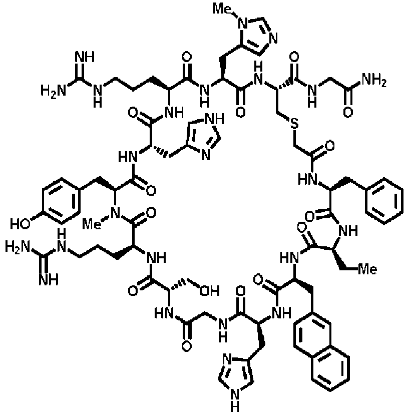 | (3R,6S,9S,12S,15S,18S,21S,27S,30S, 33S,36S)-N-(2-Amino-2-oxoethyl)-36-benzyl-33-ethyl-9,18-bis(3-guanidinopropyl)-21-(hydroxymethyl)-15-[(4-hydroxyphenyl)methyl]-12,27-bis(1H-imidazol-4-ylmethyl)-16-methyl-6-[(3-methylimidazol-4-yl)methyl]-30-(2-naphthylmethyl)-5,8,11,14,17,20,23,26,29,32,35,38-dodecaoxo-1-thia-4,7,10,13,16,19,22,25,28,31,34,37-dodecazacyclononatriacontan-3-carboxamid |
| 26 | 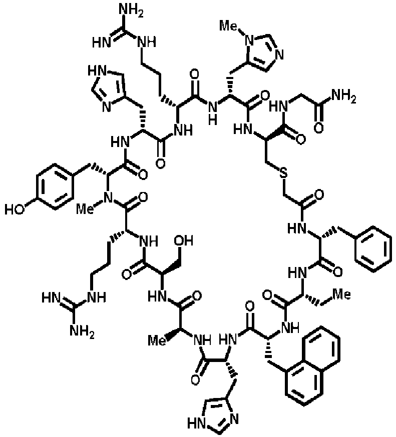 | (3R,6S,9S,12S,15S,18S,21S,24R,27S, 30S,33S,36S)-N-(2-Amino-2-oxoethyl)-36-benzyl-33-ethyl-9,18-bis(3-guanidinopropyl)-21-(hydroxymethyl)-15-[(4-hydroxyphenyl)methyl]-12,27-bis(1H-imidazol-4-ylmethyl)-16,24-dimethyl-6-[(3-methylimidazol-4-yl)methyl]-30-(1-naphthylmethyl)-5,8,11,14,17,20,23,26,29,32,35,38-dodecaoxo-1-thia-4,7,10,13,16,19,22,25,28,31,34,37-dodecazacyclononatriacontan-3-carboxamid |
| 27 | 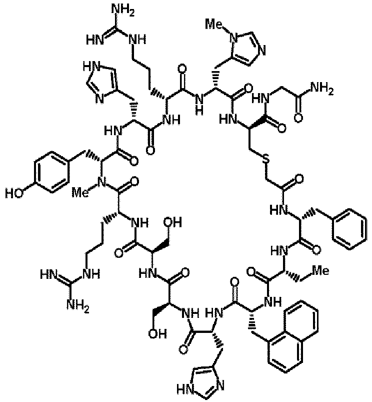 | (3R,6S,9S,12S,15S,18S,21S,24R,27S, 30S,33S,36S)-N-(2-Amino-2-oxoethyl)-36-benzyl-33-ethyl-9,18-bis(3-guanidinopropyl)-21,24-bis(hydroxymethyl)-15-[(4-hydroxyphenyl)methyl]-12,27-bis(1H-imidazol-4-ylmethyl)-16-methyl-6-[(3-methylimidazol-4-yl)methyl]-30-(1-naphthylmethyl)-5,8,11,14,17,20,23,26,29,32,35,38-dodecaoxo-1-thia-4,7,10,13,16,19,22,25,28,31,34,37-dodecazacyclononatriacontan-3-carboxamid |
| 28 | 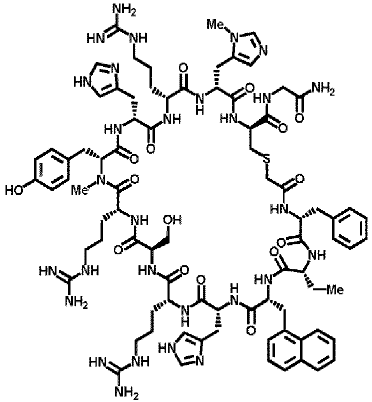 | (3R,6S,9S,12S,15S,18S,21S,24S,27S, 30S,33S,36S)-N-(2-Amino-2-oxoethyl)-36-benzyl-33-ethyl-9,18,24-tris(3-guanidinopropyl)-21-(hydroxymethyl)-15-[(4-hydroxyphenyl)methyl]-12,27-bis(1H-imidazol-4-ylmethyl)-16-methyl-6-[(3-methylimidazol-4-yl)methyl]-30-(1-naphthylmethyl)-5,8,11,14,17,20,23,26,29,32,35,38-dodecaoxo-1-thia-4,7,10,13,16,19,22,25,28,31,34,37-dodecazacyclononatriacontan-3-carboxamid |
| 29 | 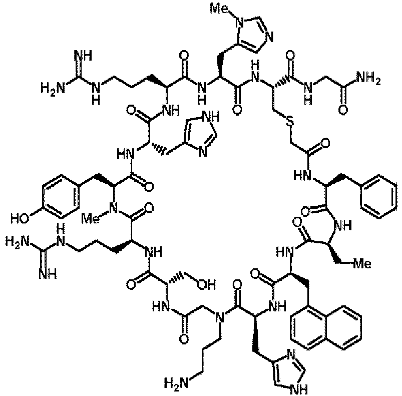 | (3R,6S,9S,12S,15S,18S,21S,27S,30S, 33S,36S)-N-(2-Amino-2-oxoethyl)-25-(3-aminopropyl)-36-benzyl-33-ethyl-9,18-bis(3-guanidinopropyl)-21-(hydroxymethyl)-15-[(4-hydroxyphenyl)methyl]-12,27-bis(1H-imidazol-4-ylmethyl)-16-methyl-6-[(3-methylimidazol-4-yl)methyl]-30-(1-naphthylmethyl)-5,8,11,14,17,20,23,26,29,32,35,38-dodecaoxo-1-thia-4,7,10,13,16,19,22,25,28,31,34,37-dodecazacyclononatriacontan-3-carboxamid |
| 30 | 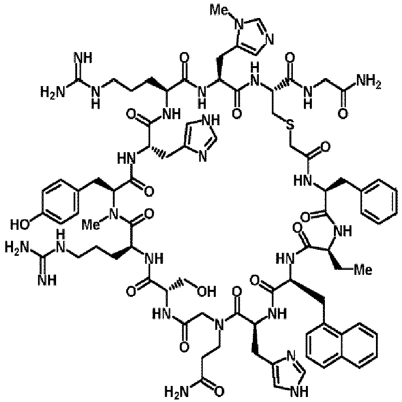 | (3R,6S,9S,12S,15S,18S,21S,27S,30S, 33 S,36S)-N-(2-Amino-2-oxoethyl)-25-(3-amino-3-oxopropyl)-36-benzyl-33-ethyl-9,18-bis(3-guanidinopropyl)-21-(hydroxymethyl)-15-[(4-hydroxyphenyl)methyl]-12,27-bis(1H-imidazol-4-ylmethyl)-16-methyl-6-[(3-methylimidazol-4-yl)methyl]-30-(1-naphthylmethyl)-5,8,11,14,17,20,23,26,29,32,35,38-dodecaoxo-1-thia-4,7,10,13,16,19,22,25,28,31,34,37-dodecazacyclononatriacontan-3-carboxamid |
| 31 | 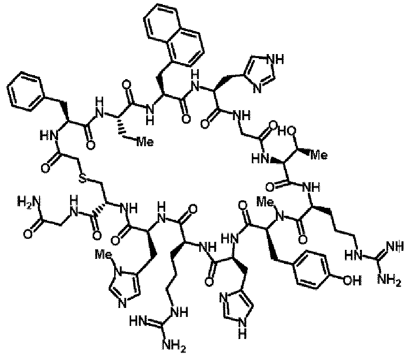 | (3R,6S,9S,12S,15S,18S,21S,27S,30S, 33S,36S)-N-(2-Amino-2-oxoethyl)-36-benzyl-33-ethyl-9,18-bis(3-guanidinopropyl)-21-[(1S)-1-hydroxyethyl]-15-[(4-hydroxyphenyl)methyl]-12,27-bis(1H-imidazol-4-ylmethyl)-16-methyl-6-[(3-methylimidazol-4-yl)methyl]-30-(1-naphthylmethyl)-5,8,11,14,17,20,23,26,29,32,35,38-dodecaoxo-1-thia-4,7,10,13,16,19,22,25,28,31,34,37-dodecazacyclononatriacontan-3-carboxamid |
| 32 | 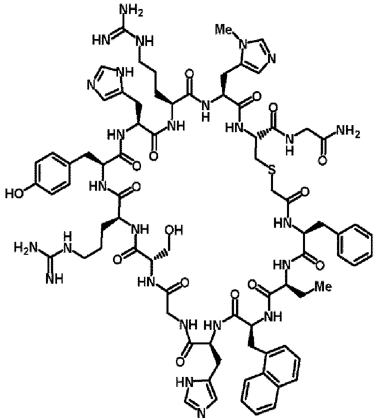 | (3R,6S,9S,12S,15S,18S,21S,27S,30S, 33S,36S)-N-(2-Amino-2-oxoethyl)-36-benzyl-33-ethyl-9,18-bis(3-guanidinopropyl)-21-(hydroxymethyl)-15-[(4-hydroxyphenyl)methyl]-12,27-bis(1H-imidazol-4-ylmethyl)-6-[(3-methylimidazol-4-yl)methyl]-30-(1-naphthylmethyl)-5,8,11,14,17,20,23,26,29,32,35,38-dodecaoxo-1-thia-4,7,10,13,16,19,22,25,28,31,34,37-dodecazacyclononatriacontan-3-carboxamid |
| 33 | 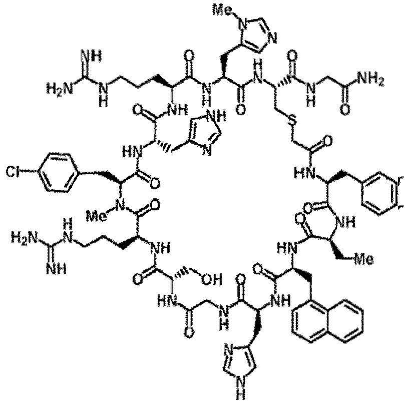 | (3R,6S,9S,12S,15S,18S,21S,27S,30S, 33S,36S)-N-(2-Amino-2-oxoethyl)-36-benzyl-15-[(4-chlorphenyl)methyl]-33-ethyl-9,18-bis(3-guanidinopropyl)-21-(hydroxymethyl)-12,27-bis(1H-imidazol-4-ylmethyl)-16-methyl-6-[(3-methylimidazol-4-yl)methyl]-30-(1-naphthylmethyl)-5,8,11,14,17,20,23,26,29,32,35,38-dodecaoxo-1-thia-4,7,10,13,16,19,22,25,28,31,34,37-dodecazacyclononatriacontan-3-carboxamid |
| 34 | 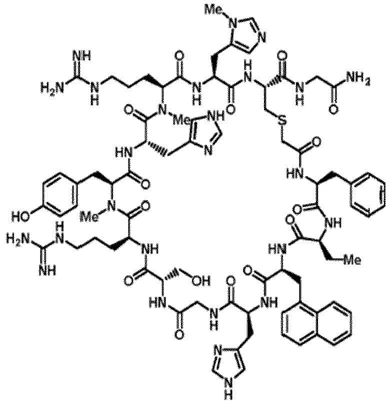 | (3R,6S,9S,12S,15S,18S,21S,27S,30S, 33S,36S)-N-(2-Amino-2-oxoethyl)-36-benzyl-33-ethyl-9,18-bis(3-guanidinopropyl)-21-(hydroxymethyl)-15-[(4-hydroxyphenyl)methyl]-12,27-bis(1H-imidazol-4-ylmethyl)-10,16-dimethyl-6-[(3-(methylimidazol-4-yl)methyl]-30-(1-naphthylmethyl)-5,8,11,14,17,20,23,26,29,32,35,38-dodecaoxo-1-thia-4,7,10,13,16,19,22,25,28,31,34,37-dodecazacyclononatriacontan-3-carboxamid |
| 35 | 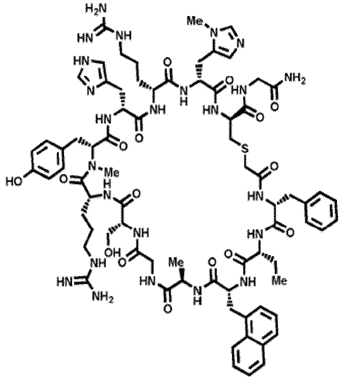 | (3R,6S,9S,12S,15S,18S,21S,27S,30S, 33S,36S)-N-(2-Amino-2-oxoethyl)-36-benzyl-33-ethyl-9,18-bis(3-guanidinopropyl)-21-(hydroxymethyl)-15-[(4-hydroxyphenyl)methyl]-12-(1H-imidazol-4-ylmethyl)-16,27-dimethyl-6-[(3-methylimidazol-4-yl)methyl]-30-(1-naphthylmethyl)-5,8,11,14,17,20,23,26,29,32,35,38-dodecaoxo-1-thia-4,7,10,13,16,19,22,25,28,31,34,37-dodecazacyclononatriacontan-3-carboxamid |
| 36 | 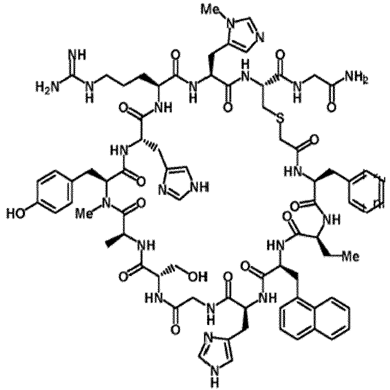 | (3R,6S,9S,12S,15S,18S,21S,27S,30S, 33S,36S)-N-(2-Amino-2-oxoethyl)-36-benzyl-33-ethyl-9-(3-guanidinopropyl)-21-(hydroxymethyl)-15-[(4-hydroxyphenyl)methyl]-12,27-bis(1H-imidazol-4-ylmethyl)-16,18-dimethyl-6-[(3-methylimidazol-4-yl)methyl]-30-(1-naphthylmethyl)-5,8,11,14,17,20,23,26,29,32,35,38-dodecaoxo-1-thia-4,7,10,13,16,19,22,25,28,31,34,37-dodecazacyclononatriacontan-3-carboxamid |
| 37 | 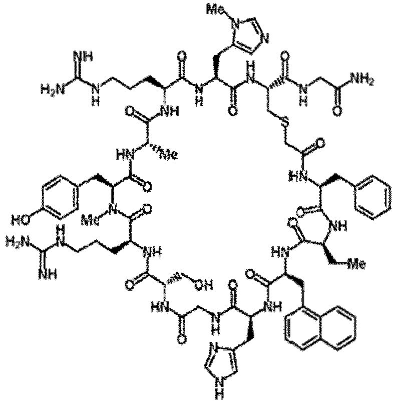 | (3R,6S,9S,12S,15S,18S,21S,27S,30S, 33S,36S)-N-(2-Amino-2-oxoethyl)-36-benzyl-33-ethyl-9,18-bis(3-guanidinopropyl)-21-(hydroxymethyl)-15-[(4-hydroxyphenyl)methyl]-27-(1H-imidazol-4-ylmethyl)-12,16-dimethyl-6-[(3-methylimidazol-4-yl)methyl]-30-(1-naphthylmethyl)-5,8,11,14,17,20,23,26,29,32,35,38-dodecaoxo-1-thia-4,7,10,13,16,19,22,25,28,31,34,37-dodecazacyclononatriacontan-3-carboxamid |
| 38 | 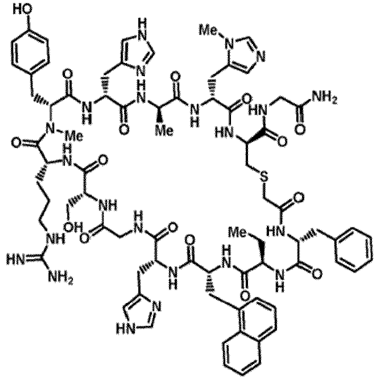 | (3R,6S,9S,12S,15S,18S,21S,27S,30S, 33S,36S)-N-(2-Amino-2-oxoethyl)-36-benzyl-33-ethyl-18-(3-guanidinopropyl)-21-(hydroxymethyl)-15-[(4-hydroxyphenyl)methyl]-12,27-bis(1H-imidazol-4-ylmethyl)-9,16-dimethyl-6-[(3-methylimidazol-4-yl)methyl]-30-(1-naphthylmethyl)-5,8,11,14,17,20,23,26,29,32,35,38-dodecaoxo-1-thia-4,7,10,13,16,19,22,25,28,31,34,37-dodecazacyclononatriacontan-3-carboxamid |
| 39 | 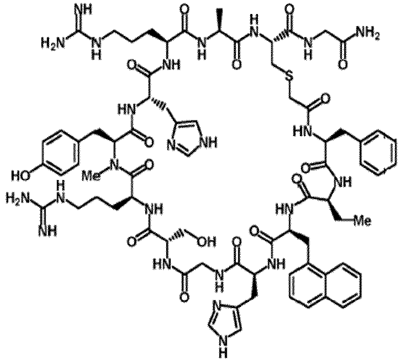 | (3R,6S,9S,12S,15S,18S,21S,27S,30S, 33S,36S)-N-(2-Amino-2-oxoethyl)-36-benzyl-33-ethyl-9,18-bis(3-guanidinopropyl)-21-(hydroxymethyl)-15-[(4-hydroxyphenyl)methyl]-12,27-bis(1H-imidazol-4-ylmethyl)-6,16-dimethyl-30-(1-naphthylmethyl)-5,8,11,14,17,20,23,26,29,32,35,38-dodecaoxo-1-thia-4,7,10,13,16,19,22,25,28,31,34,37-dodecazacyclononatriacontan-3-carboxamid |
| 40 | 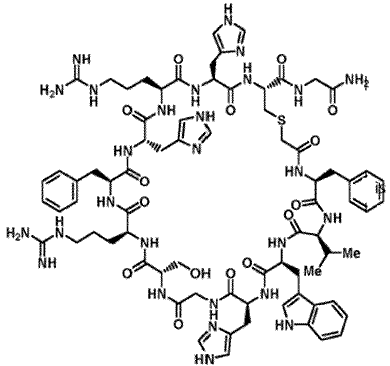 | (3R,6S,9S,12S,15S,18S,21S,27S,30S, 33 S,36S)-N-(2-Amino-2-oxoethyl)-15,36-dibenzyl-9,18-bis(3-guanidinopropyl)-21-(hydroxymethyl)-6,12,27-tris(1H-imidazol-4-ylmethyl)-30-(1H-indol-3-ylmethyl)-33-isopropyl-5,8,11,14,17,20,23,26,29,32,35,38-dodecaoxo-1-thia-4,7,10,13,16,19,22,25,28,31,34,37-dodecazacyclononatriacontan-3-carboxamid |
| 41 | 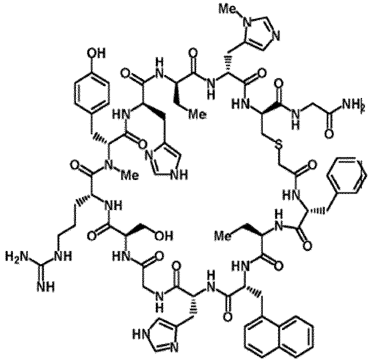 | (3R,6S,9S,12S,15S,18S,21S,27S,30S, 33S,36S)-N-(2-Amino-2-oxoethyl)-36-benzyl-9,33-diethyl-18-(3-guanidinopropyl)-21-(hydroxymethyl)-15-[(4-hydroxyphenyl)methyl]-12,27-bis(1H-imidazol-4-ylmethyl)-16-methyl-6-[(3-methylimidazol-4-yl)methyl]-30-(1-naphthylmethyl)-5,8,11,14,17,20,23,26,29,32,35,38-dodecaoxo-1-thia-4,7,10,13,16,19,22,25,28,31,34,37-dodecazacyclononatriacontan-3-carboxamid |
| 42 | 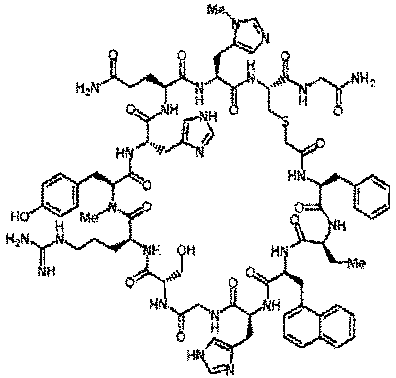 | rac-(3R,6S,9S,12S,15S,18S,21S,27S,30S, 33S,36S)-N-(2-Amino-2-oxoethyl)-9-(3-amino-3-oxopropyl)-36-benzyl-33-ethyl-18-(3-guanidinopropyl)-21-(hydroxymethyl)-15-[(4-hydroxyphenyl)methyl]-12,27-bis(1H-imidazol-4-ylmethyl)-16-methyl-6-[(3-methylimidazol-4-yl)methyl]-30-(1-naphthylmethyl)-5,8,11,14,17,20,23,26,29,32,35,38-dodecaoxo-1-thia-4,7,10,13,16,19,22,25,28,31,34,37-dodecazacyclononatriacontan-3-carboxamid |
| 43 | 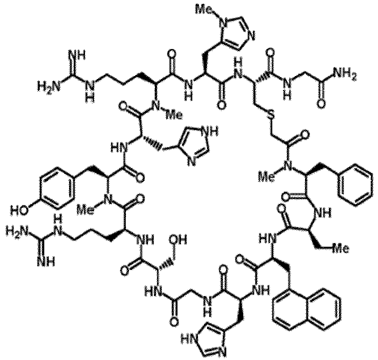 | rac-(3R,6S,9S,12S,15S,18S,21S,27S,30S, 33S,36S)-N-(2-Amino-2-oxoethyl)-36-benzyl-33-ethyl-9,18-bis(3-guanidinopropyl)-21-(hydroxymethyl)-15-[(4-hydroxyphenyl)methyl]-12,27-bis(1H-imidazol-4-ylmethyl)-10,16,37-trimethyl-6-[(3-methylimidazol-4-yl)methyl]-30-(1-naphthylmethyl)-5,8,11,14,17,20,23,26,29,32,35,38-dodecaoxo-1-thia-4,7,10,13,16,19,22,25,28,31,34,37-dodecazacyclononatriacontan-3-carboxamid |
| 44 | 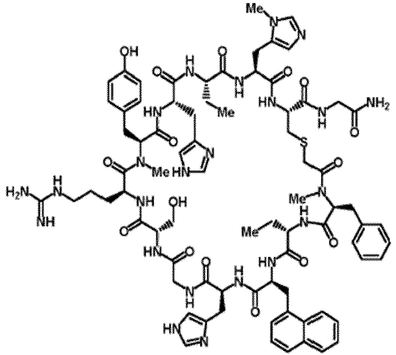 | rac-(3R,6S,9S,12S,15S,18S,21S,27S,30S, 33S,36S)-N-(2-Amino-2-oxoethyl)-36-benzyl-9,33-diethyl-18-(3-guanidinopropyl)-21-(hydroxymethyl)-15-[(4-hydroxyphenyl)methyl]-12,27-bis(1H-imidazol-4-ylmethyl)-16,37-dimethyl-6-[(3-methylimidazol-4-yl)methyl]-30-(1-naphthylmethyl)-5,8,11,14,17,20,23,26,29,32,35,38-dodecaoxo-1-thia-4,7,10,13,16,19,22,25,28,31,34,37-dodecazacyclononatriacontan-3-carboxamid |
| 45 | 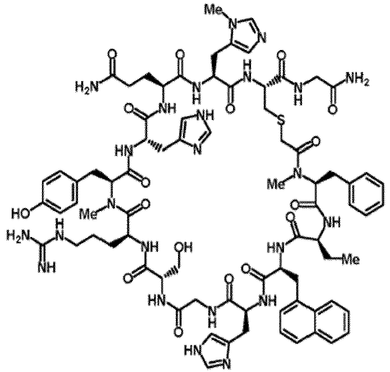 | rac-(3R,6S,9S,12S,15S,18S,21S,27S,30S, 33S,36S)-N-(2-Amino-2-oxoethyl)-9-(3-amino-3-oxopropyl)-36-benzyl-33-ethyl-18-(3-guanidinopropyl)-21-(hydroxymethyl)-15-[(4-hydroxyphenyl)methyl]-12,27-bis(1H-imidazol-4-ylmethyl)-16,37-dimethyl-6-[(3-methylimidazol-4-yl)methyl]-30-(1-naphthylmethyl)-5,8,11,14,17,20,23,26,29,32,35,38-dodecaoxo-1-thia-4,7,10,13,16,19,22,25,28,31,34,37-dodecazacyclononatriacontan-3-carboxamid |
| 46 | 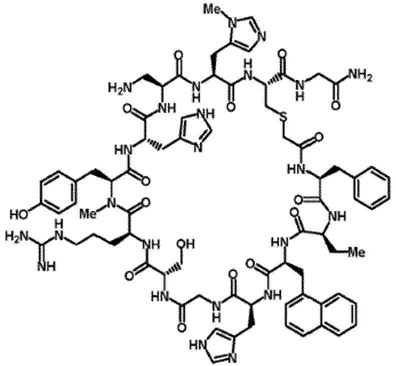 | rac-(3R,6S,9S,12S,15S,18S,21S,27S,30S, 33S,36S)-9-(Aminomethyl)-N-(2-amino-2-oxoethyl)-36-benzyl-33-ethyl-18-(3-guanidinopropyl)-21-(hydroxymethyl)-15-[(4-hydroxyphenyl)methyl]-12,27-bis(1H-imidazol-4-ylmethyl)-16-methyl-6-[(3-methylimidazol-4-yl)methyl]-30-(1-naphthylmethyl)-5,8,11,14,17,20,23,26,29,32,35,38-dodecaoxo-1-thia-4,7,10,13,16,19,22,25,28,31,34,37-dodecazacyclononatriacontan-3-carboxamid |
| 47 | 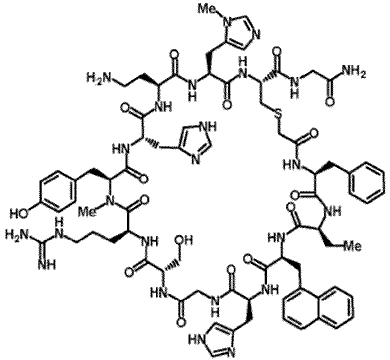 | rac-(3R,6S,9S,12S,15S,18S,21S,27S,30S, 33 S,36S)-9-(2-Aminoethyl)-N-(2-amino-2-oxoethyl)-36-benzyl-33-ethyl-18-(3-guanidinopropyl)-21-(hydroxymethyl)-15-[(4-hydroxyphenyl)methyl]-12,27-bis(1H-imidazol-4-ylmethyl)-16-methyl-6-[(3-methylimidazol-4-yl)methyl]-30-(1-naphthylmethyl)-5,8,11,14,17,20,23,26,29,32,35,38-dodecaoxo-1-thia-4,7,10,13,16,19,22,25,28,31,34,37-dodecazacyclononatriacontan-3-carboxamid |
| 48 | 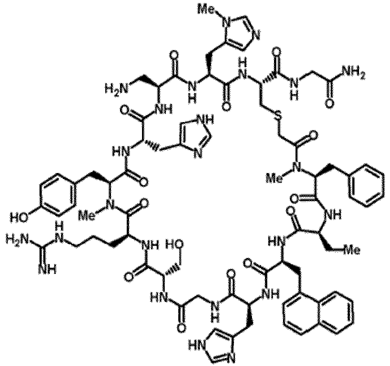 | rac-(3R,6S,9S,12S,15S,18S,21S,27S,30S, 33S,36S)-9-(Aminomethyl)-N-(2-amino-2-oxoethyl)-36-benzyl-33-ethyl-18-(3-guanidinopropyl)-21-(hydroxymethyl)-15-[(4-hydroxyphenyl)methyl]-12,27-bis(1H-imidazol-4-ylmethyl)-16,37-dimethyl-6-[(3-methylimidazol-4-yl)methyl]-30-(1-naphthylmethyl)-5,8,11,14,17,20,23,26,29,32,35,38-dodecaoxo-1-thia-4,7,10,13,16,19,22,25,28,31,34,37-dodecazacyclononatriacontan-3-carboxamid |
| 49 | 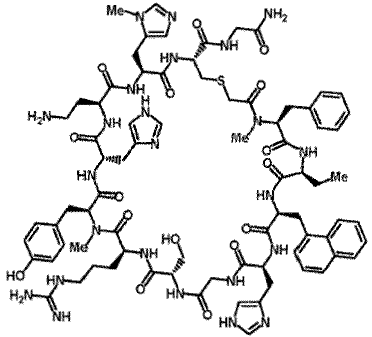 | rac-(3R,6S,9S,12S,15S,18S,21S,27S,30S, 33 S,36S)-9-(2-Aminoethyl)-N-(2-amino-2-oxoethyl)-36-benzyl-33-ethyl-18-(3-guanidinopropyl)-21-(hydroxymethyl)-15-[(4-hydroxyphenyl)methyl]-12,27-bis(1H-imidazol-4-ylmethyl)-16,37-dimethyl-6-[(3-methylimidazol-4-yl)methyl]-30-(1-naphthylmethyl)-5,8,11,14,17,20,23,26,29,32,35,38-dodecaoxo-1-thia-4,7,10,13,16,19,22,25,28,31,34,37-dodecazacyclononatriacontan-3-carboxamid |
| 50 | 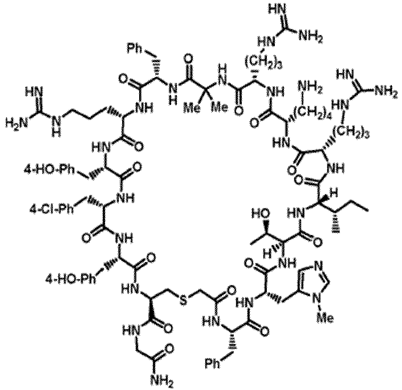 | rac-(3R,6S,9S,12S,15S,18S,21S,24S,27S, 30S,33 S,36S,42S)-27-(4-Aminobutyl)-N-(2-amino-2-oxoethyl)-6,18,42-tribenzyl-15,21,24-tris(3-guanidinopropyl)-12-[(4-hydroxyphenyl)methyl]-30-(1H-imidazol-5-ylmethyl)-9-(1H-indol-3-ylmethyl)-5,8,11,14,17,20,23,26,29,3 2,3 5,3 8,41,44 -tetradecaoxo-36-[rac-(1R)-1-hydroxyethyl]-33-[rac-(1S)-1-methylpropyl]-1-thia-4,7,10,13,16,19,22,25,28,31,34,37,40,43 -tetradecazacyclopentatetracontan-3-carboxamid |
| 51 | 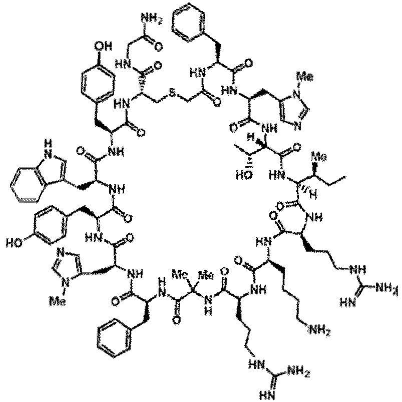 | (3R,6S,9S,12S,15S,18S,24S,27S,30S, 33S,36S,39S,42S)-27-(4-Aminobutyl)-N-(2-amino-2-oxoethyl)-18,42-dibenzyl-24,30-bis(3-guanidinopropyl)-36-[(1R)-1-hydroxyethyl]-6,12-bis[(4-hydroxyphenyl)methyl]-9-(1H-indol-3-ylmethyl)-21,21-dimethyl-15,39-bis[(3-methylimidazol-4-yl)methyl]-33-[(1S)-1-methylpropyl]-5,8,11,14,17,20,23,26,29,32,35,38,41,44 -tetradecaoxo-1-thia-4,7,10,13,16,19,22,25,28,31,34,37,40,43 -tetradecazacyclopentatetracontan-3-carboxamid |
| 52 | 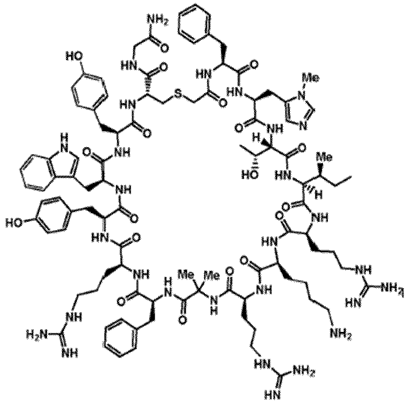 | (3R,6S,9S,12S,15S,18S,24S,27S,30S, 33 S,36S,39S,42S)-27-(4-Aminobutyl)-N-(2-amino-2-oxoethyl)-18,42-dibenzyl-15,24,30-tris(3-guanidinopropyl)-36-[(1R)-1-hydroxyethyl]-6,12-bis[(4-hydroxyphenyl)methyl]-9-(1H-indol-3-ylmethyl)-21,21-dimethyl-39-[(3-methylimidazol-4-yl)methyl]-33-[(1S)-1-methylpropyl]-5,8,11,14,17,20,23,26,29,32,35,38,41,44 -tetradecaoxo-1-thia-4,7,10,13,16,19,22,25,28,31,34,37,40,43 -tetradecazacyclopentatetracontan-3-carboxamid |
| 53 | 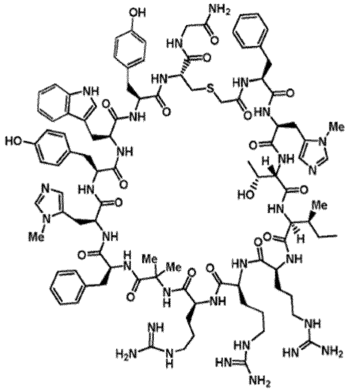 | (3R,6S,9S,12S,15S,18S,24S,27S,30S, 33 S,36S,39S,42S)-N-(2-Amino-2-oxoethyl)-18,42-dibenzyl-24,27,30-tris(3-guanidinopropyl)-36-[(1R)-1-hydroxyethyl]-6,12-bis[(4-hydroxyphenyl)methyl]-9-(1H-indol-3-ylmethyl)-21,21-dimethyl-15,39-bis[(3-methylimidazol-4-yl)methyl]-33-[(1S)-1-methylpropyl]-5,8,11,14,17,20,23,26,29,32,35,38,41,44 -tetradecaoxo-1-thia-4,7,10,13,16,19,22,25,28,31,34,37,40,43 -tetradecazacyclopentatetracontan-3-carboxamid |
| 54 | 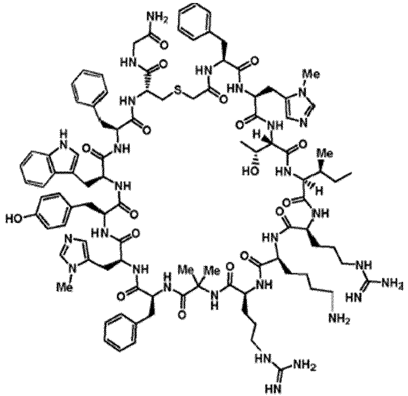 | (3R,6S,9S,12S,15S,18S,24S,27S,30S, 33 S,36S,39S,42S)-27-(4-Aminobutyl)-N-(2-amino-2-oxoethyl)-6,18,42-tribenzyl-24,30-bis(3-guanidinopropyl)-36-[(1R)-1-hydroxyethyl]-12-[(4-hydroxyphenyl)methyl]-9-(1H-indol-3-ylmethyl)-21,21-dimethyl-15,39-bis[(3-methylimidazol-4-yl)methyl]-33-[(1S)-1-methylpropyl]-5,8,11,14,17,20,23,26,29,32,35,38,41,44 -tetradecaoxo-1-thia-4,7,10,13,16,19,22,25,28,31,34,37,40,43 -tetradecazacyclopentatetracontan-3-carboxamid; 2,2,2-Trifluoressigsäure |
| 55 | 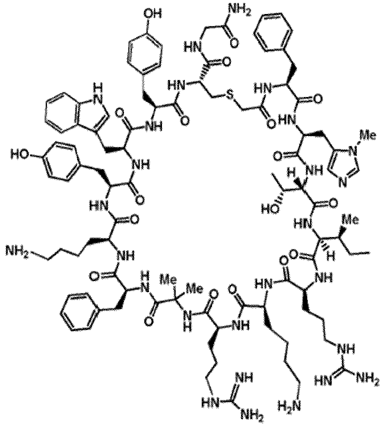 | (3R,6S,9S,12S,15S,18S,24S,27S,30S, 33S,36S,39S,42S)-15,27-Bis(4-aminobutyl)-N-(2-amino-2-oxoethyl)-18,42-dibenzyl-24,30-bis(3-guanidinopropyl)-36-[(1R)-1-hydroxyethyl]-6,12-bis[(4-hydroxyphenyl)methyl]-9-(1H-indol-3-ylmethyl)-21,21-dimethyl-39-[(3-methylimidazol-4-yl)methyl]-33-[(1S)-1-methylpropyl]-5,8,11,14,17,20,23,26,29,32,35,38,41,44 -tetradecaoxo-1-thia-4,7,10,13,16,19,22,25,28,31,34,37,40,43 -tetradecazacyclopentatetracontan-3-carboxamid |
| 56 | 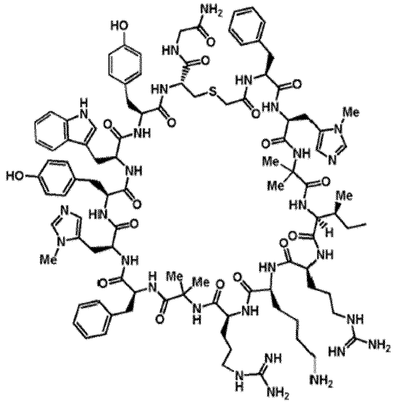 | (3R,6S,9S,12S,15S,18S,24S,27S,30S, 33S,39S,42S)-27-(4-Aminobutyl)-N-(2-amino-2-oxoethyl)-18,42-dibenzyl-24,30-bis(3-guanidinopropyl)-6,12-bis[(4-hydroxyphenyl)methyl]-9-(1H-indol-3-ylmethyl)-21,21,36,36-tetramethyl-15,39-bis[(3-methylimidazol-4-yl)methyl]-33-[(1S)-1-methylpropyl]-5,8,11,14,17,20,23,26,29,32,35,38,41,44 -tetradecaoxo-1-thia-4,7,10,13,16,19,22,25,28,31,34,37,40,43 -tetradecazacyclopentatetracontan-3-carboxamid |
| 57 | 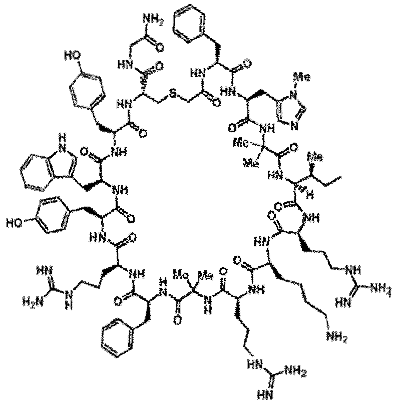 | (3R,6S,9S,12S,15S,18S,24S,27S,30S, 33S,39S,42S)-27-(4-Aminobutyl)-N-(2-amino-2-oxoethyl)-18,42-dibenzyl-15,24,30-tris(3-guanidinopropyl)-6,12-bis[(4-hydroxyphenyl)methyl]-9-(1H-indol-3-ylmethyl)-21,21,36,36-tetramethyl-39-[(3-methylimidazol-4-yl)methyl]-33-[(1S)-1-methylpropyl]-5,8,11,14,17,20,23,26,29,3 2,3 5,3 8,41,44 -tetradecaoxo-1-thia-4,7,10,13,16,19,22,25,28,31,34,37,40,43 -tetradecazacyclopentatetracontan-3-carboxamid |
| 58 | 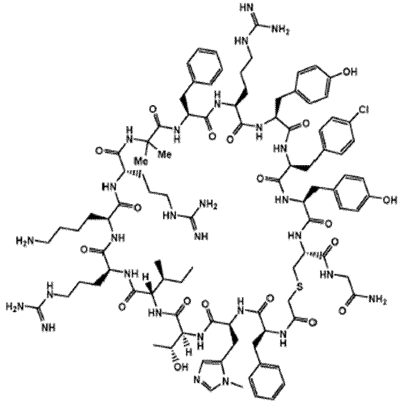 | (3R,6S,9S,12S,15S,18S,24S,27S,30S, 33 S,36S,39S,42S)-27-(4-Aminobutyl)-N-(2-amino-2-oxoethyl)-18,42-dibenzyl-9-[(4-chlorphenyl)methyl]-15,24,30-tris(3-guanidinopropyl)-36-[(1R)-1-hydroxyethyl]-6,12-bis[(4-hydroxyphenyl)methyl]-21,21-dimethyl-39-[(3-methylimidazol-4-yl)methyl]-33-[(1S)-1-methylpropyl]-5,8,11,14,17,20,23,26,29,3 2,3 5,3 8,41,44 -tetradecaoxo-1-thia-4,7,10,13,16,19,22,25,28,31,34,37,40,43 -tetradecazacyclopentatetracontan-3-carboxamid |
| 59 | 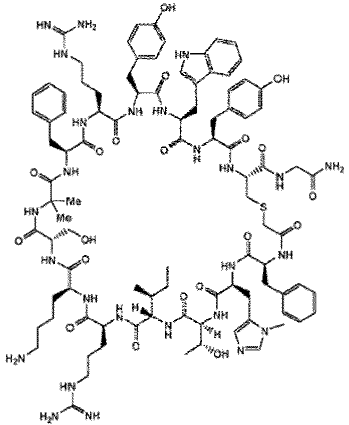 | rac-(3R,6S,9S,12S,15S,18S,24S,27S,30S, 33 S,36S,39S,42S)-27-(4-Aminobutyl)-N-(2-amino-2-oxoethyl)-18,42-dibenzyl-15,30-bis(3-guanidinopropyl)-24-(hydroxymethyl)-6,12-bis[(4-hydroxyphenyl)methyl]-9-(1H-indol-3-ylmethyl)-21,21-dimethyl-39-[(3-methylimidazol-4-yl)methyl]-5,8,11,14,17,20,23,26,29,3 2,3 5,3 8,41,44 -tetradecaoxo-36-[rac-(1R)-1-hydroxyethyl]-33-[rac-(1S)-1-methylpropyl]-1-thia-4,7,10,13,16,19,22,25,28,31,34,37,40,43 -tetradecazacyclopentatetracontan-3-carboxamid |
| 60 | 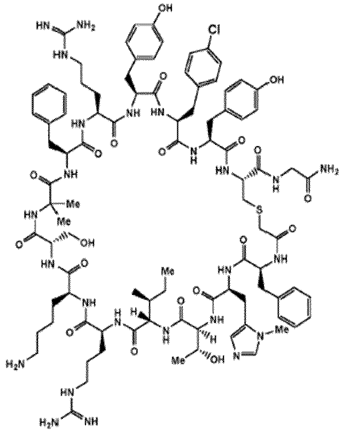 | (3R,6S,9S,12S,15S,18S,24S,27S,30S, 33 S,36S,39S,42S)-27-(4-Aminobutyl)-N-(2-amino-2-oxoethyl)-18,42-dibenzyl-9-[(4-chlorphenyl)methyl]-15,30-bis(3-guanidinopropyl)-24-(hydroxymethyl)-6,12-bis[(4-hydroxyphenyl)methyl]-21,21-dimethyl-39-[(3-methylimidazol-4-yl)methyl]-5,8,11,14,17,20,23,26,29,3 2,3 5,3 8,41,44 -tetradecaoxo-36-[rac-(1R)-1-hydroxyethyl]-33-[rac-(1S)-1-methylpropyl]-1-thia-4,7,10,13,16,19,22,25,28,31,34,37,40,43 -tetradecazacyclopentatetracontan-3-carboxamid |
| 61 | 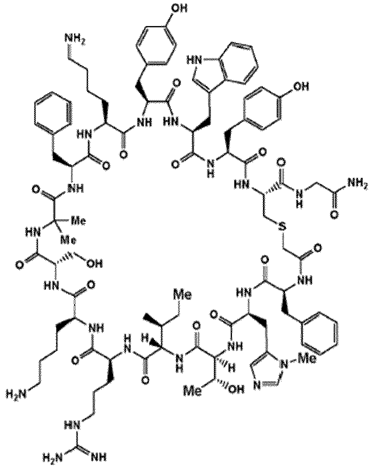 | rac-(3R,6S,9S,12S,15S,18S,24S,27S,30S, 33S,36S,39S,42S)-15,27-Bis(4-aminobutyl)-N-(2-amino-2-oxoethyl)-18,42-dibenzyl-30-(3-guanidinopropyl)-24-(hydroxymethyl)-6,12-bis[(4-hydroxyphenyl)methyl]-9-(1H-indol-3-ylmethyl)-21,21-dimethyl-39-[(3-methylimidazol-4-yl)methyl]-5,8,11,14,17,20,23,26,29,3 2,3 5,3 8,41,44 -tetradecaoxo-36-[rac-(1R)-1-hydroxyethyl]-33-[rac-(1S)-1-methylpropyl]-1-thia-4,7,10,13,16,19,22,25,28,31,34,37,40,43 -tetradecazacyclopentatetracontan-3-carboxamid |

13. Pharmazeutische Zusammensetzung, umfassend die Verbindung nach einem der Ansprüche 1 bis 12 oder ein pharmazeutisch unbedenkliches Salz, Solvat oder Stereoisomer davon und einen pharmazeutisch unbedenklichen Hilfsstoff.

14. Verbindung nach einem der Ansprüche 1 bis 12 oder ein pharmazeutisch unbedenkliches Salz, Solvat oder Stereoisomer davon zur Verwendung bei der Behandlung einer bakteriellen Infektion bei einem Säuger, umfassend das Verabreichen einer wirksamen Menge der Verbindung an den Säuger in einer Häufigkeit und für eine Dauer, die ausreichen, um dem Säuger eine vorteilhafte Wirkung bereitzustellen.

15. Verbindung zur Verwendung nach Anspruch 14, ferner umfassend das Verabreichen eines zweiten Therapeutikums, wobei das zweite Therapeutikum ein Aminoglykosid-Antibiotikum, Fluorchinolon-Antibiotikum, β-Lactam-Antibiotikum, Makrolid-Antibiotikum, Glykopeptid-Antibiotikum, Rifampicin, Chloramphenicol, Fluoramphenicol, Colistin, Mupirocin, Bacitracin, Daptomycin oder Linezolid ist.

## Revendications

1. Composé de formule I 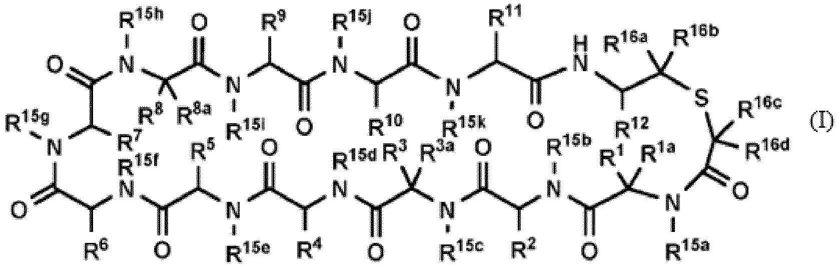
ou un stéréoisomère ou un sel pharmaceutiquement acceptable de celui-ci, dans lequel :
R¹ représente un alkyle en C₁₋₆, un hydroxylalkyle en C₁₋₆ ou un benzyle éventuellement substitué par un halogène ou un hydroxy ;
R^{1a} représente un hydrogène ;
R² représente un hydrogène, un alkyle en C₁₋₆ ou un (imidazol-4-yl)méthyle éventuellement substitué ;
R³ représente un aryle bicyclique, un hétéroaryle bicyclique, un alkyle en C₁₋₆ ou un hydroxylalkyle en C₁₋₆ dans lequel l'aryle bicyclique et l'hétéroaryle bicyclique est éventuellement substitué une ou deux fois par un alkyle en C₁₋₆, un hydroxyle ou un halogène ;
R^{3a} représente un hydrogène ou un alkyle en C₁₋₆ ;
R⁴ représente un alkyle en C₁₋₆ ou un (imidazol-4-yl)méthyle éventuellement substitué ;
R⁵ représente un hydrogène ; un alkyle en C₁₋₆, un hydroxylalkyle en C₁₋₆, un (imidazol-4-yl)méthyle éventuellement substitué ou un guanidino-alkyle en C₂₋₄ éventuellement substitué ;
R⁶ représente un hydroxylalkyle en C₁₋₆ ou un aminoalkyle en C₁₋₆ ;
R⁷ représente un alkyle en C₁₋₆, un hydroxylalkyle en C₁₋₆ ou un guanidino-alkyle en C₂₋₄ éventuellement substitué
R⁸ représente un benzyle éventuellement substitué ;
R^{8a} représente un hydrogène ou un alkyle en C₁₋₆ ;
R⁹ représente un benzyle éventuellement substitué ou un 1H-imidazol-5-ylméthyle éventuellement substitué ;
R¹⁰ représente un alkyle en C₁₋₆, un aminoalkyle en C₁₋₆, un 1H-imidazol-5-ylméthyle éventuellement substitué, un guanidino-alkyle en C₂₋₄ éventuellement substitué ou un alkyle en C₁₋₂-carboxamide éventuellement substitué ;
R¹¹ représente un benzyle éventuellement substitué ou un (imidazol-4-yl)méthyle éventuellement substitué ;
R¹² représente un N-(3-amino-3-oxo-propyl)carboxamide ou un N-(2-amino-2-oxo-éthyl)carboxamide tous deux éventuellement substitués ;
R^{b}, R^{c}, R^{d}, R^{e}, R^{f}, R^{15a}, R^{15b}, R^{15c}, R^{15d}, R^{15f}, R^{15g}, R^{15h}, R¹⁵ⁱ, R^{15j}, R^{15k}, R^{16a}, R^{16b},
R^{16c} et R^{16d} représentent chacun indépendamment un hydrogène ou un alkyle en C₁₋₆ ;
R^{15c}, représente un hydrogène, un alkyle en C₁₋₆, un aminoalkyle en C₁₋₆ ou un alkyle en C₁₋₂-carboxamide éventuellement substitué ;
R^{a} représente un hydrogène, un alkyle en C₁₋₆, un hydroxyle ou un halogène ;
*n* et *p* représentent indépendamment de 0 à 2 ;
q représente 0 ou 1 ; et
r représente 1 ou 2 ; ou
composé de formule (II) 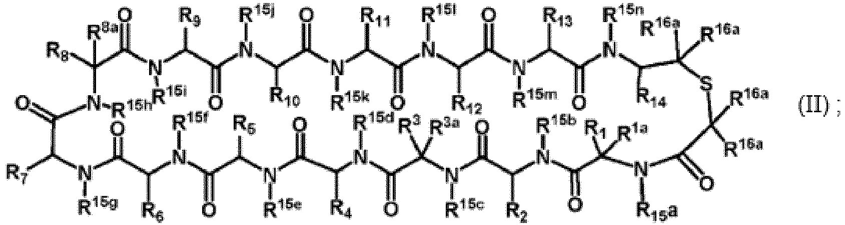
ou un stéréoisomère ou un sel pharmaceutiquement acceptable de celui-ci, dans lequel :
R¹ représente un benzyle éventuellement substitué ;
R^{1a} représente un hydrogène ou un alkyle en C₁₋₆ ;
R² représente un hydrogène ou un (imidazol-4-yl)méthyle éventuellement substitué ;
R³ représente un alkyle en C₁₋₆ ou un hydroxylalkyle en C₁₋₆ ;
R^{3a} représente un hydrogène ou un alkyle en C₁₋₆ ;
R⁴ représente un alkyle en C₁₋₆ ou un (imidazol-4-yl)méthyle éventuellement substitué ;
R⁵ représente un guanidino-alkyle en C₂₋₄ éventuellement substitué ou un (imidazol-4-yl)méthyle éventuellement substitué ;
R⁶ représente un aminoalkyle en C₁₋₆ ou un guanidino-alkyle en C₂₋₄ éventuellement substitué ;
R⁷ représente un hydroxylalkyle en C₁₋₆ ou un guanidino-alkyle en C₂₋₄ éventuellement substitué ;
R⁸ représente un méthyle ou une 3-propylguanidine ;
R^{8a} représente un hydrogène ou un méthyle ;
R⁹ représente un benzyle éventuellement substitué ;
R¹⁰ représente un aminoalkyle en C₁₋₆, un (imidazol-4-yl)méthyle éventuellement substitué ou un guanidinoalkyle en C₂₋₄ éventuellement substitué ;
R¹¹ représente un benzyle éventuellement substitué ;
R¹² représente un indolyl-3-méthyle ou un benzyle éventuellement substitué ;
R¹³ représente un benzyle éventuellement substitué ;
R¹⁴ représente un N-(3-amino-3-oxo-propyl)carboxamide ou un N-(2-amino-2-oxo-éthyl)carboxamide tous deux éventuellement substitués ;
R^{b}, R^{c}, R^{d}, R^{e}, R^{f}, R^{15a}, R^{15b}, R^{15c}, R^{15d}, R^{15e}, R^{15f}, R^{15g}, R^{15h}, R¹⁵ⁱ, R^{15j}, R^{15k}, R^{15l}, R^{15m}, R¹⁵ⁿ, R^{16a}, R^{16b}, R^{16c} et R^{16d} représentent indépendamment dans chaque occurrence un hydrogène ou un alkyle en C₁₋₆ ;
R^{a} représente un hydrogène, un alkyle en C₁₋₆, un hydroxyle ou un halogène ;
n et p représentent chacun indépendamment 0 à 2 ; et
q représente 0 ou 1.

2. Composé selon la revendication 1 dans lequel le composé est de formule I, et R¹² représente un N-(2-amino-2-oxo-éthyl)carboxamide, R⁴ et R⁹ représentent un 1H-imidazol-5-ylméthyle et R² représente un alkyle en C₁₋₆.

3. Composé selon la revendication 1 ou 2 dans lequel le composé est de formule I, et dans lequel :
R¹ représente un alkyle en C₁₋₆, un hydroxylalkyle en C₁₋₆ ou un benzyle éventuellement substitué ;
R³ représente un 1-naphtylméthyle, un 2-naphtylméthyle, un (4-phénylphényl)méthyle ou un 1H-indolyl-3-méthyle éventuellement substitué ;
R⁵ représente un hydrogène ou un 3-guanidopropyle ;
R⁶ représente un hydroxylalkyle en C₁₋₆ ou un 4-aminobutyle ;
R⁷ représente un 3-guanidopropyle ;
R¹⁰ représente un 3-guanidino-propyle, un méthyle, un éthyle, un aminométhyle, un aminoéthyle, un 4-aminobutyle, un 3-méthylimidazol-4-yl)méthyle ou un 3-amino-3-oxo-propyle ;
R¹¹ représente un 1H-imidazol-5-ylméthyle ou un 3-méthylimidazol-4-yl)méthyle ;
R^{b}, R^{c}, R^{e}, R^{1a}, R^{3a}, R^{8a}, R^{15b}, R^{15c}, R^{15d}, R^{15f}, R^{15g}, R¹⁵ⁱ, R^{15k}, R^{16a}, R^{16b}, R^{16c} et R^{16d} représentent un hydrogène ;
R^{15a}, R^{15e}, R^{15h} et R^{15j}, représentent indépendamment un hydrogène ou un méthyle ;
R^{b}, et R^{f}, représentent indépendamment un hydrogène ou un méthyle ;
R^{15h} représente un méthyle ;
ou un stéréoisomère ou un sel pharmaceutiquement acceptable de celui-ci.

4. Composé selon l'une quelconque des revendications 1 à 3 dans lequel le composé est de formule I, et dans lequel :
R¹ représente un benzyle éventuellement substitué par un halogène ou un hydroxy ;
R² représente un hydrogène, un éthyle, un isopropyle ou un (1S)-1-méthylpropyle ;
R³ représente un 1-naphtylméthyle ou un 1H-indole-3-méthyle ;
R⁴ représente un 1H-imidazol-5-ylméthyle ;
R⁵ représente un hydrogène ;
R⁶ représente un hydroxyméthyle ou un (1R)-1-hydroxyéthyle ;
R⁷ représente un 3-guanidinopropyle ;
R⁸ représente un benzyle ou un 4-hydroxybenzyle ;
R⁹ représente un 1H-imidazol-5-ylméthyle ;
R¹⁰ représente un 3-guanidopropyle, un méthyle ou un éthyle ;
R¹¹ représente un 1H-imidazol-5-ylméthyle, un 3-méthylimidazol-4-yl)méthyle ;
R¹² représente -C(=O)NHCH₂C(=O)NH₂ ;
ou un stéréoisomère ou un sel pharmaceutiquement acceptable de celui-ci.

5. Composé selon l'une quelconque des revendications 1 à 4 dans lequel le composé est de formule I, et dans lequel :
R¹ représente un benzyle ;
R² représente un éthyle ou un isopropyle ;
R³ représente un 1-naphtylméthyle ;
R⁴ représente un 1H-imidazol-5-ylméthyle ;
R⁵ représente un hydrogène ;
R⁶ représente un hydroxylméthyle ;
R⁷ représente un 3-guanidopropyle ;
R⁸ représente un 4-hydroxylbenzyle ou un 4-chlorobenzyle ;
R⁹ représente un 1-méthyl-1H-imidazole-5-méthylène ;
R¹⁰ représente un 3-guanidopropyle ;
R¹¹ représente un (3-méthylimidazol-4-yl)méthyle ;
R¹² représente un N-(2-amino-2-oxo-éthyl)carboxamide ;
R^{b}, et R^{f}, représentent indépendamment dans chaque occurrence un hydrogène ou un méthyle ; et
ou un stéréoisomère ou un sel pharmaceutiquement acceptable de celui-ci.

6. Composé selon l'une quelconque des revendications 1 à 5 dans lequel le composé est de formule Ia : 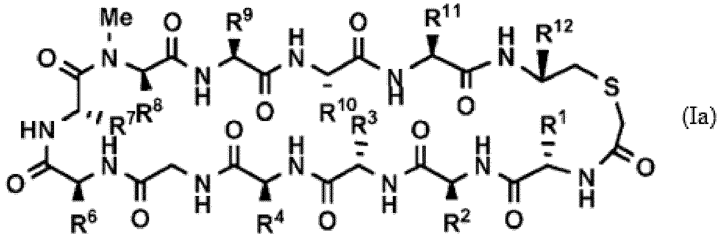 ou un stéréoisomère ou un sel pharmaceutiquement acceptable de celui-ci.

7. Composé selon la revendication 1, dans lequel le composé est de formule II, et dans lequel :
R¹ représente un benzyle éventuellement substitué ;
R^{1a} représente un hydrogène ou un alkyle en C₁₋₆ ;
R² représente un (3-méthylimidazol-4-yl)méthyle ;
R³ représente un alkyle en C₁₋₆ ou un hydroxylalkyle en C₁₋₆ ;
R^{3a} représente un hydrogène ou un alkyle en C₁₋₆ ;
R⁴ représente un alkyle en C₁₋₆ ;
R⁵ représente un 3-guanidino-propyle ;
R⁶ représente un 3-guanidino-propyle ou un aminoalkyle en C₁₋₆ ;
R⁷ représente un 3-guanidino-propyle ou un hydroxylalkyle en C₁₋₆ ;
R⁸ représente un benzyle éventuellement substitué ;
R^{8a} représente un méthyle ;
R⁹ représente un benzyle éventuellement substitué ;
R¹⁰ représente un guanidino-alkyle en C₂₋₄ éventuellement substitué, un (3-méthylimidazol-4-yl)méthyle ou un aminoalkyle en C₁₋₆ ;
R¹¹ représente un 4-hydroxybenzyle ou un 4-chlorobenzyle ;
R¹² représente un 1H-indole-3-méthyle ou un 4-chlorobenzyle ;
R¹³ représente un benzyle éventuellement substitué ;
R¹⁴ représente -C(=O)NHCH₂C(=O)NH₂ ;
R^{15a}, R^{15b}, R^{15c}, R^{15d}, R^{15e}, R^{15f}, R^{15g}, R^{15h}, R¹⁵ⁱ, R^{15j}, R^{15k}, R^{15l}, R^{15m}, R¹⁵ⁿ, R^{16a},
R^{16b}, R^{16c} et R^{16d} représentent un hydrogène ;
ou un stéréoisomère ou un sel pharmaceutiquement acceptable de celui-ci.

8. Composé selon l'une quelconque des revendications 1 ou 7, dans lequel le composé est de formule II, et dans lequel :
R¹ représente un benzyle ;
R^{1a} représente un hydrogène ;
R² représente un (3-méthylimidazol-4-yl)méthyle ;
R³ représente un méthyle ou un (1R)-1-hydroxyéthyle ;
R^{3a} représente un hydrogène ou un méthyle ;
R⁴ représente un (1S)-1-méthylpropyle ;
R⁵ représente un 3-guanidopropyle ;
R⁶ représente un 4-amino-butyle ;
R⁷ représente un 3-guanidopropyle ou un (1R)-1-hydroxyéthyle ;
R⁸ et R^{8a} représentent un méthyle ;
R⁹ représente un benzyle ;
R¹⁰ représente un guanidino-propyle, un (3-méthylimidazol-4-yl)méthyle ou un aminoalkyle en C₁₋₆ ;
R¹¹ représente un 4-hydroxybenzyle ;
R¹² représente un 1H-indole-3-méthyle ;
R¹³ représente un 4-hydroxybenzyle ;
R¹⁴ représente -C(=O)NHCH₂C(=O)NH₂ ;
R^{15a}, R^{15b}, R^{15c}, R^{15d},R^{15e}, R^{15f}, R^{15g}, R^{15h}, R¹⁵ⁱ, R^{15j}, R^{15k}, R^{15l}, R^{15m}, R¹⁵ⁿ, R^{16a}, R^{16b}, R^{16c} et R^{16d} représentent un hydrogène ;
ou un stéréoisomère ou un sel pharmaceutiquement acceptable de celui-ci.

9. Composé selon la revendication 1, 7 ou 8, dans lequel le composé est de formule II, et dans lequel :
R¹ représente un benzyle ;
R^{1a} représente un hydrogène ;
R² représente un (3-méthylimidazol-4-yl)méthyle ;
R³ représente un méthyle ou un (1R)-1-hydroxyéthyle ;
R⁴ représente un (1S)-1-méthylpropyle ;
R⁵ représente un 3-guanidino-propyle ;
R⁶ représente un 4-aminobutyle ;
R⁷ représente un rac-(1R)-1-hydroxyéthyle ;
R⁸ et R^{8a} représentent un méthyle ;
R⁹ représente un benzyle ;
R¹⁰ représente un 3-guanidinopropyle, un (3-méthylimidazol-4-yl)méthyle, un aminométhyle, un 2-amino-éthyle, un 3-aminopropyle ou un 4 aminobutyle ;
R¹¹ représente un 4-hydroxybenzyle ;
R¹² représente un 1H-indole-3-méthyle ;
R¹³ représente un 4-hydroxybenzyle ;
R¹⁴ représente -C(=O)NHCH₂C(=O)NH.

10. Composé selon la revendication 1 ou l'une quelconque des revendications 7 à 9, dans lequel le composé est de formule II, et dans lequel :
R¹ représente un benzyle ;
R^{1a} représente un hydrogène ;
R² représente un (3-méthylimidazol-4-yl)méthyle ;
R³ représente un méthyle ou un (1R)-1-hydroxyéthyle ;
R⁴ représente un (1S)-1-méthylpropyle ;
R⁵ représente un 3-guanidino-propyle ;
R⁶ représente un 4-aminobutyle ;
R⁷ représente un rac-(1R)-1-hydroxyéthyle ;
R⁸ et R^{8a} représentent un méthyle ;
R⁹ représente un benzyle ;
R¹⁰ représente un 3-guanidinopropyle, un (3-méthylimidazol-4-yl)méthyle, un 3-aminopropyle ou un 4 aminobutyle ;
R¹¹ représente un 4-hydroxybenzyle ;
R¹² représente un 1H-indole-3-méthyle ;
R¹³ représente un 4-hydroxybenzyle ;
R¹⁴ représente -C(=O)NHCH₂C(=O)NH ;
ou un stéréoisomère ou un sel pharmaceutiquement acceptable de celui-ci.

11. Composé selon la revendication 1 ou l'une quelconque des revendications 7 à 10 dans lequel le composé est de formule IIa :

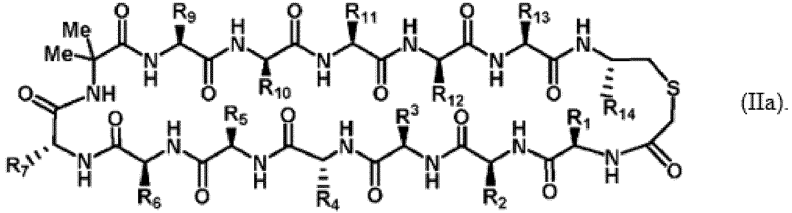

12. Composé selon la revendication 1 dans lequel ledit composé est choisi parmi :
| # | Structure | Nom du composé |
|---|---|---|
| 1 | 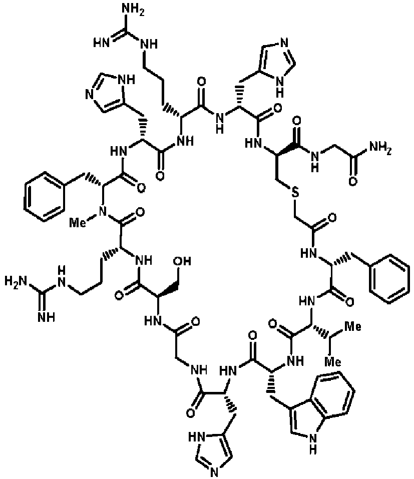 | rac-(3R,6S,9S,12S,15S,18S,21S,27S,30S, 33S,36S)-N-(2-amino-2-oxo-éthyl)-15,36-dibenzyl-9,18-bis(3-guanidinopropyl)-21-(hydroxyméthyl)-6,12,27-tris(1H-imidazol-5-ylméthyl)-30-(1H-indol-3-ylméthyl)-33-isopropyl-16-méthyl-5,8,11,14,17,20,23,26,29,32,35,38-dodécaoxo-1-thia-4,7,10,13,16,19,22,25,28,31,34,37-dodécazacyclononatriacontane-3-carboxamide |
| 2 | 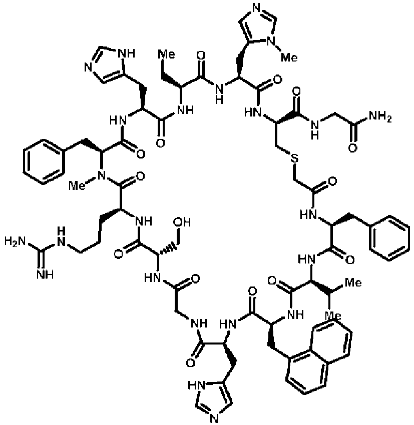 | (3R,6S,9S,12S,15S,18S,21S,27S,30S, 33S,36S)-N-(2-amino-2-oxo-éthyl)-15,36-dibenzyl-9-éthyl-18-(3-guanidinopropyl)-21-(hydroxyméthyl)-12,27-bis(1H-imidazol-4-ylméthyl)-33-isopropyl-16-méthyl-6-[(3-méthylimidazol-4-yl)méthyl]-30-(1-naphtylméthyl)-5,8,11,14,17,20,23,26,29,32,35,38-dodécaoxo-1-thia-4,7,10,13,16,19,22,25,28,31,34,37-dodécazacyclononatriacontane-3-carboxamide |
| 3 | 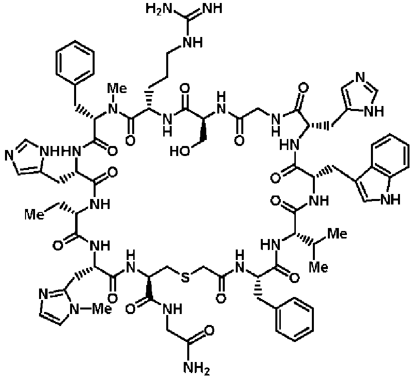 | (3R,6S,9S,12S,15S,18S,21S,27S,30S, 33S,36S)-N-(2-amino-2-oxo-éthyl)-15,36-dibenzyl-9-éthyl-18-(3-guanidinopropyl)-21-(hydroxyméthyl)-12,27-bis(1H-imidazol-4-ylméthyl)-30-(1H-indol-3-ylméthyl)-33-isopropyl-16-méthyl-6-[(3-méthylimidazol-4-yl)méthyl]-5,8,11,14,17,20,23,26,29,32,35,38-dodécaoxo-1-thia-4,7,10,13,16,19,22,25,28,31,34,37-dodécazacyclononatriacontane-3-carboxamide |
| 4 | 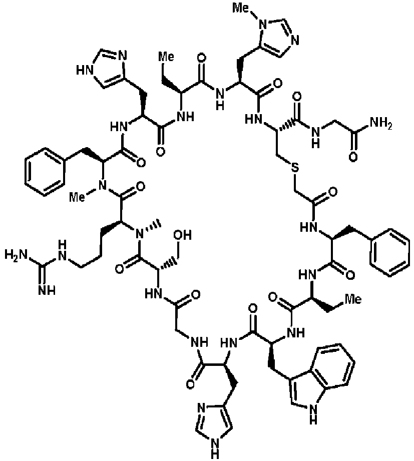 | (3R,6S,9S,12S,15S,18S,21S,27S,30S, 33S,36S)-N-(2-amino-2-oxo-éthyl)-15,36-dibenzyl-9,33-diéthyl-18-(3-guanidinopropyl)-21-(hydroxyméthyl)-12,27-bis(1H-imidazol-4-ylméthyl)-30-(1H-indol-3-ylméthyl)-16-méthyl-6-[(3-méthylimidazol-4-yl)méthyl]-5,8,11,14,17,20,23,26,29,32,35,38-dodécaoxo-1-thia-4,7,10,13,16,19,22,25,28,31,34,37-dodécazacyclononatriacontane-3-carboxamide |
| 5 | 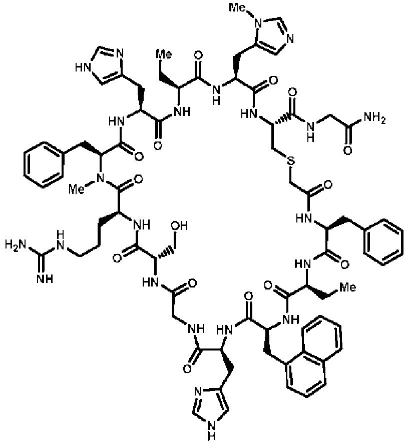 | (3R,6S,9S,12S,15S,18S,21S,27S,30S, 33S,36S)-N-(2-amino-2-oxo-éthyl)-15,36-dibenzyl-9,33-diéthyl-18-(3-guanidinopropyl)-21-(hydroxyméthyl)-12,27-bis(1H-imidazol-4-ylméthyl)-16-méthyl-6-[(3-méthylimidazol-4-yl)méthyl]-30-(1-naphtylméthyl)-5,8,11,14,17,20,23,26,29,32,35,38-dodécaoxo-1-thia-4,7,10,13,16,19,22,25,28,31,34,37-dodécazacyclononatriacontane-3-carboxamide |
| 6 | 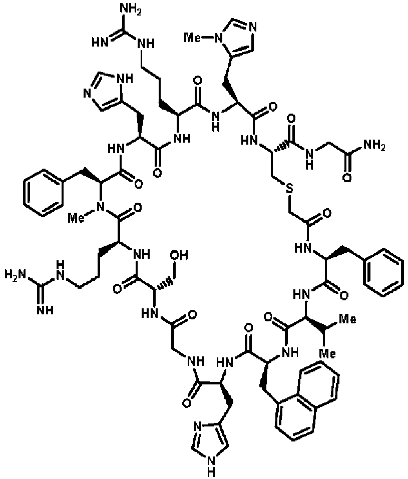 | (3R,6S,9S,12S,15S,18S,21S,27S,30S, 33S,36S)-N-(2-amino-2-oxo-éthyl)-15,36-dibenzyl-9,18-bis(3-guanidinopropyl)-21-(hydroxyméthyl)-12,27-bis(1H-imidazol-4-ylméthyl)-33-isopropyl-16-méthyl-6-[(3-méthylimidazol-4-yl)méthyl]-30-(1-naphtylméthyl)-5,8,11,14,17,20,23,26,29,32,35,38-dodécaoxo-1-thia-4,7,10,13,16,19,22,25,28,31,34,37-dodécazacyclononatriacontane-3-carboxamide |
| 7 | 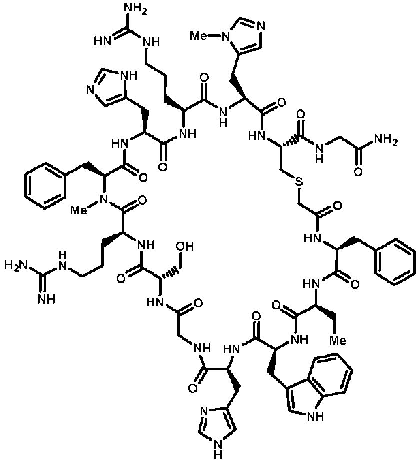 | (3R,6S,9S,12S,15S,18S,21S,27S,30S, 33S,36S)-N-(2-amino-2-oxo-éthyl)-15,36-dibenzyl-33-éthyl-9,18-bis(3-guanidinopropyl)-21-(hydroxyméthyl)-12,27-bis(1H-imidazol-4-ylméthyl)-30-(1H-indol-3-ylméthyl)-16-méthyl-6-[(3-méthylimidazol-4-yl)méthyl]-5,8,11,14,17,20,23,26,29,32,35,38-dodécaoxo-1-thia-4,7,10,13,16,19,22,25,28,31,34,37-dodécazacyclononatriacontane-3-carboxamide |
| 8 | 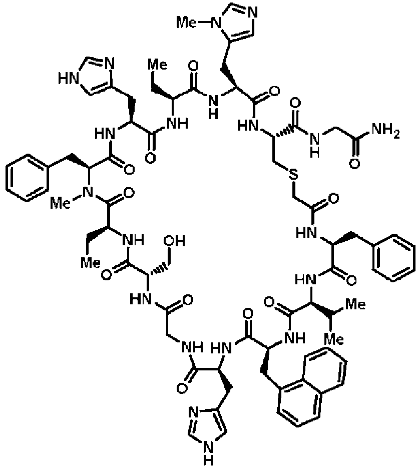 | le (3R,6S,9S,12S,15S,18S,21S,27S,30S, 33S,36S)-N-(2-amino-2-oxo-éthyl)-15,36-dibenzyl-9,18-diéthyl-21-(hydroxyméthyl)-12,27-bis(1H-imidazol-4-ylméthyl)-33-isopropyl-16-méthyl-6-[(3-méthylimidazol-4-yl)méthyl]-30-(1-naphtylméthyl)-5,8,11,14,17,20,23,26,29,32,35,38-dodécaoxo-1-thia-4,7,10,13,16,19,22,25,28,31,34,37-dodécazacyclononatriacontane-3-carboxamide |
| 9 | 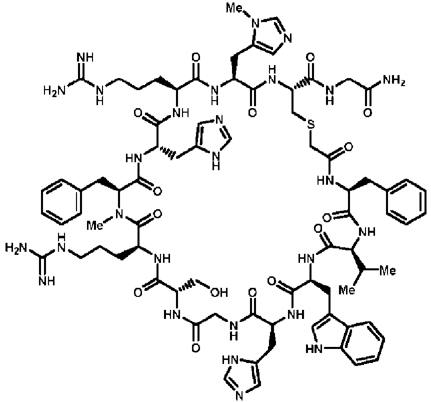 | rac-(3R,6S,9S,12S,15S,18S,21S,27S,30S, 33S,36S)-N-(2-amino-2-oxo-éthyl)-15,36-dibenzyl-9,18-bis(3-guanidinopropyl)-21-(hydroxyméthyl)-12,27-bis(1H-imidazol-5-ylméthyl)-30-(1H-indol-3-ylméthyl)-33-isopropyl-16-méthyl-6-[(3-méthylimidazol-4-yl)méthyl]-5,8,11,14,17,20,23,26,29,32,35,38-dodécaoxo-1-thia-4,7,10,13,16,19,22,25,28,31,34,37-dodécazacyclononatriacontane-3-carboxamide |
| 10 | 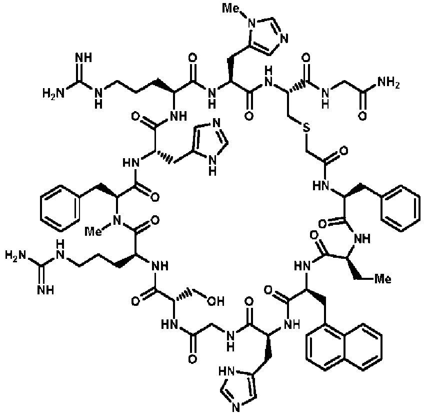 | rac-(3R,6S,9S,12S,15S,18S,21S,27S,30S, 33S,36S)-N-(2-amino-2-oxo-éthyl)-15,36-dibenzyl-33-éthyl-9,18-bis(3-guanidinopropyl)-21-(hydroxyméthyl)-12,27-bis(1H-imidazol-5-ylméthyl)-16-méthyl-6-[(3-méthylimidazol-4-yl)méthyl]-30-(1-naphtylméthyl)-5,8,11,14,17,20,23,26,29,32,35,38-dodécaoxo-1-thia-4,7,10,13,16,19,22,25,28,31,34,37-dodécazacyclononatriacontane-3-carboxamide |
| 11 | 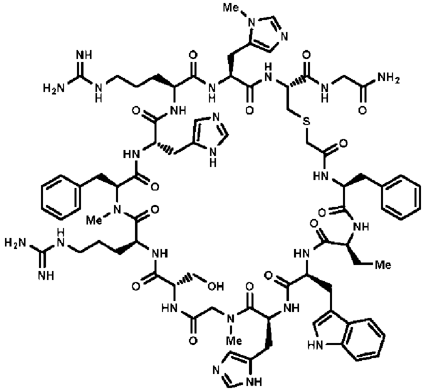 | rac-(3R,6S,9S,12S,15S,18S,21S,27S,30S, 33S,36S)-N-(2-amino-2-oxo-éthyl)-15,36-dibenzyl-33-éthyl-9,18-bis(3-guanidinopropyl)-21-(hydroxyméthyl)-12,27-bis(1H-imidazol-5-ylméthyl)-30-(1H-indol-3-ylméthyl)-16,25-diméthyl-6-[(3-méthylimidazol-4-yl)méthyl]-5,8,11,14,17,20,23,26,29,32,35,38-dodécaoxo-1-thia-4,7,10,13,16,19,22,25,28,31,34,37-dodécazacyclononatriacontane-3-carboxamide |
| 12 | 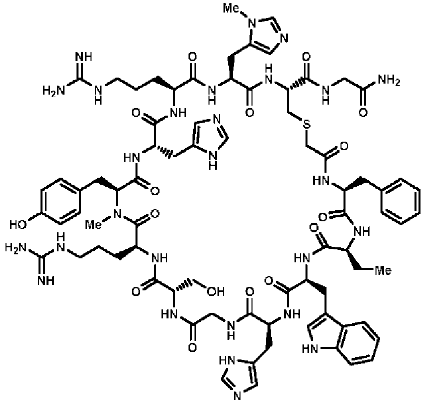 | rac-(3R,6S,9S,12S,15S,18S,21S,27S,30S, 33S,36S)-N-(2-amino-2-oxo-éthyl)-36-benzyl-33-éthyl-9,18-bis(3-guanidinopropyl)-21-(hydroxyméthyl)-15-[(4-hydroxyphényl)méthyl]-12,27-bis(1H-imidazol-5-ylméthyl)-30-(1H-indol-3-ylméthyl)-16-méthyl-6-[(3-méthylimidazol-4-yl)méthyl]-5,8,11,14,17,20,23,26,29,32,35,38-dodécaoxo-1-thia-4,7,10,13,16,19,22,25,28,31,34,37-dodécazacyclononatriacontane-3-carboxamide |
| 13 | 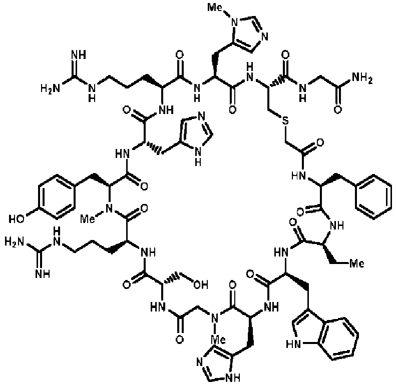 | rac-(3R,6S,9S,12S,15S,18S,21S,27S,30S, 33S,36S)-N-(2-amino-2-oxo-éthyl)-36-benzyl-33-éthyl-9,18-bis(3-guanidinopropyl)-21-(hydroxyméthyl)-15-[(4-hydroxyphényl)méthyl]-12,27-bis(1H-imidazol-5-ylméthyl)-30-(1H-indol-3-ylméthyl)-16,25-diméthyl-6-[(3-méthylimidazol-4-yl)méthyl]-5,8,11,14,17,20,23,26,29,32,35,38-dodécaoxo-1-thia-4,7,10,13,16,19,22,25,28,31,34,37-dodécazacyclononatriacontane-3-carboxamide |
| 14 | 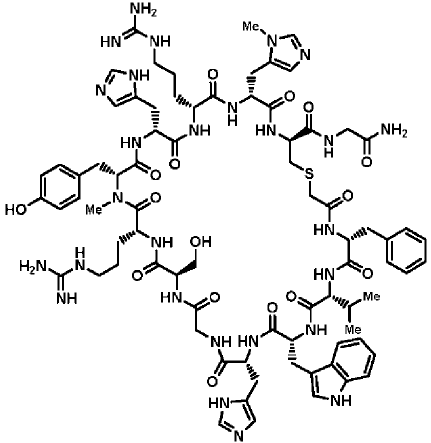 | rac-(3R,6S,9S,12S,15S,18S,21S,27S,30S, 33S,36S)-N-(2-amino-2-oxo-éthyl)-36-benzyl-9,18-bis(3-guanidinopropyl)-21-(hydroxyméthyl)-15-[(4-hydroxyphényl)méthyl]-12,27-bis(1H-imidazol-5-ylméthyl)-30-(1H-indol-3-ylméthyl)-33-isopropyl-16-méthyl-6-[(3-méthylimidazol-4-yl)méthyl]-5,8,11,14,17,20,23,26,29,32,35,38-dodécaoxo-1-thia-4,7,10,13,16,19,22,25,28,31,34,37-dodécazacyclononatriacontane-3-carboxamide |
| 15 | 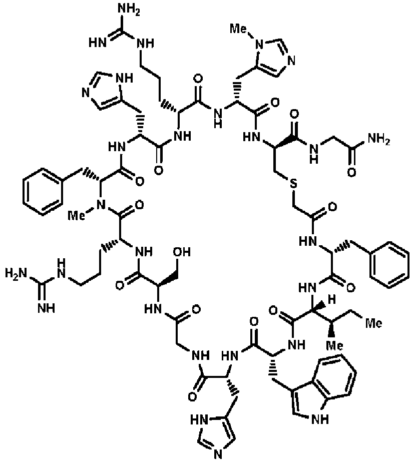 | rac-(3R,6S,9S,12S,15S,18S,21S,27S,30S, 33S,36S)-N-(2-amino-2-oxo-éthyl)-15,36-dibenzyl-9,18-bis(3-guanidinopropyl)-21-(hydroxyméthyl)-12,27-bis(1H-imidazol-5-ylméthyl)-30-(1H-indol-3-ylméthyl)-16-méthyl-6-[(3-méthylimidazol-4-yl)méthyl]-5,8,11,14,17,20,23,26,29,32,35,38-dodécaoxo-33-[rac-(1S)-1-méthylpropyl]-1-thia-4,7,10,13,16,19,22,25,28,31,34,37-dodécazacyclononatriacontane-3-carboxamide |
| 16 | 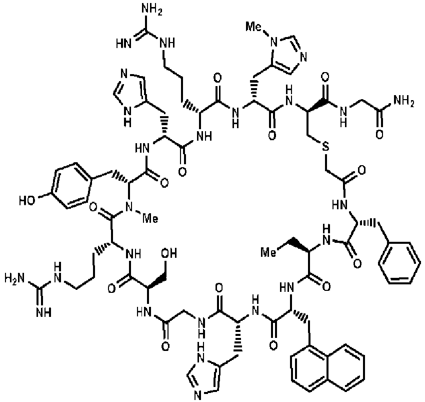 | rac-(3R,6S,9S,12S,15S,18S,21S,27S,30S, 33S,36S)-N-(2-amino-2-oxo-éthyl)-36-benzyl-33-éthyl-9,18-bis(3-guanidinopropyl)-21-(hydroxyméthyl)-15-[(4-hydroxyphényl)méthyl]-12,27-bis(1H-imidazol-5-ylméthyl)-16-méthyl-6-[(3-méthylimidazol-4-yl)méthyl]-30-(1-naphtylméthyl)-5,8,11,14,17,20,23,26,29,32,35,38-dodécaoxo-1-thia-4,7,10,13,16,19,22,25,28,31,34,37-dodécazacyclononatriacontane-3-carboxamide |
| 17 | 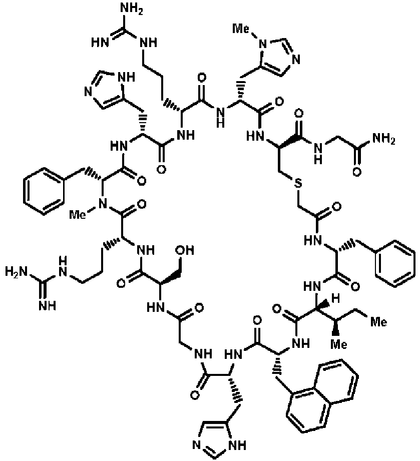 | rac-(3R,6S,9S,12S,15S,18S,21S,27S,30S, 33S,36S)-N-(2-amino-2-oxo-éthyl)-15,36-dibenzyl-9,18-bis(3-guanidinopropyl)-21-(hydroxyméthyl)-12,27-bis(1H-imidazol-5-ylméthyl)-16-méthyl-6-[(3-méthylimidazol-4-yl)méthyl]-30-(1-naphtylméthyl)-5,8,11,14,17,20,23,26,29,32,35,38-dodécaoxo-33-[rac-(1S)-1-méthylpropyl]-1-thia-4,7,10,13,16,19,22,25,28,31,34,37-dodécazacyclononatriacontane-3-carboxamide |
| 18 | 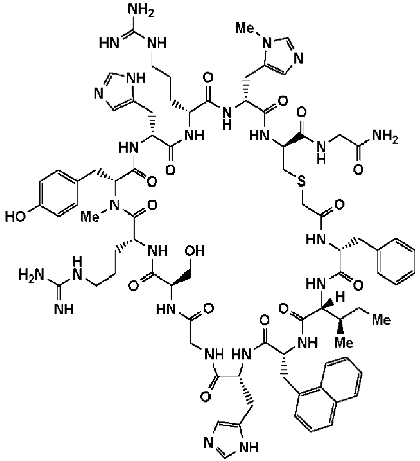 | rac-(3R,6S,9S,12S,15S,18S,21S,27S,30S, 33S,36S)-N-(2-amino-2-oxo-éthyl)-36-benzyl-9,18-bis(3-guanidinopropyl)-21-(hydroxyméthyl)-15-[(4-hydroxyphényl)méthyl]-12,27-bis(1H-imidazol-5-ylméthyl)-16-méthyl-6-[(3-méthylimidazol-4-yl)méthyl]-30-(1-naphtylméthyl)-5,8,11,14,17,20,23,26,29,32,35,38-dodécaoxo-33-[rac-(1S)-1-méthylpropyl]-1-thia-4,7,10,13,16,19,22,25,28,31,34,37-dodécazacyclononatriacontane-3-carboxamide |
| 19 | 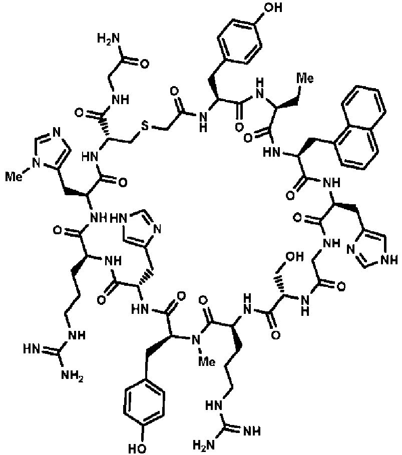 | (3R,6S,9S,12S,15S,18S,21S,27S,30S, 33S,36S)-N-(2-amino-2-oxo-éthyl)-33-éthyl-9,18-bis(3-guanidinopropyl)-21-(hydroxyméthyl)-15,36-bis[(4-hydroxyphényl)méthyl]-12,27-bis(1H-imidazol-4-ylméthyl)-16-méthyl-6-[(3-méthylimidazol-4-yl)méthyl]-30-(1-naphtylméthyl)-5,8,11,14,17,20,23,26,29,32,35,38-dodécaoxo-1-thia-4,7,10,13,16,19,22,25,28,31,34,37-dodécazacyclononatriacontane-3-carboxamide |
| 20 | 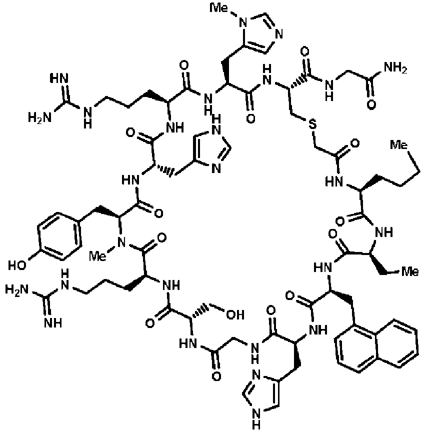 | (3R,6S,9S,12S,15S,18S,21S,27S,30S, 33S,36S)-N-(2-amino-2-oxo-éthyl)-36-butyl-33-éthyl-9,18-bis(3-guanidinopropyl)-21-(hydroxyméthyl)-15-[(4-hydroxyphényl)méthyl]-12,27-bis(1H-imidazol-4-ylméthyl)-16-méthyl-6-[(3-méthylimidazol-4-yl)méthyl]-30-(1-naphtylméthyl)-5,8,11,14,17,20,23,26,29,32,35,38-dodécaoxo-1-thia-4,7,10,13,16,19,22,25,28,31,34,37-dodécazacyclononatriacontane-3-carboxamide |
| 21 | 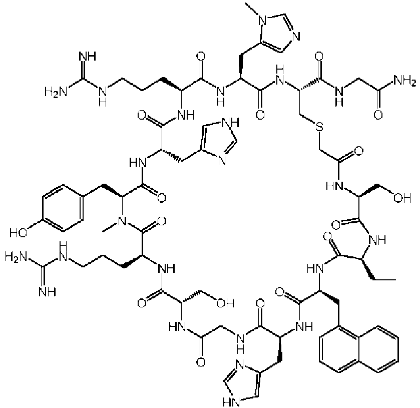 | (3R,6S,9S,12S,15S,18S,21S,27S,30S, 33S,36S)-N-(2-amino-2-oxo-éthyl)-33-éthyl-9,18-bis(3-guanidinopropyl)-21,36-bis(hydroxyméthyl)-15-[(4-hydroxyphényl)méthyl]-12,27-bis(1H-imidazol-4-ylméthyl)-16-méthyl-6-[(3-méthylimidazol-4-yl)méthyl]-30-(1-naphtylméthyl)-5,8,11,14,17,20,23,26,29,32,35,38-dodécaoxo-1-thia-4,7,10,13,16,19,22,25,28,31,34,37-dodécazacyclononatriacontane-3-carboxamide |
| 22 | 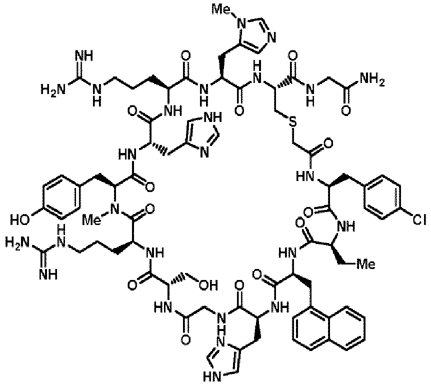 | (3R,6S,9S,12S,15S,18S,21S,27S,30S, 33S,36S)-N-(2-amino-2-oxo-éthyl)-36-[(4-chlorophényl)méthyl]-33-éthyl-9,18-bis(3-guanidinopropyl)-21-(hydroxyméthyl)-15-[(4-hydroxyphényl)méthyl]-12,27-bis(1H-imidazol-4-ylméthyl)-16-méthyl-6-[(3-méthylimidazol-4-yl)méthyl]-30-(1-naphtylméthyl)-5,8,11,14,17,20,23,26,29,32,35,38-dodécaoxo-1-thia-4,7,10,13,16,19,22,25,28,31,34,37-dodécazacyclononatriacontane-3-carboxamide |
| 23 | 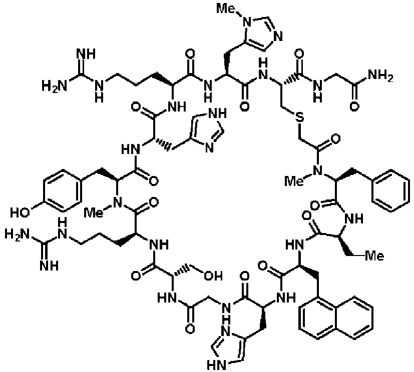 | (3R,6S,9S,12S,15S,18S,21S,27S,30S, 33S,36S)-N-(2-amino-2-oxo-éthyl)-36-benzyl-33-éthyl-9,18-bis(3-guanidinopropyl)-21-(hydroxyméthyl)-15-[(4-hydroxyphényl)méthyl]-12,27-bis(1H-imidazol-4-ylméthyl)-16,37-diméthyl-6-[(3-méthylimidazol-4-yl)méthyl]-30-(1-naphtylméthyl)-5,8,11,14,17,20,23,26,29,32,35,38-dodécaoxo-1-thia-4,7,10,13,16,19,22,25,28,31,34,37-dodécazacyclononatriacontane-3-carboxamide |
| 24 | 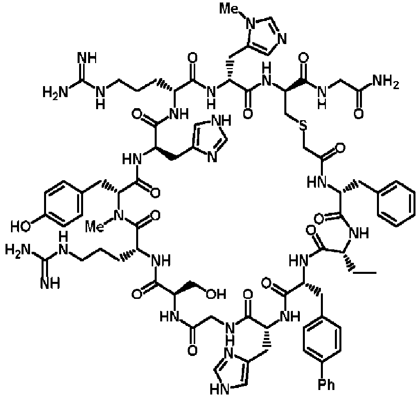 | (3R,6S,9S,12S,15S,18S,21S,27S,30S, 33S,36S)-N-(2-amino-2-oxo-éthyl)-36-benzyl-33-éthyl-9,18-bis(3-guanidinopropyl)-21-(hydroxyméthyl)-15-[(4-hydroxyphényl)méthyl]-12,27-bis(1H-imidazol-4-ylméthyl)-16-méthyl-6-[(3-méthylimidazol-4-yl)méthyl]-5,8,11,14,17,20,23,26,29,32,35,38-dodécaoxo-30-[(4-phénylphényl)méthyl]-1-thia-4,7,10,13,16,19,22,25,28,31,34,37-dodécazacyclononatriacontane-3-carboxamide |
| 25 | 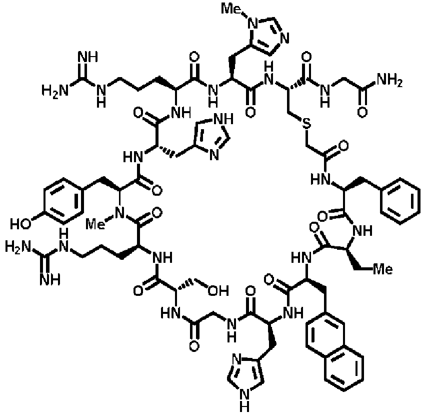 | (3R,6S,9S,12S,15S,18S,21S,27S,30S, 33S,36S)-N-(2-amino-2-oxo-éthyl)-36-benzyl-33-éthyl-9,18-bis(3-guanidinopropyl)-21-(hydroxyméthyl)-15-[(4-hydroxyphényl)méthyl]-12,27-bis(1H-imidazol-4-ylméthyl)-16-méthyl-6-[(3-méthylimidazol-4-yl)méthyl]-30-(2-naphtylméthyl)-5,8,11,14,17,20,23,26,29,32,35,38-dodécaoxo-1-thia-4,7,10,13,16,19,22,25,28,31,34,37-dodécazacyclononatriacontane-3-carboxamide |
| 26 | 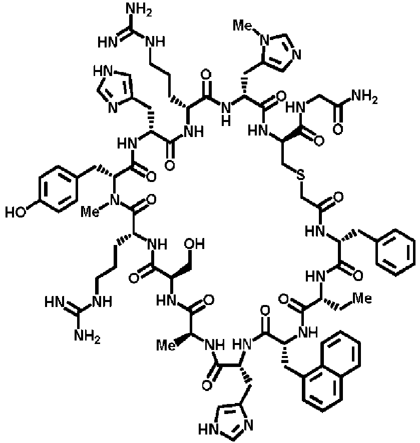 | (3R,6S,9S,12S,15S,18S,21S,24R,27S, 30S,33S,36S)-N-(2-amino-2-oxo-éthyl)-36-benzyl-33-éthyl-9,18-bis(3-guanidinopropyl)-21-(hydroxyméthyl)-15-[(4-hydroxyphényl)méthyl]-12,27-bis(1H-imidazol-4-ylméthyl)-16,24-diméthyl-6-[(3-méthylimidazol-4-yl)méthyl]-30-(1-naphtylméthyl)-5,8,11,14,17,20,23,26,29,32,35,38-dodécaoxo-1-thia-4,7,10,13,16,19,22,25,28,31,34,37-dodécazacyclononatriacontane-3-carboxamide |
| 27 | 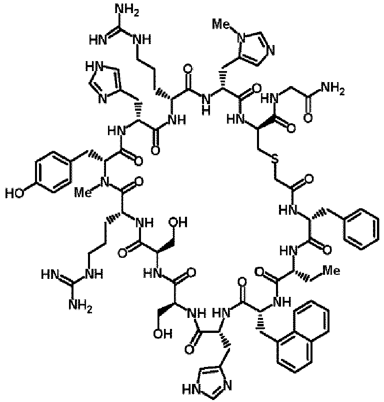 | (3R,6S,9S,12S,15S,18S,21S,24R,27S, 30S,33S,36S)-N-(2-amino-2-oxo-éthyl)-36-benzyl-33-éthyl-9,18-bis(3-guanidinopropyl)-21,24-bis(hydroxyméthyl)-15-[(4-hydroxyphényl)méthyl]-12,27-bis(1H-imidazol-4-ylméthyl)-16-méthyl-6-[(3-méthylimidazol-4-yl)méthyl]-30-(1-naphtylméthyl)-5,8,11,14,17,20,23,26,29,32,35,38-dodécaoxo-1-thia-4,7,10,13,16,19,22,25,28,31,34,37-dodécazacyclononatriacontane-3-carboxamide |
| 28 | 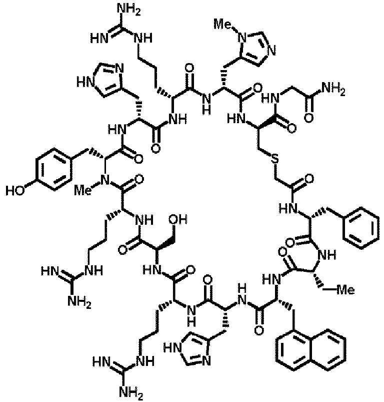 | (3R,6S,9S,12S,15S,18S,21S,24S,27S, 30S,33S,36S)-N-(2-amino-2-oxo-éthyl)-36-benzyl-33-éthyl-9,18,24-tris(3-guanidinopropyl)-21-(hydroxyméthyl)-15-[(4-hydroxyphényl)méthyl]-12,27-bis(1H-imidazol-4-ylméthyl)-16-méthyl-6-[(3-méthylimidazol-4-yl)méthyl]-30-(1-naphtylméthyl)-5,8,11,14,17,20,23,26,29,32,35,38-dodécaoxo-1-thia-4,7,10,13,16,19,22,25,28,31,34,37-dodécazacyclononatriacontane-3-carboxamide |
| 29 | 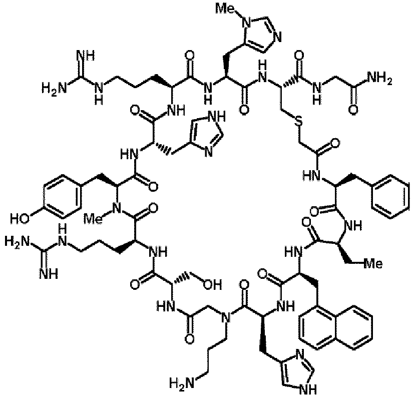 | (3R,6S,9S,12S,15S,18S,21S,27S,30S, 33S,36S)-N-(2-amino-2-oxo-éthyl)-25-(3-aminopropyl)-36-benzyl-33-éthyl-9,18-bis(3-guanidinopropyl)-21-(hydroxyméthyl)-15-[(4-hydroxyphényl)méthyl]-12,27-bis(1H-imidazol-4-ylméthyl)-16-méthyl-6-[(3-méthylimidazol-4-yl)méthyl]-30-(1-naphtylméthyl)-5,8,11,14,17,20,23,26,29,32,35,38-dodécaoxo-1-thia-4,7,10,13,16,19,22,25,28,31,34,37-dodécazacyclononatriacontane-3-carboxamide |
| 30 | 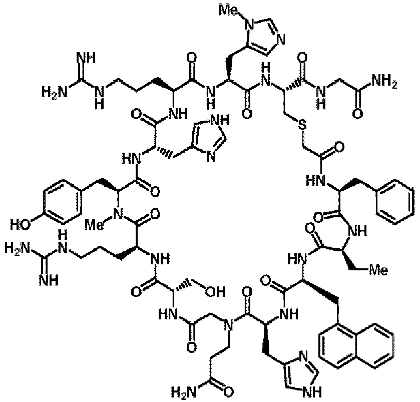 | (3R,6S,9S,12S,15S,18S,21S,27S,30S, 33S,36S)-N-(2-amino-2-oxo-éthyl)-25-(3-amino-3-oxo-propyl)-36-benzyl-33-éthyl-9,18-bis(3-guanidinopropyl)-21-(hydroxyméthyl)-15-[(4-hydroxyphényl)méthyl]-12,27-bis(1H-imidazol-4-ylméthyl)-16-méthyl-6-[(3-méthylimidazol-4-yl)méthyl]-30-(1-naphtylméthyl)-5,8,11,14,17,20,23,26,29,32,35,38-dodécaoxo-1-thia-4,7,10,13,16,19,22,25,28,31,34,37-dodécazacyclononatriacontane-3-carboxamide |
| 31 | 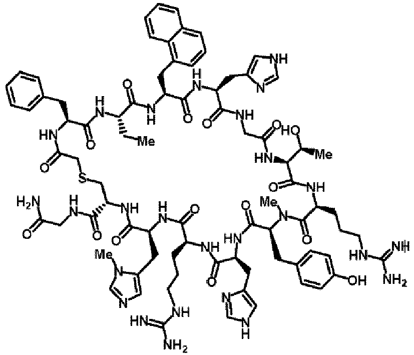 | (3R,6S,9S,12S,15S,18S,21S,27S,30S, 33S,36S)-N-(2-amino-2-oxo-éthyl)-36-benzyl-33-éthyl-9,18-bis(3-guanidinopropyl)-21-[(1S)-1-hydroxyéthyl]-15-[(4-hydroxyphényl)méthyl]-12,27-bis(1H-imidazol-4-ylméthyl)-16-méthyl-6-[(3-méthylimidazol-4-yl)méthyl]-30-(1-naphtylméthyl)-5,8,11,14,17,20,23,26,29,32,35,38-dodécaoxo-1-thia-4,7,10,13,16,19,22,25,28,31,34,37-dodécazacyclononatriacontane-3-carboxamide |
| 32 | 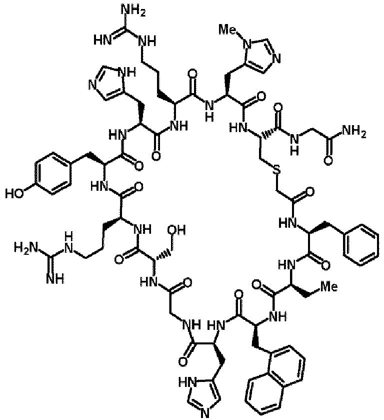 | (3R,6S,9S,12S,15S,18S,21S,27S,30S, 33S,36S)-N-(2-amino-2-oxo-éthyl)-36-benzyl-33-éthyl-9,18-bis(3-guanidinopropyl)-21-(hydroxyméthyl)-15-[(4-hydroxyphényl)méthyl]-12,27-bis(1H-imidazol-4-ylméthyl)-6-[(3-méthylimidazol-4-yl)méthyl]-30-(1-naphtylméthyl)-5,8,11,14,17,20,23,26,29,32,35,38-dodécaoxo-1-thia-4,7,10,13,16,19,22,25,28,31,34,37-dodécazacyclononatriacontane-3-carboxamide |
| 33 | 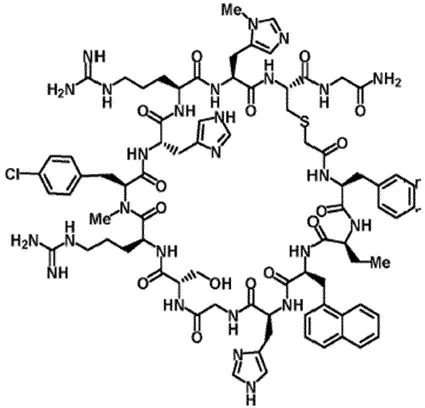 | (3R,6S,9S,12S,15S,18S,21S,27S,30S, 33S,36S)-N-(2-amino-2-oxo-éthyl)-36-benzyl-15-[(4-chlorophényl)méthyl]-33-éthyl-9,18-bis(3-guanidinopropyl)-21-(hydroxyméthyl)-12,27-bis(1H-imidazol-4-ylméthyl)-16-méthyl-6-[(3-méthylimidazol-4-yl)méthyl]-30-(1-naphtylméthyl)-5,8,11,14,17,20,23,26,29,32,35,38-dodécaoxo-1-thia-4,7,10,13,16,19,22,25,28,31,34,37-dodécazacyclononatriacontane-3-carboxamide |
| 34 | 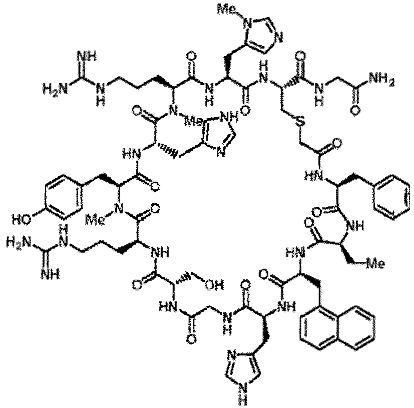 | (3R,6S,9S,12S,15S,18S,21S,27S,30S, 33S,36S)-N-(2-amino-2-oxo-éthyl)-36-benzyl-33-éthyl-9,18-bis(3-guanidinopropyl)-21-(hydroxyméthyl)-15-[(4-hydroxyphényl)méthyl]-12,27-bis(1H-imidazol-4-ylméthyl)-10,16-diméthyl-6-[(3-(méthylimidazol-4-yl)méthyl]-30-(1-naphtylméthyl)-5,8,11,14,17,20,23,26,29,32,35,38-dodécaoxo-1-thia-4,7,10,13,16,19,22,25,28,31,34,37-dodécazacyclononatriacontane-3-carboxamide |
| 35 | 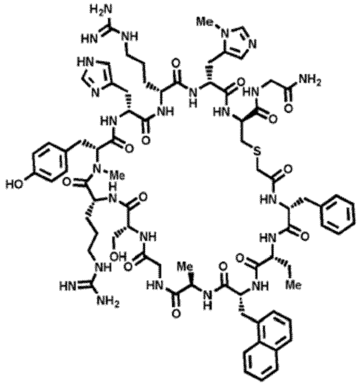 | (3R,6S,9S,12S,15S,18S,21S,27S,30S, 33S,36S)-N-(2-amino-2-oxo-éthyl)-36-benzyl-33-éthyl-9,18-bis(3-guanidinopropyl)-21-(hydroxyméthyl)-15-[(4-hydroxyphényl)méthyl]-12-(1H-imidazol-4-ylméthyl)-16,27-diméthyl-6-[(3-méthylimidazol-4-yl)méthyl]-30-(1-naphtylméthyl)-5,8,11,14,17,20,23,26,29,32,35,38-dodécaoxo-1-thia-4,7,10,13,16,19,22,25,28,31,34,37-dodécazacyclononatriacontane-3-carboxamide |
| 36 | 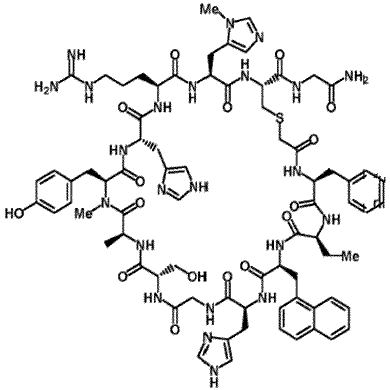 | (3R,6S,9S,12S,15S,18S,21S,27S,30S, 33S,36S)-N-(2-amino-2-oxo-éthyl)-36-benzyl-33-éthyl-9-(3-guanidinopropyl)-21-(hydroxyméthyl)-15-[(4-hydroxyphényl)méthyl]-12,27-bis(1H-imidazol-4-ylméthyl)-16,18-diméthyl-6-[(3-méthylimidazol-4-yl)méthyl]-30-(1-naphtylméthyl)-5,8,11,14,17,20,23,26,29,32,35,38-dodécaoxo-1-thia-4,7,10,13,16,19,22,25,28,31,34,37-dodécazacyclononatriacontane-3-carboxamide |
| 37 | 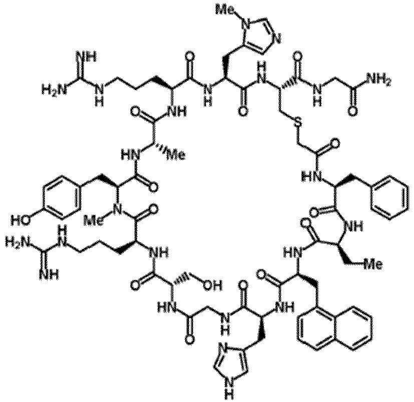 | (3R,6S,9S,12S,15S,18S,21S,27S,30S, 33S,36S)-N-(2-amino-2-oxo-éthyl)-36-benzyl-33-éthyl-9,18-bis(3-guanidinopropyl)-21-(hydroxyméthyl)-15-[(4-hydroxyphényl)méthyl]-27-(1H-imidazol-4-ylméthyl)-12,16-diméthyl-6-[(3-méthylimidazol-4-yl)méthyl]-30-(1-naphtylméthyl)-5,8,11,14,17,20,23,26,29,32,35,38-dodécaoxo-1-thia-4,7,10,13,16,19,22,25,28,31,34,37-dodécazacyclononatriacontane-3-carboxamide |
| 38 | 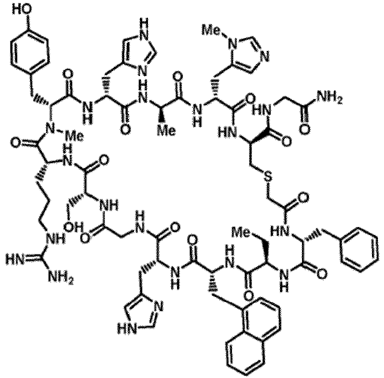 | (3R,6S,9S,12S,15S,18S,21S,27S,30S, 33S,36S)-N-(2-amino-2-oxo-éthyl)-36-benzyl-33-éthyl-18-(3-guanidinopropyl)-21-(hydroxyméthyl)-15-[(4-hydroxyphényl)méthyl]-12,27-bis(1H-imidazol-4-ylméthyl)-9,16-diméthyl-6-[(3-méthylimidazol-4-yl)méthyl]-30-(1-naphtylméthyl)-5,8,11,14,17,20,23,26,29,32,35,38-dodécaoxo-1-thia-4,7,10,13,16,19,22,25,28,31,34,37-dodécazacyclononatriacontane-3-carboxamide |
| 39 | 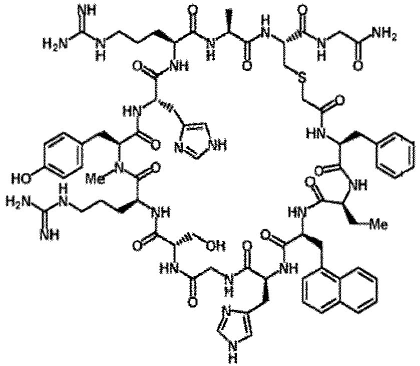 | (3R,6S,9S,12S,15S,18S,21S,27S,30S, 33S,36S)-N-(2-amino-2-oxo-éthyl)-36-benzyl-33-éthyl-9,18-bis(3-guanidinopropyl)-21-(hydroxyméthyl)-15-[(4-hydroxyphényl)méthyl]-12,27-bis(1H-imidazol-4-ylméthyl)-6,16-diméthyl-30-(1-naphtylméthyl)-5,8,11,14,17,20,23,26,29,32,35,38-dodécaoxo-1-thia-4,7,10,13,16,19,22,25,28,31,34,37-dodécazacyclononatriacontane-3-carboxamide |
| 40 | 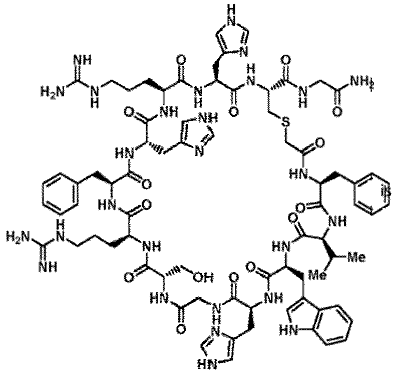 | (3R,6S,9S,12S,15S,18S,21S,27S,30S, 33S,36S)-N-(2-amino-2-oxo-éthyl)-15,36-dibenzyl-9,18-bis(3-guanidinopropyl)-21-(hydroxyméthyl)-6,12,27-tris(1H-imidazol-4-ylméthyl)-30-(1H-indol-3-ylméthyl)-33-isopropyl-5,8,11,14,17,20,23,26,29,32,35,38-dodécaoxo-1-thia-4,7,10,13,16,19,22,25,28,31,34,37-dodécazacyclononatriacontane-3-carboxamide |
| 41 | 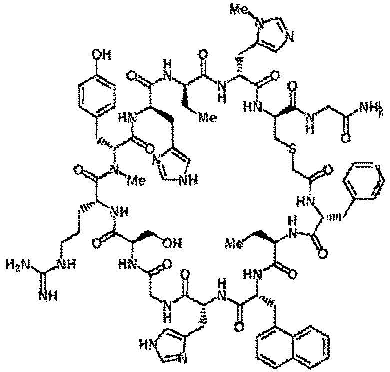 | (3R,6S,9S,12S,15S,18S,21S,27S,30S, 33S,36S)-N-(2-amino-2-oxo-éthyl)-36-benzyl-9,33-diéthyl-18-(3-guanidinopropyl)-21-(hydroxyméthyl)-15-[(4-hydroxyphényl)méthyl]-12,27-bis(1H-imidazol-4-ylméthyl)-16-méthyl-6-[(3-méthylimidazol-4-yl)méthyl]-30-(1-naphtylméthyl)-5,8,11,14,17,20,23,26,29,32,35,38-dodécaoxo-1-thia-4,7,10,13,16,19,22,25,28,31,34,37-dodécazacyclononatriacontane-3-carboxamide |
| 42 | 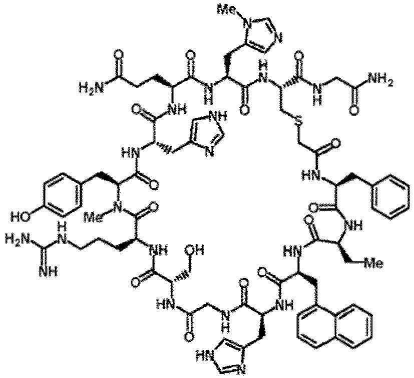 | rac-(3R,6S,9S,12S,15S,18S,21S,27S,30S, 33S,36S)-N-(2-amino-2-oxo-éthyl)-9-(3-amino-3-oxo-propyl)-36-benzyl-33-éthyl-18-(3-guanidinopropyl)-21-(hydroxyméthyl)-15-[(4-hydroxyphényl)méthyl]-12,27-bis(1H-imidazol-4-ylméthyl)-16-méthyl-6-[(3-méthylimidazol-4-yl)méthyl]-30-(1-naphtylméthyl)-5,8,11,14,17,20,23,26,29,32,35,38-dodécaoxo-1-thia-4,7,10,13,16,19,22,25,28,31,34,37-dodécazacyclononatriacontane-3-carboxamide |
| 43 | 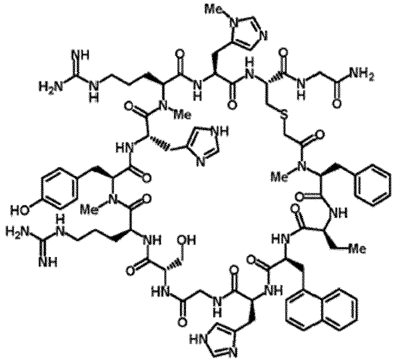 | rac-(3R,6S,9S,12S,15S,18S,21S,27S,30S, 33S,36S)-N-(2-amino-2-oxo-éthyl)-36-benzyl-33-éthyl-9,18-bis(3-guanidinopropyl)-21-(hydroxyméthyl)-15-[(4-hydroxyphényl)méthyl]-12,27-bis(1H-imidazol-4-ylméthyl)-10,16,37-triméthyl-6-[(3-méthylimidazol-4-yl)méthyl]-30-(1-naphtylméthyl)-5,8,11,14,17,20,23,26,29,32,35,38-dodécaoxo-1-thia-4,7,10,13,16,19,22,25,28,31,34,37-dodécazacyclononatriacontane-3-carboxamide |
| 44 | 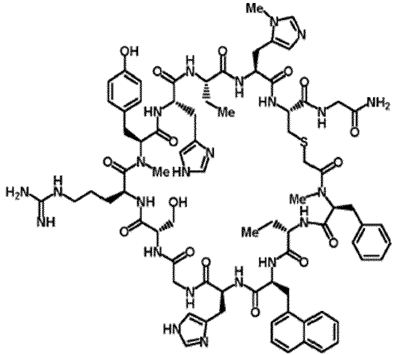 | rac-(3R,6S,9S,12S,15S,18S,21S,27S,30S, 33S,36S)-N-(2-amino-2-oxo-éthyl)-36-benzyl-9,33-diéthyl-18-(3-guanidinopropyl)-21-(hydroxyméthyl)-15-[(4-hydroxyphényl)méthyl]-12,27-bis(1H-imidazol-4-ylméthyl)-16,37-diméthyl-6-[(3-méthylimidazol-4-yl)méthyl]-30-(1-naphtylméthyl)-5,8,11,14,17,20,23,26,29,32,35,38-dodécaoxo-1-thia-4,7,10,13,16,19,22,25,28,31,34,37-dodécazacyclononatriacontane-3-carboxamide |
| 45 | 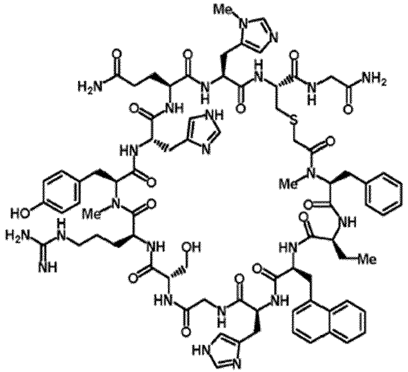 | rac-(3R,6S,9S,12S,15S,18S,21S,27S,30S, 33S,36S)-N-(2-amino-2-oxo-éthyl)-9-(3-amino-3-oxo-propyl)-36-benzyl-33-éthyl-18-(3-guanidinopropyl)-21-(hydroxyméthyl)-15-[(4-hydroxyphényl)méthyl]-12,27-bis(1H-imidazol-4-ylméthyl)-16,37-diméthyl-6-[(3-méthylimidazol-4-yl)méthyl]-30-(1-naphtylméthyl)-5,8,11,14,17,20,23,26,29,32,35,38-dodécaoxo-1-thia-4,7,10,13,16,19,22,25,28,31,34,37-dodécazacyclononatriacontane-3-carboxamide |
| 46 | 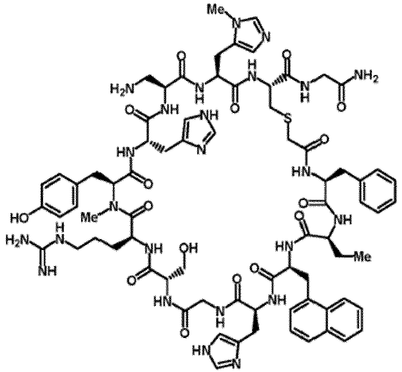 | rac-(3R,6S,9S,12S,15S,18S,21S,27S,30S, 33 S,36S)-9-(aminométhyl)-N-(2-amino-2-oxo-éthyl)-36-benzyl-33-éthyl-18-(3-guanidinopropyl)-21-(hydroxyméthyl)-15-[(4-hydroxyphényl)méthyl]-12,27-bis(1H-imidazol-4-ylméthyl)-16-méthyl-6-[(3-méthylimidazol-4-yl)méthyl]-30-(1-naphtylméthyl)-5,8,11,14,17,20,23,26,29,32,35,38-dodécaoxo-1-thia-4,7,10,13,16,19,22,25,28,31,34,37-dodécazacyclononatriacontane-3-carboxamide |
| 47 | 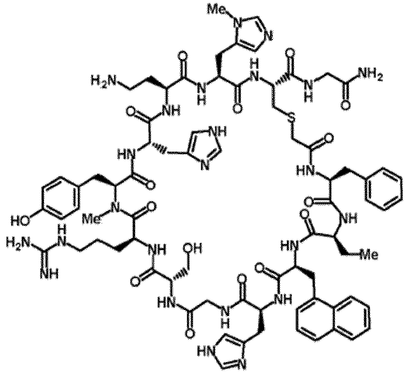 | rac-(3R,6S,9S,12S,15S,18S,21S,27S,30S, 33 S,36S)-9-(2-aminoéthyl)-N-(2-amino-2-oxo-éthyl)-36-benzyl-33-éthyl-18-(3-guanidinopropyl)-21-(hydroxyméthyl)-15-[(4-hydroxyphényl)méthyl]-12,27-bis(1H-imidazol-4-ylméthyl)-16-méthyl-6-[(3-méthylimidazol-4-yl)méthyl]-30-(1-naphtylméthyl)-5,8,11,14,17,20,23,26,29,32,35,38-dodécaoxo-1-thia-4,7,10,13,16,19,22,25,28,31,34,37-dodécazacyclononatriacontane-3-carboxamide |
| 48 | 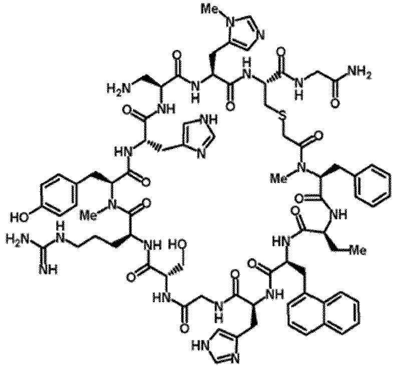 | rac-(3R,6S,9S,12S,15S,18S,21S,27S,30S, 33 S,36S)-9-(aminométhyl)-N-(2-amino-2-oxo-éthyl)-36-benzyl-33-éthyl-18-(3-guanidinopropyl)-21-(hydroxyméthyl)-15-[(4-hydroxyphényl)méthyl]-12,27-bis(1H-imidazol-4-ylméthyl)-16,37-diméthyl-6-[(3-méthylimidazol-4-yl)méthyl]-30-(1-naphtylméthyl)-5,8,11,14,17,20,23,26,29,32,35,38-dodécaoxo-1-thia-4,7,10,13,16,19,22,25,28,31,34,37-dodécazacyclononatriacontane-3-carboxamide |
| 49 | 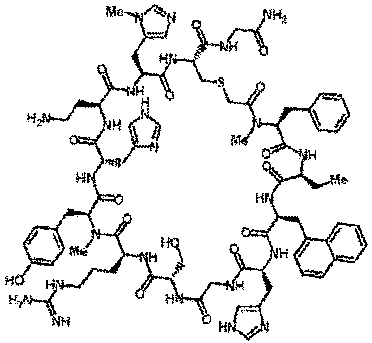 | rac-(3R,6S,9S,12S,15S,18S,21S,27S,30S, 33 S,36S)-9-(2-aminoéthyl)-N-(2-amino-2-oxo-éthyl)-36-benzyl-33-éthyl-18-(3-guanidinopropyl)-21-(hydroxyméthyl)-15-[(4-hydroxyphényl)méthyl]-12,27-bis(1H-imidazol-4-ylméthyl)-16,37-diméthyl-6-[(3-méthylimidazol-4-yl)méthyl]-30-(1-naphtylméthyl)-5,8,11,14,17,20,23,26,29,32,35,38-dodécaoxo-1-thia-4,7,10,13,16,19,22,25,28,31,34,37-dodécazacyclononatriacontane-3-carboxamide |
| 50 | 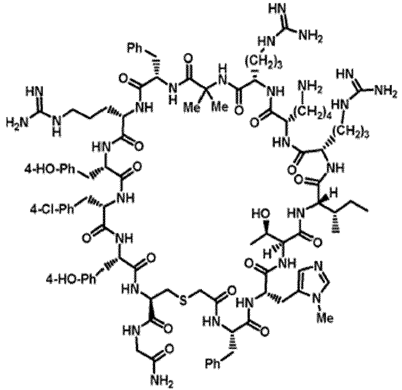 | rac-(3R,6S,9S, 12S, 15S, 18S,21S,24S,27S, 30S,33 S,36S,42S)-27-(4-aminobutyl)-N-(2-amino-2-oxo-éthyl)-6,18,42-tribenzyl-15,21,24-tris(3-guanidinopropyl)-12-[(4-hydroxyphényl)méthyl]-30-(1H-imidazol-5-ylméthyl)-9-(1H-indol-3-ylméthyl)-5,8,11,14,17,20,23,26,29,32,35,38,41, 44-tétradécaoxo-36-[rac-(1R)-1-hydroxyéthyl]-33-[rac-(1S)-1-méthylpropyl]-1-thia-4,7,10,13,16,19,22,25,28,31,34,37,40, 43-tétradécazacyclopentatétracontane-3-carboxamide |
| 51 | 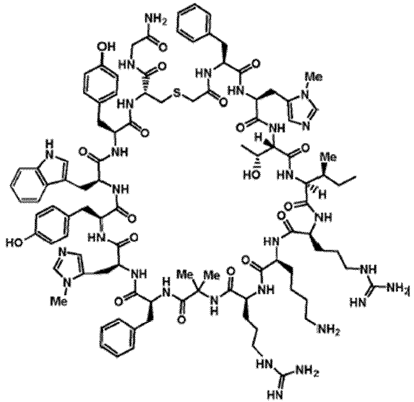 | (3R,6S,9S,12S,15S,18S,24S,27S,30S, 33 S,36S,39S,42S)-27-(4-aminobutyl)-N-(2-amino-2-oxo-éthyl)-18,42-dibenzyl-24,30-bis(3-guanidinopropyl)-36-[(1R)-1-hydroxyéthyl]-6,12-bis[(4-hydroxyphényl)méthyl]-9-(1H-indol-3-ylméthyl)-21,21-diméthyl-15,39-bis[(3-méthylimidazol-4-yl)méthyl]-33-[(1S)-1-méthylpropyl]-5,8,11,14,17,20,23,26,29,32,35,38,41, 44-tétradécaoxo-1-thia-4,7,10,13,16,19,22,25,28,31,34,37,40, 43-tétradécazacyclopentatétracontane-3-carboxamide |
| 52 | 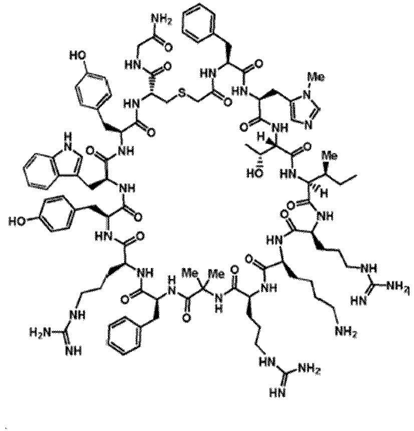 | (3R,6S,9S,12S,15S,18S,24S,27S,30S, 33 S,36S,39S,42S)-27-(4-aminobutyl)-N-(2-amino-2-oxo-éthyl)-18,42-dibenzyl-15,24,30-tris(3-guanidinopropyl)-36-[(1R)-1-hydroxyéthyl]-6,12-bis[(4-hydroxyphényl)méthyl]-9-(1H-indol-3-ylméthyl)-21,21-diméthyl-39-[(3-méthylimidazol-4-yl)méthyl]-33-[(1S)-1-méthylpropyl]-5,8,11,14,17,20,23,26,29,32,35,38,41, 44-tétradécaoxo-1-thia-4,7,10,13,16,19,22,25,28,31,34,37,40, 43-tétradécazacyclopentatétracontane-3-carboxamide |
| 53 | 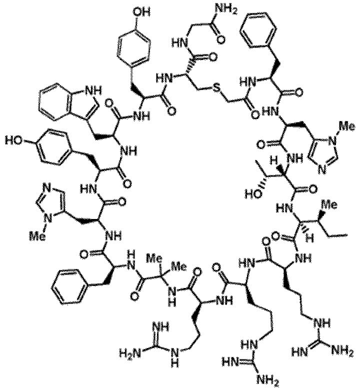 | (3R,6S,9S,12S,15S,18S,24S,27S,30S, 33S,36S,39S,42S)-N-(2-amino-2-oxo-éthyl)-18,42-dibenzyl-24,27,30-tris(3-guanidinopropyl)-36-[(1R)-1-hydroxyéthyl]-6,12-bis[(4-hydroxyphényl)méthyl]-9-(1H-indol-3-ylméthyl)-21,21-diméthyl-15,39-bis[(3-méthylimidazol-4-yl)méthyl]-33-[(1S)-1-méthylpropyl]-5,8,11,14,17,20,23,26,29,32,35,38,41, 44-tétradécaoxo-1-thia-4,7,10,13,16,19,22,25,28,31,34,37,40, 43-tétradécazacyclopentatétracontane-3-carboxamide |
| 54 | 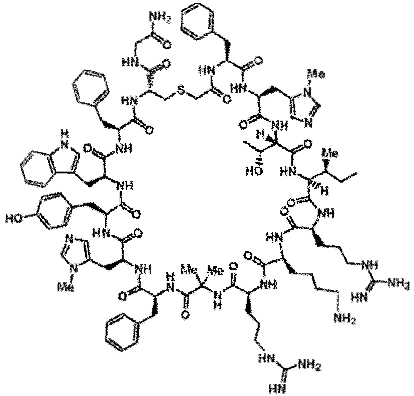 | (3R,6S,9S,12S,15S,18S,24S,27S,30S, 33 S,36S,39S,42S)-27-(4-aminobutyl)-N-(2-amino-2-oxo-éthyl)-6,18,42-tribenzyl-24,30-bis(3-guanidinopropyl)-36-[(1R)-1-hydroxyéthyl]-12-[(4-hydroxyphényl)méthyl]-9-(1H-indol-3-ylméthyl)-21,21-diméthyl-15,39-bis[(3-méthylimidazol-4-yl)méthyl]-33-[(1S)-1-méthylpropyl]-5,8,11,14,17,20,23,26,29,32,35,38,41, 44-tétradécaoxo-1-thia-4,7,10,13,16,19,22,25,28,31,34,37,40, 43-tétradécazacyclopentatétracontane-3-carboxamide ; acide 2,2,2-trifluoroacétique |
| 55 | 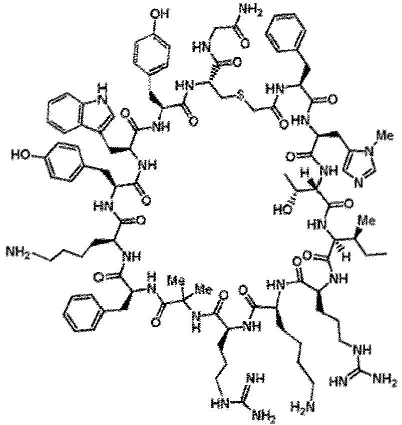 | (3R,6S,9S,12S,15S,18S,24S,27S,30S, 33S,36S,39S,42S)-15,27-bis(4-aminobutyl)-N-(2-amino-2-oxo-éthyl)-18,42-dibenzyl-24,30-bis(3-guanidinopropyl)-36-[(1R)-1-hydroxyéthyl]-6,12-bis[(4-hydroxyphényl)méthyl]-9-(1H-indol-3-ylméthyl)-21,21-diméthyl-39-[(3-méthylimidazol-4-yl)méthyl]-33-[(1S)-1-méthylpropyl]-5,8,11,14,17,20,23,26,29,32,35,38,41, 44-tétradécaoxo-1-thia-4,7,10,13,16,19,22,25,28,31,34,37,40, 43-tétradécazacyclopentatétracontane-3-carboxamide |
| 56 | 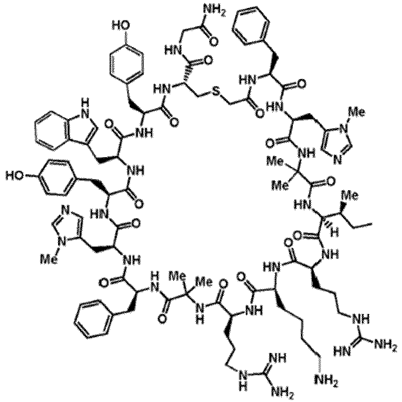 | (3R,6S,9S,12S,15S,18S,24S,27S,30S, 33S,39S,42S)-27-(4-aminobutyl)-N-(2-amino-2-oxo-éthyl)-18,42-dibenzyl-24,30-bis(3-guanidinopropyl)-6,12-bis[(4-hydroxyphényl)méthyl]-9-(1H-indol-3-ylméthyl)-21,21,36,36-tétraméthyl-15,39-bis[(3-méthylimidazol-4-yl)méthyl]-33-[(1S)-1-méthylpropyl]-5,8,11,14,17,20,23,26,29,32,35,38,41, 44-tétradécaoxo-1-thia-4,7,10,13,16,19,22,25,28,31,34,37,40, 43-tétradécazacyclopentatétracontane-3-carboxamide |
| 57 | 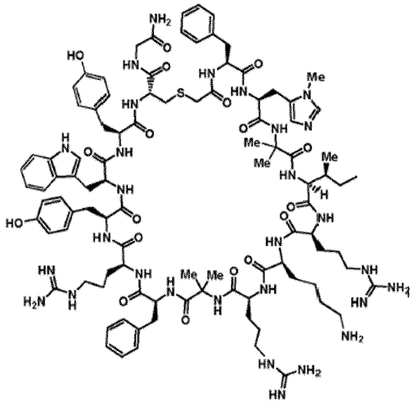 | (3R,6S,9S,12S,15S,18S,24S,27S,30S, 33S,39S,42S)-27-(4-aminobutyl)-N-(2-amino-2-oxo-éthyl)-18,42-dibenzyl-15,24,30-tris(3-guanidinopropyl)-6,12-bis[(4-hydroxyphényl)méthyl]-9-(1H-indol-3-ylméthyl)-21,21,36,36-tétraméthyl-39-[(3-méthylimidazol-4-yl)méthyl]-33-[(1S)-1-méthylpropyl]-5,8,11,14,17,20,23,26,29,32,35,38,41, 44-tétradécaoxo-1-thia-4,7,10,13,16,19,22,25,28,31,34,37,40, 43-tétradécazacyclopentatétracontane-3-carboxamide |
| 58 | 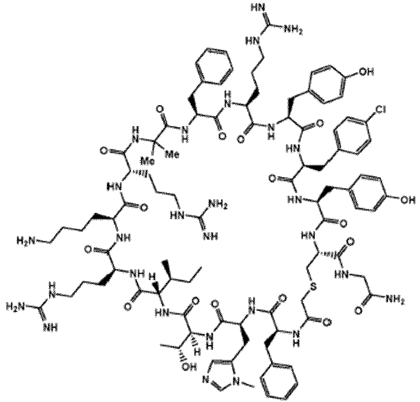 | (3R,6S,9S,12S,15S,18S,24S,27S,30S, 33 S,36S,39S,42S)-27-(4-aminobutyl)-N-(2-amino-2-oxo-éthyl)-18,42-dibenzyl-9-[(4-chlorophényl)méthyl]-15,24,30-tris(3-guanidinopropyl)-36-[(1R)-1-hydroxyéthyl]-6,12-bis[(4-hydroxyphényl)méthyl]-21,21-diméthyl-39-[(3-méthylimidazol-4-yl)méthyl]-33-[(1S)-1-méthylpropyl]-5,8,11,14,17,20,23,26,29,32,35,38,41, 44-tétradécaoxo-1-thia-4,7,10,13,16,19,22,25,28,31,34,37,40, 43-tétradécazacyclopentatétracontane-3-carboxamide |
| 59 | 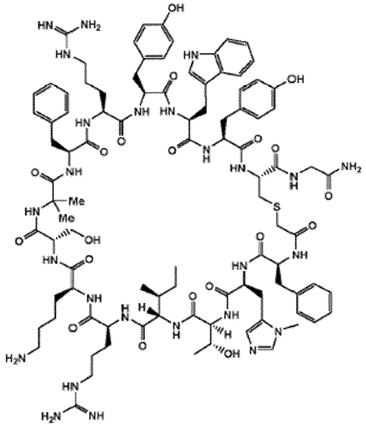 | rac-(3R,6S,9S,12S,15S,18S,24S,27S,30S, 33 S,36S,39S,42S)-27-(4-aminobutyl)-N-(2-amino-2-oxo-éthyl)-18,42-dibenzyl-15,30-bis(3-guanidinopropyl)-24-(hydroxyméthyl)-6,12-bis[(4-hydroxyphényl)méthyl]-9-(1H-indol-3-ylméthyl)-21,21-diméthyl-39-[(3-méthylimidazol-4-yl)méthyl]-5,8,11,14,17,20,23,26,29,32,35,38,41, 44-tétradécaoxo-36-[rac-(1R)-1-hydroxyéthyl]-33-[rac-(1S)-1-méthylpropyl]-1-thia-4,7,10,13,16,19,22,25,28,31,34,37,40, 43-tétradécazacyclopentatétracontane-3-carboxamide |
| 60 | 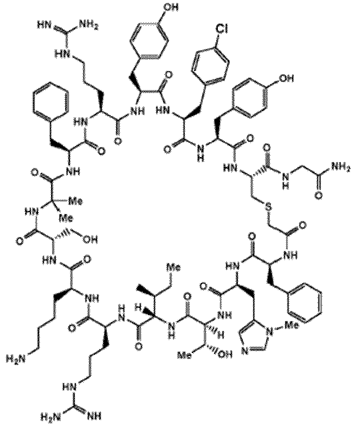 | (3R,6S,9S,12S,15S,18S,24S,27S,30S, 33 S,36S,39S,42S)-27-(4-aminobutyl)-N-(2-amino-2-oxo-éthyl)-18,42-dibenzyl-9-[(4-chlorophényl)méthyl]-15,30-bis(3-guanidinopropyl)-24-(hydroxyméthyl)-6,12-bis[(4-hydroxyphényl)méthyl]-21,21-diméthyl-39-[(3-méthylimidazol-4-yl)méthyl]-5,8,11,14,17,20,23,26,29,32,35,38,41, 44-tétradécaoxo-36-[rac-(1R)-1-hydroxyéthyl]-33-[rac-(1S)-1-méthylpropyl]-1-thia-4,7,10,13,16,19,22,25,28,31,34,37,40, 43-tétradécazacyclopentatétracontane-3-carboxamide |
| 61 | 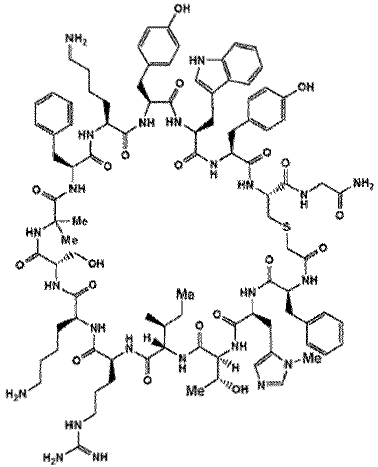 | rac-(3R,6S,9S,12S,15S,18S,24S,27S,30S, 33S,36S,39S,42S)-15,27-bis(4-aminobutyl)-N-(2-amino-2-oxo-éthyl)-18,42-dibenzyl-30-(3-guanidinopropyl)-24-(hydroxyméthyl)-6,12-bis[(4-hydroxyphényl)méthyl]-9-(1H-indol-3-ylméthyl)-21,21-diméthyl-39-[(3-méthylimidazol-4-yl)méthyl]-5,8,11,14,17,20,23,26,29,32,35,38,41, 44-tétradécaoxo-36-[rac-(1R)-1-hydroxyéthyl]-33-[rac-(1S)-1-méthylpropyl]-1-thia-4,7,10,13,16,19,22,25,28,31,34,37,40, 43-tétradécazacyclopentatétracontane-3-carboxamide |

13. Composition pharmaceutique comprenant le composé selon l'une quelconque des revendications 1 à 12, ou un sel pharmaceutiquement acceptable, un solvate ou un stéréoisomère de celui-ci, et un excipient pharmaceutiquement acceptable.

14. Composé selon l'une quelconque des revendications 1 à 12, ou un sel pharmaceutiquement acceptable, un solvate ou un stéréoisomère de celui-ci, pour une utilisation dans le traitement d'une infection bactérienne chez un mammifère, comprenant l'administration au mammifère d'une quantité efficace dudit composé à une fréquence et pendant une durée suffisantes pour procurer un effet bénéfique au mammifère.

15. Composé pour une utilisation selon la revendication 14 comprenant en outre l'administration d'un second agent thérapeutique, dans lequel ledit second agent thérapeutique est un antibiotique aminoglycoside, un antibiotique fluoroquinolone, un antibiotique β-lactame, un antibiotique macrolide, un antibiotique glycopeptide, la rifampicine, le chloramphénicol, le fluoramphénicol, la colistine, la mupirocine, la bacitracine, la daptomycine ou le linézolide.
